# EUROPEAN PATENT APPLICATION

(11) **EP 4 847 510 A1**
(43) Date of publication of application: **02.12.2026**
(21) Application number: 25751493.5
(22) Date of filing: 05.02.2025
(51) Int. Cl.: C07D 471/16, A61K 31/4375, A61P 25/00

(54) **TETRACYCLIC COMPOUND, AND PREPARATION METHOD, PHARMACEUTICAL COMPOSITION, AND USE THEREOF**

(30) Priority: 05.02.2024 CN 202410165703; 12.08.2024 CN 202411101069; 04.12.2024 CN 202411772195; 24.01.2025 CN 202510119032
(71) Applicant: JING MEDICINE TECHNOLOGY (SHANGHAI) LTD., Shanghai 201210 (CN)
(72) Inventor: XUN, Guoliang, Shanghai 201203 (CN); XI, Jianbei, Shanghai 201203 (CN); LIU, Yao, Shanghai 201203 (CN); LIU, Zhi, Shanghai 201203 (CN); CHEN, Jinju, Shanghai 201203 (CN); FANG, Tao, Shanghai 201203 (CN); ZHAO, Shu, Shanghai 201203 (CN); ZHAO, Cunliang, Shanghai 201203 (CN); CHEN, Yanni, Shanghai 201203 (CN); ZHOU, Yuan, Shanghai 201203 (CN)
(74) Representative: Johnson, Stephen William
(86) International application number: PCT/CN2025/075755
(87) International publication number: WO 2025/167894

(57) **Abstract**

Disclosed in the present invention are a tetracyclic compound, and a preparation method, a pharmaceutical composition, and a use thereof. Specifically disclosed is a tetracyclic compound as shown in formula I or a pharmaceutically acceptable salt thereof. The compound of the present invention can effectively treat depression, has good pharmacokinetic properties, and has less drug interactions.

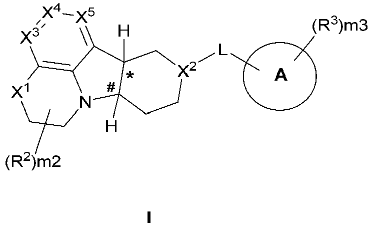

## Description

### CROSS-REFERENCE TO RELATED APPLICATIONS

This application is a continuation-in-part application of PCT/CN2025/075755, filed on February 05, 2025, which claims priority to Chinese Patent Application No. 2024101657033 filed on February 5, 2024, Chinese Patent Application No. 2024111010693 filed on August 12, 2024, Chinese Patent Application No. 2024117721951 filed on December 4, 2024, and Chinese Patent Application No. 2025101190321 filed on January 24, 2025, which are incorporated herein by reference in their entirety.

### TECHNICAL FIELD

The present disclosure relates to a tetracyclic compound, a preparation method therefor, a pharmaceutical composition thereof, and use thereof.

### BACKGROUND

5-Hydroxytryptamine is a monoamine neurotransmitter that achieves various biological functions, including mood, cognition, rewarding, learning, memory, etc., through different receptor subtypes (Young SN. JPsychiatry Neurosci. 2007 Nov; 32(6):394-9). The 5-HT2A receptor belongs to a 5-hydroxytryptamine receptor family that currently consists of more than 15 different receptors encoded by different genes, and these receptors are classified into 7 major categories: 5-HT1, 5-HT2, 5-HT3, 5-HT4, 5-HT5, 5-HT6, and 5-HT7 (Roth BL, Lopez E, The Neuroscientist. 2000; 6(4):252-262).

The 5-hydroxytryptamine 2A receptor is one of the receptors for 5-hydroxytryptamine, and there are mainly two downstream signaling pathways. One of the pathways is Gq/11-coupled, and it activates phospholipase C, resulting in an increase in the formation of inositol triphosphate and diacylglycerol and triggering their downstream signaling events. The other pathway is the activation of downstream signals such as ERK by β-arrestin as a signal transduction scaffold (McCorvy JD, Roth BL. Pharmacol Ther. 2015 Jun; 150:129-42). Non-classical pathways also include atypical signaling pathways related to the 5-HT2A receptor, including phospholipase A2 and β-arrestin-coupled Src/Akt pathways, etc. (Maroteaux L, Ayme-Dietrich E, Aubertin-Kirch G, et al. Pharmacol Ther. 2017 Feb; 170:14-36).

Stimulation of the 5-HT2A receptor can lead to neuronal excitation in various brain regions. Among all 14 5-hydroxytryptamine receptor subtypes, the 5-HT2A receptor is widely present in the central nervous system; it has the highest concentration level among the monoaminergic receptors in the cerebral cortex, and is distributed in structural regions involved in mood regulation, such as the median nucleus/dorsal raphe nucleus, locus coeruleus, and ventral tegmental area (Zi 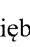 ba A, St 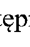 pnicki P, Matosiuk D, et al. Int J Mol Sci. 2021 Dec 21; 23(1): 10). Consistent with their locations in the brain, they are associated not only with various central physiological functions, including memory, sleep, nociception, eating, and rewarding behavior, but also with numerous neuropsychiatric diseases, such as schizophrenia, depression, and anxiety (Guiard BP, Di Giovanni G. Front Pharmacol. 2015 Mar 17; 6:46). Many novel antipsychotics (e.g., aripiprazole, brexpiprazole, and pimavanserin) have high affinity for the 5-HT2A receptor, which may be the reason why they have better efficacy and relatively small side effects (McCreary AC, Newman-Tancredi A. Curr Pharm Des. 2015; 21(26):3725-31). Experiments on 5-HT2A receptor knockout mice have confirmed that cortical 5-HT2AR can play a certain role in anxiety regulation, and 5-hydroxytryptamine signaling in the cortex can significantly influence behavior in conflict anxiety tests (Weisstaub NV, Zhou M, Lira A, et al. Science. 2006 Jul 28; 313(5786):536-40).

Depression is one of the chronic mental disorders affecting humanity today. In 2019, the U.S. FDA approved esketamine, which is the first rapidly acting antidepressant on the market and can be used in combination with oral antidepressants to treat refractory depression. However, as an analog of ketamine, esketamine involves a certain risk of addiction and must be used under medical supervision. In addition, hallucinogens such as lysergic acid diethylamide and psilocybin, which act on the target of 5-HT2A, were widely used by psychotherapists as adjunctive drugs for the treatment of depression, anxiety-related diseases, and addiction in the 1960s, and the outcomes were generally encouraging. Currently, drugs targeting 5-HT2A for the treatment of depression still have problems such as slow action, limited efficacy, significant individual variability, and relatively significant side effects.

### BRIEF SUMMARY OF THE INVENTION

The technical problem to be solved by the present disclosure is to overcome the defect of slow action or hallucinogenic side effects of existing antidepressants. Therefore, the present disclosure provides a tetracyclic compound, a preparation method therefor, a pharmaceutical composition thereof, and use thereof. The compound of the present disclosure has the advantages of rapid action, absence of hallucinogenic side effects, etc., while maintaining a relatively good antidepressant effect, and has a relatively low level of drug interaction in the body.

The present disclosure overcomes the above technical problem through the following technical solutions.

The present disclosure provides a tetracyclic compound represented by formula I or a pharmaceutically acceptable salt thereof: 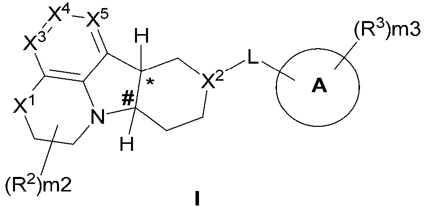
wherein the carbon atom with "*" represents a chiral carbon atom that has an S configuration, an R configuration, or a mixture thereof;
the carbon atom with "#" represents a chiral carbon atom that has an S configuration, an R configuration, or a mixture thereof;
X¹ is -NR^{X1}-, -O-, -CR^{X2}R^{X3}-, -S-, -S(O)-, or -S(O)₂-;
R^{X1} is H, C₁-C₆ alkyl, C₁-C₆ alkyl substituted with one or more R^{X1-1}, -C(O)-C₁-C₆ alkyl, or "3-12 membered heterocycloalkyl containing 1, 2, or 3 heteroatoms selected from 1, 2, or 3 of N, O, and S";
R^{X1-1} is independently OH;
R^{X2} and R^{X3} are independently H or C₁-C₆ alkyl;
X² is N or CR^{X4};
R^{X4} is H or C₁-C₆ alkyl;
X³ is N or CR¹⁻¹;
X⁴ is N or CR¹⁻²;
X⁵ is N or CR¹⁻³;
R¹⁻¹, R¹⁻², and R¹⁻³ are independently H, CN, halogen, C₁-C₆ alkyl, C₁-C₆ haloalkyl, C₁-C₆ alkoxy, or C₁-C₆ haloalkoxy;
L is -(CR^{L1}R^{L2})n1-, -(CR^{L1}R^{L2})n2-Y¹-(CR^{L1}R^{L2})n3-, or -(CR^{L1}R^{L2})n4-Y²-(CR^{L1}R^{L2})n5-;
   R^{L1} and R^{L2} are independently H or C₁-C₆ alkyl;
n1 is 1, 2, 3, or 4;
Y¹ is -O-, -NR^{L3}-, -C(O)-, -CR^{L4}R^{L5}-, 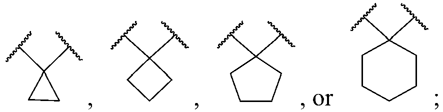
n2 is 0, 1, 2, or 3;
n3 is 0, 1, 2, or 3;
n2 + n3 = 1, 2, or 3;
Y² is -C≡C-, -C(O)-NR^{L3}-, -CR^{L6}=CR^{L7}-, 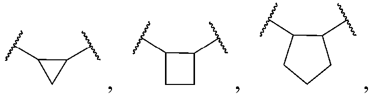 or 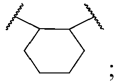
n4 is 0, 1, or 2;
n5 is 0, 1, or 2;
n4 + n5 = 1 or 2;
R^{L3} is independently H or C₁-C₆ alkyl;
R^{L4} is H or halogen;
R^{L5} is halogen, OH, or C₁-C₆ alkoxy;
R^{L6} and R^{L7} are independently H or halogen;
R² is independently C₁-C₆ alkyl, -C(O)-C₁-C₆ alkyl, or oxo (=O);
m2 is 0, 1, 2, 3, or 4;
ring A is "9-12 membered bicyclic heterocyclyl containing 1, 2, or 3 heteroatoms selected from 1, 2, or 3 of N, O, and S", "5-6 membered monocyclic heterocyclyl containing 1, 2, or 3 heteroatoms selected from 1, 2, or 3 of N, O, and S", C₆-C₁₀ aryl, or C₃-C₆ cycloalkyl;
and when L is -(CR^{L1}R^{L2})n1-, -(CR^{L1}R^{L2})n2-O-, or -(CR^{L1}R^{L2})n2-C(O)-, 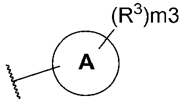 is 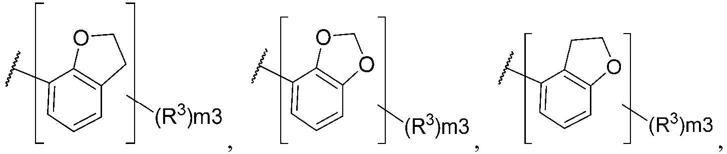 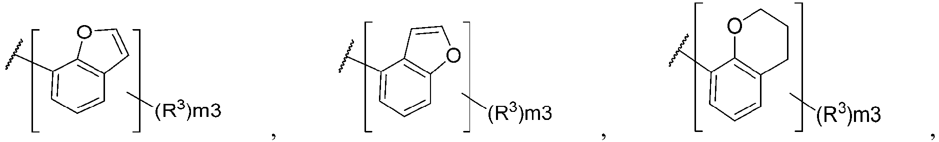 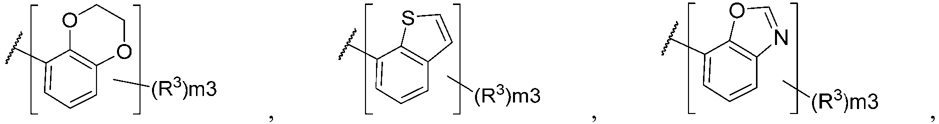 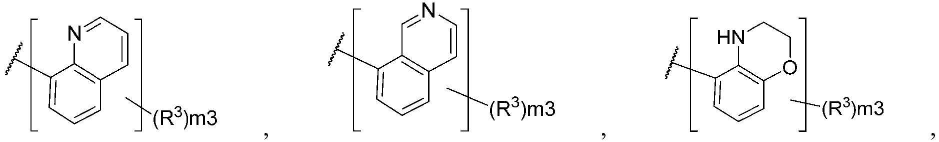 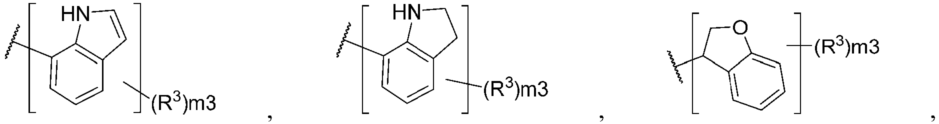 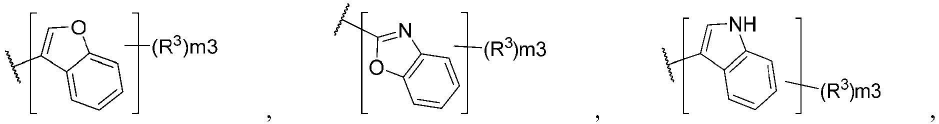 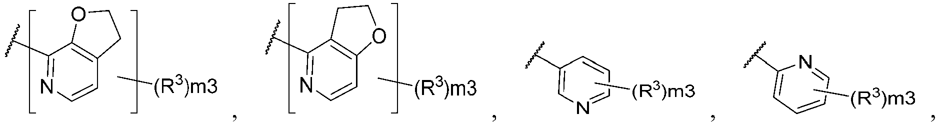 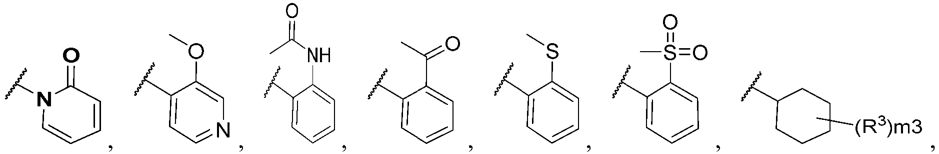 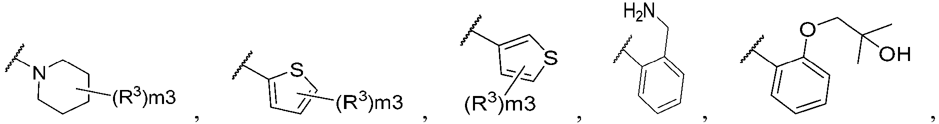 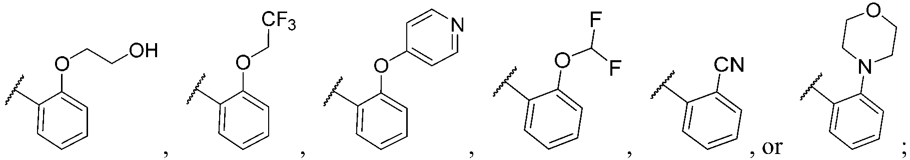
R³ is independently oxo (=O), C₁-C₆ alkyl, C₁-C₆ alkoxy, -NR⁴R⁵, -C(O)R⁶, -SR⁷, - S(O)₂R⁸, halogen, C₁-C₆ alkoxy substituted with one or more R⁹, C₁-C₆ alkyl substituted with one or more R¹⁰, CN, "3-12 membered heterocycloalkyl containing 1, 2, or 3 heteroatoms selected from 1, 2, or 3 of N, O, and S", or -OR¹¹;
R⁴ is independently H or C₁-C₆ alkyl;
R⁵ is independently H, C₁-C₆ alkyl, or -C(O)R⁵⁻¹;
R⁶, R⁵⁻¹, R⁷, and R⁸ are independently C₁-C₆ alkyl;
R⁹ and R¹⁰ are independently OH, -NR⁴R⁵, or halogen;
R¹¹ is H or "5-10 membered heteroaryl containing 1, 2, or 3 heteroatoms selected from 1, 2, or 3 of N, O, and S";
m3 is 0, 1, 2, 3, or 4.

The present disclosure further provides a tetracyclic compound represented by formula I-0 or a pharmaceutically acceptable salt thereof: 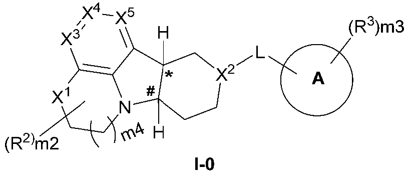
wherein the carbon atom with "*" represents a chiral carbon atom that has an S configuration, an R configuration, or a mixture thereof;
the carbon atom with "#" represents a chiral carbon atom that has an S configuration, an R configuration, or a mixture thereof;
X¹ is -NR^{X1}-, -O-, -CR^{X2}R^{X3}-, -S-, -S(O)-, or -S(O)₂-;
R^{X1} is H, C₁-C₆ alkyl, C₁-C₆ alkyl substituted with one or more R^{X1-1}, -C(O)-C₁-C₆ alkyl, C₃-C₆ cycloalkyl, or "3-12 membered heterocycloalkyl containing 1, 2, or 3 heteroatoms selected from 1, 2, or 3 of N, O, and S";
R^{X1-1} is independently deuterium or OH;
R^{X2} and R^{X3} are independently H, deuterium, C₁-C₆ alkyl, or C₁-C₆ deuterated alkyl;
X² is N, N⁺O⁻, or CR^{X4};
R^{X4} is H or C₁-C₆ alkyl;
X³ is N or CR¹⁻¹;
X⁴ is N or CR¹⁻²;
X⁵ is N or CR¹⁻³;
R¹⁻¹, R¹⁻², and R¹⁻³ are independently H, CN, halogen, C₁-C₆ alkyl, C₁-C₆ haloalkyl, C₁-C₆ alkoxy, or C₁-C₆ haloalkoxy;
L is -(CR^{L1}R^{L2})n1-, -(CR^{L1}R^{L2})n2-Y¹-(CR^{L1}R^{L2})n3-, or -(CR^{L1}R^{L2})n4-Y²-(CR^{L1}R^{L2})n5-;
R^{L1} and R^{L2} are independently H or C₁-C₆ alkyl;
n1 is 3 or 4;
Y¹ is -O-, -NR^{L3}-, -C(O)-, -CR^{L4}R^{L5}-, 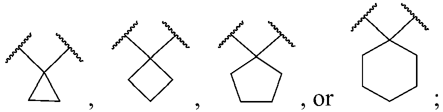
n2 is 0, 1, 2, or 3;
n3 is 0, 1, 2, or 3;
n2 + n3 = 1, 2, or 3;
Y² is -C≡C-, -C(O)-NR^{L3}-, -CR^{L6}=CR^{L7}-, 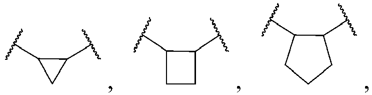 or 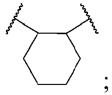
n4 is 0, 1, or 2;
n5 is 0, 1, or 2;
n4 + n5 = 1 or 2;
R^{L3} is independently H or C₁-C₆ alkyl;
R^{L4} is H or halogen;
R^{L5} is halogen, OH, or C₁-C₆ alkoxy;
R^{L6} and R^{L7} are independently H or halogen;
R² is independently deuterium, C₁-C₆ alkyl, C₁-C₆ deuterated alkyl, -C(O)-C₁-C₆ alkyl, oxo (=O), thio (=S), or hydroxyl;
m2 is 0, 1, 2, 3, or 4;
ring A is "8-12 membered bicyclic heterocyclyl containing 1, 2, or 3 heteroatoms selected from 1, 2, or 3 of N, O, and S", "5-6 membered monocyclic heterocyclyl containing 1, 2, or 3 heteroatoms selected from 1, 2, or 3 of N, O, and S", "9-16 membered polycyclic heterocyclyl containing 1, 2, or 3 heteroatoms selected from 1, 2, or 3 of N, O, and S", or C₃-C₆ cycloalkyl;
and when L is -(CR^{L1}R^{L2})n1-, -(CR^{L1}R^{L2})n2-O-, or -(CR^{L1}R^{L2})n2-C(O)-, 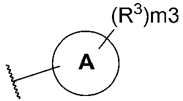 is 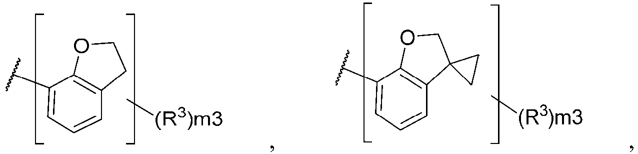 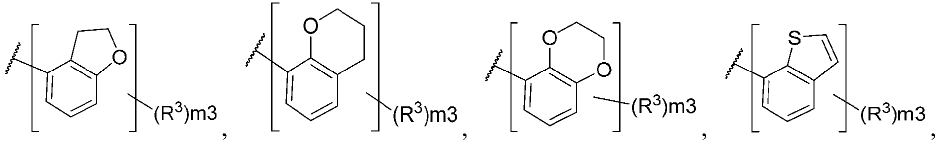 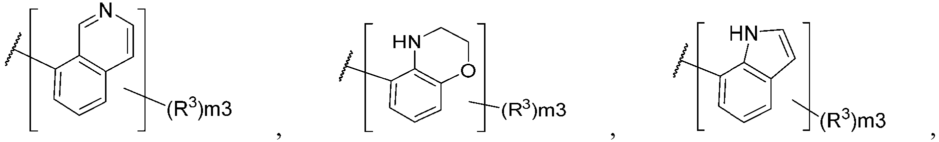 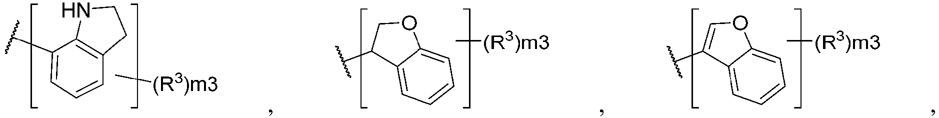 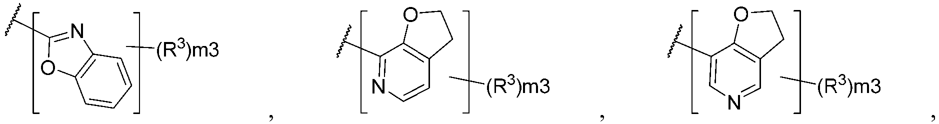 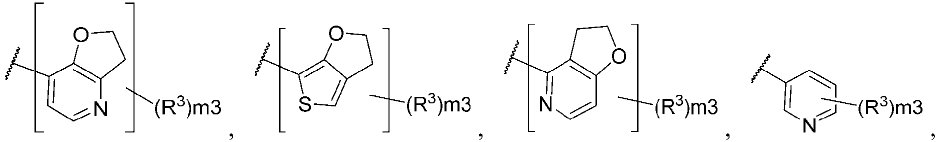 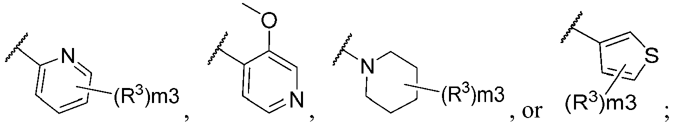
R³ is independently oxo (=O), C₁-C₆ alkyl, C₁-C₆ alkoxy, -NR⁴R⁵, -C(O)R⁶, -SR⁷, - S(O)₂R⁸, halogen, C₁-C₆ alkoxy substituted with one or more R⁹, C₁-C₆ alkyl substituted with one or more R¹⁰, CN, "3-12 membered heterocycloalkyl containing 1, 2, or 3 heteroatoms selected from 1, 2, or 3 of N, O, and S", or -OR¹¹;
R⁴ is independently H or C₁-C₆ alkyl;
R⁵ is independently H, C₁-C₆ alkyl, or -C(O)R⁵⁻¹;
R⁶, R⁵⁻¹, R⁷, and R⁸ are independently C₁-C₆ alkyl;
R⁹ and R¹⁰ are independently OH, -NR⁴R⁵, or halogen;
R¹¹ is H or "5-10 membered heteroaryl containing 1, 2, or 3 heteroatoms selected from 1, 2, or 3 of N, O, and S";
m3 is 0, 1, 2, 3, or 4;
m4 is 0, 1, or 2.

In certain preferred embodiments of the present disclosure, certain groups in the tetracyclic compound or the pharmaceutically acceptable salt thereof are defined below, groups not mentioned are the same as those described in any one of the embodiments of the present disclosure (abbreviated as "in a certain embodiment of the present disclosure"), and X¹ is - NR^{X1}-.

In a certain embodiment of the present disclosure, R^{X2} and R^{X3} are independently H.

In a certain embodiment of the present disclosure, R^{X4} is H.

In a certain embodiment of the present disclosure, X³ is CR¹⁻¹.

In a certain embodiment of the present disclosure, X⁴ is CR¹⁻².

In a certain embodiment of the present disclosure, X⁵ is CR¹⁻³.

In a certain embodiment of the present disclosure, R¹⁻¹, R¹⁻², and R¹⁻³ are independently H, CN, halogen, or C₁-C₆ alkoxy; for example, R¹⁻¹ and R¹⁻³ are H, and R¹⁻² is H, F, CN, or C₁-C₆ alkoxy; in another example, R¹⁻¹, R¹⁻², and R¹⁻³ are independently H.

In a certain embodiment of the present disclosure, R¹⁻¹, R¹⁻², and R¹⁻³ are independently H, CN, or C₁-C₆ alkoxy; for example, R¹⁻¹ and R¹⁻³ are H, and R¹⁻² is H, CN, or C₁-C₆ alkoxy.

In a certain embodiment of the present disclosure, m4 is 1.

In a certain embodiment of the present disclosure, R^{L1} and R^{L2} are independently H.

In a certain embodiment of the present disclosure, n1 is 3.

In a certain embodiment of the present disclosure, Y¹ is -O-, -NR^{L3}-, -C(O)-, - CR^{L4}R^{L5}-, or 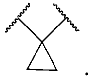 For example, Y¹ is -C(O)- or -CR^{L4}R^{L5}-,

In a certain embodiment of the present disclosure, n2 is 0, 1, or 2; for example, n2 is 1 or 2; in another example, n2 is 2.

In a certain embodiment of the present disclosure, n3 is 0, 1, or 2; for example, n3 is 0 or 1; in another example, n3 is 0.

In a certain embodiment of the present disclosure, n2 + n3 = 2.

In a certain embodiment of the present disclosure, Y² is -C≡C-, -C(O)-NR^{L3}-, - CR^{L6}=CR^{L7}-, or 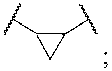 for example, Y² is -CR^{L6}=CR^{L7}-.

In a certain embodiment of the present disclosure, n4 is 0 or 1; for example, n4 is 1.

In a certain embodiment of the present disclosure, n5 is 0 or 1; for example, n5 is 0.

In a certain embodiment of the present disclosure, n4 + n5 = 1.

In a certain embodiment of the present disclosure, R^{L3} is H.

In a certain embodiment of the present disclosure, R^{L4} is H.

In a certain embodiment of the present disclosure, R^{L5} is halogen.

In a certain embodiment of the present disclosure, R^{L6} and R^{L7} are independently H.

In a certain embodiment of the present disclosure, R² is independently deuterium, C₁₋C₆ alkyl, oxo (=O), thio (=S), or hydroxyl, e.g., oxo (=O).

In a certain embodiment of the present disclosure, R² is independently oxo (=O).

In a certain embodiment of the present disclosure, m2 is 0, 1, 2, or 3; for example, m2 is 1.

In a certain embodiment of the present disclosure, m2 is 0 or 1; for example, m2 is 0.

In a certain embodiment of the present disclosure, when L is -(CR^{L1}R^{L2})n1-, - (CR^{L1}R^{L2})n2-O-, or -(CR^{L1}R^{L2})n2-C(O)-, 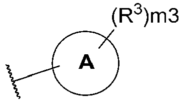 is

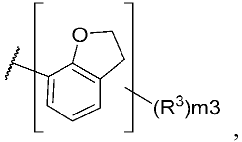

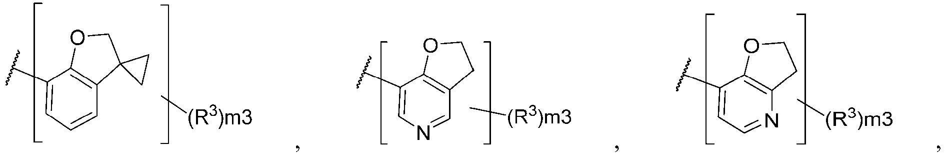

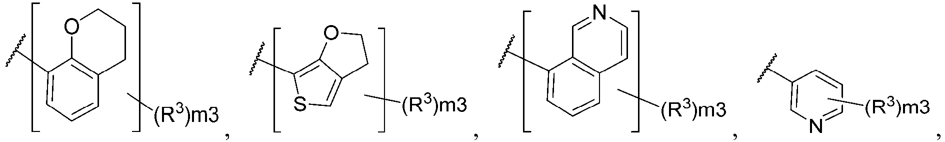

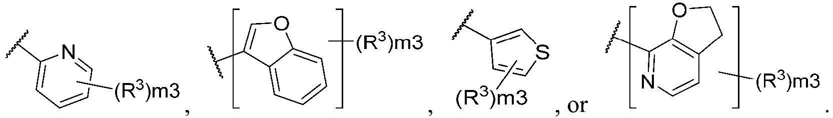

In a certain embodiment of the present disclosure, when L is -(CR^{L1}R^{L2})n1-, - (CR^{L1}R^{L2})n2-O-, or -(CR^{L1}R^{L2})n2-C(O)-, 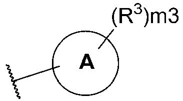 is

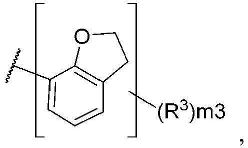

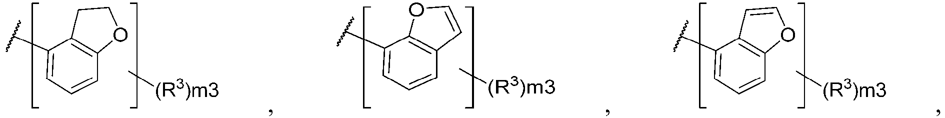

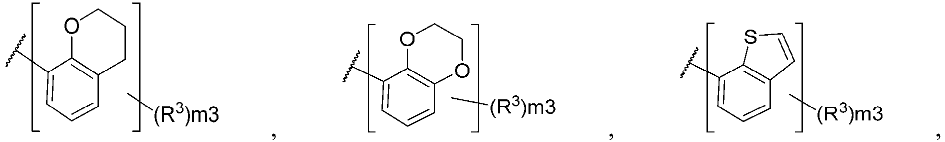

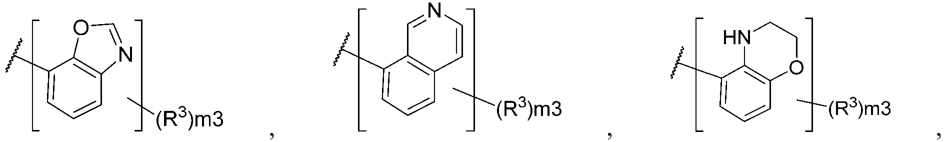

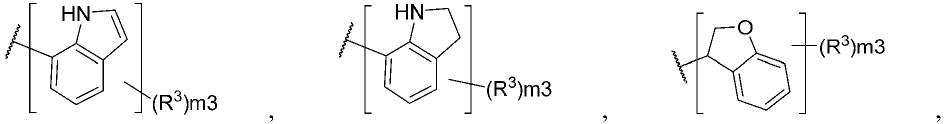

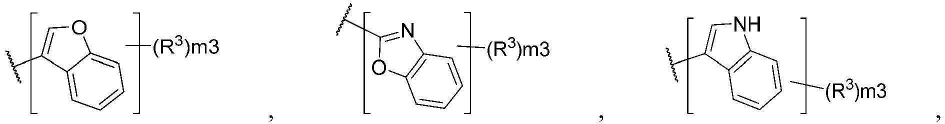

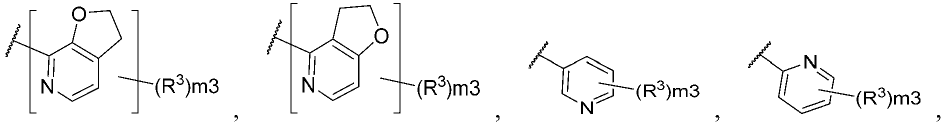

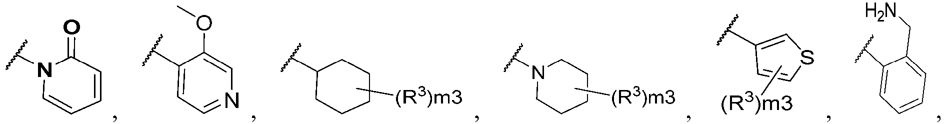

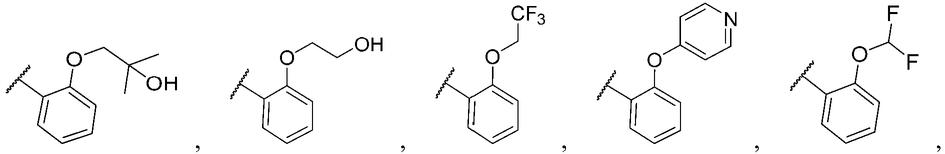

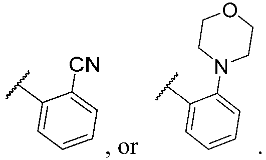

In a certain embodiment of the present disclosure, when L is -(CR^{L1}R^{L2})n1-, - (CR^{L1}R^{L2})n2-O-, or -(CR^{L1}R^{L2})n2-C(O)-, 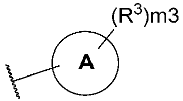 is

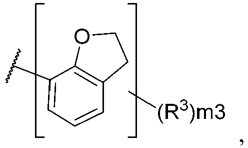

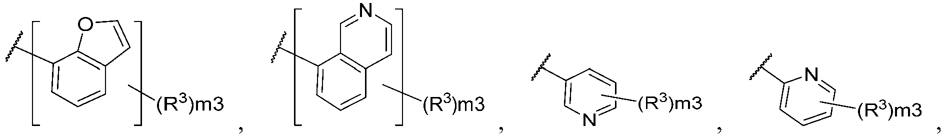

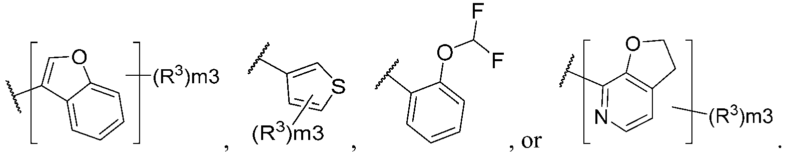

In a certain embodiment of the present disclosure, R³ is independently oxo (=O), C₁-C₆ alkoxy, -NR⁴R⁵, -C(O)R⁶, -SR⁷, -S(O)₂R⁸, halogen, C₁-C₆ alkoxy substituted with one or more R⁹, C₁-C₆ alkyl substituted with one or more R¹⁰, CN, "3-12 membered heterocycloalkyl containing 1, 2, or 3 heteroatoms selected from 1, 2, or 3 of N, O, and S", or -OR¹¹; for example, R³ is independently C₁-C₆ alkoxy, C₁-C₆ alkoxy substituted with one or more R⁹, or halogen.

In a certain embodiment of the present disclosure, R³ is independently -OR¹¹, C₁-C₆ alkyl, C₁-C₆ alkoxy, C₁-C₆ alkoxy substituted with one or more R⁹, or halogen.

In a certain embodiment of the present disclosure, R⁴ is independently H.

In a certain embodiment of the present disclosure, R⁵ is independently H or -C(O)R⁵⁻¹; for example, R⁵ is independently H.

In a certain embodiment of the present disclosure, R⁹ is independently halogen.

In a certain embodiment of the present disclosure, m3 is 0 or 1; for example, m3 is 0.

In a certain embodiment of the present disclosure, the "C₁-C₆ alkyl" in each "C₁-C₆ alkyl", "substituted C₁-C₆ alkyl", and "-C(O)-C₁-C₆ alkyl" is independently methyl, ethyl, n-propyl, isopropyl, n-butyl, isobutyl, sec-butyl, or tert-butyl, e.g., methyl or ethyl.

In a certain embodiment of the present disclosure, the "C₁-C₆ alkoxy" in each "C₁-C₆ alkoxy" and "substituted C₁-C₆ alkoxy" is independently methoxy, ethoxy, n-propoxy, isopropoxy, n-butoxy, isobutoxy, sec-butoxy, or tert-butoxy, e.g., methoxy or ethoxy.

In a certain embodiment of the present disclosure, each "3-12 membered heterocycloalkyl containing 1, 2, or 3 heteroatoms selected from 1, 2, or 3 of N, O, and S" is independently "5-6 membered heterocycloalkyl containing 1 or 2 heteroatoms selected from 1 or 2 of N and O", e.g., piperidinyl (e.g., 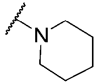 ), morpholinyl ( 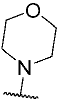 ), or oxetanyl (e.g., 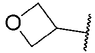 ).

In a certain embodiment of the present disclosure, each halogen is independently F, Cl, Br, or I, e.g., F or Cl.

In a certain embodiment of the present disclosure, each C₃-C₆ cycloalkyl is independently cyclopropyl, cyclobutyl, cyclopentyl, or cyclohexyl, e.g., cyclobutyl.

In a certain embodiment of the present disclosure, in ring A, the "9-12 membered bicyclic heterocyclyl containing 1, 2, or 3 heteroatoms selected from 1, 2, or 3 of N, O, and S" is 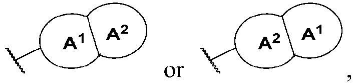 wherein ring A¹ is independently phenyl or pyridyl, and ring A² is independently "5-6 membered heterocycloalkyl containing 1 or 2 heteroatoms selected from 1 or 2 of N and O" or "5-6 membered heteroaryl containing 1 or 2 heteroatoms selected from 1 or 2 of N and O"; for example, ring A² is independently "5-membered heterocycloalkyl containing 1 or 2 heteroatoms selected from O" or "5-6 membered heteroaryl containing 1 or 2 heteroatoms selected from 1 or 2 of N and O"; for example, in ring A, the "9-12 membered bicyclic heterocyclyl containing 1, 2, or 3 heteroatoms selected from 1, 2, or 3 of N, O, and S" is

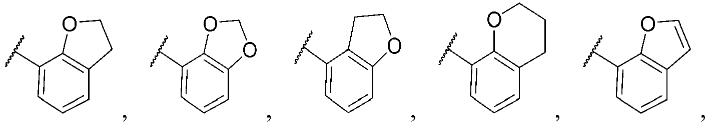

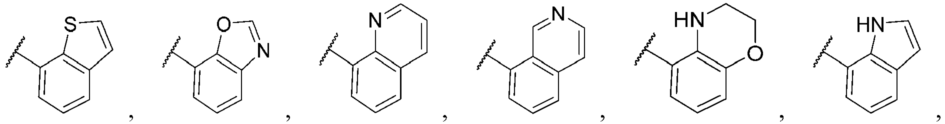

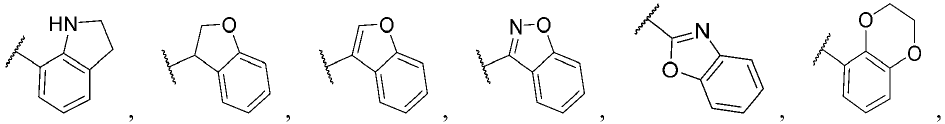

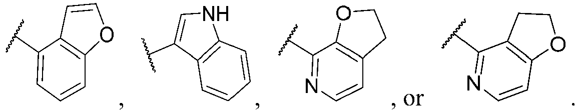

In a certain embodiment of the present disclosure, in ring A, the "8-12 membered bicyclic heterocyclyl containing 1, 2, or 3 heteroatoms selected from 1, 2, or 3 of N, O, and S" is "8-membered bicyclic heterocyclyl containing 1, 2, or 3 heteroatoms selected from 1, 2, or 3 of N, O, and S" or "9-12 membered bicyclic heterocyclyl containing 1, 2, or 3 heteroatoms selected from 1, 2, or 3 of N, O, and S", wherein the "9-12 membered bicyclic heterocyclyl containing 1, 2, or 3 heteroatoms selected from 1, 2, or 3 of N, O, and S" is as defined in any one of the embodiments of the present disclosure. For example, in ring A, the "8-12 membered bicyclic heterocyclyl containing 1, 2, or 3 heteroatoms selected from 1, 2, or 3 of N, O, and S" is 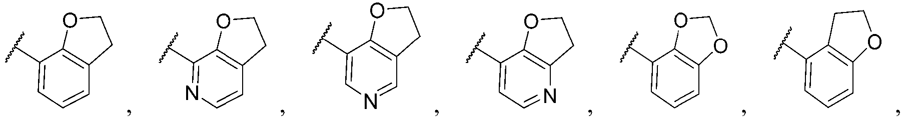 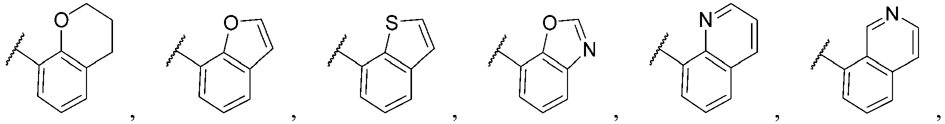 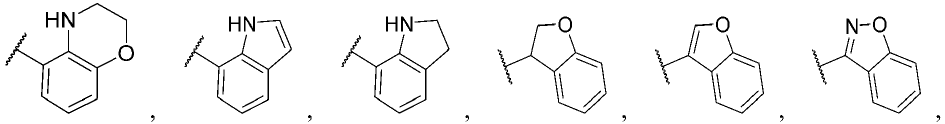 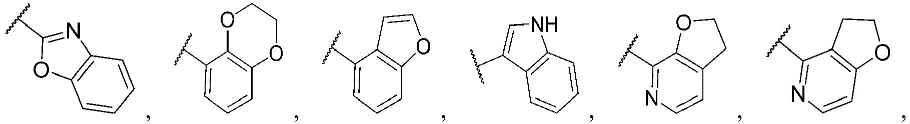 or

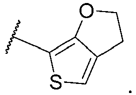

In a certain embodiment of the present disclosure, in ring A, the "5-6 membered monocyclic heterocyclyl containing 1, 2, or 3 heteroatoms selected from 1, 2, or 3 of N, O, and S" is "5-6 membered monocyclic heteroaryl containing 1 or 2 heteroatoms selected from 1 or 2 of N and S" or "5-6 membered monocyclic heterocycloalkyl containing 1 or 2 heteroatoms selected from N", e.g., pyridyl (e.g., 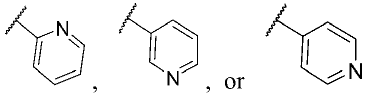 ), thienyl (e.g., 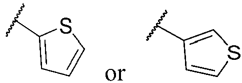 ), or piperidinyl (e.g., 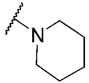 ); preferably, in ring A, the "5-6 membered monocyclic heterocyclyl containing 1, 2, or 3 heteroatoms selected from 1, 2, or 3 of N, O, and S" is

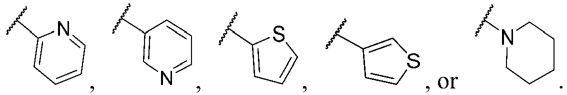

In a certain embodiment of the present disclosure, in ring A, the "9-16 membered polycyclic heterocyclyl containing 1, 2, or 3 heteroatoms selected from 1, 2, or 3 of N, O, and S" is "10-12 membered tricyclic heterocyclyl containing 1 or 2 heteroatoms selected from 1 or 2 of N and O", e.g.,

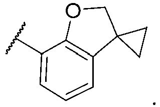

In a certain embodiment of the present disclosure, in ring A, the C₆-C₁₀ aryl is phenyl or naphthyl, e.g., phenyl.

In a certain embodiment of the present disclosure, in ring A, the C₃-C₆ cycloalkyl is cyclopropyl, cyclobutyl, cyclopentyl, or cyclohexyl, e.g., cyclohexyl.

In a certain embodiment of the present disclosure, each "5-10 membered heteroaryl containing 1, 2, or 3 heteroatoms selected from 1, 2, or 3 of N, O, and S" is independently "5-6 membered heteroaryl containing 1 or 2 heteroatoms selected from N", e.g., pyridyl (e.g.,

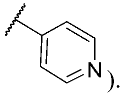

In a certain embodiment of the present disclosure, 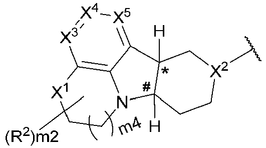 is one or more of 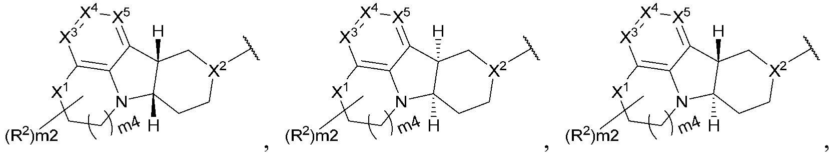 and

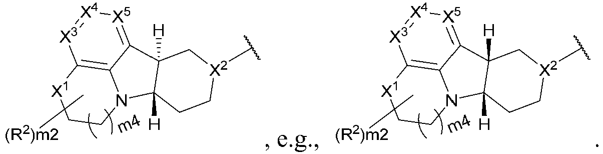

In a certain embodiment of the present disclosure, 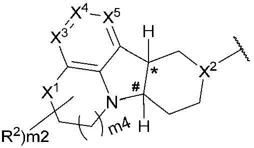 is

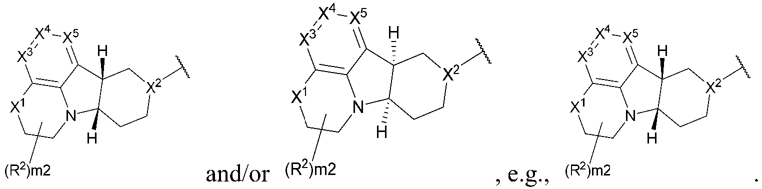

In a certain embodiment of the present disclosure, 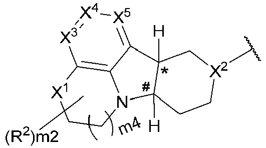 is

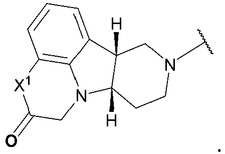

In a certain embodiment of the present disclosure, 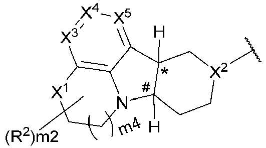 is

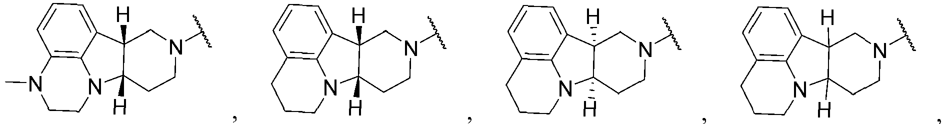

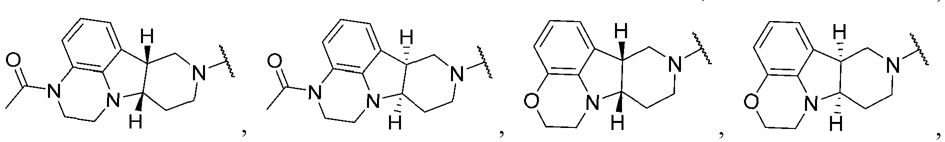

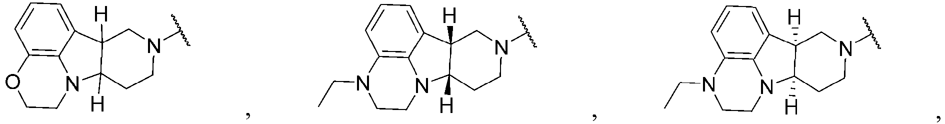

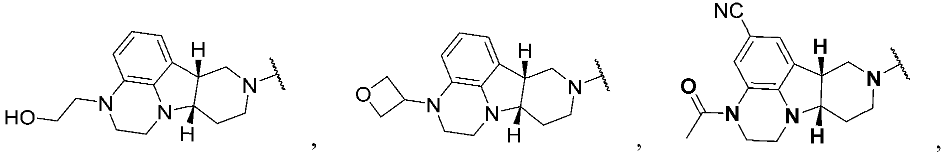

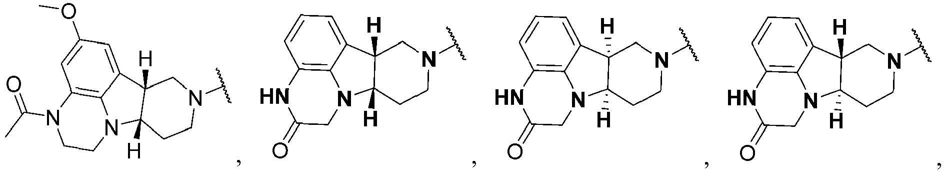

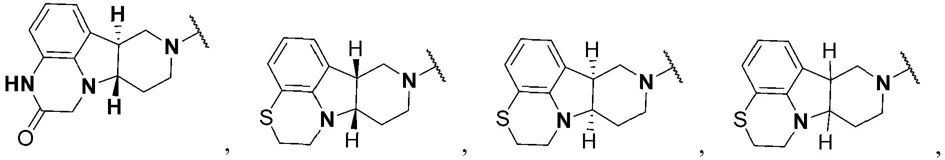

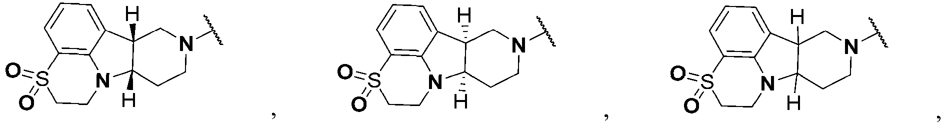

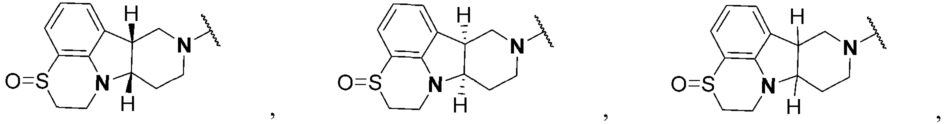

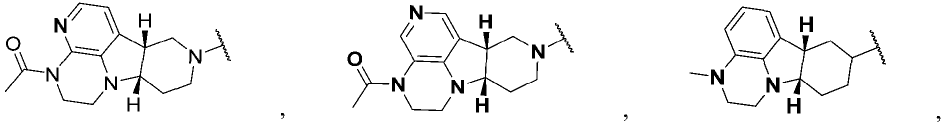

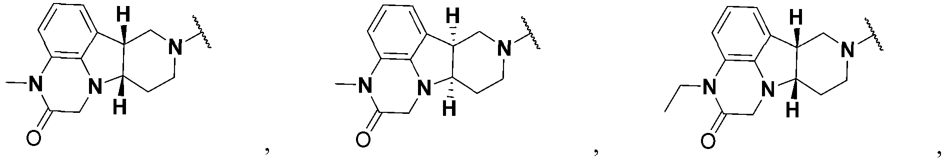

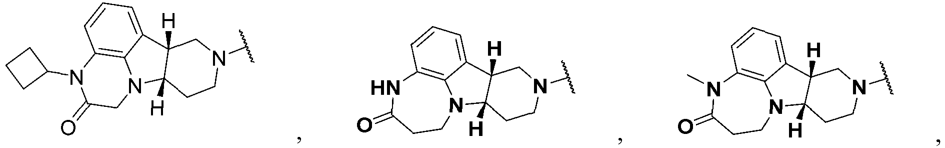

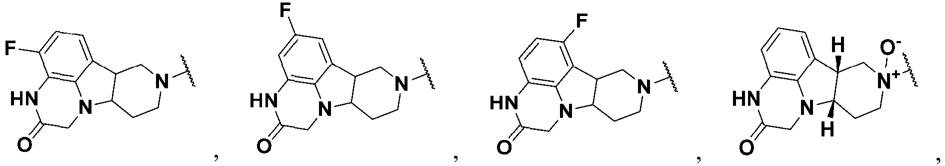

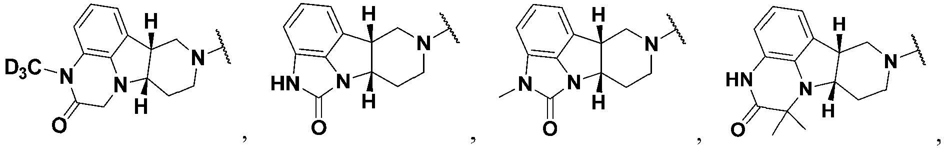

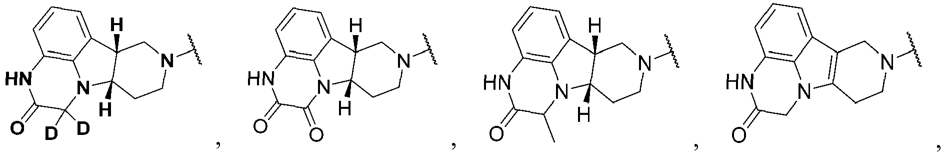

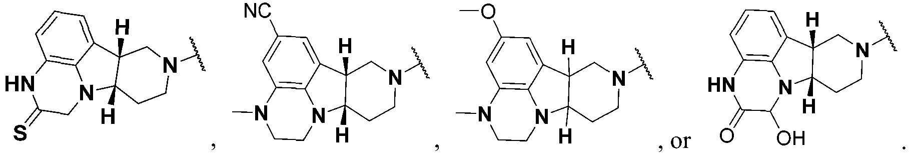

In a certain embodiment of the present disclosure, 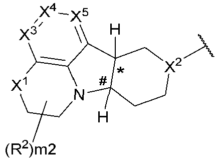 is 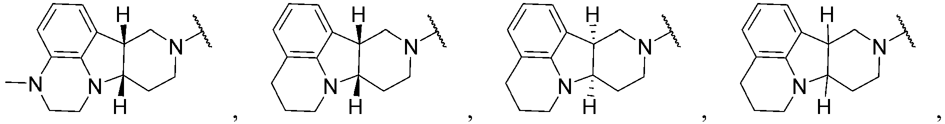 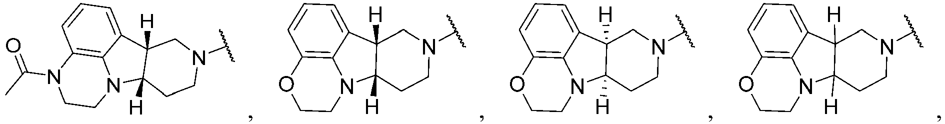 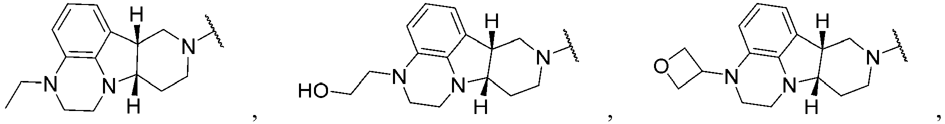 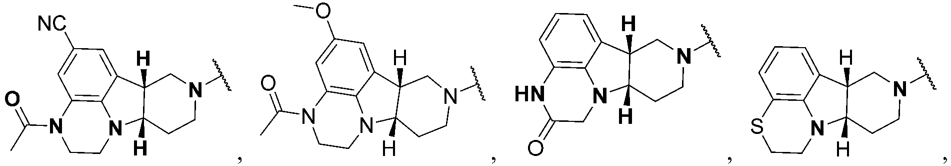 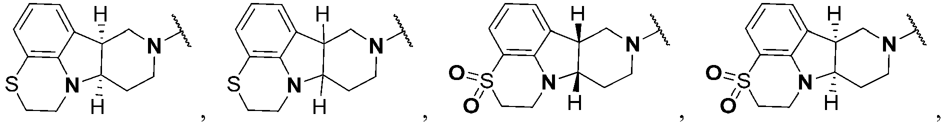 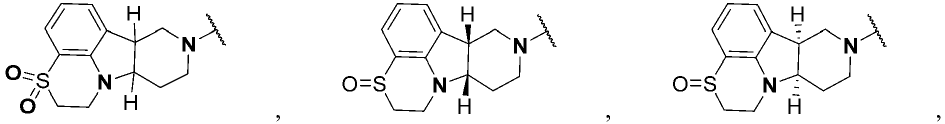 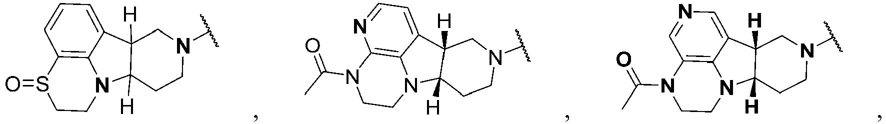 or

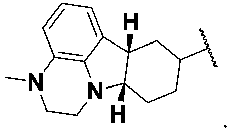

In a certain embodiment of the present disclosure, L is 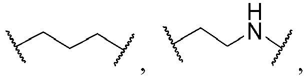 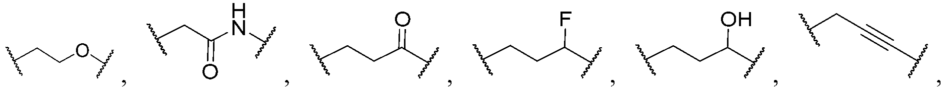 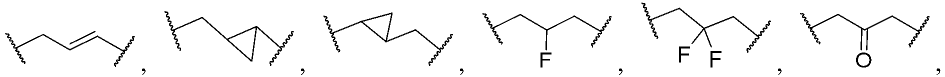 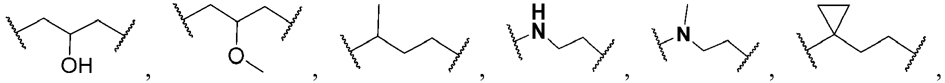 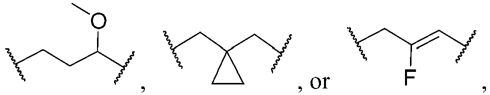 e.g.,

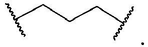

In a certain embodiment of the present disclosure, L is

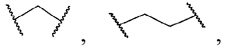

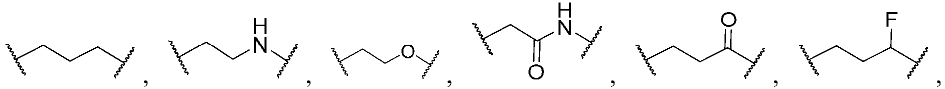

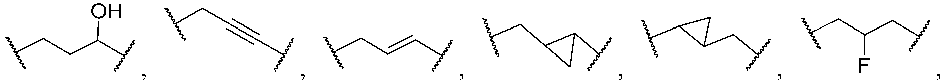

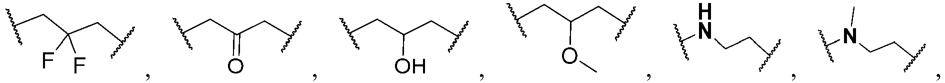

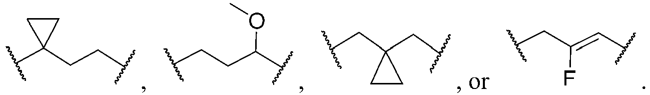

In a certain embodiment of the present disclosure, 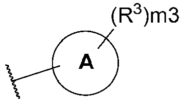 is

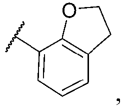

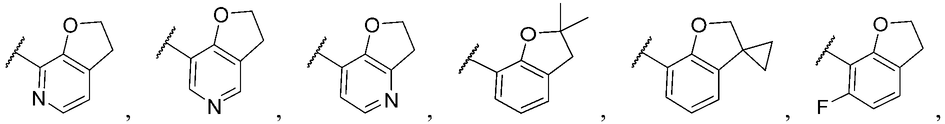

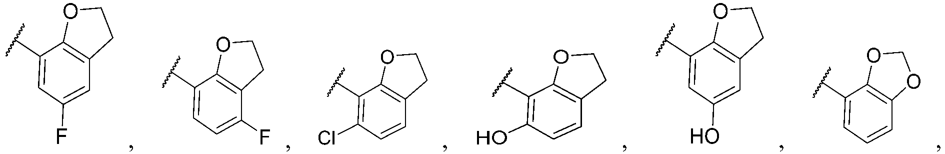

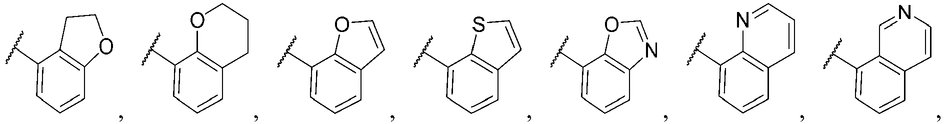

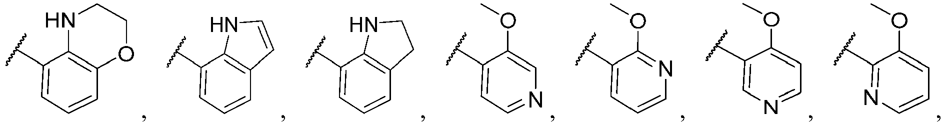

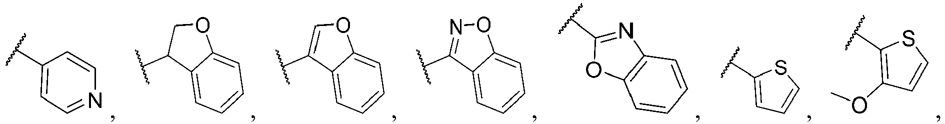

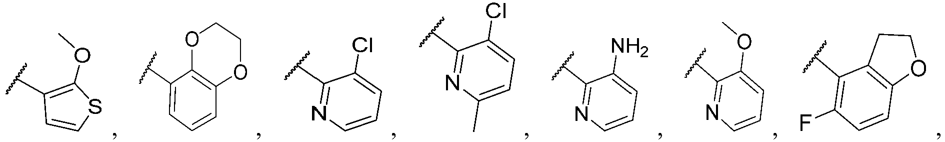

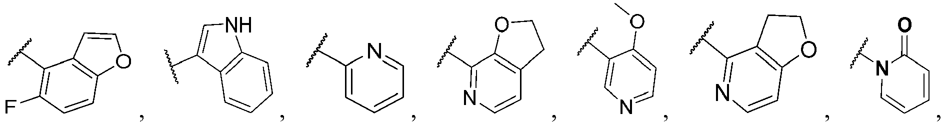

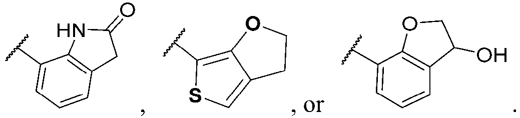

In a certain embodiment of the present disclosure, 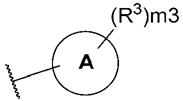 is

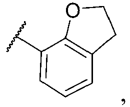

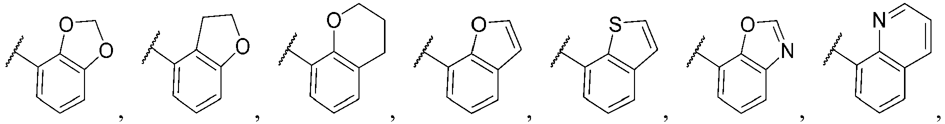

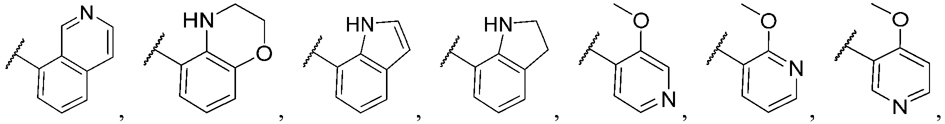

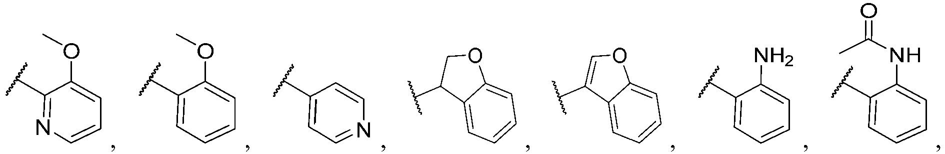

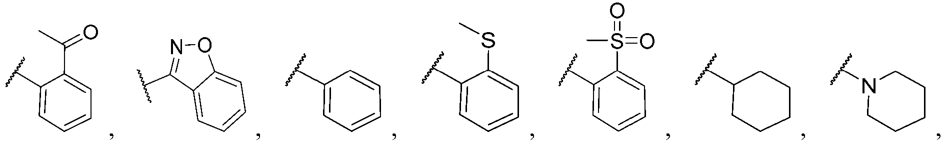

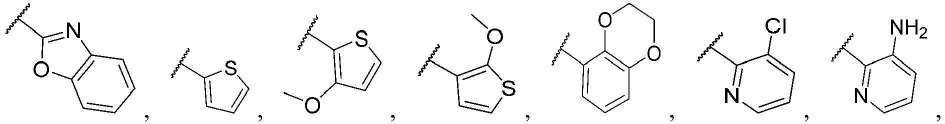

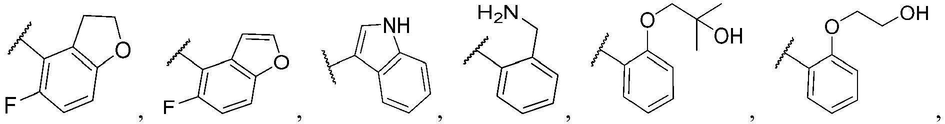

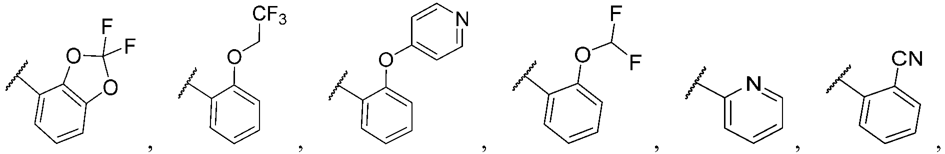

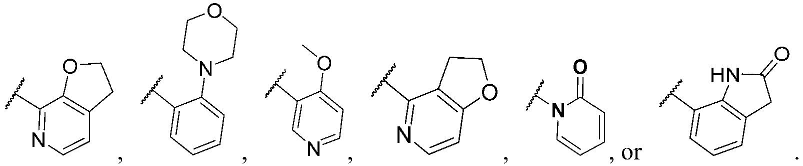

In a certain embodiment of the present disclosure, 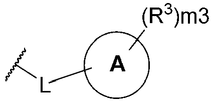 is

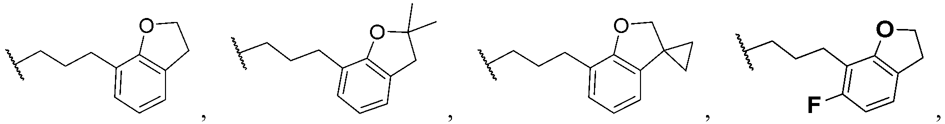

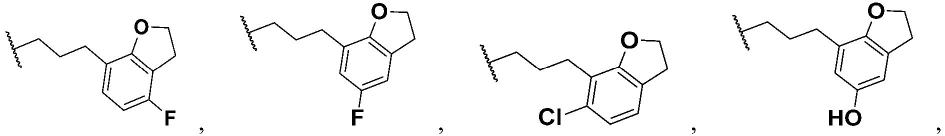

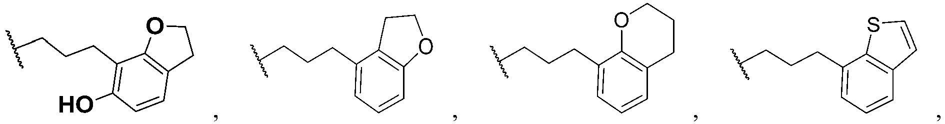

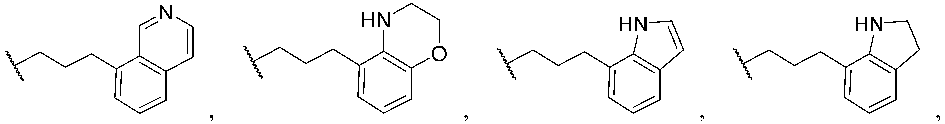

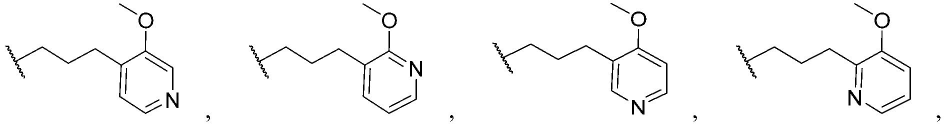

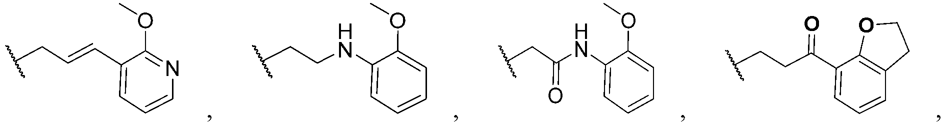

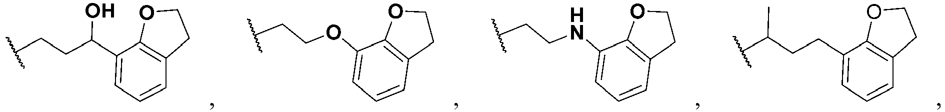

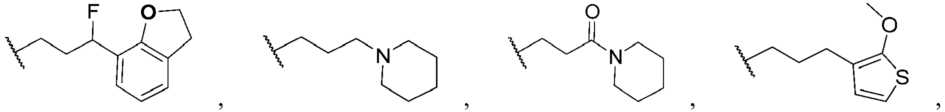

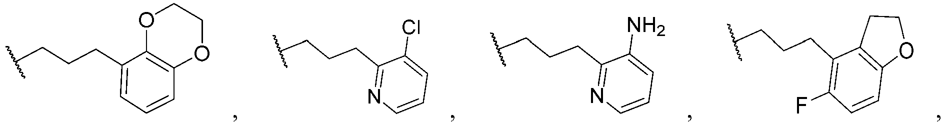

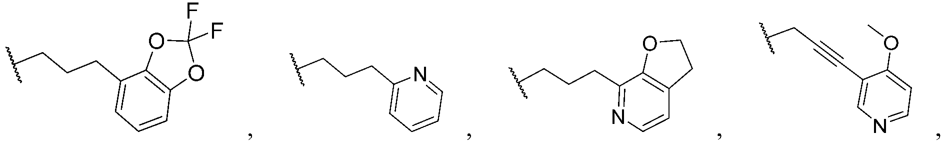

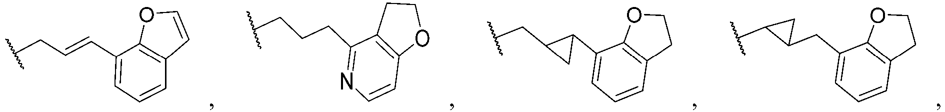

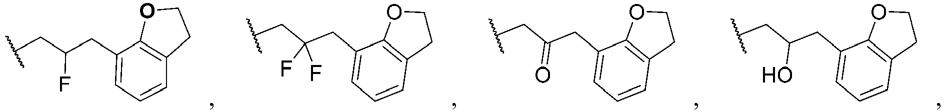

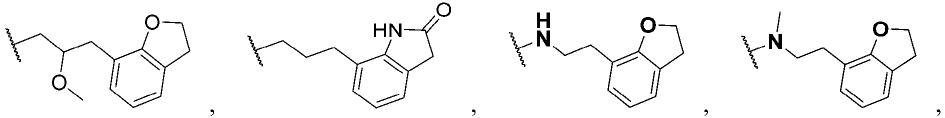

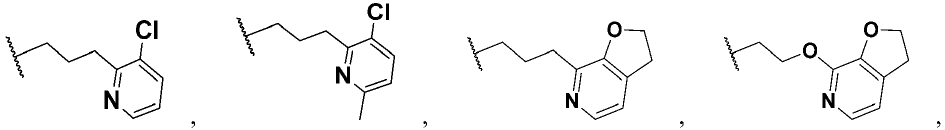

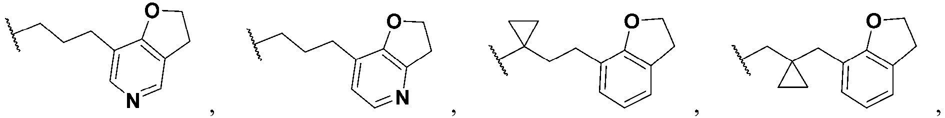

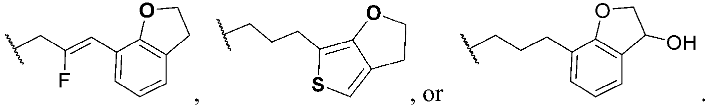

In a certain embodiment of the present disclosure, 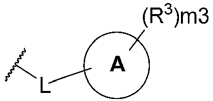 is

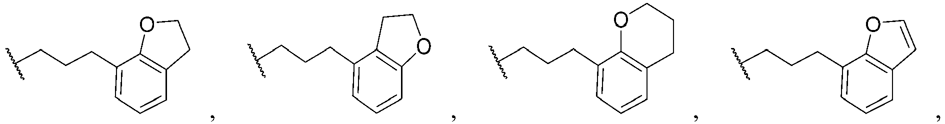

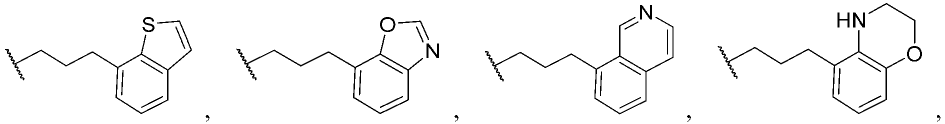

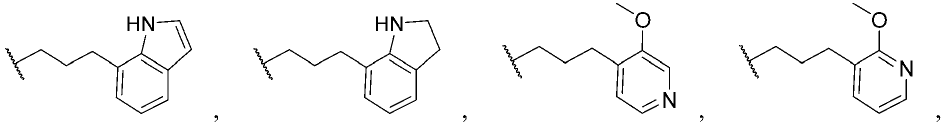

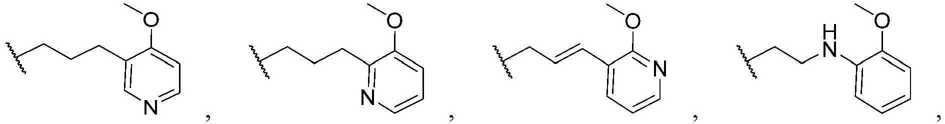

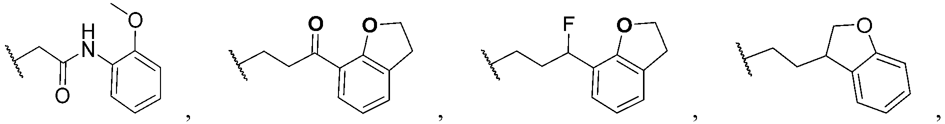

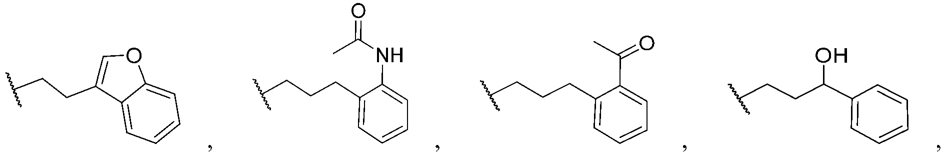

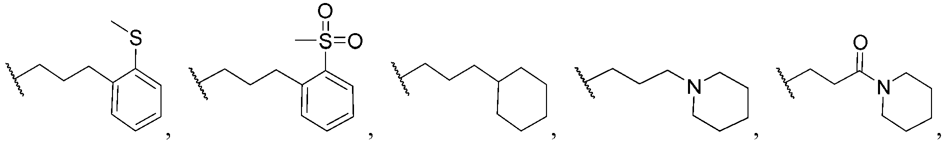

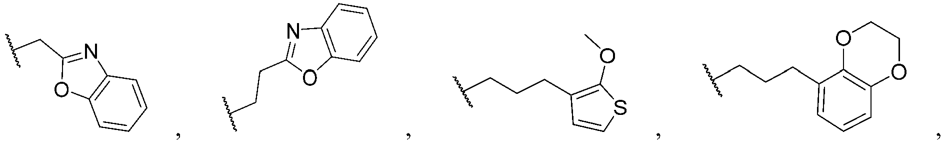

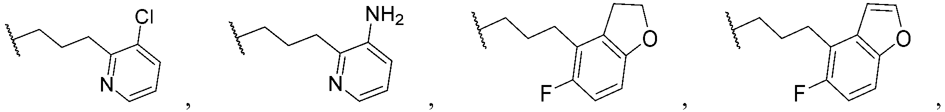

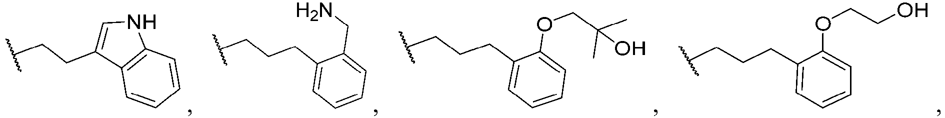

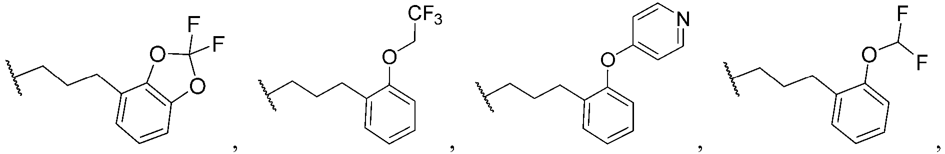

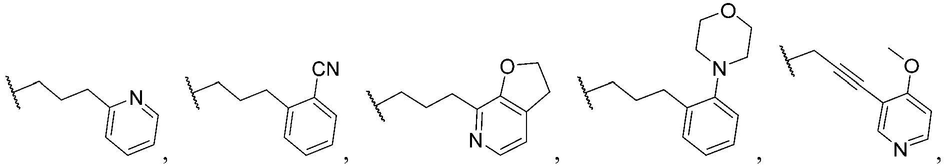

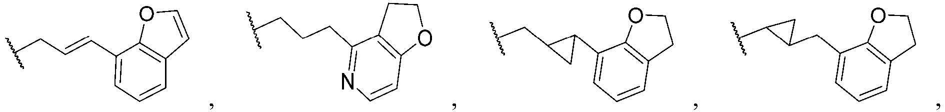

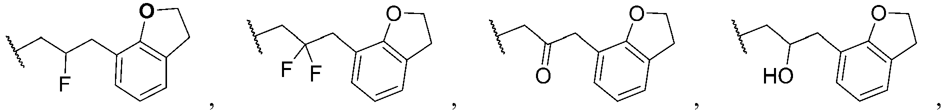

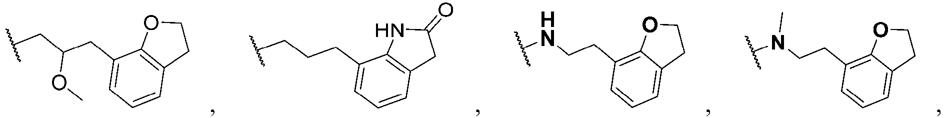

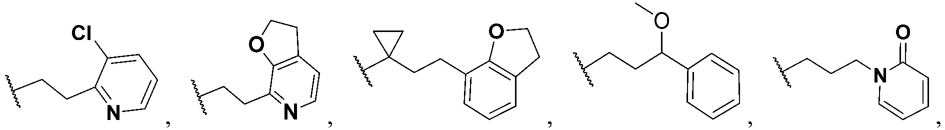

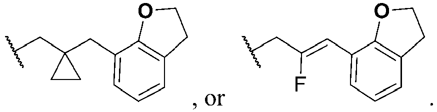

In a certain embodiment of the present disclosure, the tetracyclic compound is a compound represented by formula I-1, I-2, or I-3: 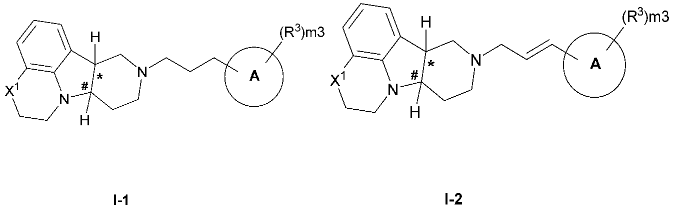 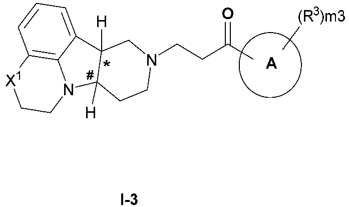 wherein *, #, X¹, ring A, R³, and m3 are as defined in any one of the embodiments of the present disclosure.

In a certain embodiment of the present disclosure, the tetracyclic compound is a compound represented by formula I-4: 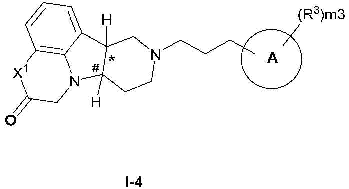 wherein *, #, X¹, ring A, R³, and m3 are as defined in any one of the embodiments of the present disclosure.

In a certain embodiment of the present disclosure, 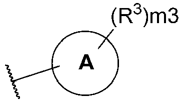 is

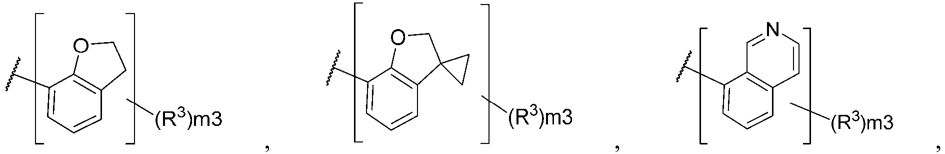

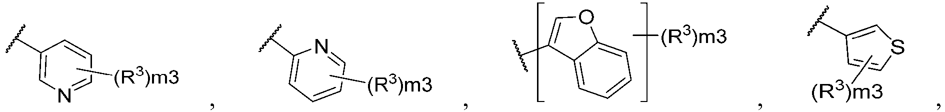

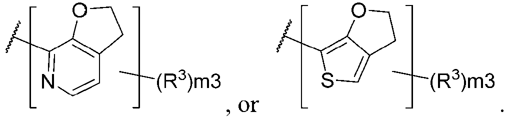

In a certain embodiment of the present disclosure, 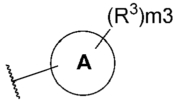 is 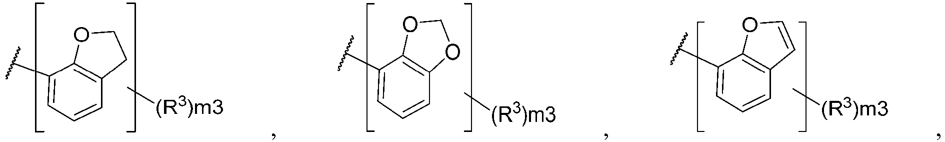 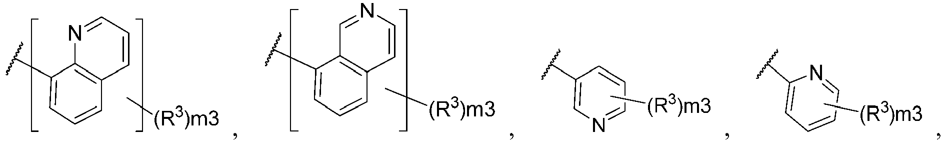 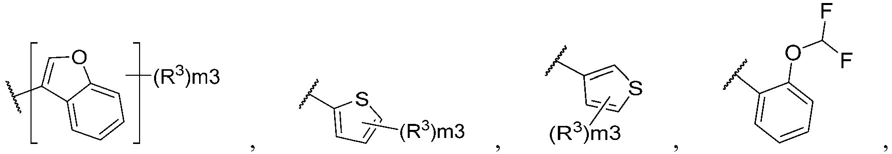 or

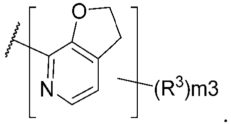

In a certain embodiment of the present disclosure, the compound of the present disclosure is not the following compounds: 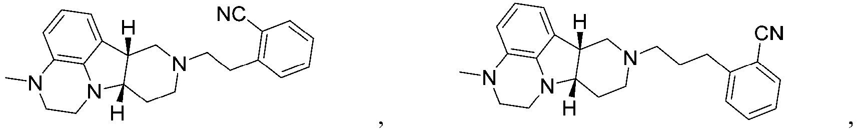 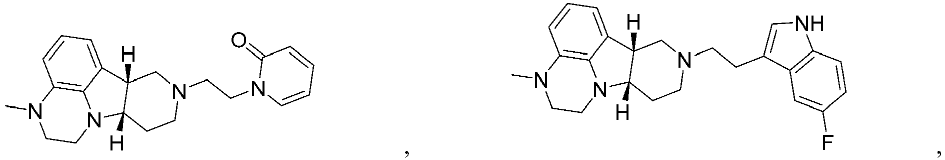 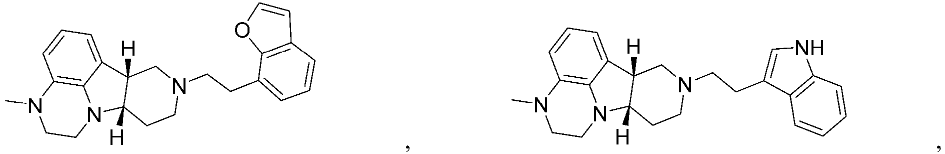 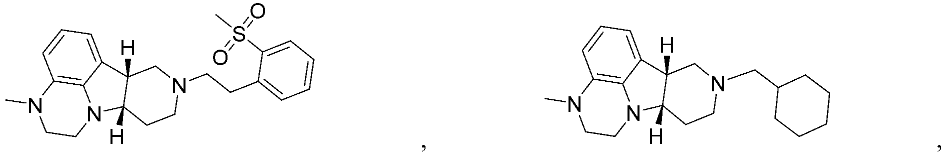 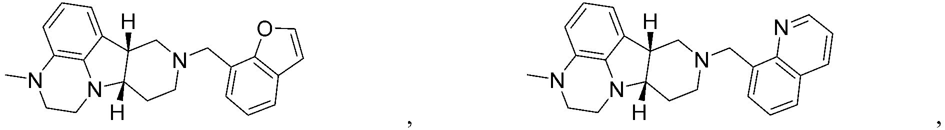 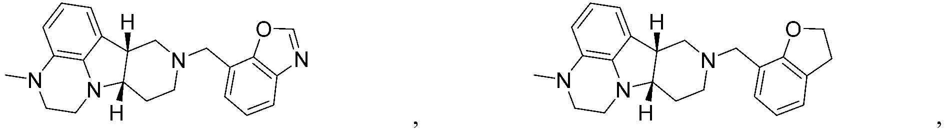 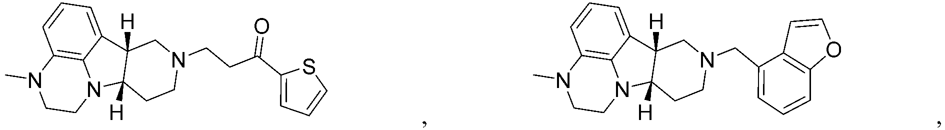 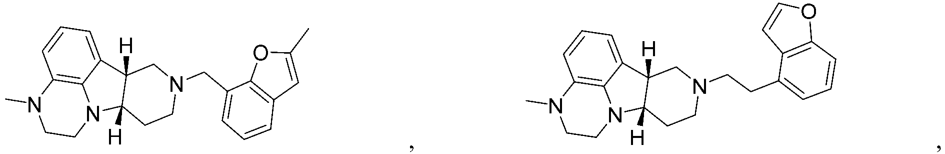 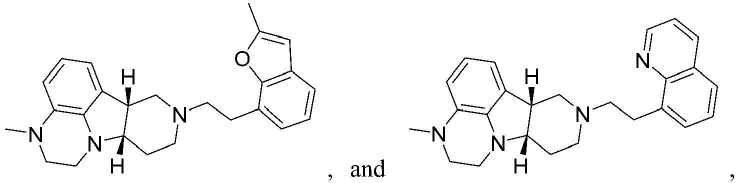 and stereoisomers thereof.

The present disclosure further provides a tetracyclic compound represented by any one of the following or a pharmaceutically acceptable salt thereof:

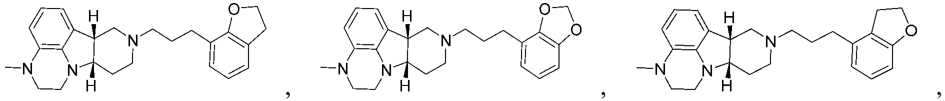

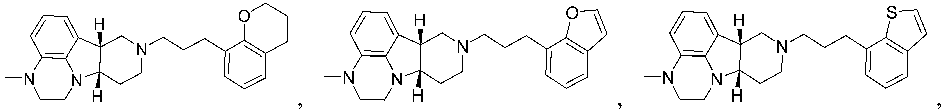

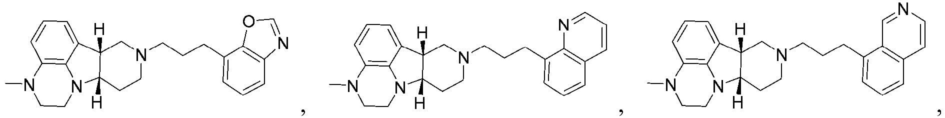

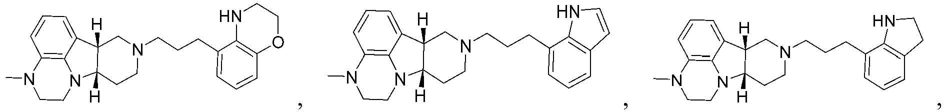

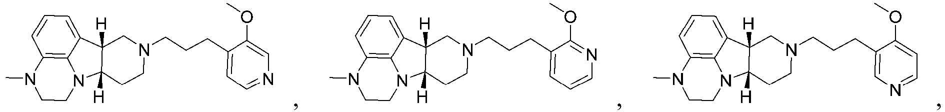

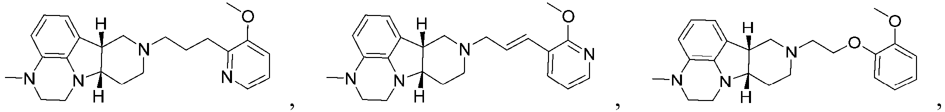

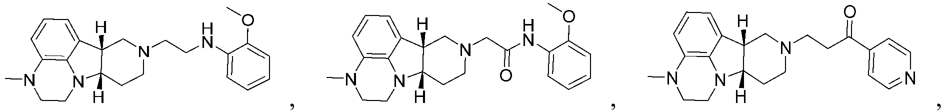

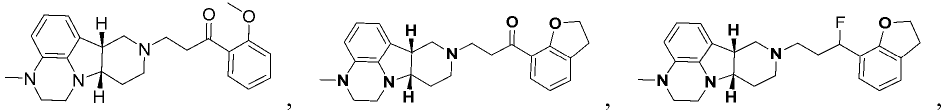

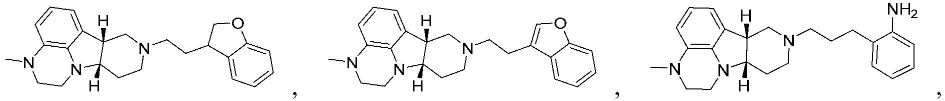

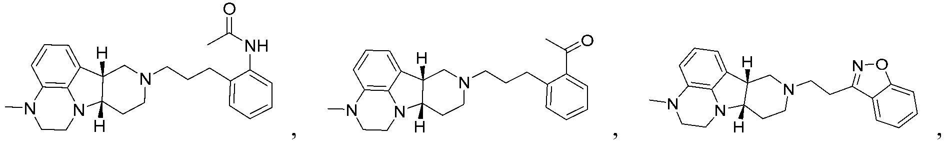

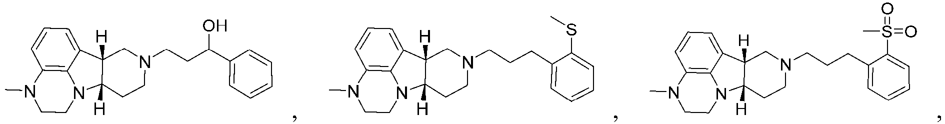

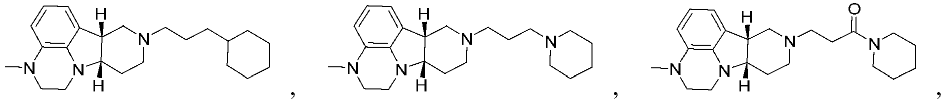

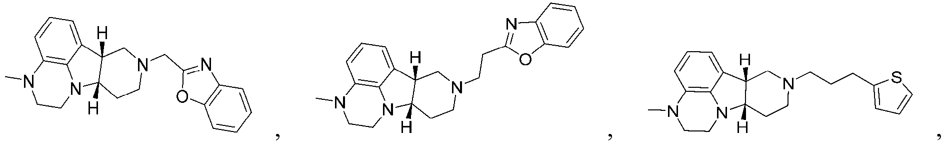

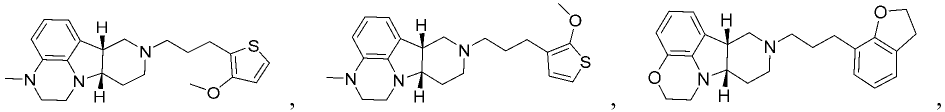

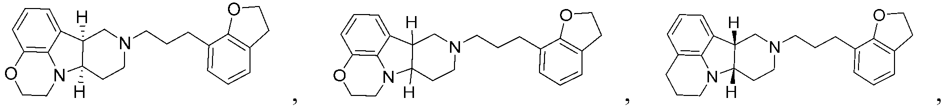

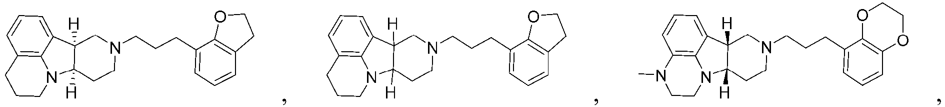

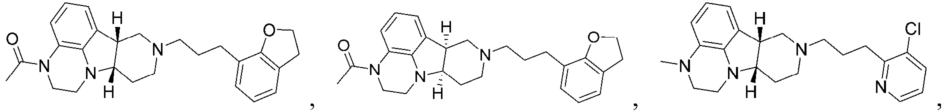

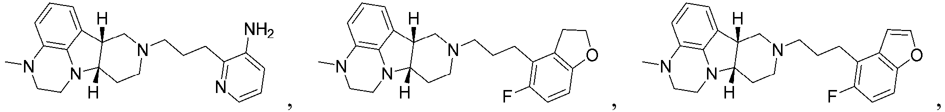

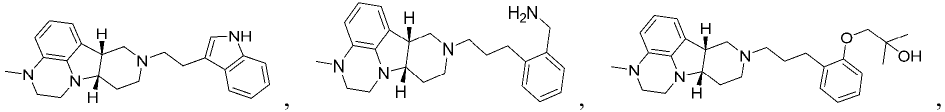

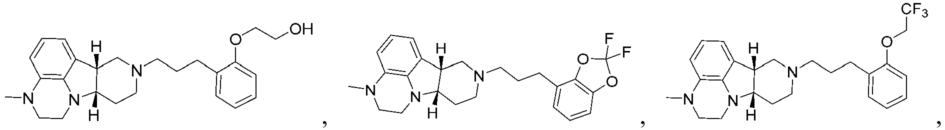

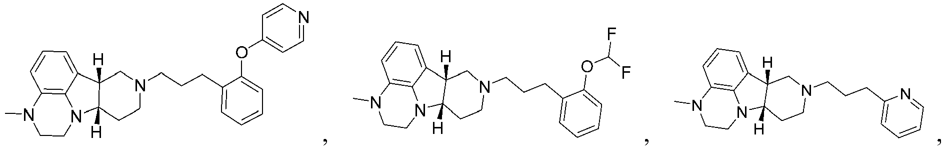

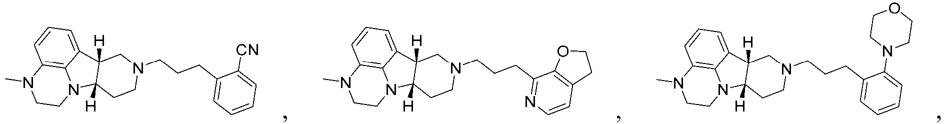

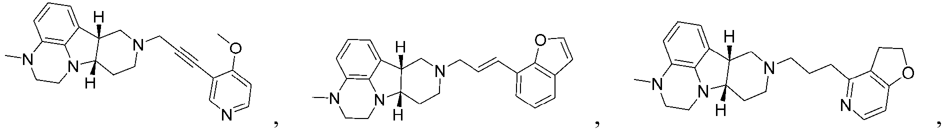

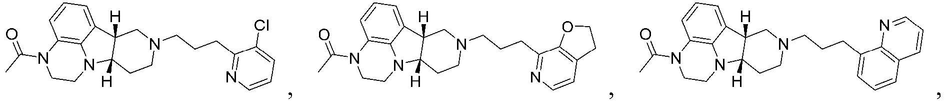

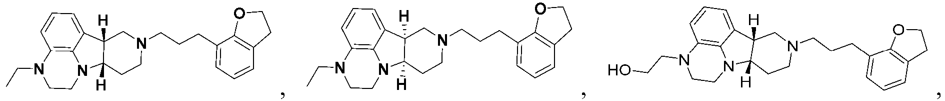

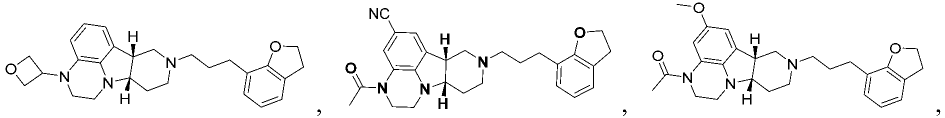

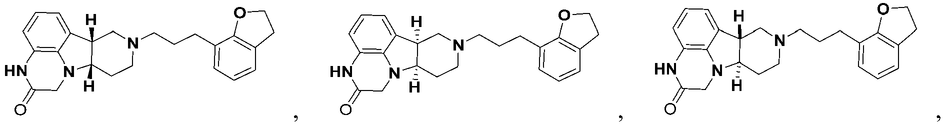

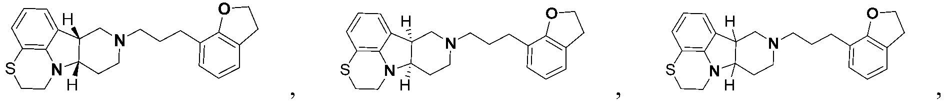

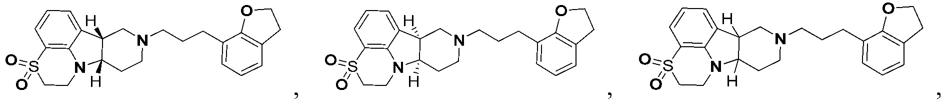

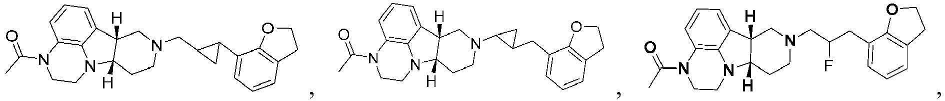

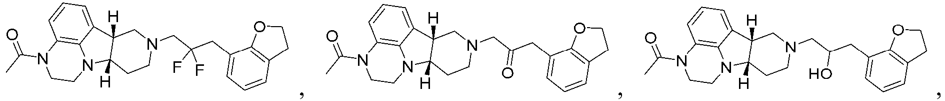

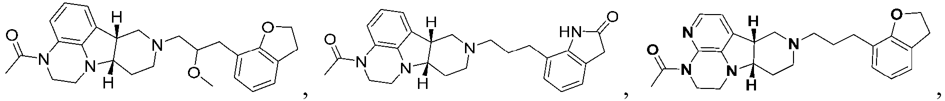

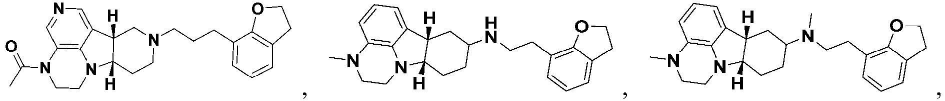

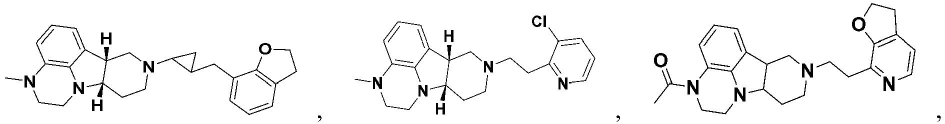

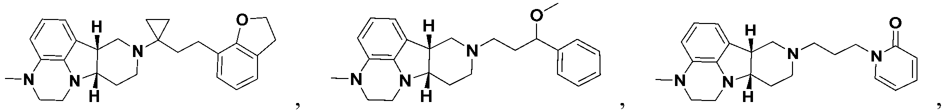

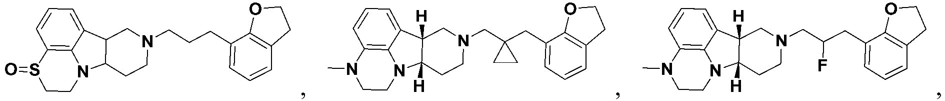

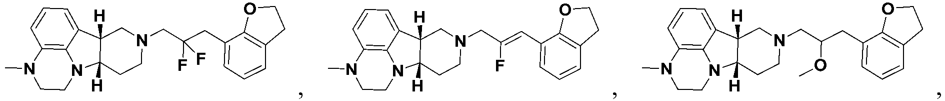

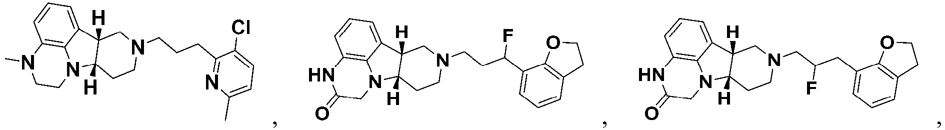

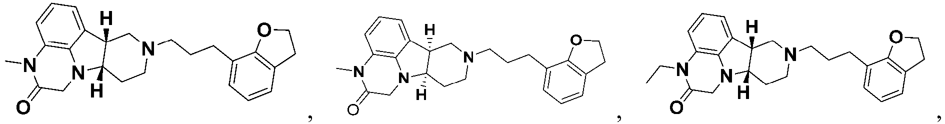

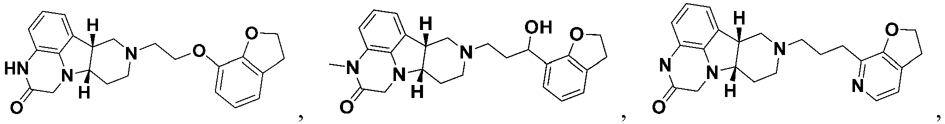

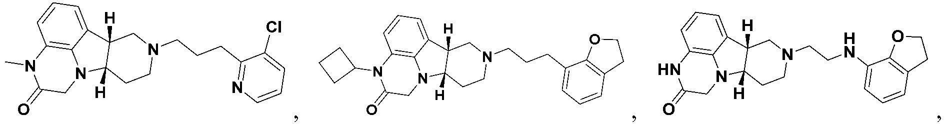

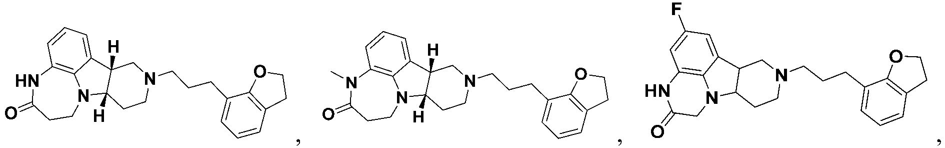

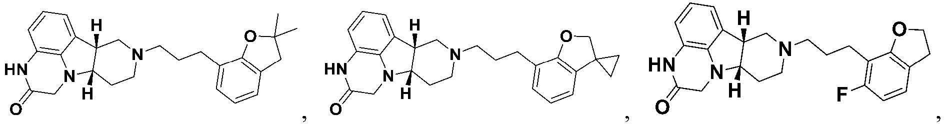

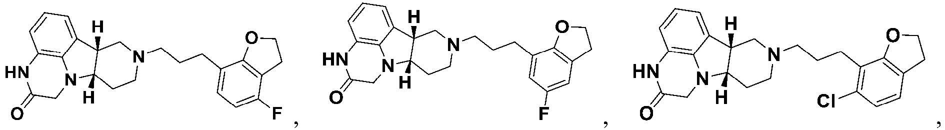

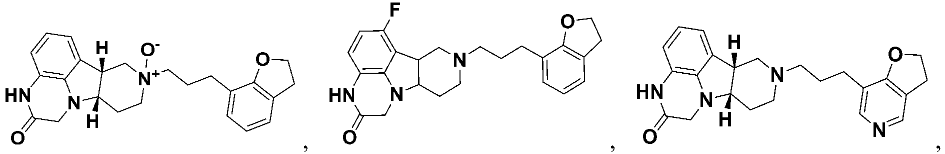

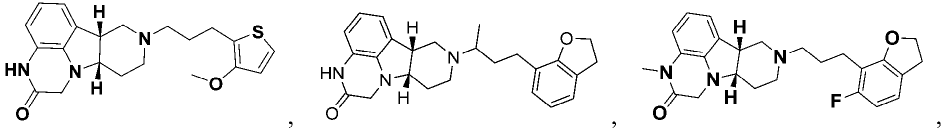

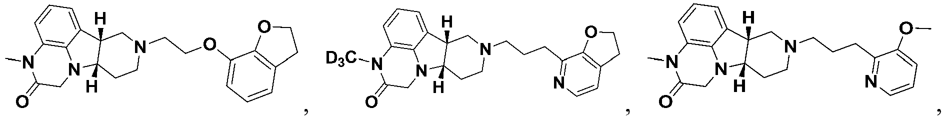

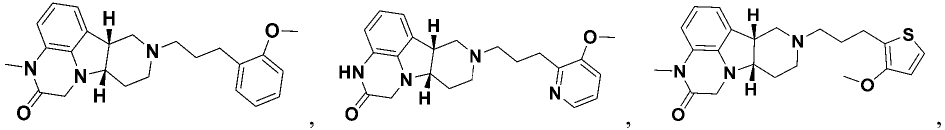

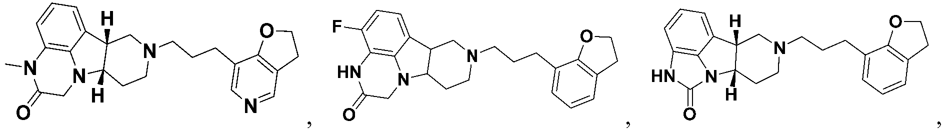

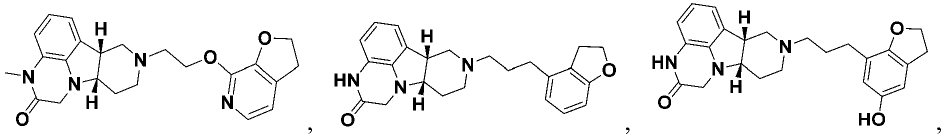

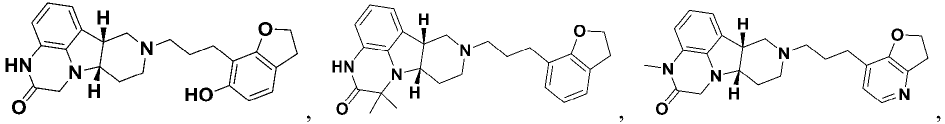

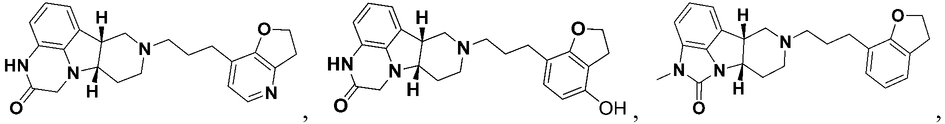

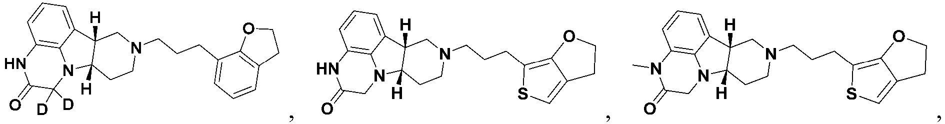

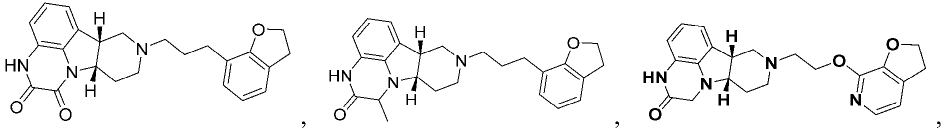

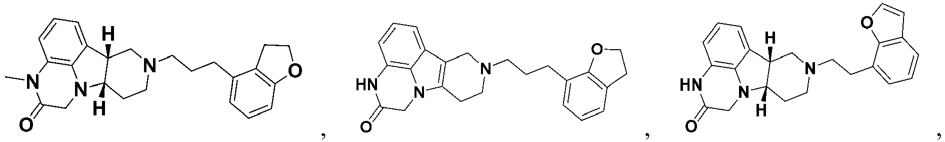

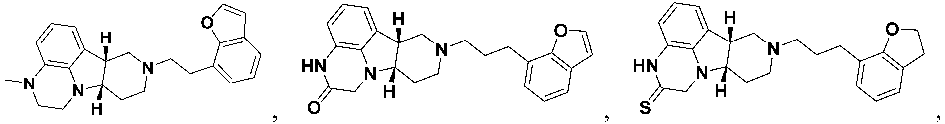

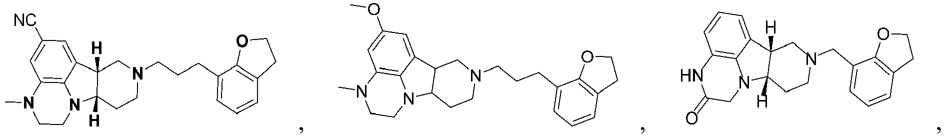

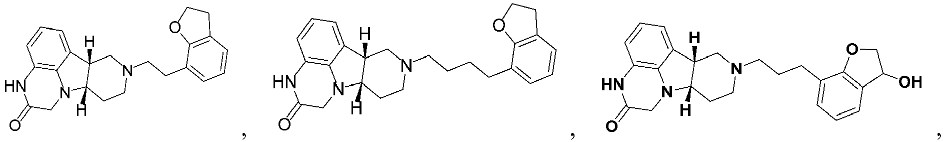

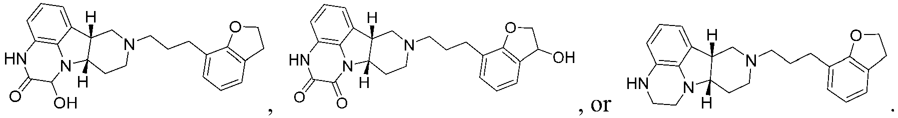

The present disclosure provides a pharmaceutical composition comprising:
a) the tetracyclic compound or the pharmaceutically acceptable salt thereof according to any one of the embodiments of the present disclosure, and
b) a pharmaceutically acceptable auxiliary material.

The present disclosure further provides use of the tetracyclic compound or the pharmaceutically acceptable salt thereof according to any one of the embodiments of the present disclosure or the pharmaceutical composition described above, wherein the use is selected from:
a) use for preparing a 5-HT_{2A} receptor agonist;
b) use for preparing a medicament for treating and/or preventing a 5-HT_{2A} receptor-related disease; and
c) use for preparing a medicament for treating and/or preventing depression.

The present disclosure further provides a method for treating and/or preventing a 5-HT_{2A} receptor-related disease, comprising: administering to a patient in need thereof a therapeutically effective amount of the tetracyclic compound represented by formula I or the pharmaceutically acceptable salt thereof according to any one of the embodiments of the present disclosure or the pharmaceutical composition described above.

The present disclosure further provides a method for treating and/or preventing depression, comprising: administering to a patient in need thereof a therapeutically effective amount of the tetracyclic compound represented by formula I or the pharmaceutically acceptable salt thereof according to any one of the embodiments of the present disclosure or the pharmaceutical composition described above.

In a certain embodiment of the present disclosure, the 5-HT_{2A} receptor-related disease is depression.

### Terminology

Unless otherwise defined, the terms used in the present disclosure have the following meanings:
The term "pharmaceutically acceptable" refers to being relatively non-toxic, safe, and suitable for use in a patient.

The term "pharmaceutically acceptable salt" refers to a salt obtained from a reaction of the compound with a pharmaceutically acceptable acid or base. When the compound contains a relatively acidic functional group, a base addition salt can be obtained by making the compound come into contact with a sufficient amount of a pharmaceutically acceptable base in a suitable inert solvent. When the compound contains a relatively basic functional group, an acid addition salt can be obtained by making the compound come into contact with a sufficient amount of a pharmaceutically acceptable acid in a suitable inert solvent. See Handbook of Pharmaceutical Salts: Properties, Selection, and Use (P. Heinrich Stahl, Camille G. Wermuth, 2011, 2nd Revised Edition) for details.

The mark "-" at the end of a group means that the group is linked to the rest of the molecule via the site, and the mark " 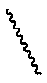 " at the end of a structural fragment means that the structural fragment is linked to the rest of the molecule via the site; for example, 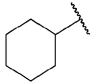 refers to cyclohexyl.

When the direction in which a linking group enumerated in the present disclosure is linked is not specified, it is linked in a direction that is the same as the left-to-right reading direction. For example, when the linking group L in 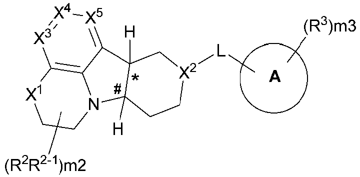 is - (CR^{L1}R^{L2})n2-Y¹-(CR^{L1}R^{L2})n3-, then -(CR^{L1}R^{L2})n2-Y¹-(CR^{L1}R^{L2})n3- is linked in a direction that is the same as the left-to-right reading direction to form 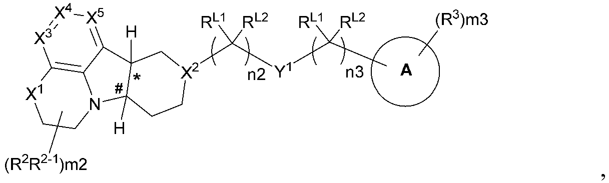 rather than

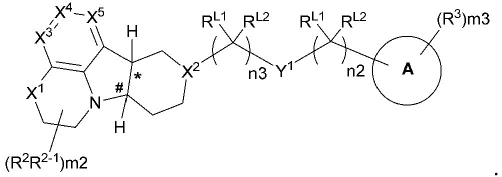

The term "one or more" refers to 1, 2, or 3.

The term "halogen" refers to fluorine, chlorine, bromine, or iodine.

The term "oxo" refers to =O, which means that an oxygen atom replaces two hydrogens on the same carbon atom; that is, a carbonyl group replaces a methylene group.

The term "alkyl" refers to a linear or branched, saturated, monovalent hydrocarbon group having a specified number of carbon atoms (e.g., C₁-C₆). Alkyl groups include, but are not limited to, methyl, ethyl, n-propyl, isopropyl, n-butyl, isobutyl, sec-butyl, tert-butyl, n-pentyl, n-hexyl, etc.

The term "haloalkyl" refers to an alkyl group substituted with one or more halogens, wherein the definition of the halogens is the same as that of the term "halogen", and the definition of the alkyl group is the same as that of the term "alkyl". Haloalkyl groups include, but are not limited to: -CF₃, -CHF₂, -CH₂CF₃, etc.

The term "alkoxy" refers to the group -O-R^{X}, wherein the definition of R^{X} is the same as that of the term "alkyl". Alkoxy groups include, but are not limited to: methoxy, ethoxy, n-propoxy, isopropoxy, etc.

The term "haloalkoxy" refers to the group -O-R^{X}', wherein the definition of R^{X}' is the same as that of the term "haloalkyl". Haloalkoxy groups include, but are not limited to: -O-CF₃, -O-CHF₂, -O-CH₂CF₃, etc.

The term "cycloalkyl" refers to a saturated, cyclic hydrocarbon group having a specified number of carbon atoms (e.g., C₃-C₆), which is monocyclic. Cycloalkyl groups include, but are not limited to: 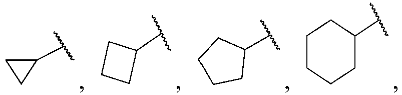 etc.

The term "aryl" refers to an unsaturated, cyclic hydrocarbon group having a specified number of carbon atoms (e.g., C₆-C₁₀), which is monocyclic or polycyclic (e.g., 2 rings); when it is polycyclic, two atoms and one bond are shared between adjacent monocyclic rings, and each ring is aromatic. Aryl groups include, but are not limited to: phenyl, naphthyl, etc.

The term "heterocycloalkyl" refers to a cyclic group having a specified number of ring atoms (e.g., 3-12 membered), a specified number of heteroatoms (e.g., 1, 2, or 3 heteroatoms), and specified heteroatom types (one, two, or three of N, O, and S), which is monocyclic, bridged, or spiro, and each ring is saturated. A heterocycloalkyl group is linked to the rest of the molecule via a carbon atom or a heteroatom. Heterocycloalkyl groups include, but are not limited to: 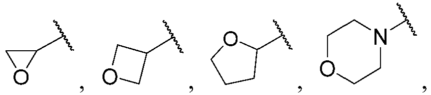 etc.

The term "monocyclic heterocyclyl" refers to a saturated or unsaturated, aromatic or non-aromatic, cyclic group having a specified number of ring atoms (e.g., 5-6 membered), a specified number of heteroatoms (e.g., 1, 2, or 3 heteroatoms), and specified heteroatom types (one, two, or three of N, O, and S), which is monocyclic. A heterocyclyl group is linked to the rest of the molecule via a carbon atom or a heteroatom.

The term "bicyclic heterocyclyl" refers to a cyclic group having a specified number of ring atoms (e.g., 5-6 membered), a specified number of heteroatoms (e.g., 1, 2, or 3 heteroatoms), and specified heteroatom types (one, two, or three of N, O, and S), which is bicyclic, and each ring is independently saturated or unsaturated, and aromatic or non-aromatic. A heterocyclyl group is linked to the rest of the molecule via a carbon atom or a heteroatom. Bicyclic heterocyclyl groups include, but are not limited to: 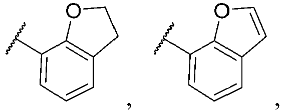 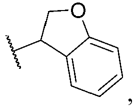 etc.

The term "heteroaryl" refers to an unsaturated, cyclic group having a specified number of ring atoms (e.g., 5-10 membered or 5-6 membered), a specified number of heteroatoms (e.g., 1, 2, or 3 heteroatoms), and specified heteroatom types (one, two, or three of N, O, and S), which is monocyclic or polycyclic; when it is polycyclic, two atoms and one bond are shared between monocyclic rings, and each ring is aromatic. A heteroaryl group is linked to the rest of the molecule via a carbon atom or a heteroatom; when the heteroaryl group is polycyclic, it is linked to the rest of the molecule via a ring having a heteroatom or a ring having no heteroatom. Heteroaryl groups include, but are not limited to, 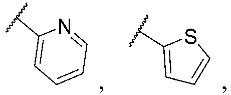 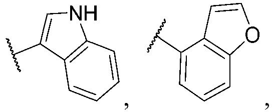 etc.

The term "therapeutically effective amount" refers to an amount administered to a patient that is sufficient to effectively treat a disease. The therapeutically effective amount will vary depending on the type of the compound, the type of the disease, the severity of the disease, the age of the patient, etc., but can be adjusted as appropriate by those skilled in the art.

The term "pharmaceutically acceptable auxiliary material" refers to all substances, other than the active pharmaceutical ingredient, contained in a pharmaceutical formulation, which are generally classified into two main categories: excipients and additives. See the Pharmacopoeia of the People's Republic of China (2020 Edition) and Handbook of Pharmaceutical Excipients (Paul J Sheskey, Bruno C Hancock, Gary P Moss, DavidJ Goldfarb, 2020, 9th Edition) for details.

The term "treating" refers to eliminating causes or alleviating symptoms.

The term "preventing" refers to reducing the risk of developing a disease.

The term "patient" refers to any animal, generally a mammal, e.g., a human, in need of treatment or prevention of a disease. Mammals include, but are not limited to, cows, horses, sheep, pigs, cats, dogs, mice, rats, domestic rabbits, guinea pigs, monkeys, humans, etc.

The above preferred conditions may be combined arbitrarily to obtain preferred examples of the present disclosure without departing from the general knowledge in the art.

The reagents and starting materials used in the present disclosure are all commercially available.

The positive and progressive effect of the present disclosure is that the compound of the present disclosure can reduce depression and hallucinogenic effects while maintaining a relatively good antidepressant effect, and has a relatively low level of drug interaction in the body.

### DETAILED DESCRIPTION OF THE INVENTION

The present disclosure is further illustrated by the following examples; however, these examples should not be construed as limiting the present disclosure. In the following examples, if no specific conditions are indicated in an experimental method, conventional methods and conditions were followed, or conditions were selected according to commodity instructions.

### Example 1: (6bR,10aS)-8-(3-(2,3-Dihydrobenzofuran-7-yl)propyl)-3-methyl-2,3,6b,7,8,9,10,10a-octahydro-1H-pyrido[3',4':4,5]pyrrolo[1,2,3-de]quinoxaline

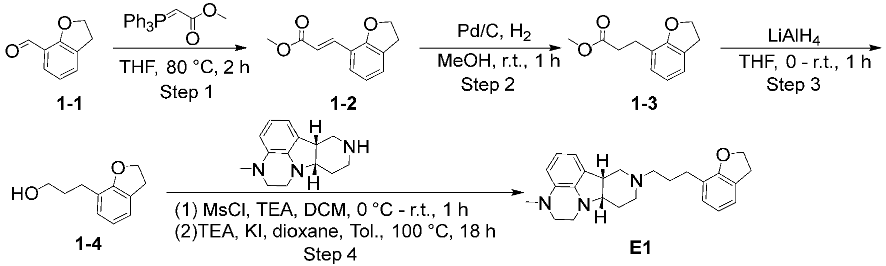

Step 1: 2,3-Dihydrobenzofuran-7-carbaldehyde (1.0 g, 6.7 mmol) and methyl (triphenylphosphoranylidene)acetate (3.36 g, 10.05 mmol) were dissolved in anhydrous tetrahydrofuran (15 mL) at room temperature. The reaction solution was stirred at 80 °C for 2 h. The reaction solution was cooled to room temperature and concentrated *in vacuo* to give a crude product. The crude product was separated and purified by column chromatography (EtOAc/PE = 0-10%) to give methyl (E)-3-(2,3-dihydrobenzofuran-7-yl)acrylate (1.18 g, yield: 76.1%) as a white solid. MS m/z (ESI): 205.1 [M+H]⁺.

Step 2: At room temperature, methyl (E)-3-(2,3-dihydrobenzofuran-7-yl)acrylate (1.18 g, 5.8 mmol) was dissolved in methanol (10 mL), and 10% wet palladium on carbon (310 mg, 0.29 mmol) was then added. The reaction solution was stirred at room temperature for 1 h in a hydrogen atmosphere. The reaction solution was filtered through diatomaceous earth, and the filter cake was washed with methanol. The filtrates were combined and concentrated *in vacuo* to give methyl 3-(2,3-dihydrobenzofuran-7-yl)propanoate (crude, 1.15 g, yield: 96.6%) as a colorless viscous oil. The crude product was directly used in the next step without purification. MS m/z (ESI): 207.1 [M+H]⁺.

Step 3: Methyl 3-(2,3-dihydrobenzofuran-7-yl)propanoate (500 mg, 2.4 mmol) was dissolved in tetrahydrofuran (8 mL) at room temperature, and the solution was cooled to 0 °C in a nitrogen atmosphere. A 1 M solution of lithium aluminum hydride in tetrahydrofuran (8 mL) was then added, and the mixture was stirred at room temperature for 1 h. The reaction solution was stirred at 0 °C. Ethyl acetate (20 mL) was slowly added for dilution, and sodium sulfate decahydrate was added. The mixture was filtered through diatomaceous earth, and the filter cake was washed with methanol. The filtrate was concentrated *in vacuo* to give a crude product. The crude product was separated and purified by column chromatography (EtOAc/PE = 0-15%) to give 3-(2,3-dihydrobenzofuran-7-yl)propan-1-ol (340 mg, yield: 78.7%) as a colorless oily liquid. MS m/z (ESI): 179.1 [M+H]⁺.

Step 4: 3-(2,3-Dihydrobenzofuran-7-yl)propan-1-ol (150 mg, 0.841 mmol) and triethylamine (256 mg, 2.525 mmol) were dissolved in dichloromethane (4 mL) at room temperature, and the solution was cooled to 0 °C. Methanesulfonyl chloride (145 mg, 1.262 mmol) was added, and the mixture was warmed to room temperature and stirred for 1 h. Then, the reaction solution was concentrated *in vacuo* at room temperature, and (6bR,10aS)-3-methyl-2,3,6b,7,8,9,10,10a-octahydro-1H-pyrido[3',4':4,5]pyrrolo[1,2,3-de]quinoxaline (97 mg, 0.421 mmol), potassium iodide (140 mg, 0.842 mmol), dioxane (3 mL), and triethylamine (256 mg, 2.525 mmol) were added. The reaction solution was stirred at 100 °C for 18 h. The reaction solution was cooled to room temperature and concentrated *in vacuo* to give a crude product. The crude product was purified by a preparative alkaline method to give (6bR,10aS)-8-(3-(2,3-dihydrobenzofuran-7-yl)propyl)-3-methyl-2,3,6b,7,8,9,10,10a-octahydro-1H-pyrido[3',4':4,5]pyrrolo[1,2,3-de]quinoxaline (28.61 mg, yield: 17.32%) as a yellow oily liquid. ¹H NMR (400 MHz, CDCl₃) δ 7.03 (d, *J* = 7.3 Hz, 1H), 6.92 (d, *J =* 7.5 Hz, 1H), 6.76 (t, *J* = 7.4 Hz, 1H), 6.65 (t*, J =* 7.6 Hz, 1H), 6.51 (d, *J* = 6.8 Hz, 1H), 6.40 (d, *J =* 7.7 Hz, 1H), 4.53 (t, *J =* 8.7 Hz, 2H), 3.63-3.55 (m, 1H), 3.32-3.15 (m, 6H), 2.92-2.79 (m, 5H), 2.69 (m, 1H), 2.57 (t, *J =* 7.6 Hz, 2H), 2.39 (s, 2H), 2.24 (s, 1H), 1.89 (d, *J =* 35.6 Hz, 5H). MS m/z (ESI): 390.2 [M+H]⁺.

### Example 2: (6bR,10aS)-8-(3-(Benzo[d][1,3]dioxol-4-yl)propyl)-3-methyl-2,3,6b,7,8,9,10,10a-octahydro-1H-pyrido[3',4':4,5]pyrrolo[1,2,3-de]quinoxaline

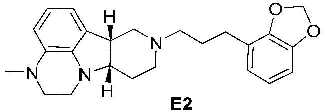

E2 was prepared with reference to the synthesis method of Example 1. ¹H NMR(400 MHz, CDCl₃) δ 6.77-6.71 (m, 1H), 6.70-6.62 (m, 3H), 6.51 (d, *J =* 6.8 Hz, 1H), 6.40 (d, *J =* 7.9 Hz, 1H), 5.91 (s, 2H), 3.63-3.56 (m, 1H), 3.33-3.12 (m, 4H), 2.92-2.78 (m, 5H), 2.62-2.59 (m, 3H), 2.45-2.24 (m, 3H), 1.94-1.86 (m, 5H). MS m/z (ESI): 392.2 [M+H]⁺.

### Example 3: (6bR,10aS)-8-(3-(2,3-Dihydrobenzofuran-4-yl)propyl)-3-methyl-2,3,6b,7,8,9,10,10a-octahydro-1H-pyrido[3',4':4,5]pyrrolo[1,2,3-de]quinoxaline

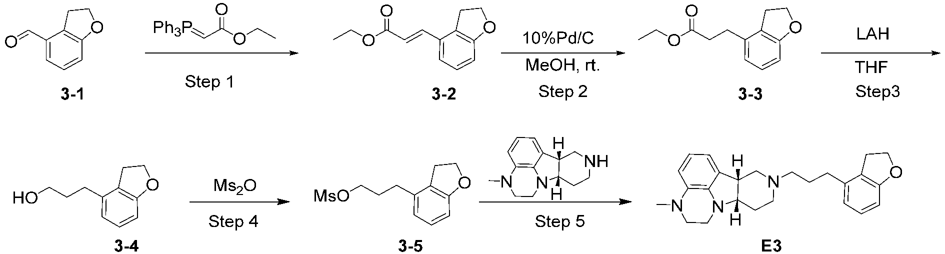

Step 1: The compounds 2,3-dihydrobenzofuran-4-carbaldehyde (200 mg, 1.35 mmol) and (ethoxycarbonylmethylene)triphenylphosphorane (940 mg, 2.7 mmol) were added to a 50 mL reaction flask, and THF (10 mL) was subsequently added. The mixture was then heated to 70 °C and stirred for 2 h. After LCMS analysis showed that the reaction had been complete, the reaction mixture was concentrated, and the resulting residue was purified by column chromatography (petroleum ether:ethyl acetate = 10:1) to give ethyl (*E*)-3-(2,3-dihydrobenzofuran-4-yl)acrylate (colorless liquid, 200 mg, yield: 68%). MS m/z (ESI): 219.3 [M+H]⁺.

Step 2: The compound ethyl (*E*)-3-(2,3-dihydrobenzofuran-4-yl)acrylate (200 mg, 0.92 mmol) was added to a 50 mL reaction flask, and methanol (10 mL) and wet palladium on carbon (10%, 97 mg) were subsequently added. The mixture was then stirred at room temperature for 1 h in a hydrogen atmosphere. After LCMS analysis showed that the reaction had been complete, the reaction mixture was filtered and concentrated to give ethyl 3-(2,3-dihydrobenzofuran-4-yl)propanoate (colorless liquid, 200 mg, yield: 99%). MS m/z (ESI): 221.4 [M+H]⁺.

Step 3: The compound ethyl 3-(2,3-dihydrobenzofuran-4-yl)propanoate (180 mg, 0.82 mmol) was added to a 50 mL three-neck reaction flask, and THF (5 mL) was subsequently added. Lithium aluminum hydride (1.5 mL, 1 mol/L) was slowly added dropwise at 0 °C, and the mixture was stirred at 0 °C for 20 min. The reaction solution was quenched with sodium sulfate decahydrate, filtered, and concentrated, and the residue was purified and separated by normal-phase column chromatography (petroleum ether:ethyl acetate = 3:1) to give 3-(2,3-dihydrobenzofuran-4-yl)propan-1-ol (colorless liquid, 100 mg, yield: 68.7%). MS m/z (ESI): 179.3 [M+H]⁺.

Step 4: The compound 3-(2,3-dihydrobenzofuran-4-yl)propan-1-ol (30 mg, 0.17 mmol) was added to a 25 mL reaction flask, and DCM (5 mL) was subsequently added. DIEA (65 mg, 0.5 mmol) and Ms₂O (44 mg, 0.5 mmol) were then added sequentially. After the addition, the mixture was stirred at room temperature for 1 h. The reaction mixture was extracted with DCM (2 × 20 mL), and the organic phase was washed with saturated brine (2 × 20 mL) and concentrated to give 3-(2,3-dihydrobenzofuran-4-yl)propyl mesylate (colorless liquid, 35 mg, yield: 81.4%). MS m/z (ESI): 257.3 [M+H]⁺.

Step 5: The compound 3-(2,3-dihydrobenzofuran-4-yl)propyl mesylate (35 mg, 0.14 mmol) and the starting material (6bR,10aS)-3-methyl-2,3,6b,7,8,9,10,10a-octahydro-1H-pyrido[3',4':4,5]pyrrolo[1,2,3-de]quinoxaline (31 mg, 0.14 mmol) were added to a 25 mL reaction flask, and DMSO (3 mL) was subsequently added. Then, DIPEA (52 mg, 0.4 mmol) was added. After the addition, the mixture was stirred at 60 °C for 16 h. The reaction mixture was concentrated, and the resulting residue was purified by reversed-phase column chromatography (acetonitrile/(water + 0.05% NH₄HCO₃)) to give (6bR,10aS)-8-(3-(2,3-dihydrobenzofuran-4-yl)propyl)-3-methyl-2,3,6b,7,8,9,10,10a-octahydro-1H-pyrido[3',4':4,5]pyrrolo[1,2,3-de]quinoxaline (gray solid, 20 mg, yield: 37.7%). ¹H NMR (500 MHz, DMSO-*d*₆) δ 7.00 (t, *J =* 7.6 Hz, 1H), 6.66 (d, *J =* 7.6 Hz, 1H), 6.56 (t*, J =* 11.2 Hz, 1H), 6.51 (t, *J =* 7.6 Hz, 1H), 6.42 (d, *J =* 7.2 Hz, 1H), 6.33 (d, *J =* 7.6 Hz, 1H), 4.50 (s, 2H), 3.46-3.38 (m, 1H), 3.27 (d, *J =* 11.4 Hz, 1H), 3.12 (t, *J =* 8.4 Hz, 3H), 3.01 (s, 1H), 2.78 (s, 4H), 2.67 (ddd, *J =* 16.4, 6.8, 2.2 Hz, 1H), 2.61-2.51 (m, 4H), 2.24 (s, 2H), 2.08 (s, 1H), 1.88-1.70 (m, 5H). MS m/z (ESI): 390.8 [M+H]⁺.

### Example 4: (6bR,10aS)-8-(3-(Chroman-8-yl)propyl)-3-methyl-2,3,6b,7,8,9,10,10a-octahydro-1H-pyrido[3',4':4,5]pyrrolo[1,2,3-de]quinoxaline

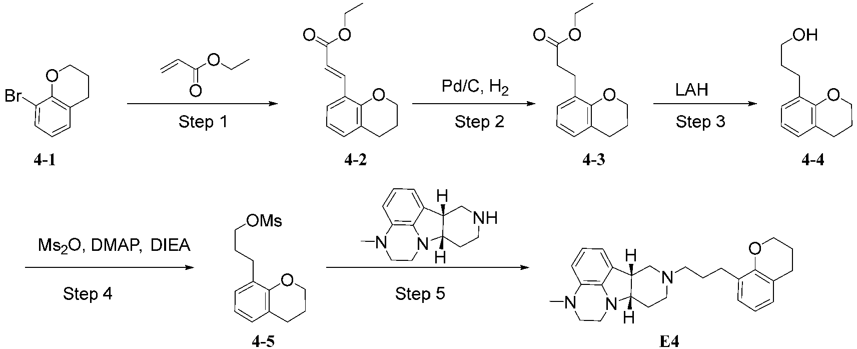

Step 1: The compounds 8-bromochromane (500 mg, 2.35 mmol), ethyl acrylate (260 mg, 2.58 mmol), Pd₂(dba)₃ (43 mg, 0.05 mmol), tri-tert-butylphosphine tetrafluoroborate (14 mg, 0.05 mmol), and *N*-methyldicyclohexylamine (1370 mg, 7.05 mmol) were added together to a 50 mL single-neck reaction flask, and anhydrous dioxane (10 mL) was subsequently added. After the addition, the mixture was purged 3 times with nitrogen, slowly heated to 100 °C, and stirred for 16 h. After TLC analysis showed that the reaction had been complete, water (20 mL) was added to the reaction solution, and extraction was performed with ethyl acetate (3 × 20 mL). The organic phases were combined, washed with saturated brine (1 × 20 mL), dried over anhydrous sodium sulfate, and concentrated to dryness by rotary evaporation under reduced pressure, and the crude product was purified and separated by normal-phase column chromatography (Biotage-12 g, eluent gradient: 10% EA/PE) to give ethyl (E)-3-(chroman-8-yl)acrylate (pale yellow oil, 150 mg, yield: 28%).

Step 2: The compound ethyl (E)-3-(chroman-8-yl)acrylate (150 mg, 0.65 mmol) was added to a 25 mL single-neck reaction flask, and subsequently, ethyl acetate (15 mL) and 10% Pd/C (wet basis, 15 mg) were separately added. After the addition, the mixture was purged 3 times with hydrogen and stirred at room temperature for 16 h. After LCMS analysis showed that the reaction had been complete, the reaction solution was filtered under vacuum, and the filtrate was concentrated under reduced pressure to give ethyl 3-(chroman-8-yl)propanoate (crude, 150 mg, yield: 99%). The crude product was directly used in the next step without further purification. MS m/z (ESI): 235.5 [M+H]⁺.

Step 3: The compound ethyl 3-(chroman-8-yl)propanoate (150 mg, 0.64 mmol) was added to a 25 mL three-neck reaction flask, and anhydrous tetrahydrofuran (10 mL) was subsequently added. A 1 M solution of lithium aluminum hydride in THF (48 mg, 1.28 mmol, 1 mol/L) was added portionwise under nitrogen and ice-water bath conditions, and the mixture was stirred at 0 °C for 0.5 h. After TLC analysis showed that the reaction had been complete, Na₂SO₄.10H₂O was slowly added to the reaction solution to quench the reaction, and the mixture was filtered. The filter cake was washed with 10% MeOH/DCM (30 mL), and the filtrate was concentrated under reduced pressure. The crude product was purified and separated by normal-phase column chromatography (eluent gradient: 30% EA/PE) to give 3-(chroman-8-yl)propan-1-ol (colorless oil, 110 mg, yield: 89%).

Step 4: The compounds 3-(chroman-8-yl)propan-1-ol (15 mg, 0.08 mmol), DMAP (2 mg, 0.01 mmol), DIEA (30 mg, 0.24 mmol), and anhydrous dichloromethane (5 mL) were added together to a 25 mL three-neck reaction flask, and methanesulfonic anhydride (27 mg, 0.16 mmol) was slowly added under nitrogen and ice-water bath conditions. After the addition, the mixture was stirred at room temperature for 2 h. After TLC analysis showed that the reaction had been complete, water (10 mL) was added to the reaction solution, and extraction was performed with dichloromethane (2 × 10 mL). The organic phases were combined, washed with saturated brine (1 × 10 mL), dried over anhydrous sodium sulfate, and concentrated to dryness by rotary evaporation under reduced pressure to give 3-(chroman-8-yl)propyl mesylate (colorless oil, crude, 20 mg). The crude product was directly used in the next step without further purification.

Step 5: The compounds 3-(chroman-8-yl)propyl mesylate (20 mg, 0.07 mmol) and (6bR,10aS)-3-methyl-2,3,6b,7,8,9,10,10a-octahydro-1H-pyrido[3',4':4,5]pyrrolo[1,2,3-de]quinoxaline (20 mg, 0.08 mmol) were added to a 10 mL reaction flask, and subsequently, anhydrous DMSO (2 mL) and DIPEA (30 mg, 0.21 mmol) were separately added. After the addition, the mixture was slowly heated to 60 °C and stirred for 16 h. After the reaction was complete, the reaction mixture was filtered, and the filtrate was directly separated by reversed-phase preparative chromatography (C18 column, eluent gradient: acetonitrile/(water + 0.05% NH₄HCO₃)) to give ((6bR,10aS)-8-(3-(chroman-8-yl)propyl)-3-methyl-2,3,6b,7,8,9,10,10a-octahydro-1H-pyrido[3',4':4,5]pyrrolo[1,2,3-de]quinoxaline (off-white solid, 4.5 mg, yield: 15%). ¹H NMR (500 MHz, CDCl₃) δ 6.93 (d, *J* = 7.3 Hz, 1H), 6.88 (d, *J* = 7.0 Hz, 1H), 6.74 (t, *J =* 7.4 Hz, 1H), 6.65 (t, *J* = 7.6 Hz, 1H), 6.51 (d, *J* = 7.1 Hz, 1H), 6.40 (d, *J* = 7.8 Hz, 1H), 4.21-4.14 (m, 2H), 3.64-3.56 (m, 1H), 3.33-3.16 (m, 4H), 2.95-2.89 (m, 1H), 2.86 (s, 3H), 2.85-2.76 (m, 3H), 2.75-2.69 (m, 1H), 2.60-2.51 (m, 2H), 2.48-2.34 (m, 2H), 2.32-2.22 (m, 1H), 2.02-1.90 (m, 5H), 1.84-1.78 (m, 2H). MS m/z (ESI): 404.7 [M+H]⁺.

### Example 5: (6bR,10aS)-8-(3-(Benzofuran-7-yl)propyl)-3-methyl-2,3,6b,7,8,9,10,1Oa-octahydro-1H-pyrido[3',4':4,5]pyrrolo[1,2,3-de]quinoxaline

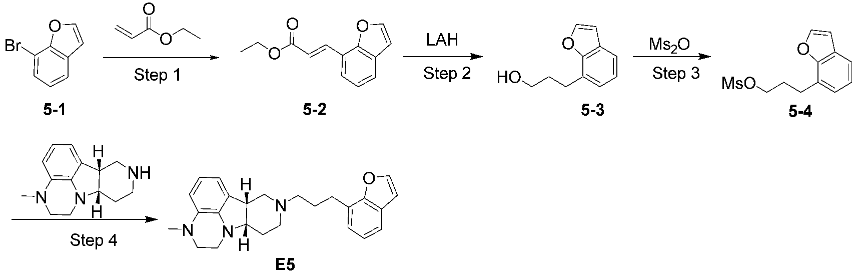

Step 1: The compound 7-bromobenzofuran (400 mg, 2.0 mmol) was dissolved in DMF (5.0 mL), and triethylamine (1.40 g, 14.0 mmol), ethyl acrylate (220 mg, 2.2 mmol), tri(o-tolyl)phosphine (122 mg, 0.4 mmol), and palladium acetate (44 mg, 0.2 mmol) were sequentially added in a nitrogen atmosphere. The mixture was stirred at 100 °C for 4 h. After TLC analysis showed that the reaction had been complete, the reaction mixture was quenched with water and extracted with ethyl acetate. The organic phase was washed with saturated brine, dried over anhydrous sodium sulfate, and purified by normal-phase chromatography to give the compound ethyl (E)-3-(benzofuran-7-yl)acrylate (yellow oil, 220 mg, yield: 50%). MS m/z (ESI): 217.3 [M+H]⁺.

Step 2: Ethyl (E)-3-(benzofuran-7-yl)acrylate (45 mg, 0.2 mmol) was dissolved in THF (2.0 mL), and the solution was cooled to 0 °C. A 1 M solution of lithium aluminum hydride in tetrahydrofuran (0.2 mL, 0.2 mmol) was added, and the mixture was stirred at room temperature for 1 h. Sodium sulfate decahydrate was added, and the mixture was stirred for 30 min, filtered, and concentrated. The resulting residue was purified by normal-phase chromatography to give the compound 3-(benzofuran-7-yl)propan-1-ol (yellow oil, 25 mg, yield: 70%). MS m/z (ESI): 177.2 [M+H]⁺.

Step 3: The compound 3-(benzofuran-7-yl)propan-1-ol (25 mg, 0.14 mmol) was dissolved in DCM (2.0 mL), and DIEA (90 mg, 0.7 mmol) was added. The mixture was cooled to 0 °C. After methanesulfonic anhydride (45 mg, 0.26 mmol) was added, the mixture was warmed to room temperature, stirred for 3 h, quenched with water, and extracted with DCM, and the extract was dried over sodium sulfate and filtered to give the compound 3-(4-fluoro-2-methoxyphenyl)propyl mesylate (yellow oil, 23 mg), which was directly used in the next step.

Step 4: At room temperature, (6bR,10aS)-3-methyl-2,3,6b,7,8,9,10,10a-octahydro-1H-pyrido[3',4':4,5]pyrrolo[1,2,3-de]quinoxaline (25 mg, 0.10 mmol) was dissolved in DMSO (2.5 mL), and DIEA (0.5 mL, 3.0 mmol) and 3-(4-fluoro-2-methoxyphenyl)propyl mesylate (23 mg, 0.1 mmol) were sequentially added. The mixture was heated to 65 °C and stirred for 18 h, cooled to room temperature, filtered, and then purified and separated by prep-HPLC (eluent (v/v): acetonitrile/(water + 0.05% NH₄HCO₃) = 30%-70%) to give the compound (6bR,10aS)-8-(3-(benzofuran-7-yl)propyl)-3-methyl-2,3,6b,7,8,9,10,10a-octahydro-1H-pyrido[3',4':4,5]pyrrolo[1,2,3-de]quinoxaline (colorless oil, 2.0 mg, yield: 5%). ¹H NMR (500 MHz, DMSO-*d*₆) δ 7.98 (d*, J =* 5.0 Hz, 1H), 7.48 (dd, *J =* 10.0, 5.0 Hz, 1H), 7.19-7.12 (m, 2H), 6.95 (d, *J* = 5.0 Hz, 1H), 6.67-6.52 (m, 1H), 6.43 (d, *J =* 5.0 Hz, 1H), 6.34 (d*, J =* 10.0 Hz, 1H), 3.36-3.26 (m, 7H), 3.12-3.04 (m, 2H), 2.89-2.79 (m, 3H), 2.71 (s, 3H), 2.69-2.67 (m, 2H), 1.98-1.79 (m, 4H). MS m/z (ESI): 388.8 [M+H]⁺.

### Example 6: (6bR,10aS)-8-(3-(Benzo[b]thiophen-7-yl)propyl)-3-methyl-2,3,6b,7,8,9,10,10a-octahydro-1H-pyrido[3',4':4,5]pyrrolo[1,2,3-de]quinoxaline

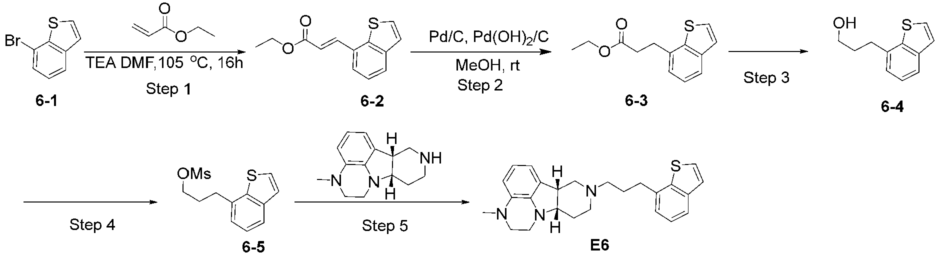

Step 1: 7-Bromobenzo[b]thiophene (500 mg, 2.35 mmol), ethyl acrylate (580 mg, 5.79 mmol), Pd₂(dab)₃ (110 mg, 0.12 mmol), and tri(o-tolyl)phosphine (220 mg, 0.72 mmol) were added to a three-neck flask containing 10 mL of DMF, and the mixture was purged three times with nitrogen and left to react at 105 °C for 16 h. After TLC analysis showed that the reaction had been complete, water and EA were added, and the aqueous phase was isolated and extracted twice with EA. The organic phases were combined, washed three times with saturated sodium chloride, dried over anhydrous sodium sulfate, filtered, and concentrated, and the resulting residue was purified by normal-phase column chromatography (PE/EA = 100%-10/1) to give the compound ethyl (E)-3-(benzo[b]thiophen-7-yl)acrylate (yellow solid, 487 mg, yield: 87%).

Step 2: Ethyl (E)-3-(benzo[b]thiophen-7-yl)acrylate (470 mg, 2.02 mmol), 50 mg of Pd/C, and 50 mg of Pd(OH)₂/C were added to a three-neck flask containing 30 mL of MeOH. The mixture was purged three times with H₂ and left to react at room temperature for 16 h, and TLC and LCMS analysis showed that the reaction had been complete. The reaction mixture was filtered and concentrated, and the resulting residue was purified by normal-phase column chromatography (PE/EA = 100%-5/1) to give the compound ethyl 3-(benzo[b]thiophen-7-yl)propanoate (410 mg, colorless oil, yield: 87%).

Step 3: The compound ethyl 3-(benzo[b]thiophen-7-yl)propanoate (110 mg, 0.469 mmol) was added to a three-neck flask containing 5 mL of THF. The mixture was purged three times with nitrogen and cooled to 0 °C, and 0.9 mL of a 1 M solution of LAH in THF was injected. Then, the mixture was left to react for 1 h, and TLC analysis showed that the reaction had been complete. Sodium sulfate decahydrate was added to quench the reaction, and EA and Na₂SO₄ were then added. The mixture was stirred at room temperature for half an hour, filtered, and concentrated, and the residue was purified by normal-phase column chromatography (PE/EA = 100%-10/1) to give the compound 3-(benzo[b]thiophen-7-yl)propan-1-ol (90 mg, colorless oil, yield: 90%).

Step 4: The compound 3-(benzo[b]thiophen-7-yl)propyl mesylate was prepared with reference to the synthesis method in step 4 of Example 3.

Step 5: The compound (6bR,10aS)-8-(3-(benzo[b]thiophen-7-yl)propyl)-3-methyl-2,3,6b,7,8,9,10,10a-octahydro-1H-pyrido[3',4':4,5]pyrrolo[1,2,3-de]quinoxaline was prepared with reference to the synthesis method in step 5 of Example 3. ¹H NMR (500 MHz, CDCl₃) δ7.68 (d, *J* = 8.0 Hz 1H), 7.42(d, *J* = 5.5Hz 1H), 7.35(d, *J* = 5.5Hz, 1H), 7.31(t, *J* = 8.0 Hz 1H), 7.16 (d, *J* = 7.0 Hz 1H), 6.64(t, *J* = 7.5 Hz 1H), 6.51(d, *J =* 5.5 Hz 1H), 6.40(d, *J =* 7.5 Hz 1H), 3.62-3.56 (m, 1H), 3.31-3.20 (m, 4H), 2.92(t, *J* = 8.0 Hz 1H)2.86 (s, 3H), 2.85-2.72 (m, 2H), 2.58-2.30 (m, 3H), 2.07-1.94 (m, 5H). MS m/z (ESI): 405.4 [M+H]⁺.

Example 7: 7-(3-((6bR,10aS)-3-Methyl-2,3,6b,9,10,10a-hexahydro-1H-pyrido[3',4':4,5]pyrrolo[1,2,3-de]quinoxalin-8(7H)-yl)propyl)benzo[d]oxazole

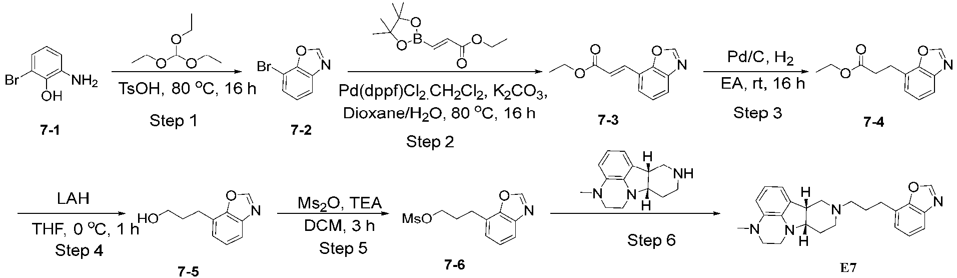

Step 1: The compounds 2-amino-6-bromophenol (1000 mg, 5.32 mmol), anhydrous p-toluenesulfonic acid (100 mg, 0.53 mmol), and triethyl orthoformate (10 mL) were added together to a 25 mL single-neck reaction flask, and the mixture was slowly heated to 80 °C and stirred for 16 h in a nitrogen atmosphere. After TLC analysis showed that the reaction had been complete, a saturated NaHCO₃ solution was added to the reaction solution to make the system alkaline, and extraction was subsequently performed with ethyl acetate (3 × 20 mL). The organic phases were combined, washed with saturated brine (1 × 20 mL), dried over anhydrous sodium sulfate, and concentrated to dryness by rotary evaporation under reduced pressure, and the crude product was purified and separated by normal-phase column chromatography (eluent gradient: 3% EA/PE) to give the compound 7-bromobenzo[d]oxazole (pale yellow solid, 900 mg, yield: 85%).

Step 2: The compounds 7-bromobenzo[d]oxazole (250 mg, 1.26 mmol), ethyl (E)-3-(4,4,5,5-tetramethyl-1,3,2-dioxaborolan-2-yl)acrylate (340 mg, 1.51 mmol), Pd(dppf)Cl₂.CH₂Cl₂ complex (20 mg, 0.03 mmol), and sodium carbonate (265 mg, 2.52 mmol) were added together to a 25 mL single-neck reaction flask, and subsequently, dioxane (5 mL) and water (1 mL) were separately added. After the addition, the mixture was purged 3 times with nitrogen, slowly heated to 80 °C, and stirred for 16 h. After LCMS analysis showed that the reaction had been complete, the reaction solution was filtered. Subsequently, water (20 mL) was added to the reaction solution, and extraction was performed with ethyl acetate (3 × 10 mL). The organic phases were combined, washed with saturated brine (1 × 20 mL), dried over anhydrous sodium sulfate, and concentrated to dryness by rotary evaporation under reduced pressure, and the crude product was purified and separated by normal-phase column chromatography (eluent gradient: 10% EA/PE) to give the compound ethyl (E)-3-(benzo[d]oxazol-7-yl)acrylate (pale yellow oil, 150 mg, yield: 55%). MS m/z (ESI): 218.5 [M+H]⁺.

Step 3: The compound ethyl (E)-3-(benzo[d]oxazol-7-yl)acrylate (100 mg, 0.46 mmol) was added to a 25 mL single-neck reaction flask, and subsequently, ethyl acetate (10 mL) and 10% Pd/C (wet basis, 10 mg) were separately added. After the addition, the mixture was purged 3 times with hydrogen and stirred at room temperature for 16 h. After LCMS analysis showed that the reaction had been complete, the reaction solution was filtered under vacuum, and the filtrate was concentrated under reduced pressure to give the compound ethyl 3-(benzo[d]oxazol-7-yl)propanoate (crude, 100 mg, yield: 99%). The crude product was directly used in the next step without further purification. MS m/z (ESI): 220.4 [M+H]⁺.

Step 4: 3-(Benzo[d]oxazol-7-yl)propan-1-ol was prepared with reference to the synthesis method in step 3 of Example 3. The compound ethyl 3-(benzo[d]oxazol-7-yl)propanoate (50 mg, 0.23 mmol) was added to a 25 mL three-neck reaction flask, and anhydrous tetrahydrofuran (3 mL) was subsequently added. Lithium aluminum hydride (17 mg, 0.46 mmol) was added portionwise under nitrogen and ice-water bath conditions, and the mixture was stirred for 1 h in the ice-water bath. After LCMS analysis showed that the reaction had been complete, Na₂SO₄.10H₂O was slowly added to the reaction solution to quench the reaction, and the mixture was filtered. The filter cake was washed with 10% MeOH/DCM (10 mL), and the filtrate was concentrated under reduced pressure. The crude product was purified and separated by normal-phase column chromatography (eluent gradient: 40% EA/PE) to give the compound 3-(benzo[d]oxazol-7-yl)propan-1-ol (colorless oil, 25 mg, yield: 62%). MS m/z (ESI): 178.4 [M+H]⁺.

Step 5: The compounds 3-(benzo[d]oxazol-7-yl)propan-1-ol (10 mg, 0.06 mmol), triethylamine (12 mg, 0.12 mmol), and anhydrous dichloromethane (3 mL) were added together to a 10 mL single-neck reaction flask, and methanesulfonic anhydride (12 mg, 0.07 mmol) was added under ice-water bath conditions. After the addition, the mixture was stirred at room temperature for 3 h. After LCMS analysis showed that the reaction had been complete, a saturated ammonium chloride solution (5 mL) was added to the reaction solution, and extraction was performed with dichloromethane (2 × 10 mL). The organic phases were combined, washed with saturated brine (1 × 10 mL), dried over anhydrous sodium sulfate, and concentrated to dryness by rotary evaporation under reduced pressure, and the crude product was purified by normal-phase chromatography (eluent gradient: 30% EA/PE) to give the compound 3-(benzo[d]oxazol-7-yl)propyl mesylate (pale yellow oil, 8 mg, yield: 60%).

Step 6: The compounds 3-(benzo[d]oxazol-7-yl)propyl mesylate (8 mg, 0.03 mmol, crude) and (6bR,10aS)-3-methyl-2,3,6b,7,8,9,10,10a-octahydro-1H-pyrido[3',4':4,5]pyrrolo[1,2,3-de]quinoxaline (8 mg, 0.04 mmol) were added to a 10 mL reaction flask, and DIEA (11 mg, 0.09 mmol) and anhydrous DMSO (1 mL) were subsequently added. After the addition, the mixture was heated to 60 °C and stirred for 6 h. After LCMS analysis showed that the reaction had been complete, the reaction solution was filtered, and the filtrate was directly separated by reversed-phase preparative chromatography (C18 column, eluent gradient: acetonitrile/(water + 0.05% NH₄HCO₃)) to give the compound 7-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)benzo[d]oxazole (pale yellow solid, 2.0 mg, yield: 15%). ¹H NMR (500 MHz, CDCl₃) 6 8.08 (s, 1H), 7.63 (d, *J =* 8.0 Hz, 1H), 7.29 (d, *J* = 7.6 Hz, 1H), 7.19 (d, *J* = 7.4 Hz, 1H), 6.65 (t, *J =* 7.6 Hz, 1H), 6.51 (d, *J* = 7.2 Hz, 1H), 6.40 (d, *J* = 7.8 Hz, 1H), 3.58 (dd, *J* = 14.7, 6.5 Hz, 1H), 3.33-3.14 (m, 4H), 2.93 (t, *J* = 7.6 Hz, 3H), 2.87-2.78 (m, 4H), 2.78-2.64 (m, 1H), 2.52-2.26 (m, 3H), 2.06-1.96 (m, 5H). MS m/z (ESI): 389.7 [M+H]⁺.

### Example 8: (6bR,10aS)-3-Methyl-8-(3-(quinolin-8-yl)propyl)-2,3,6b,7,8,9,10,10a-octahydro-1H-pyrido[3',4':4,5]pyrrolo[1,2,3-de]quinoxaline

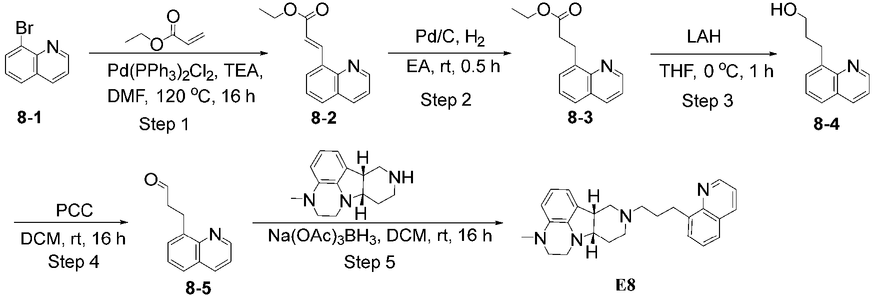

Step 1: The compounds 8-bromoquinoline (500 mg, 2.40 mmol), ethyl acrylate (480 mg, 4.80 mmol), Pd(PPh₃)₂Cl₂ (17 mg, 0.02 mmol), and triethylamine (730 mg, 7.20 mmol) were added together to a 25 mL single-neck reaction flask, and anhydrous DMF (8 mL) was subsequently added. After the addition, the mixture was purged 3 times with nitrogen, slowly heated to 120 °C, and stirred for 16 h. After LCMS analysis showed that the reaction had been complete, water (20 mL) was added to the reaction solution, and extraction was performed with ethyl acetate (3 × 20 mL). The organic phases were combined, washed with saturated brine (1 × 20 mL), dried over anhydrous sodium sulfate, and concentrated to dryness by rotary evaporation under reduced pressure, and the crude product was purified and separated by normal-phase column chromatography (eluent gradient: 5% EA/PE) to give the compound ethyl (E)-3-(quinolin-8-yl)acrylate (reddish brown oil, 350 mg, yield: 64%). MS m/z (ESI): 228.4 [M+H]⁺.

Step 2: The compound ethyl (E)-3-(quinolin-8-yl)acrylate (100 mg, 0.44 mmol) was added to a 25 mL single-neck reaction flask, and subsequently, ethyl acetate (10 mL) and 10% Pd/C (wet basis, 10 mg) were separately added. After the addition, the mixture was purged 3 times with hydrogen and stirred at room temperature for 0.5 h. After LCMS analysis showed that the reaction had been complete, the reaction solution was filtered under vacuum, and the filtrate was concentrated under reduced pressure to give the compound ethyl 3-(quinolin-8-yl)propanoate (crude, 100 mg, yield: 99%). The crude product was directly used in the next step without further purification. MS m/z (ESI): 230.4 [M+H]⁺.

Step 3: The compound ethyl 3-(quinolin-8-yl)propanoate (100 mg, 0.44 mmol) was added to a 25 mL three-neck reaction flask, and anhydrous tetrahydrofuran (5 mL) was subsequently added. Lithium aluminum hydride (33 mg, 0.88 mmol, 1 mol/L) was added portionwise under nitrogen and ice-water bath conditions, and the mixture was stirred for 0.5 h in the ice-water bath. After LCMS analysis showed that the reaction had been complete, Na₂SO₄.10H₂O was slowly added to the reaction solution to quench the reaction, and the mixture was filtered. The filter cake was washed with 10% MeOH/DCM (10 mL), and the filtrate was concentrated under reduced pressure. The crude product was purified and separated by normal-phase column chromatography (eluent gradient: 30% EA/PE) to give the compound 3-(quinolin-8-yl)propan-1-ol (colorless oil, 60 mg, yield: 74%). MS m/z (ESI): 188.4 [M+H]⁺.

Step 4: The compounds 3-(quinolin-8-yl)propan-1-ol (30 mg, 0.16 mmol), pyridinium chlorochromate (65 mg, 0.32 mmol), and anhydrous dichloromethane (3 mL) were added together to a 10 mL single-neck reaction flask, and the mixture was stirred at room temperature for 2 h. After LCMS analysis showed that the reaction had been complete, a saturated aqueous sodium bicarbonate solution was added to the reaction solution to make the system alkaline, and extraction was performed with dichloromethane (2 × 10 mL). The organic phases were combined, washed with saturated brine (1 × 10 mL), dried over anhydrous sodium sulfate, and concentrated to dryness by rotary evaporation under reduced pressure to give the compound 3-(quinolin-8-yl)propanal (blackish brown oil, crude, 25 mg, yield: 83%). The crude product was directly used in the next step without further purification. MS m/z (ESI): 186.4 [M+H]⁺.

Step 5: The compounds 3-(quinolin-8-yl)propanal (25 mg, 0.13 mmol) and (6bR,10aS)-3-methyl-2,3,6b,7,8,9,10,10a-octahydro-1H-pyrido[3',4':4,5]pyrrolo[1,2,3-de]quinoxaline (32 mg, 0.14 mmol) were added to a 10 mL reaction flask, and the mixture was stirred at room temperature for 0.5 h. Subsequently, sodium triacetoxyborohydride (55 mg, 0.26 mmol) was added to the reaction solution. After the addition, the mixture was stirred at room temperature for 16 h. After the reaction was complete, the reaction solvent was removed under reduced pressure, and subsequently, the crude product was directly separated by reversed-phase preparative chromatography (C18 column, eluent gradient: acetonitrile/(water + 0.05% NH₄HCO₃)) to give the compound (6bR,10aS)-3-methyl-8-(3-(quinolin-8-yl)propyl)-2,3,6b,7,8,9,10,10a-octahydro-1H-pyrido[3',4':4,5]pyrrolo[1,2,3-de]quinoxaline (off-white solid, 6.0 mg, yield: 18%). ¹H NMR (500 MHz, MeOD) δ 8.86 (dd, *J =* 4.2, 1.8 Hz, 1H), 8.29 (dd, *J* = 8.3, 1.7 Hz, 1H), 7.77 (dd, *J =* 8.2, 1.3 Hz, 1H), 7.62 (d, *J =* 7.0 Hz, 1H), 7.54-7.46 (m, 2H), 6.64-6.55 (m, 1H), 6.47 (d, *J* = 7.2 Hz, 1H), 6.40 (d, *J =* 7.9 Hz, 1H), 3.49 (ddd, *J* = 11.4, 10.1, 2.9 Hz, 1H), 3.36-3.32 (m, 1H), 3.29-3.25 (m, 1H), 3.25-3.21 (m, 2H), 3.14-3.05 (m, 2H), 2.92-2.87 (m, 1H), 2.82 (s, 3H), 2.78-2.70 (m, 2H), 2.54-2.44 (m, 2H), 2.31 (td, *J =* 12.1, 3.0 Hz, 1H), 2.05-1.86 (m, 5H). MS m/z (ESI): 399.9 [M+H]⁺.

### Example 9: (6bR,10aS)-8-(3-(Isoquinolin-8-yl)propyl)-3-methyl-2,3,6b,7,8,9,10,10a-octahydro-1H-pyrido[3',4':4,5]pyrrolo[1,2,3-de]quinoxaline

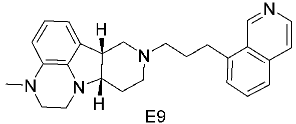

E9 was prepared with reference to the synthesis method of Example 3. ¹H NMR (500 MHz, DMSO) δ 9.54 (s, 1H), 8.51 (d, *J* = 5.6 Hz, 1H), 7.87-7.76 (m, 2H), 7.69 (dd, *J* = 8.2, 7.0 Hz, 1H), 7.51 (dd, *J* = 7.0, 1.1 Hz, 1H), 6.51 (t, *J* = 7.6 Hz, 1H), 6.42 (d, *J* = 7.2 Hz, 1H), 6.33 (d, *J* = 7.9 Hz, 1H), 3.45-3.40 (m, 1H), 3.27 (dd, *J =* 11.5, 2.9 Hz, 3H), 3.19 (dd, *J* = 8.6, 6.6 Hz, 2H), 3.13 (s, 1H), 3.05 (s, 1H), 2.78 (s, 3H), 2.71-2.55 (m, 2H), 2.45-2.22 (m, 2H), 2.09 (d, *J* = 21.6 Hz, 1H), 1.84 (d, *J =* 29.7 Hz, 5H). MS m/z (ESI): 399.9 [M+H]⁺.

### Example 10: (6bR,10aS)-3-Methyl-8-(3-(1,2,3,4-tetrahydroquinolin-8-yl)propyl)-2,3,6b,7,8,9,10,10a-octahydro-1H-pyrido[3',4':4,5]pyrrolo[1,2,3-de]quinoxaline

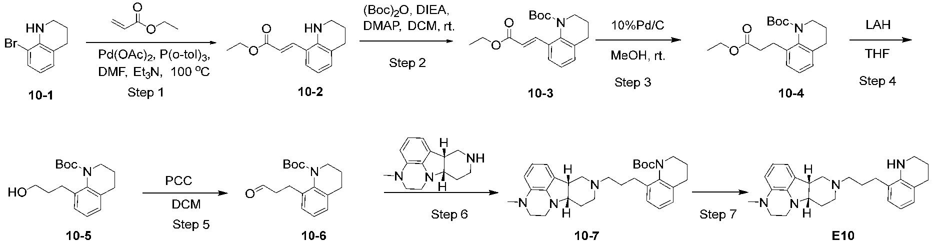

Step 1: The compound 5-bromo-3,4-dihydro-2H-benzo[b][1,4]oxazine (420 mg, 2.0 mmol) was dissolved in DMF (10.0 mL), and triethylamine (1.6 g, 16.0 mmol), ethyl acrylate (300 mg, 1.5 mmol), tri(o-tolyl)phosphine (120 mg, 0.4 mmol), and palladium acetate (44 mg, 0.2 mmol) were sequentially added in a nitrogen atmosphere. The mixture was stirred at 100 °C for 3 h. After TLC analysis showed that the reaction had been complete, the reaction mixture was quenched with water and extracted with ethyl acetate. The organic phase was washed with saturated brine, dried over anhydrous sodium sulfate, and purified by normal-phase chromatography to give the compound ethyl (E)-3-(3,4-dihydro-2H-benzo[b][1,4]oxazin-5-yl)acrylate (yellow oil, 230 mg, yield: 49%). MS m/z (ESI): 234.3 [M+H]⁺.

Step 2: The compound ethyl (E)-3-(3,4-dihydro-2H-benzo[b][1,4]oxazin-5-yl)acrylate (190 mg, 0.81 mmol) was dissolved in DCM (20.0 mL). After TEA (800 mg, 8.0 mmol) was added, (Boc)₂O (700 mg, 3.2 mmol) was added. The mixture was stirred at 20 °C for 16 h and concentrated under reduced pressure, and the residue was purified by normal-phase chromatography to give the compound tert-butyl (E)-5-(3-ethoxy-3-oxoprop-1-en-1-yl)-2,3-dihydro-4H-benzo[b][1,4]oxazine-4-carboxylate (yellow oil, 180 mg, yield: 67%). MS m/z (ESI): 334.4 [M+H]⁺.

Step 3: The compound tert-butyl (E)-5-(3-ethoxy-3-oxoprop-1-en-1-yl)-2,3-dihydro-4H-benzo[b][1,4]oxazine-4-carboxylate was dissolved in MeOH (10.0 mL), and 10% palladium on carbon (90 mg) was added. The mixture was stirred at room temperature for 18 h, filtered, and concentrated to give the compound tert-butyl 5-(3-ethoxy-3-oxopropyl)-2,3-dihydro-4H-benzo[b][1,4]oxazine-4-carboxylate (yellow oil, 160 mg, yield: 88%). MS m/z (ESI): 336.4 [M+H]⁺.

Step 4: The compound tert-butyl 5-(3-ethoxy-3-oxopropyl)-2,3-dihydro-4H-benzo[b][1,4]oxazine-4-carboxylate (145 mg, 0.43 mmol) was dissolved in THF (2.0 mL), and the solution was cooled to 0 °C. After a lithium aluminum hydride solution (0.45 mL, 0.15 mmol) was added, the mixture was heated to 20 °C, stirred for 1 h, dried over sodium sulfate decahydrate, filtered, and then purified by normal-phase chromatography to give the compound tert-butyl 5-(3-hydroxypropyl)-2,3-dihydro-4H-benzo[b][1,4]oxazine-4-carboxylate (yellow oil, 85 mg, yield: 67%). MS m/z (ESI): 294.3 [M+H]⁺.

Step 5: The compound tert-butyl 5-(3-hydroxypropyl)-2,3-dihydro-4H-benzo[b][1,4]oxazine-4-carboxylate (85 mg, 0.29 mmol) was dissolved in DCM (5.0 mL), and PCC (128 mg, 0.59 mmol) was added. The mixture was stirred at 20 °C for 3 h. The reaction mixture was quenched with saturated sodium bicarbonate and extracted with DCM, and the extract was dried over sodium sulfate, filtered, and concentrated to give the compound tert-butyl 5-(3-oxopropyl)-2,3-dihydro-4H-benzo[b][1,4]oxazine-4-carboxylate (colorless oil, 66 mg, yield: 80%), which was directly used in the next step. MS m/z (ESI): 292.4 [M+H]⁺.

Step 6: (6bR,10aS)-3-Methyl-2,3,6b,7,8,9,10,10a-octahydro-1H-pyrido[3',4':4,5]pyrrolo[1,2,3-de]quinoxaline (62 mg, 0.27 mmol) and the compound tert-butyl 5-(3-oxopropyl)-2,3-dihydro-4H-benzo[b][1,4]oxazine-4-carboxylate (66 mg, 0.23 mmol) were dissolved in DCM (3.0 mL), and 5 mg of triethylamine and 5 mg of acetic acid were added. After 10 min of stirring, sodium triacetoxyborohydride (230 mg, 4.0 mmol) was added, and the mixture was stirred at 20 °C for 18 h, quenched with water, and extracted with ethyl acetate. The organic phase was washed with saturated brine, dried over anhydrous sodium sulfate, and purified by normal-phase chromatography to give an intermediate. The intermediate was dissolved in DCM (3.0 mL), and TFA (0.3 mL, 1.1 mmol) was added. The mixture was stirred at room temperature for 0.5 h and concentrated, and the resulting residue was purified and separated by prep-HPLC (eluent (v/v): acetonitrile/(water + 0.05% NH₄HCO₃) = 30%-70%) to give the compound (6bR,10aS)-3-methyl-8-(3-(1,2,3,4-tetrahydroquinolin-8-yl)propyl)-2,3,6b,7,8,9,10,10a-octahydro-1H-pyrido[3',4':4,5]pyrrolo[1,2,3-de]quinoxaline (off-white solid, 8.0 mg, yield: 9%). ¹H NMR (500 MHz, CD₃OD) δ 6.61-6.59 (m, 2H), 6.57-6.49 (m, 3H), 7.00 (d, *J =* 5.0 Hz, 1H), 4.15 (t, *J =* 5.0 Hz, 2H), 3.54-3.48 (m, 1H), 3.37 (t, *J =* 5.0 Hz, 3H), 3.23-3.16 (m, 2H), 3.06-3.00 (m, 1H), 2.93-2.88 (m, 1H), 2.84 (s, 3H), 2.79-2.72 (m, 1H), 2.57-2.47 (m, 5H), 2.17-2.07 (m, 2H), 2.00-1.84 (m, 3H). MS m/z (ESI): 405.8 [M+H]⁺.

### Example 11: (6bR,10aS)-8-(3-(1H-Indol-7-yl)propyl)-3-methyl-2,3,6b,7,8,9,10,10a-octahydro-1H-pyrido[3',4':4,5]pyrrolo[1,2,3-de]quinoxaline

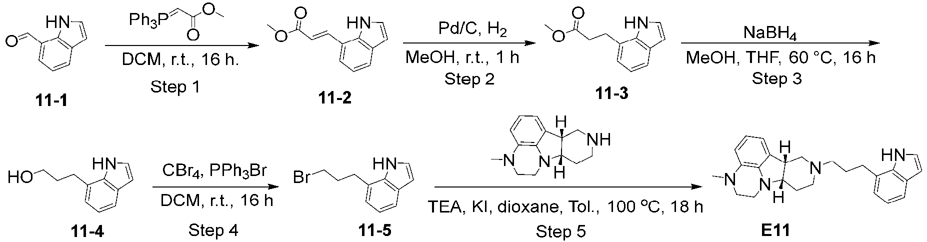

Step 1: 1H-Indole-7-carbaldehyde (500.0 mg, 3.44 mmol) and methyl (triphenylphosphoranylidene)acetate (1.4 g, 4.13 mmol) were dissolved in DCM (3 mL) at room temperature, and the reaction mixture was stirred at room temperature for 16 h. 80 mL of water was added to the reaction solution, and extraction was performed with DCM (70 mL × 3). The organic phases were combined and concentrated *in vacuo* to give a crude product, and the crude product was purified by silica gel column chromatography (PE/EA = 100/0-90/10) to give methyl (E)-3-(1H-indol-7-yl)acrylate (580 mg, yield: 83.7%) as a yellow solid. MS m/z (ESI): 202.1 [M+H]⁺.

Step 2: Methyl (E)-3-(1H-indol-7-yl)acrylate (480.0 mg, 2.39 mmol) was added to methanol (5 mL), and 10% palladium on carbon (101.5 mg) was added. The reaction solution was stirred at room temperature for 1 h in a hydrogen atmosphere. After the reaction was complete, the reaction solution was filtered through diatomaceous earth and washed with methanol to give methyl 3-(1H-indol-7-yl)propanoate (450 mg, yield: 92.8%) as a gray solid. MS m/z (ESI): 204.1 [M+H]⁺.

Step 3: At room temperature, methyl 3-(1H-indol-7-yl)propanoate (450.0 mg, 2.21 mmol) was added to methanol (5 mL) and tetrahydrofuran (5 mL), and sodium borohydride (418.8 mg, 11.07 mmol) was added. The reaction solution was stirred at room temperature for 16 h in a nitrogen atmosphere. After the reaction was complete, the reaction mixture was quenched with an excessive amount of methanol and concentrated *in vacuo* to give a crude product, and the crude product was purified by silica gel column chromatography (PE/EA = 100/0-60/40) to give 3-(1H-indol-7-yl)propan-1-ol (380 mg, yield: 97.9%) as a pale yellow oil. MS m/z (ESI): 176.2 [M+H]⁺.

Step 4: At room temperature, 3-(1H-indol-7-yl)propan-1-ol (50.0 mg, 0.29 mmol) and carbon tetrabromide (141.9 mg, 0.43 mmol) were dissolved in dichloromethane (10 mL), and triphenylphosphine (112.2 mg, 0.43 mmol) was added. The reaction solution was stirred at room temperature for 16 h. After the reaction was complete, the reaction mixture was concentrated *in vacuo* to give a crude product. The crude product was purified by silica gel column chromatography (PE/EA = 100/0-90/10) to give 7-(3-bromopropyl)-1H-indole (40 mg, yield: 58.9%) as a transparent oil. MS m/z (ESI): 238.1 [M+H]⁺.

Step 5: 7-(3-Bromopropyl)-1H-indole (40.5 mg, 0.17 mmol) was dissolved in 1,4-dioxane/toluene (1.5 mL/1.5 mL), and (6bR,10aS)-3-methyl-2,3,6b,7,8,9,10,10a-octahydro-1H-pyrido[3',4':4,5]pyrrolo[1,2,3-de]quinoxaline (39.0 mg, 0.17 mmol), potassium iodide (56.4 mg, 0.34 mmol), and triethylamine (51.6 mg, 0.51 mmol) were added. The reaction solution was stirred at 100 °C for 18 h. The reaction solution was cooled to room temperature and concentrated *in vacuo* to give a crude product. The crude product was purified by preparative chromatography to give (6bR,10aS)-8-(3-(1H-indol-7-yl)propyl)-3-methyl-2,3,6b,7,8,9,10,10a-octahydro-1H-pyrido[3',4':4,5]pyrrolo[1,2,3-de]quinoxaline (13.51 mg, yield: 20.16%) as a light yellow solid. ¹H NMR (400 MHz, CDCl₃) δ 10.85 (s, 1H), 7.52 (dd, *J =* 7.8, 0.8 Hz, 1H), 7.22 (d, *J =* 2.9 Hz, 1H), 7.06-7.01 (m, 1H), 6.96 (d, *J* = 6.8 Hz, 1H), 6.68 (t, *J =* 7.6 Hz, 1H), 6.55 (dd, *J =* 6.6, 5.4 Hz, 2H), 6.43 (d, *J =* 7.8 Hz, 1H), 3.64-3.58 (m, 1H), 3.39-3.28 (m, 4H), 3.04-2.96 (m, 3H), 2.92-2.86 (m, 4H), 2.78 (s, 1H), 2.41-2.38 (m, 3H), 2.11-2.07 (m, 3H), 2.02-1.97 (m, 2H). MS m/z (ESI): 387.3 [M+H]⁺.

### Example 12: (6bR,10aS)-8-(3-(Dihydroindol-7-yl)propyl)-3-methyl-2,3,6b,7,8,9,10,10a-octahydro-1H-pyrido[3',4':4,5]pyrrolo[1,2,3-de]quinoxaline

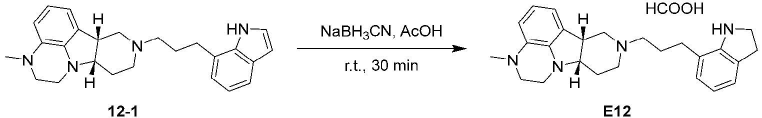

At room temperature, (6bR,10aS)-8-(3-(1H-indol-7-yl)propyl)-3-methyl-2,3,6b,7,8,9,10,10a-octahydro-1H-pyrido[3',4':4,5]pyrrolo[1,2,3-de]quinoxaline (55.0 mg, 0.14 mmol) was dissolved in acetic acid (3 mL), and sodium triacetoxyborohydride (13.4 mg, 0.21 mmol) was added. The reaction mixture was stirred at room temperature for 30 min. After the reaction was complete, the reaction mixture was concentrated *in vacuo* and added to 10 mL of water, and a sodium hydroxide solution was added dropwise to adjust the pH to 8. The mixture was diluted with 30 mL of water and extracted with ethyl acetate (30 mL × 3) to give a crude product, and the crude product was purified by preparative chromatography to give (6bR,10aS)-8-(3-(dihydroindol-7-yl)propyl)-3-methyl-2,3,6b,7,8,9,10,10a-octahydro-1H-pyrido[3',4':4,5]pyrrolo[1,2,3-de]quinoxaline formate (14.04 mg, yield: 24.2%) as a brown solid. ¹H NMR (400 MHz, CDCl₃) δ 8.50 (s, 1H), 6.99 (d, *J* = 7.2 Hz, 1H), 6.78 (d, *J =* 7.5 Hz, 1H), 6.69 (t, *J* = 7.7 Hz, 1H), 6.63 (t, *J* = 7.4 Hz, 1H), 6.52 (d, *J* = 7.4 Hz, 1H), 6.43 (d, *J* = 8.0 Hz, 1H), 3.65-3.50 (m, 4H), 3.41 (dd, *J* = 11.8, 6.2 Hz, 1H), 3.36-3.24 (m, 4H), 3.03 (t, *J* = 8.4 Hz, 2H), 2.95-2.78 (m, 7H), 2.56 (t, *J =* 7.3 Hz, 2H), 2.39-2.34 (m, 2H), 2.09-2.03 (m, 3H). MS m/z (ESI): 389.3 [M+H]⁺.

### Example 13: (6bR,10aS)-8-(3-(3-Methoxypyridin-4-yl)propyl)-3-methyl-2,3,6b,7,8,9,10,10a-octahydro-1H-pyrido[3',4':4,5]pyrrolo[1,2,3-de]quinoxaline

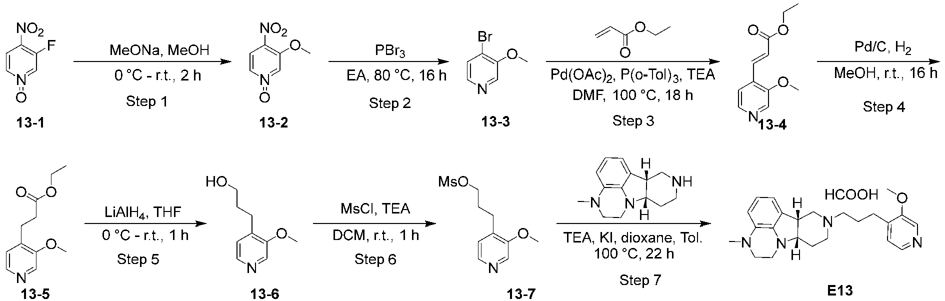

Step 1: 3-Fluoro-4-nitropyridine 1-oxide (5 g, 31.6 mmol) was dissolved in absolute methanol (55 mL) at room temperature, and a 5.4 M solution of sodium methoxide in methanol (5.9 mL, 31.6 mmol) was then added at 0 °C. The reaction solution was stirred at room temperature for 2 h. The reaction solution was concentrated *in vacuo* and diluted with water (80 mL). The aqueous phase was extracted with dichloromethane (50 mL × 3). The combined organic phases were washed with brine (50 mL), dried over anhydrous sodium sulfate, filtered, and concentrated *in vacuo* to give 3-methoxy-4-nitropyridine 1-oxide (5 g, yield: 77.8%) as a pale yellow solid. The crude product was directly used in the next step without purification. MS m/z (ESI): 171.1 [M+H]⁺.

Step 2: At room temperature, 3-methoxy-4-nitropyridine 1-oxide (5.0 g, 29.4 mmol) was dissolved in ethyl acetate (50 mL), and phosphorus tribromide (22.1 mL, 235.2 mmol) was added dropwise. The mixture was stirred at room temperature for 10 min, then heated to 80 °C, and stirred at this temperature for 16 h. The reaction solution was cooled to room temperature, added dropwise to iced water, and extracted with ethyl acetate. The pH of the aqueous phase was adjusted to 11 with a 10 M aqueous NaOH solution, and extraction was then performed three times with ethyl acetate. The combined organic phases were dried over anhydrous sodium sulfate, filtered, and concentrated *in vacuo* to give a crude product. The crude product was separated and purified by column chromatography (EtOAc/PE = 0-15%) to give 4-bromo-3-methoxypyridine (470 mg, yield: 8.5%) as a brown solid. MS m/z (ESI): 188.1 [M+H]⁺.

Step 3: DMF (5 mL) was added to a reaction flask containing 4-bromo-3-methoxypyridine (470 mg, 2.5 mmol), ethyl acrylate (860.79 mg, 9.99 mmol), palladium acetate (56.62 mg, 0.25 mmol), triethylamine (2.53 g, 25.0 mmol), and tri(o-tolyl)phosphine (228.25 mg, 0.75 mmol) at room temperature. After the reaction flask was purged three times with nitrogen, the mixture was heated to 100 °C and stirred for 18 h in a nitrogen atmosphere. The reaction solution was cooled to room temperature and filtered through diatomaceous earth. The filtrate was diluted with water (40 mL) and extracted with ethyl acetate (30 mL × 3). The combined organic phases were dried over anhydrous sodium sulfate, filtered, and concentrated *in vacuo* to give a crude product. The crude product was separated and purified by column chromatography (EtOAc/PE = 0-20%) to give ethyl 3-(3-methoxypyridin-4-yl)acrylate (300 mg, yield: 60.3%) as a pale yellow solid. MS m/z (ESI): 208.1 [M+H]⁺.

Step 4: At room temperature, ethyl 3-(3-methoxypyridin-4-yl)acrylate (270 mg, 14.5 mmol) was dissolved in methanol (5 mL), and 10% wet palladium on carbon (69 mg, 0.65 mmol) was then added. The reaction solution was stirred at room temperature for 16 h in a hydrogen atmosphere. The reaction solution was filtered through diatomaceous earth. The filter cake was washed with methanol, and the filtrates were combined and concentrated *in vacuo* to give ethyl 3-(3-methoxypyridin-4-yl)propanoate (50 mg, yield: 87.6%) as a yellow oil. The crude product was directly used in the next step without purification. MS m/z (ESI): 210.1 [M+H]⁺.

Step 5: Ethyl 3-(3-methoxypyridin-4-yl)propanoate (200.0 mg, 0.96 mmol) was dissolved in anhydrous tetrahydrofuran (5 mL) at room temperature. The temperature was lowered to below 0 °C, and a 1 M solution of lithium aluminum hydride in tetrahydrofuran (2.4 mL, 2.39 mmol) was added dropwise. The reaction solution was stirred at room temperature for 1 h in a nitrogen atmosphere. The reaction solution was diluted with tetrahydrofuran, quenched with sodium sulfate decahydrate in an ice bath, and filtered through diatomaceous earth. The filter cake was ultrasonicated with methanol, and the mixture was filtered again. The combined filtrates were concentrated *in vacuo* to give 3-(3-methoxypyridin-4-yl)propan-1-ol (160 mg, theoretical amount) as a yellow oil. MS m/z (ESI): 168.2 [M+H]⁺.

Step 6: At room temperature, 3-(3-methoxypyridin-4-yl)propan-1-ol (130.0 mg, 0.78 mmol) and triethylamine (236.0 mg, 2.33 mmol) were dissolved in anhydrous DCM (1.5 mL), and a solution of MsCl (133.59 mg, 1.17 mmol) in DCM (0.5 mL) was added dropwise. The reaction solution was stirred at room temperature for 1 h. The reaction solution was concentrated *in vacuo* to give 3-(3-methoxypyridin-4-yl)propyl mesylate (191 mg, theoretical amount) as a white solid. MS m/z (ESI): 246.1 [M+H]⁺.

Step 7: At room temperature, (6bR,10aS)-3-methyl-2,3,6b,7,8,9,10,10a-octahydro-1H-pyrido[3',4':4,5]pyrrolo[1,2,3-de]quinoxaline (90.0 mg, 0.30 mmol), 3-(3-methoxypyridin-4-yl)propyl mesylate (192.6 mg, 0.78 mmol), and potassium iodide (130.2 mg, 0.78 mmol) were dissolved in anhydrous 1,4-dioxane (1.5 mL) and anhydrous toluene (1.5 mL), and triethylamine (238.3 mg, 2.36 mmol) was then added. The reaction solution was stirred at 100 °C for 22 h. The reaction solution was cooled to room temperature and concentrated *in vacuo* to give a crude product. The crude product was purified by preparative chromatography with formic acid to give (6bR,10aS)-8-(3-(3-methoxypyridin-4-yl)propyl)-3-methyl-2,3,6b,7,8,9,10,10a-octahydro-1H-pyrido[3',4':4,5]pyrrolo[1,2,3-de]quinoxaline formate (6.53 mg, yield: 3.8%) as a yellow oil. ¹H NMR (400 MHz, CDCl₃) δ 8.41 (s, 1H), 8.19 (s, 1H), 8.16 (d, *J =* 4.7 Hz, 1H), 7.06 (d, *J* = 4.8 Hz, 1H), 6.69 (t, *J =* 7.7 Hz, 1H), 6.52 (d, *J =* 7.4 Hz, 1H), 6.43 (d, *J =* 7.9 Hz, 1H), 3.90 (s, 3H), 3.62-3.49 (m, 2H), 3.37 (dd, *J* = 11.6, 6.7 Hz, 1H), 3.28 (d, *J =* 9.4 Hz, 4H), 2.87-2.78 (m, 6H), 2.68-2.63 (m, 2H), 2.36 (s, 2H), 2.08-2.02 (m, 4H). MS m/z (ESI): 379.2 [M+H]⁺.

### Example 14: (6bR,10aS)-8-(3-(2-Methoxypyridin-3-yl)propyl)-3-methyl-2,3,6b,7,8,9,10,10a-octahydro-1H-pyrido[3',4':4,5]pyrrolo[1,2,3-de]quinoxaline

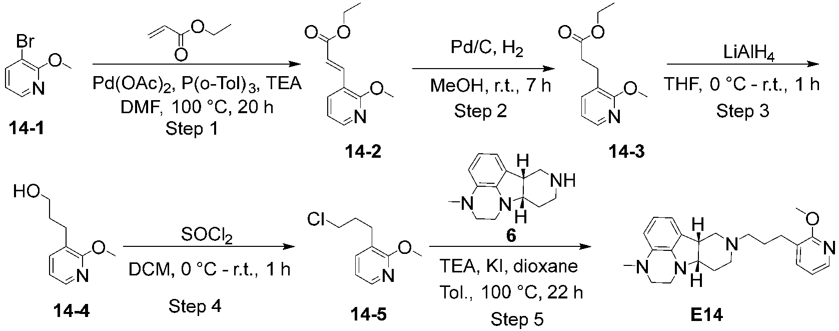

Step 1: DMF (100 mL) was added to a reaction flask containing 3-bromo-2-methoxypyridine (5 g, 26.6 mmol), ethyl acrylate (10.65 g, 106.4 mmol), palladium acetate (0.6 g, 2.66 mmol), triethylamine (26.92 g, 266 mmol), and tri(o-tolyl)phosphine (2.43 g, 7.98 mmol) at room temperature. After the reaction flask was purged three times with nitrogen, the mixture was heated to 100 °C and stirred for 20 h in a nitrogen atmosphere. The reaction solution was cooled to room temperature and filtered through diatomaceous earth. The filtrate was diluted with water (80 mL) and extracted with ethyl acetate (50 mL × 3). The combined organic phases were dried over anhydrous sodium sulfate, filtered, and concentrated *in vacuo* to give a crude product. The crude product was separated and purified by column chromatography (EtOAc/PE = 0-6%) to give ethyl (E)-3-(2-methoxypyridin-3-yl)acrylate (4 g, yield: 71.8%) as a pale yellow oil. MS m/z (ESI): 208.1 [M+H]⁺.

Step 2: At room temperature, ethyl (E)-3-(2-methoxypyridin-3-yl)acrylate (3 g, 14.5 mmol) was dissolved in methanol (30 mL), and 10% wet palladium on carbon (770 mg, 0.725 mmol) was then added. The reaction solution was stirred at room temperature for 7 h in a hydrogen atmosphere. The reaction solution was filtered through diatomaceous earth. The filter cake was washed with methanol, and the filtrates were combined and concentrated *in vacuo* to give ethyl 3-(2-methoxypyridin-3-yl)propanoate (3 g, yield: 98.6%) as a colorless viscous oil. The crude product was directly used in the next step without purification. MS m/z (ESI): 210.1 [M+H]⁺.

Step 3: Ethyl 3-(2-methoxypyridin-3-yl)propanoate (1 g, 4.8 mmol) was dissolved in anhydrous tetrahydrofuran (15 mL) at room temperature. The temperature was lowered to below 0 °C, and a 1 M solution of lithium aluminum hydride in tetrahydrofuran (12 mL, 12 mmol) was added dropwise. The reaction solution was stirred at room temperature for 1 h in a nitrogen atmosphere. The reaction solution was diluted with tetrahydrofuran, quenched with sodium sulfate decahydrate in an ice bath, and filtered through diatomaceous earth. The filter cake was ultrasonicated with methanol, and the mixture was filtered again. The combined filtrates were concentrated *in vacuo* to give a crude product. The crude product was separated and purified by column chromatography (EtOAc/PE = 0-18%) to give 3-(2-methoxypyridin-3-yl)propan-1-ol (705 mg, yield: 87.5%) as a colorless viscous oil. MS m/z (ESI): 168.2 [M+H]⁺.

Step 4: 3-(2-Methoxypyridin-3-yl)propan-1-ol (200 mg, 1.196 mmol) was dissolved in anhydrous DCM (2 mL) at room temperature, and thionyl chloride (398.4 mg, 3.35 mmol) was added dropwise under ice bath conditions. The reaction solution was stirred at room temperature for 1 h. The reaction solution was concentrated *in vacuo* to give 3-(3-chloropropyl)-2-methoxypyridine (220 mg, theoretical amount) as a white solid. The crude product was directly used in the next step without purification. MS m/z (ESI): 186.1 [M+H]⁺.

Step 5: At room temperature, (6bR,10aS)-3-methyl-2,3,6b,7,8,9,10,10a-octahydro-1H-pyrido[3',4':4,5]pyrrolo[1,2,3-de]quinoxaline (68 mg, 0.296 mmol), 3-(3-chloropropyl)-2-methoxypyridine (110.09 mg, 0.593 mmol), and potassium iodide (98.36 mg, 0.593 mmol) were dissolved in anhydrous 1,4-dioxane (1.5 mL) and anhydrous toluene (1.5 mL), and triethylamine (180.02 mg, 1.779 mmol) was then added. The reaction solution was stirred at 100 °C for 22 h. The reaction solution was cooled to room temperature and concentrated *in vacuo* to give a crude product. The crude product was purified by a preparative alkaline method to give (6bR,10aS)-8-(3-(2-methoxypyridin-3-yl)propyl)-3-methyl-2,3,6b,7,8,9,10,10a-octahydro-1H-pyrido[3',4':4,5]pyrrolo[1,2,3-de]quinoxaline (12.10 g, yield: 10.7%) as a yellow oil. ¹H NMR (400 MHz, CDCl₃) δ 8.01 (dd, *J =* 5.0, 1.7 Hz, 1H), 7.38 (d, *J* = 5.8 Hz, 1H), 6.80 (dd, *J* = 7.1, 5.1 Hz, 1H), 6.65 (t, *J =* 7.6 Hz, 1H), 6.51 (d, *J =* 7.3 Hz, 1H), 6.41 (d, *J* = 7.9 Hz, 1H), 3.93 (s, 3H), 3.59 (dd, *J* = 14.9, 6.3 Hz, 1H), 3.46-3.00 (m, 5H), 2.91-2.69 (m, 6H), 2.58 (t, *J* = 7.6 Hz, 2H), 2.47-2.18 (m, 3H), 1.89-1.83 (m, 4H). MS m/z (ESI): 379.2 [M+H]⁺.

### Example 15: (6bR,10aS)-8-(3-(4-Methoxypyridin-3-yl)propyl)-3-methyl-2,3,6b,7,8,9,10,10a-octahydro-1H-pyrido[3',4':4,5]pyrrolo[1,2,3-de]quinoxaline

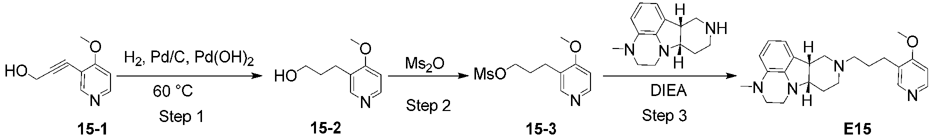

Step 1: The compound 3-(4-methoxypyridin-3-yl)prop-2-yn-1-ol (150 mg, 0.92 mmol) was added to a 50 mL three-neck reaction flask, and methanol (10 mL) and wet palladium on carbon (10%, 50 mg) were subsequently added. Then, the mixture was heated to 60 °C and stirred for 4 h in a hydrogen atmosphere. After LCMS analysis showed that the reaction had been complete, the reaction mixture was filtered and concentrated, and the resulting residue was purified by column chromatography (DCM:MeOH = 3:1) to give the title compound 3-(4-methoxypyridin-3-yl)propan-1-ol (colorless liquid, 80 mg, yield: 80%). MS m/z (ESI): 168.4 [M+H]⁺.

Step 2: 3-(4-Methoxypyridin-3-yl)propyl mesylate was prepared with reference to the synthesis method in step 3 of Example 3.

Step 3: (6bR,10aS)-8-(3-(4-Methoxypyridin-3-yl)propyl)-3-methyl-2,3,6b,7,8,9,10,10a-octahydro-1H-pyrido[3',4':4,5]pyrrolo[1,2,3-de]quinoxaline was prepared with reference to the synthesis method in step 4 of Example 3. ¹H NMR (500 MHz, DMSO) δ 8.30 (d, *J* = 5.6 Hz, 1H), 8.20 (s, 1H), 6.98 (d, *J* = 5.6 Hz, 1H), 6.51 (t, *J* = 7.6 Hz, 1H), 6.42 (d, *J* = 7.2 Hz, 1H), 6.33 (d, *J =* 7.6 Hz, 1H), 3.85 (s, 3H), 3.45-3.39 (m, 1H), 3.31-3.25 (m, 2H), 3.11 (s, 1H), 3.02 (s, 1H), 2.78 (s, 4H), 2.67-2.63 (m, 1H), 2.53-.2.49 (m, 3H), 2.18-2.10 (m, 2H), 2.03-1.85 (m, 2H), 1.81-1.61 (m, 4H). MS m/z (ESI): 379.6 [M+H]⁺.

### Example 16: (6bR,10aS)-8-(3-(3-Methoxypyridin-2-yl)propyl)-3-methyl-2,3,6b,7,8,9,10,10a-octahydro-1H-pyrido[3',4':4,5]pyrrolo[1,2,3-de]quinoxaline

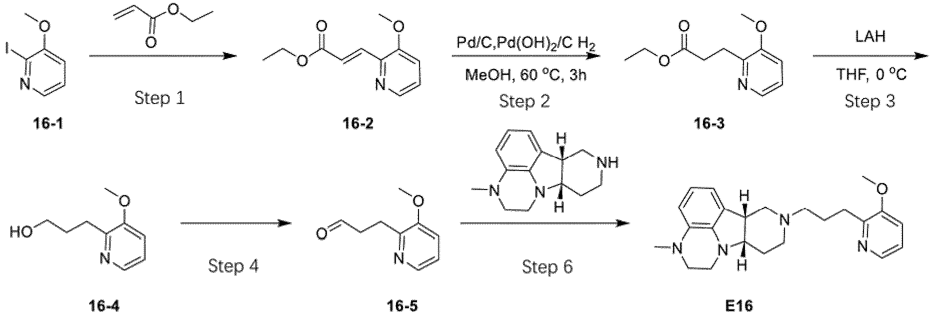

Preparation was performed with reference to the synthesis method of Example 8. 1H NMR (500 MHz, DMSO) δ 8.03 (dd, *J* = 4.7, 1.3 Hz, 1H), 7.31 (dd, *J* = 8.3, 1.3 Hz, 1H), 7.18 (dd, *J* = 8.2, 4.7 Hz, 1H), 6.54-6.46 (m, 1H), 6.41 (dd, *J =* 7.3, 0.9 Hz, 1H), 6.32 (dd, *J =* 8.0, 1.0 Hz, 1H), 3.80 (s, 3H), 3.44-3.39 (m, 1H), 3.29 (m, 2H), 3.09 (m, 1H), 2.98 (m, 1H), 2.77 (s, 3H), 2.76-2.64 (m, 4H), 2.60-2.54 (m, 1H), 2.27 (m, 2H), 2.06 (m, 1H), 1.92-1.85 (m, 1H), 1.80-1.70 (m, 4H). MS m/z (ESI): 379.8 [M+H]⁺.

### Example 17: Preparation of (6bR,10aS)-8-((E)-3-(2-Methoxypyridin-3-yl)allyl)-3-methyl-2,3,6b,7,8,9,10,10a-octahydro-1H-pyrido[3',4":4,5]pyrrolo[1,2,3-de]quinoxaline

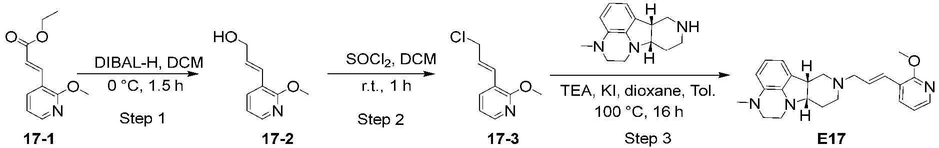

Step 1: Ethyl (E)-3-(2-methoxypyridin-3-yl)acrylate (800.0 mg, 3.82 mmol) was dissolved in dichloromethane (10 mL) at room temperature. The temperature was lowered to below 0 °C, and a 1 M solution of diisobutylaluminum hydride (7.6 mL, 7.65 mmol) in THF was added dropwise. The reaction solution was stirred at room temperature for 1 h in a nitrogen atmosphere. The reaction solution was diluted with dichloromethane, quenched by dropwise addition of a saturated ammonium chloride solution in an ice bath, diluted with 40 mL of water, and extracted with dichloromethane (40 mL × 3). The organic phases were combined and concentrated *in vacuo* to give a crude product. The crude product was separated and purified by column chromatography (EtOAc/PE = 0-18%) to give (E)-3-(2-methoxypyridin-3-yl)prop-2-en-1-ol (437 mg, yield: 69.3%) as a light yellow oil. MS m/z (ESI): 166.2 [M+H]⁺.

Step 2: (E)-3-(2-Methoxypyridin-3-yl)prop-2-en-1-ol (100 mg, 0.60 mmol) was dissolved in anhydrous DCM (3 mL) at room temperature, and SOCl₂ (144 mg, 1.21 mmol) was added dropwise under ice bath conditions. The reaction solution was stirred at room temperature for 1 h. The reaction solution was concentrated *in vacuo* to give (E)-3-(3-chloroprop-1-en-1-yl)-2-methoxypyridine (110 mg, theoretical amount) as a white solid. The crude product was directly used in the next step without purification. MS m/z (ESI): 184.1 [M+H]⁺.

Step 3: At room temperature, (E)-3-(3-chloroprop-1-en-1-yl)-2-methoxypyridine (112.1 mg, 0.61 mmol) was dissolved in 1,4-dioxane/toluene (1.5 mL/1.5 mL), and (6bR,10aS)-3-methyl-2,3,6b,7,8,9,10,10a-octahydro-1H-pyrido[3',4':4,5]pyrrolo[1,2,3-de]quinoxaline (70.0 mg, 0.30 mmol), potassium iodide (101 mg, 0.61 mmol), and triethylamine (185.3 mg, 1.83 mmol) were added. The reaction solution was stirred at 100 °C for 18 h. The reaction solution was cooled to room temperature and concentrated *in vacuo* to give a crude product. The crude product was purified by a preparative alkaline method to give (6bR,10aS)-8-((E)-3-(2-methoxypyridin-3-yl)allyl)-3-methyl-2,3,6b,7,8,9,10,10a-octahydro-1H-pyrido[3',4':4,5]pyrrolo[1,2,3-de]quinoxaline (26.15 mg, yield: 22.5%) as a yellow solid. ¹H NMR (400 MHz, CDCl₃) δ 8.05 (dd, *J =* 4.9, 1.7 Hz, 1H), 7.67 (d, *J =* 6.4 Hz, 1H), 6.86 (dd, *J =* 7.3, 5.0 Hz, 1H), 6.70-6.62 (m, 2H), 6.52 (d, *J =* 6.8 Hz, 1H), 6.41 (d, *J =* 7.6 Hz, 2H), 3.97 (s, 3H), 3.58-3.61 (m, 1H), 3.35-3.28 (m, 6H), 2.96 (s, 1H), 2.88-2.73 (m, 5H), 2.30 (s, 1H), 1.97 (s, 3H). MS m/z (ESI): 377.2 [M+H]⁺.

### Example 18: Preparation of (6bR,10aS)-8-(2-(2-Methoxyphenoxy)ethyl)-3-methyl-2,3,6b,7,8,9,10,10a-octahydro-1H-pyrido[3',4':4,5]pyrrolo[1,2,3-de]quinoxaline

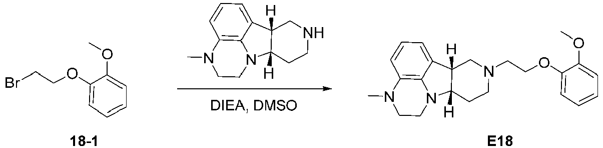

At room temperature, (6bR,10aS)-3-methyl-2,3,6b,7,8,9,10,10a-octahydro-1H-pyrido[3',4':4,5]pyrrolo[1,2,3-de]quinoxaline (23 mg, 0.1 mmol) was dissolved in DMSO (2.5 mL), and DIEA (0.5 mL, 3.0 mmol) and 1-(2-bromoethoxy)-2-methoxybenzene (23 mg, 0.1 mmol) were sequentially added. The mixture was heated to 65 °C and stirred for 18 h, cooled to room temperature, filtered, and then purified and separated by prep-HPLC (eluent (v/v): acetonitrile/(water + 0.05% NH₄HCO₃) = 30%-70%) to give the compound (6bR,10aS)-8-(2-(2-methoxyphenoxy)ethyl)-3-methyl-2,3,6b,7,8,9,10,10a-octahydro-1H-pyrido[3',4':4,5]pyrrolo[1,2,3-de]quinoxaline (yellow solid, 10.2 mg, yield: 27%). 1H NMR (500 MHz, CD₃OD) δ 7.09-6.90 (m, 4H), 6.88-6.57 (m, 3H), 4.41 (t, *J =* 5.0 Hz, 1H), 3.75-3.69 (m, 4H), 3.55-3.46 (m, 8H), 3.39-3.21 (m, 2H), 2.86 (s, 3H), 2.82-2.77 (m, 2H), 2.35-2.24 (m, 2H). MS m/z (ESI): 380.8 [M+H]⁺.

### Example 19: 2-Methoxy-N-(2-((6bR,10aS)-3-methyl-2,3,6b,9,10,10a-hexahydro-1H-pyrido[3',4':4,5]pyrrolo[1,2,3-de]quinoxalin-8(7H)-yl)ethyl)aniline

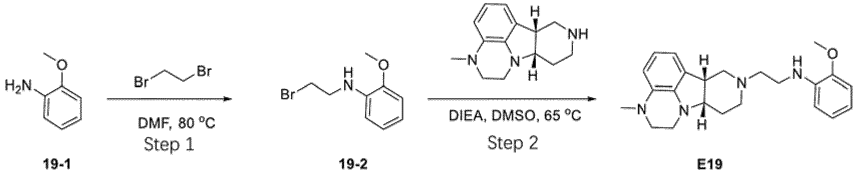

Step 1: The compounds 2-methoxyaniline (505 mg, 2.0 mmol) and dibromoethane (1.104 g, 6.0 mmol) were dissolved in DMF (5.0 mL), and the solution was stirred at 60 °C for 3 h. After TLC analysis showed that the reaction had been complete, the reaction mixture was quenched with water and extracted with ethyl acetate. The organic phase was washed with saturated brine, dried over anhydrous sodium sulfate, filtered, and concentrated, and the resulting crude product was purified by normal-phase chromatography to give the compound 1-(2-bromoethoxy)-2-methoxybenzene (yellow solid, 115 mg, yield: 25%). MS m/z (ESI): 232.3 [M+H]⁺.

Step 2: 2-Methoxy-N-(2-((6bR,10aS)-3-methyl-2,3,6b,9,10,10a-hexahydro-1H-pyrido[3',4':4,5]pyrrolo[1,2,3-de]quinoxalin-8(7H)-yl)ethyl)aniline was synthesized with reference to the synthesis method in step 4 of Example 3. ¹H NMR (500 MHz, CD₃OD) δ 6.81-6.77 (m, 2H), 6.59-6.50 (m, 3H), 6.44-6.42 (m, 1H), 6.35-6.33 (m, 1H), 4.78 (brs, 1H), 3.77 (s, 3H), 3.47-3.42 (m, 1H), 3.32-3.26 (m, 3H), 3.15-3.05 (m, 4H), 2.79-2.75 (m, 4H), 2.72-2.55 (m, 3H), 2.26-2.24 (m, 1H), 1.98-1.75 (m, 3H). MS m/z (ESI): 379.9 [M+H]⁺.

### Example 20: N-(2-Methoxyphenyl)-2-((6bR,10aS)-3-methyl-2,3,6b,9,10,10a-hexahydro-1H-pyrido[3',4':4,5]pyrrolo[1,2,3-de]quinoxalin-8(7H)-yl)acetamide

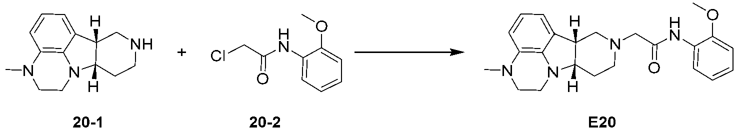

The compounds (6bR,10aS)-3-methyl-2,3,6b,7,8,9,10,10a-octahydro-1H-pyrido[3',4':4,5]pyrrolo[1,2,3-de]quinoxaline (25 mg, 0.11 mmol), 2-chloro-N-(2-methoxyphenyl)acetamide (22 mg, 0.11 mmol), DIEA (78 mg, 0.60 mmol), and sodium iodide (17 mg, 0.11 mmol) were dissolved in DMSO (3 mL), and the solution was stirred at 60 °C for 12 h. The reaction solution was filtered and then purified by preparative reversed-phase chromatography (eluent (v/v): acetonitrile/(water + 0.05% NH₄HCO₃)). Acetonitrile was removed under reduced pressure, and the residue was lyophilized to give the compound N-(2-methoxyphenyl)-2-((6bR,10aS)-3-methyl-2,3,6b,9,10,10a-hexahydro-1H-pyrido[3',4':4,5]pyrrolo[1,2,3-de]quinoxalin-8(7H)-yl)acetamide (pale yellow solid, 20 mg, yield: 46%). 1H NMR (500 MHz, DMSO) δ 9.78 (s, 1H), 8.29-8.11 (m, 1H), 7.09-7.03 (m, 2H), 6.93 (m, 1H), 6.51 (t, *J =* 7.6 Hz, 1H), 6.43 (d, *J =* 7.2 Hz, 1H), 6.35 (d, *J* = 7.8 Hz, 1H), 3.86 (s, 3H), 3.45 (m, 1H), 3.36 (m, 1H), 3.29 (m, 1H), 3.24-3.18 (m, 1H), 3.16-3.11 (m, 1H), 3.09 (s, 2H), 2.84 (m, 1H), 2.79 (s, 3H), 2.71 (m, 1H), 2.62 (d, *J* = 11.4 Hz, 1H), 2.41 (m, 1H), 2.11 (t, *J =* 11.0 Hz, 1H), 2.01 (dd, *J* = 14.5, 2.8 Hz, 1H), 1.88 (m, 1H). MS m/z (ESI): 393.6 [M+H]⁺.

### Example 21: 3-((6bR,10aS)-3-Methyl-2,3,6b,9,10,10a-hexahydro-1H-pyrido[3',4':4,5]pyrrolo[1,2,3-de]quinoxalin-8(7H)-yl)-1-(pyridin-4-yl)propan-1-one

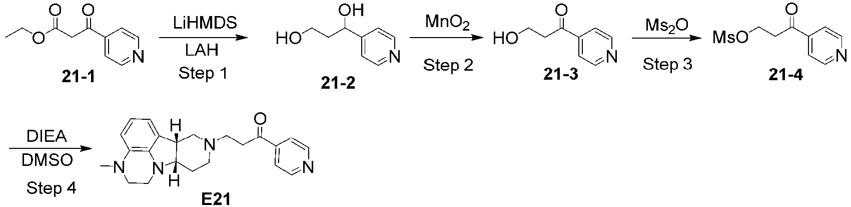

Step 1: The compound ethyl 3-carbonyl-3-(pyridin-4-yl)propanoate (300 mg, 1.55 mmol) was added to a 50 mL three-neck reaction flask, and THF (5 mL) was subsequently added. LiHMDS (1.7 mL, 1 mol/L) was slowly added dropwise at -78 °C, and this reaction was stirred at 0 °C for 0.5 h. Then, lithium aluminum hydride (3.1 mL, 1 mol/L) was slowly added dropwise at 0 °C, and the mixture was slowly warmed to 0 °C and stirred for 1 h. The reaction solution was quenched with a saturated aqueous ammonium chloride solution and extracted with dichloromethane (2 × 50 mL). After concentration, the residue was purified by column chromatography to give the compound 1-(pyridin-4-yl)propane-1,3-diol (yellow liquid, 200 mg, yield: 84.0%). MS m/z (ESI): 154.0 [M+H]⁺.

Step 2: The compound 1-(pyridin-4-yl)propane-1,3-diol (200 mg, 1.3 mmol) was added to a 100 mL reaction flask, and dichloromethane (20 mL) and MnO₂ (568 mg, 6.5 mmol) were subsequently added. The mixture was stirred at 40 °C for 4 h. The reaction solution was filtered and concentrated, and the resulting residue was purified by reversed-phase column chromatography to give the compound 3-hydroxy-1-(pyridin-4-yl)propan-1-one (colorless solid, 100 mg, yield: 50.1%). MS m/z (ESI): 152.1 [M+H]⁺.

Step 3: 3-Carbonyl-3-(pyridin-4-yl)propyl mesylate was prepared with reference to the synthesis method in step 3 of Example 3.

Step 4: 3-((6bR,10aS)-3-Methyl-2,3,6b,9,10,10a-hexahydro-1H-pyrido[3',4':4,5]pyrrolo[1,2,3-de]quinoxalin-8(7H)-yl)-1-(pyridin-4-yl)propan-1-one was prepared with reference to the synthesis method in step 4 of Example 3. ¹H NMR (500 MHz, CD₃OD) δ 8.90 (d, *J* = 6.0 Hz, 2H), 7.98 (t, *J* =8.0 Hz, 2H), 6.63 (t, *J* = 7.6 Hz, 1H), 6.52 (dd, *J =* 32.8, 7.6 Hz, 2H), 3.75 (d, *J =* 7.2 Hz, 4H), 3.28-3.02 (m, 7H), 2.84 (s, 3H), 2.80-2.71 (m, 1H), 2.65 (dd, *J =* 3.7, 1.8 Hz, 1H), 2.33 (dd, *J =* 45.0, 7.0 Hz, 3H). MS m/z (ESI): 363.6 [M+H]⁺.

### Example 22: 1-(2-Methoxyphenyl)-3-((6bR,10aS)-3-methyl-2,3,6b,9,10,10a-hexahydro-1H-pyrido[3',4':4,5]pyrrolo[1,2,3-de]quinoxalin-8(7H)-yl)propan-1-one

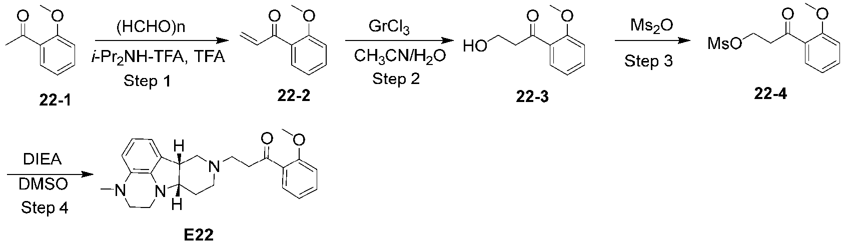

Step 1: The compounds 1-(2-methoxyphenyl)ethan-1-one (500 mg, 3.33 mmol) and paraformaldehyde (200 mg, 6.66 mmol) were dissolved in tetrahydrofuran (10 mL), and N,N-diisopropylethylamine trihydrofluoride (716 mg, 3.33 mmol) and TFA (40 mg, 0.33 mmol) were then added. The mixture was stirred at 70 °C for 12 h. Water (80 mL) was added to the reaction solution, and extraction was performed with ethyl acetate (80 mL × 2). The organic phase was dried over anhydrous sodium sulfate and then filtered, and the filtrate was concentrated. The resulting residue was purified and separated by normal-phase column chromatography (petroleum ether:ethyl acetate = 10:1) to give the compound 1-(2-methoxyphenyl)prop-2-en-1-one (yellow solid, 430 mg, crude, yield: 80%). MS m/z (ESI): 163.2 [M+H]⁺.

Step 2: The compound 1-(2-methoxyphenyl)prop-2-en-1-one (100 mg, 0.62 mmol) was dissolved in acetonitrile (5 mL), and water (67 mg, 3.70 mmol) and chromium trichloride (20 mg, 0.12 mmol) were then added. The mixture was stirred at room temperature for 24 h. Water (30 mL) was added to the reaction solution, and extraction was performed with ethyl acetate (30 mL × 2). The organic phase was dried over anhydrous sodium sulfate and then filtered, and the filtrate was concentrated. The resulting residue was purified by reversed-phase preparative chromatography (eluent (v/v): acetonitrile/(water + 0.05% NH₄HCO₃)). Acetonitrile was removed under reduced pressure, and the residue was lyophilized to give the compound 3-hydroxy-1-(2-methoxyphenyl)propan-1-one (white solid, 10 mg, yield: 9%).

Step 3: 3-(2-Methoxyphenyl)-3-oxopropyl mesylate was prepared with reference to the synthesis method in step 3 of Example 3.

Step 4: 1-(2-Methoxyphenyl)-3-((6bR,10aS)-3-methyl-2,3,6b,9,10,10a-hexahydro-1H-pyrido[3',4':4,5]pyrrolo[1,2,3-de]quinoxalin-8(7H)-yl)propan-1-one was prepared with reference to the synthesis method in step 4 of Example 3. ¹H NMR (500 MHz, DMSO) δ 7.58-7.47 (m, 2H), 7.16 (d, *J* = 8.3 Hz, 1H), 7.05-6.99 (m, 1H), 6.58-6.49 (m, 1H), 6.42 (d, *J* = 6.9 Hz, 1H), 6.34 (d, *J* = 8.0 Hz, 1H), 3.87 (s, 3H), 3.46-3.42 (m, 3H), 3.30-3.25 (m, 2H), 3.25-3.14 (m, 2H), 3.10 (s, 2H), 2.98-2.87 (m, 1H), 2.80 (s, 1H), 2.78 (s, 3H), 2.71-2.63 (m, 2H), 2.03-1.89 (m, 2H), 1.87-1.72 (m, 1H). MS m/z (ESI): 392.9 [M+H]⁺.

### Example 23: 1-(2,3-Dihydrobenzofuran-7-yl)-3-((6bR,10aS)-3-methyl-2,3,6b,9,10,10a-hexahydro-1H-pyrido[3',4':4,5]pyrrolo[1,2,3-de]quinoxalin-8(7H)-yl)propan-1-one

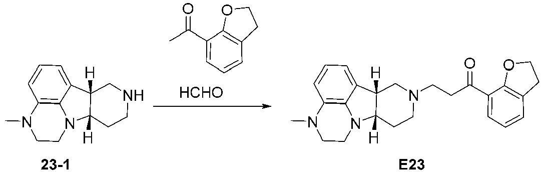

Compound 23-1 (115 mg, 0.5 mmol) was dissolved in EtOH (5 mL), and paraformaldehyde (120 mg, 4.0 mmol) and concentrated hydrochloric acid (0.125 mL, 1.5 mmol) were added. The mixture was heated, stirred, and refluxed for 16 h. After concentration, the residue was purified by normal-phase column chromatography to give the compound 1-(2,3-dihydrobenzofuran-7-yl)-3-((6bR,10aS)-3-methyl-2,3,6b,9,10,10a-hexahydro-1H-pyrido[3'1,2,3-de]quinoxalin-8(7H)-yl)propan-1-one (gray solid, 45 mg, yield: 22%). ¹H NMR (500 MHz, CD₃OD) δ 7.63 (d, *J =* 5.0 Hz, 1H), 7.43 (d, *J =* 10.0 Hz, 1H), 6.91 (t, *J* = 7.5 Hz, 1H), 6.65-6.60 (m, 1H), 6.53-6.49 (m, 1H), 6.43 (d, *J* = 10.0 Hz, 1H), 4.70 (d, *J* = 7.5 Hz, 2H), 3.58-3.50 (m, 3H), 3.41-3.34 (m, 2H), 3.29-3.23 (m, 3H), 3.21-3.14 (m, 2H), 3.11-3.04 (m, 1H), 2.87 (s, 3H), 2.84-2.80 (m, 2H), 2.65-2.58 (m, 2H), 2.22-2.18 (m, 1H), 2.12-2.09 (m, 1H), 2.03-1.97 (m, 1H). MS m/z (ESI): 404.9 [M+H]⁺.

### Example 24: (6bR,10aS)-8-(3-(2,3-Dihydrobenzofuran-7-yl)-3-fluoropropyl)-3-methyl-2,3,6b,7,8,9,10,10a-octahydro-1H-pyrido[3',4':4,5]pyrrolo[1,2,3-de]quinoxaline

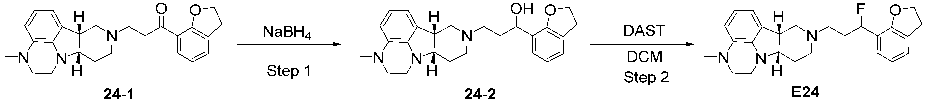

Step 1: The compound 1-(2,3-dihydrobenzofuran-7-yl)-3-((6bR,10aS)-3-methyl-2,3,6b,9,10,10a-hexahydro-1H-pyrido[3',4':4,5]pyrrolo[1,2,3-de]quinoxalin-8(7H)-yl)propan-1-one (25 mg, 0.062 mmol) was dissolved in MeOH (2 mL), and sodium borohydride (10 mg, 0.26 mmol) was added. The mixture was stirred at 20 °C for 1 h, then filtered, and purified and separated by prep-HPLC (eluent (v/v): acetonitrile/(water + 0.05% NH₄HCO₃) = 30%-70%) to give the compound 1-(2,3-dihydrobenzofuran-7-yl)-3-((6bR,10aS)-3-methyl-2,3,6b,9,10,10a-hexahydro-1H-pyrido[3',4':4,5]pyrrolo[1,2,3-de]quinoxalin-8(7H)-yl)propan-1-ol (off-white solid, 12.5 mg, yield: 50%).

Step 2: The compound 1-(2,3-dihydrobenzofuran-7-yl)-3-((6bR,10aS)-3-methyl-2,3,6b,9,10,10a-hexahydro-1H-pyrido[3',4':4,5]pyrrolo[1,2,3-de]quinoxalin-8(7H)-yl)propan-1-ol was dissolved in DCM (2 mL), and the solution was cooled to 0 °C. DAST (10 mg, 0.06 mmol) was added, and the mixture was stirred at 0 °C for 0.5 h, quenched with saturated sodium bicarbonate, and extracted with DCM. After concentration, the residue was purified and separated by prep-HPLC (eluent (v/v): acetonitrile/(water + 0.05% NH₄HCO₃) = 30%-70%) to give the compound (6bR,10aS)-8-(3-(2,3-dihydrobenzofuran-7-yl)-3-fluoropropyl)-3-methyl-2,3,6b,7,8,9,10,10a-octahydro-1H-pyrido[3',4':4,5]pyrrolo[1,2,3-de]quinoxaline (off-white solid, 2.23 mg, yield: 18%). ¹H NMR (500 MHz, CD₃OD) δ 7.17 (d, *J* = 5.0 Hz, 1H), 7.12 (d, *J* = 5.0 Hz, 1H), 6.85 (t, *J* = 7.5 Hz, 1H), 6.60 (t, *J* = 7.5 Hz, 1H), 6.48 (d, *J* = 5.0 Hz, 1H), 6.42 (d, *J* = 10.0 Hz, 1H),5.68-5.59 (m, 2H), 5.25-5.15 (m, 2H), 4.66-4.55 (m, 1H), 3.52-3.48 (m, 1H), 3.34-3.31 (m, 4H), 3.22-3.14 (m, 4H), 2.91-2.73 (m, 5H), 2.67-2.54 (m, 1H), 2.37-2.16 (m, 2H), 2.05-1.93 (m, 2H), MS m/z (ESI): 408.8 [M+H]⁺.

### Example 25: (6bR,10aS)-8-(2-(2,3-Dihydrobenzofuran-3-yl)ethyl)-3-methyl-2,3,6b,7,8,9,10,10a-octahydro-1H-pyrido[3',4':4,5]pyrrolo[1,2,3-de]quinoxaline

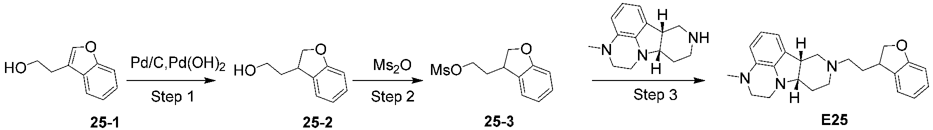

Step 1: The compound 2-(benzofuran-3-yl)ethan-1-ol (150 mg, 1.3 mmol) was added to a 50 mL three-neck reaction flask, and methanol (10 mL), wet palladium on carbon (10%, 0.1 g), and palladium hydroxide on carbon (20%, 0.1 g) were subsequently added. Then, the mixture was heated to 40 °C and stirred for 16 h in a hydrogen atmosphere. After LCMS analysis showed that the reaction had been complete, the reaction mixture was filtered and concentrated, and the resulting residue was purified by column chromatography (petroleum ether:ethyl acetate = 3:1) to give the title compound 2-(2,3-dihydrobenzofuran-3-yl)ethan-1-ol (colorless liquid, 0.1 g, yield: 66%).

Step 2: 2-(2,3-Dihydrobenzofuran-3-yl)ethyl mesylate was prepared with reference to the synthesis method in step 3 of Example 3.

Step 3: (6bR,10aS)-8-(2-(2,3-Dihydrobenzofuran-3-yl)ethyl)-3-methyl-2,3,6b,7,8,9,10,10a-octahydro-1H-pyrido[3',4':4,5]pyrrolo[1,2,3-de]quinoxaline was prepared with reference to the synthesis method in step 4 of Example 3. ¹H NMR (500 MHz, CD₃OD) δ 7.39-7.23 (m, 3H), 7.21-7.02 (m, 1H), 6.94 (t, *J* = 7.8 Hz, 1H), 6.89-6.81 (m, 1H), 6.72 (dd, *J* = 15.4, 7.9 Hz, 1H), 4.65-4.54 (m, 1H), 4.39-4.16 (m, 1H), 3.98-3.81 (m, 2H), 3.76-3.62 (m, 4H), 3.58-3.50 (m, 2H), 3.31 (dd, *J =* 3.2, 1.6 Hz, 4H), 3.29-3.06 (m, 3H), 2.83-2.69 (m, 1H), 2.51-2.19 (m, 3H), 2.17-2.00 (m, 1H). MS m/z (ESI): 376.8 [M+H]⁺.

### Example 26: (6bR,10aS)-8-(2-(Benzofuran-3-yl)ethyl)-3-methyl-2,3,6b,7,8,9,10,10a-octahydro-1H-pyrido[3',4':4,5]pyrrolo[1,2,3-de]quinoxaline

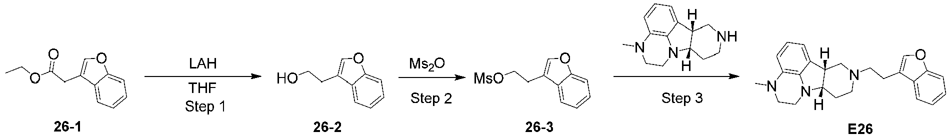

Step 1: The compound ethyl 2-(benzofuran-3-yl)acetate (1000 mg, 4.87 mmol) was added to a 50 mL three-neck reaction flask, and THF (20 mL) was subsequently added. Lithium aluminum hydride (2 mL, 1 mol/L) was slowly added dropwise at 0 °C, and the mixture was stirred at 0 °C for 0.2 h. The reaction solution was quenched with sodium sulfate decahydrate, filtered, and concentrated, and the residue was purified and separated by normal-phase column chromatography (petroleum ether:ethyl acetate = 3:1) to give the compound 2-(benzofuran-3-yl)ethan-1-ol (colorless liquid, 700 mg, yield: 88.7%).

Step 2: The compound 2-(benzofuran-3-yl)ethan-1-ol (200 mg, 1.23 mmol) was added to a 25 mL reaction flask, and DCM (50 mL) was subsequently added. DIEA (635 mg, 4.9 mmol) and Ms₂O (430 mg, 2.47 mmol) were then added sequentially. After the addition, the mixture was stirred at room temperature for 1 h. The reaction mixture was extracted with DCM (2 × 20 mL), and the organic phase was washed with saturated brine (2 × 20 mL) and concentrated to give the compound 2-(benzofuran-3-yl)ethyl mesylate (colorless liquid, 0.25 g, yield: 84.7%).

Step 3: The compound 2-(benzofuran-3-yl)ethyl mesylate (60 mg, 0.25 mmol) and the starting material (6bR,10aS)-3-methyl-2,3,6b,7,8,9,10,10a-octahydro-1H-pyrido[3',4':4,5]pyrrolo[1,2,3-de]quinoxaline (68 mg, 0.3 mmol) were added to a 25 mL reaction flask, and DMSO (5 mL) was subsequently added. Then, DIPEA (0.2 g, 1.58 mmol) was added. After the addition, the mixture was stirred at 60 °C for 16 h. The reaction mixture was added to ethyl acetate (30 mL) and washed with water (30 mL) and brine (50 mL). The organic phase was concentrated, and the resulting residue was preliminarily purified by normal-phase column chromatography (dichloromethane:methanol = 10:1) and then purified by reversed-phase column chromatography (acetonitrile/(water + 0.05% HCl)) to give the compound (6bR,10aS)-8-(2-(benzofuran-3-yl)ethyl)-3-methyl-2,3,6b,7,8,9,10,10a-octahydro-1H-pyrido[3',4':4,5]pyrrolo[1,2,3-de]quinoxaline (gray solid, 20 mg, yield: 21.5%). 1H NMR (500 MHz, CD₃OD) δ 7.73 (s, 1H), 7.70 (d, *J =* 7.4 Hz, 1H), 7.48 (d, *J =* 8.2 Hz, 1H), 7.37-7.13 (m, 4H), 6.93 (s, 1H), 3.81 (s, 2H), 3.67 (d, *J =* 10.7 Hz,3H), 3.52-3.42 (m, 3H), 3.38-3.36 (m, 2H), 3.31 (d, *J* = 1.6 Hz, 3H), 3.27 (d, *J =* 8.3 Hz, 3H), 2.85 (d, *J* = 11.7 Hz, 1H), 2.47 (s, 2H). MS m/z (ESI): 374.6 [M+H]⁺.

### Example 27: 2-(3-((6bR,10aS)-3-Methyl-2,3,6b,9,10,10a-hexahydro-1H-pyrido[3',4':4,5]pyrrolo[1,2,3-de]quinoxalin-8(7H)-yl)propyl)aniline

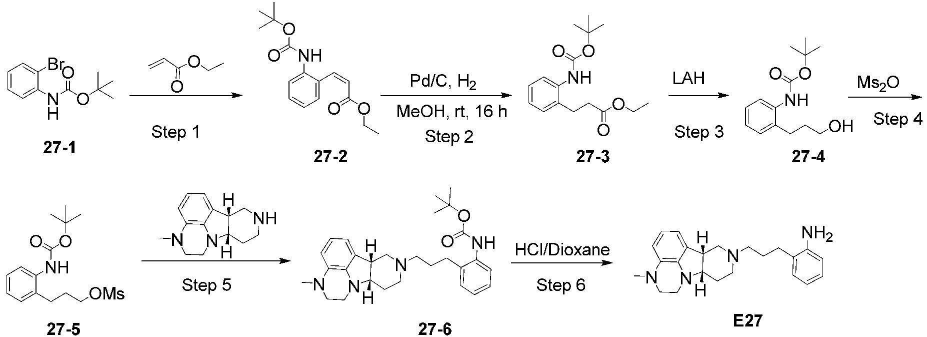

Step 1: The compounds tert-butyl (2-bromophenyl)carbamate (1 g, 3.68 mmol), ethyl acrylate (400 mg, 4.00 mmol), tri(o-tolyl)phosphine (110 mg, 0.36 mmol), palladium acetate (80 mg, 0.36 mmol), and triethylamine (728 mg, 7.19 mmol) were added to a 100 mL reaction flask, and DMF (15 mL) was subsequently added. The mixture was stirred at 100 °C for 12 h. Water (80 mL) was added to the reaction solution, and extraction was performed with ethyl acetate (2 × 80 mL). The organic phase was washed with brine (3 × 80 mL) and then concentrated, and the resulting residue was purified and separated by normal-phase column chromatography (petroleum ether:ethyl acetate = 10:1) to give the compound ethyl (Z)-3-(2-((tert-butoxycarbonyl)amino)phenyl)acrylate (yellow oil, 290 mg, yield: 27%). 1H NMR (500 MHz, DMSO) δ 9.13 (s, 1H), 7.82-7.75 (m, 2H), 7.42-7.35 (m, 1H), 7.34-7.29 (m, 1H), 7.25-7.17 (m, 1H), 6.53 (d, *J* = 16.0 Hz, 1H), 4.19 (q, *J* = 7.1 Hz, 2H), 1.45 (s, 9H), 1.26 (t, *J* = 7.1 Hz, 3H).

Step 2: The compound ethyl (Z)-3-(2-((tert-butoxycarbonyl)amino)phenyl)acrylate (290 mg, 0.995 mmol) was added to a 100 mL reaction flask, and methanol (10 mL) was subsequently added. Then, 10% wet Pd/C (11 mg, 0.0995 mmol) and Pd(OH)₂/C (14 mg, 0.0995 mmol) were sequentially added. After the addition, the mixture was stirred at 60 °C for 3 h in a hydrogen atmosphere. After the reaction solution was filtered, the filtrate was concentrated, and the resulting residue was purified and separated by normal-phase column chromatography (petroleum ether:ethyl acetate = 3:1) to give the compound ethyl 3-(2-((tert-butoxycarbonyl)amino)phenyl)propanoate (white solid, 240 mg, yield: 82%). MS m/z (ESI): 294.4 [M+H]⁺.

Step 3: The compound ethyl 3-(2-((tert-butoxycarbonyl)amino)phenyl)propanoate (140 mg, 0.48 mmol) was added to a 50 mL three-neck flask, and anhydrous tetrahydrofuran (5 mL) was subsequently added. Then, a 1 M solution of LiAlH₄ in tetrahydrofuran (0.95 mL, 0.95 mmol, 1 M) was added at 0 °C in a nitrogen atmosphere. After the addition, the mixture was stirred at room temperature for 2 h. Sodium sulfate decahydrate (50 mg) was added to the reaction solution. After 10 min of stirring at room temperature, the reaction solution was filtered, and the filtrate was concentrated. The resulting residue was purified and separated by normal-phase column chromatography (petroleum ether:ethyl acetate = 3:1) to give the compound tert-butyl (2-(3-hydroxypropyl)phenyl)carbamate (colorless oil, 100 mg, yield: 83%).

Step 4: The compound tert-butyl (2-(3-hydroxypropyl)phenyl)carbamate (100 mg, 0.40 mmol) was added to a 50 mL reaction flask, and dichloromethane (4 mL) was subsequently added. Then, DIEA (155 mg, 1.20 mmol), DMAP (25 mg, 0.20 mmol), and Ms₂O (174 mg, 1.00 mmol) were added. After 1 h of stirring at room temperature, water (10 mL) was added to the reaction solution, and extraction was performed with dichloromethane (10 mL × 2). The organic phase was washed twice with saturated brine (10 mL), dried over anhydrous sodium sulfate, and then concentrated to give the compound 3-(2-(((tert-butoxycarbonyl)amino)phenyl)propyl mesylate (yellow oil, 100 mg, yield: 77%). MS m/z (ESI): 230.5 [M+H-Boc]⁺.

Step 5: The compound 3-(2-(((tert-butoxycarbonyl)amino)phenyl)propyl mesylate (100 mg, 0.30 mmol) was added to a 50 mL reaction flask, and DMSO (4 mL) was subsequently added. Then, DIEA (118 mg, 0.91 mmol) and (6bR,10aS)-3-methyl-2,3,6b,7,8,9,10,10a-octahydro-1H-pyrido[3',4':4,5]pyrrolo[1,2,3-de]quinoxaline (83 mg, 0.36 mmol) were added. After 12 h of stirring at 60 °C, water (10 mL) was added to the reaction solution, and extraction was performed with ethyl acetate (10 mL × 2). The organic phase was washed twice with saturated brine (10 mL), then dried over anhydrous sodium sulfate, and concentrated, and the residue was purified and separated by normal-phase column chromatography (dichloromethane:methanol = 10:1) to give the compound tert-butyl (2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenyl)carbamate (white solid, 80 mg, yield: 57%). MS m/z (ESI): 463.8 [M+H]⁺.

Step 6: The compound tert-butyl (2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenyl)carbamate (20 mg, 0.043 mmol) was dissolved in HCl/dioxane (2 mL), and the solution was stirred at 25 °C for 1 h. After the reaction solution was concentrated to dryness by rotary evaporation, the residue was dissolved in MeOH (2 mL), and the solution was then purified by reversed-phase preparative chromatography (eluent (v/v): acetonitrile/(water + 0.05% NH₄HCO₃)). Acetonitrile was removed under reduced pressure, and the residue was lyophilized to give the compound 2-(3-(6bR,10aS)-3-methyl-2,3,6b,9,10,10a-hexahydropyrido[3',4':4,5]pyrrolo[1,2,3-de]quinoxaline-8(7H)-propyl)aniline (yellow solid, 7 mg, yield: 25%). ¹H NMR (500 MHz, DMSO) δ 6.93-6.83 (m, 2H), 6.59 (dd, *J* = 7.9, 1.3 Hz, 1H), 6.54-6.40 (m, 3H), 6.33 (d, *J* = 7.8 Hz, 1H), 4.98 (s, 2H), 3.44-3.40 (m, 1H), 3.31-3.25 (m, 2H), 3.16-3.10 (m, 1H), 3.08-2.99 (m, 1H), 2.78 (s, 4H), 2.72-2.65 (m, 1H), 2.62 (d, *J =* 8.9 Hz, 1H), 2.43 (t, *J =* 7.3 Hz, 2H), 2.18 (d, *J =* 64.8 Hz, 3H), 1.96-1.88 (m, 1H), 1.84-1.74 (m, 2H), 1.72-1.63 (m, 2H). MS m/z (ESI): 363.8 [M+H]⁺.

### Example 28: N-(2-(3-(6bR,10aS)-3-Methyl-2,3,6b,9,10,10a-hexahydro-1H-pyrido[3',4':4,5]pyrrolo[1,2,3-de]quinoxaline-8(7H)-propyl)propyl)phenyl)acetamide

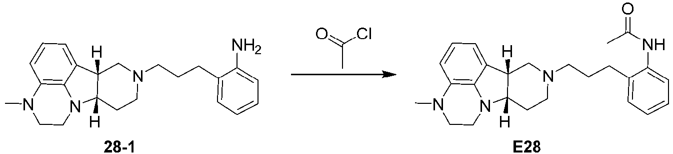

The compounds 2-(3-(6bR,10aS)-3-methyl-2,3,6b,9,10,10a-hexahydropyrido[3',4':4,5]pyrrolo[1,2,3-de]quinoxaline-8(7H)-propyl)aniline (20 mg, 0.055 mmol), DIEA (22 mg, 0.17 mmol), and acetyl chloride (5 mg, 0.066 mmol) were dissolved in dichloromethane (3 mL), and the solution was stirred at 25 °C for 2 h. Water (10 mL) was added to the reaction solution, and extraction was performed with dichloromethane (2 × 10 mL). After the organic phase was concentrated, the residue was dissolved in MeOH (2 mL), and the solution was then purified by reversed-phase preparative chromatography (eluent (v/v): acetonitrile/(water + 0.05% NH₄HCO₃)). Acetonitrile was removed under reduced pressure, and the residue was lyophilized to give the compound N-(2-(3-(6bR,10aS)-3-methyl-2,3,6b,9,10,10a-hexahydro-1H-pyrido[3',4":4,5]pyrrolo[1,2,3-de]quinoxaline-8(7H)-propyl)propyl)phenyl)acetamide (yellow oil, 5 mg, yield: 22%). ¹H NMR (500 MHz, DMSO) δ 9.42 (s, 1H), 7.32 (dd, *J* = 7.7, 1.5 Hz, 1H), 7.22 (dd, *J* = 7.3, 1.8 Hz, 1H), 7.13 (m, 2H), 6.52 (t, *J =* 7.6 Hz, 1H), 6.43 (d, *J* = 7.3 Hz, 1H), 6.34 (d, *J =* 7.9 Hz, 1H), 3.46-3.40 (m, 1H), 3.32 (d, *J =* 3.7 Hz, 2H), 3.27 (m, 2H), 3.13 (s, 2H), 2.78 (s, 3H), 2.68 (m, 1H), 2.57 (t, *J =* 7.6 Hz, 2H), 2.19 (d, *J =* 31.3 Hz, 2H), 2.04 (s, 3H), 1.95-1.62 (m, 5H), 1.23 (s, 1H). MS m/z (ESI): 405.9 [M+H]⁺.

### Example 29: 1-(2-(3-((6bR,10aS)-3-Methyl-2,3,6b,9,10,10a-hexahydro-1H-pyrido[3',4':4,5]pyrrolo[1,2,3-de]quinoxalin-8(7H)-yl)propyl)phenyl)ethan-1-one

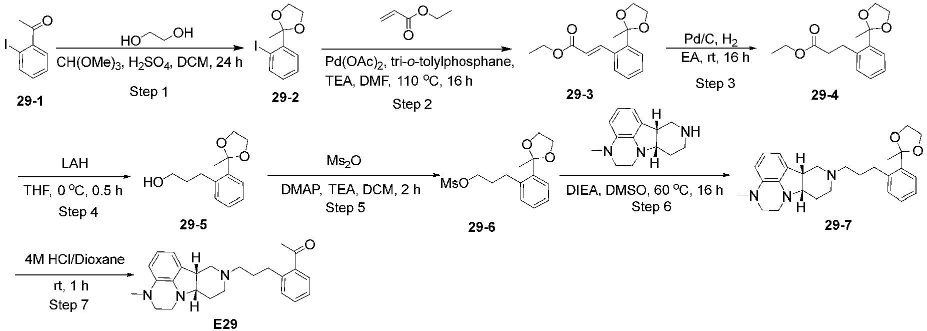

Step 1: The compounds 1-(2-iodophenyl)ethan-1-one (500 mg, 2.03 mmol), trimethyl orthoformate (1 mL), and anhydrous ethylene glycol (2 mL) were added together to a 25 mL three-neck flask, and anhydrous dichloromethane (10 mL) was subsequently added. The mixture was stirred for 0.5 h in an ice-water bath in a nitrogen atmosphere. Finally, concentrated sulfuric acid (0.5 mL) was slowly added dropwise. After the dropwise addition, the mixture was stirred at room temperature for 24 h. After TLC analysis showed that the reaction had been complete, a saturated NaHCO₃ solution was slowly added dropwise to the reaction solution to make the system alkaline, and extraction was subsequently performed with DCM (3 × 20 mL). The organic phases were combined, washed with saturated brine (1 × 20 mL), dried over anhydrous sodium sulfate, and concentrated to dryness by rotary evaporation under reduced pressure, and the crude product was purified and separated by normal-phase column chromatography (eluent gradient: 5% EA/PE) to give the compound 2-(2-iodophenyl)-2-methyl-1,3-dioxolane (pale yellow oil, 350 mg, yield: 59%).

Step 2: The compounds 2-(2-iodophenyl)-2-methyl-1,3-dioxolane (350 mg, 1.20 mmol), ethyl acrylate (360 mg, 3.60 mmol), Pd(OAc)2 (13 mg, 0.06 mmol), tri(o-tolyl)phosphine (18 mg, 0.06 mmol), and triethylamine (360 mg, 3.60 mmol) were added together to a 25 mL single-neck reaction flask, and anhydrous DMF (5 mL) was subsequently added. After the addition, the mixture was purged 3 times with nitrogen, slowly heated to 100 °C, and stirred for 16 h. After TLC analysis showed that the reaction had been complete, the reaction solution was filtered. Subsequently, water (20 mL) was added to the reaction solution, and extraction was performed with ethyl acetate (3 × 20 mL). The organic phases were combined, washed with saturated brine (1 × 20 mL), dried over anhydrous sodium sulfate, and concentrated to dryness by rotary evaporation under reduced pressure, and the crude product was purified and separated by normal-phase column chromatography (eluent gradient: 3% EA/PE) to give the compound ethyl (E)-3-(2-(2-methyl-1,3-dioxolan-2-yl)phenyl)acrylate (pale yellow oil, 200 mg, yield: 63%).

Step 3: The compound ethyl (E)-3-(2-(2-methyl-1,3-dioxolan-2-yl)phenyl)acrylate (200 mg, 0.76 mmol) was added to a 25 mL single-neck reaction flask, and subsequently, ethyl acetate (15 mL) and 10% Pd/C (wet basis, 20 mg) were separately added. After the addition, the mixture was purged 3 times with hydrogen and stirred at room temperature for 0.5 h. After TLC analysis showed that the reaction had been complete, the reaction solution was filtered under vacuum, and the filtrate was concentrated under reduced pressure to give the compound ethyl 3-(2-(2-methyl-1,3-dioxolan-2-yl)phenyl)propanoate (crude, 200 mg, yield: 99%). The crude product was directly used in the next step without further purification.

Step 4: 3-(2-(2-Methyl-1,3-dioxolan-2-yl)phenyl)propan-1-ol was prepared with reference to the synthesis method in step 1 of Example 26.

Step 5: The compounds 3-(2-(2-methyl-1,3-dioxolan-2-yl)phenyl)propan-1-ol (35 mg, 0.16 mmol), DMAP (2 mg, 0.02 mmol), DIEA (62 mg, 0.48 mmol), and anhydrous dichloromethane (5 mL) were added together to a 25 mL three-neck reaction flask, and methanesulfonic anhydride (55 mg, 0.32 mmol) was slowly added under nitrogen and ice-water bath conditions. After the addition, the mixture was stirred at room temperature for 2 h. After TLC analysis showed that the reaction had been complete, water (10 mL) was added to the reaction solution, and extraction was performed with dichloromethane (2 × 10 mL). The organic phases were combined, washed with saturated brine (1 × 10 mL), dried over anhydrous sodium sulfate, and concentrated to dryness by rotary evaporation under reduced pressure to give the compound 3-(2-(2-methyl-1,3-dioxolan-2-yl)phenyl)propyl mesylate (colorless oil, crude, 50 mg). The crude product was directly used in the next step without further purification.

Step 6: (6bR,10aS)-3-Methyl-8-(3-(2-(2-methyl-1,3-dioxolan-2-yl)phenyl)propyl)-2,3,6b,7,8,9,10,10a-octahydro-1H-pyrido[3',4':4,5]pyrrolo[1,2,3-de]quinoxaline was prepared with reference to the synthesis method in step 3 of Example 26.

Step 7: The compound (6bR,10aS)-3-methyl-8-(3-(2-(2-methyl-1,3-dioxolan-2-yl)phenyl)propyl)-2,3,6b,7,8,9,10,10a-octahydro-1H-pyrido[3',4':4,5]pyrrolo[1,2,3-de]quinoxaline (5 mg, 0.75 mmol) was added to a 10 mL single-neck reaction flask, and 4 M HCl/dioxane (1 mL) was subsequently added. After the addition, the mixture was stirred at room temperature for 1.0 h. After LCMS analysis showed that the reaction had been complete, the solvent was removed under reduced pressure, the crude product was dissolved in MeOH (1 mL), and the solution was directly separated by reversed-phase preparative chromatography (C18 column, eluent gradient: acetonitrile/(water + 0.05% NH₄HCO₃)) to give the compound 1-(2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenyl)ethan-1-one (white solid, 3.0 mg, yield: 66%). 1H NMR (500 MHz, MeOD) δ 7.78 (d, *J* = 7.3 Hz, 1H), 7.49-7.39 (m, 1H), 7.31 (t, *J =* 7.4 Hz, 2H), 6.59 (t, *J =* 7.6 Hz, 1H), 6.48 (d, *J =* 7.2 Hz, 1H), 6.41 (d, *J =* 7.6 Hz, 1H), 4.61 (s, 1H), 3.54-3.47 (m, 1H), 3.38-3.32 (m, 2H), 3.27 (t, *J =* 2.8 Hz, 1H), 3.18-3.06 (m, 2H), 2.92-2.86 (m, 1H), 2.85-2.80 (m, 5H), 2.78-2.72 (m, 2H), 2.57-2.53 (m, 1H), 2.48-2.42 (m, 2H), 2.30 (td, *J=* 12.3, 3.0 Hz, 1H), 2.04-1.99 (m, 1H), 1.96-1.88 (m, 2H), 1.84-1.76 (m, 2H). MS m/z (ESI): 390.4 [M+H]⁺.

### Example 30: 3-(2-((6bR,10aS)-3-Methyl-2,3,6b,9,10,10a-hexahydro-1H-pyrido[3',4':4,5]pyrrolo[1,2,3-de]quinoxalin-8(7H)-yl)ethyl)benzo[d]isoxazole

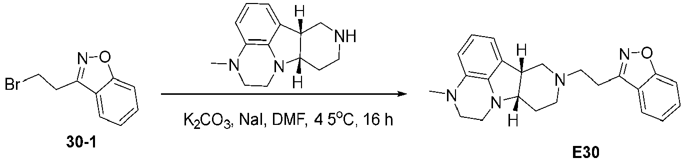

The compounds 3-(2-bromoethyl)benzo[d]isoxazole (15 mg, 0.07 mmol) and (6bR,10aS)-3-methyl-2,3,6b,7,8,9,10,10a-octahydro-1H-pyrido[3',4':4,5]pyrrolo[1,2,3-de]quinoxaline (15 mg, 0.07 mmol) were added to a 10 mL reaction flask, and subsequently, anhydrous DMF (2 mL), K₂CO₃ (19 mg, 0.14 mmol), and NaI (10 mg, 0.07 mmol) were separately added. After the addition, the mixture was slowly heated to 45 °C and stirred for 16 h. After the reaction was complete, the reaction mixture was filtered, and the filtrate was directly separated by reversed-phase preparative chromatography (C18 column, eluent gradient: acetonitrile/(water + 0.05% NH₄HCO₃)) to give the compound 3-(2-((6bR,10aS)-3-methyl-2,3,6b,9,10,10a-hexahydro-1H-pyrido[3',4':4,5]pyrrolo[1,2,3-de]quinoxalin-8(7H)-yl)ethyl)benzo[d]isoxazole (pale yellow oil, 8 mg, yield: 33%). ¹H NMR (500 MHz, CDCl₃) *δ* 7.69 (d, *J* = 7.9 Hz, 1H), 7.59-7.51 (m, 2H), 7.30 (t, *J* = 7.1 Hz, 1H), 6.70-6.64 (m, 1H), 6.54 (d, *J* = 7.3 Hz, 1H), 6.42 (d, *J =* 7.9 Hz, 1H), 3.66-3.59 (m, 1H), 3.34-3.20 (m, 6H), 3.06-2.98 (m, 1H), 2.93-2.81 (m, 7H), 2.50-2.40 (m, 1H), 2.16 (t, *J =* 10.8 Hz, 1H), 2.02-1.96 (m, 2H). MS m/z (ESI): 375.6 [M+H]⁺.

### Example 31: 3-((6bR,10aS)-3-Methyl-2,3,6b,9,10,10a-hexahydro-1H-pyrido[3',4':4,5]pyrrolo[1,2,3-de]quinoxalin-8(7H)-yl)-1-phenylpropan-1-ol

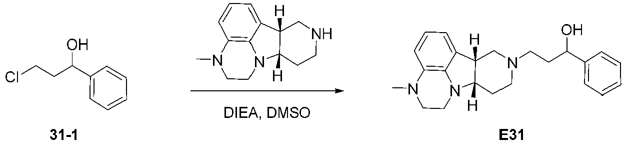

At room temperature, (6bR,10aS)-3-methyl-2,3,6b,7,8,9,10,10a-octahydro-1H-pyrido[3',4':4,5]pyrrolo[1,2,3-de]quinoxaline (18 mg, 0.078 mmol) was dissolved in DMSO (2.0 mL), and DIEA(0.5 mL, 3.0 mmol) and 3-chloro-1-phenylpropan-1-ol (14 mg, 0.08 mmol) were sequentially added. The mixture was heated to 65 °C and stirred for 18 h, cooled to room temperature, filtered, and then purified and separated by prep-HPLC (eluent (v/v): acetonitrile/(water + 0.05% NH₄HCO₃) = 30%-70%) to give the compound 3-((6bR,10aS)-3-methyl-2,3,6b,9,10,10a-hexahydro-1H-pyrido[3',4':4,5]pyrrolo[1,2,3-de]quinoxalin-8(7H)-yl)-1-phenylpropan-1-ol (pale yellow oil, 9.8 mg, yield: 33%). ¹H NMR (500 MHz, DMSO-*d*₆) δ 7.34-7.30 (m, 4H), 7.23-7.20 (m, 1H), 6.52 (t, *J* = 7.5 Hz, 1H), 6.43 (d, *J =* 5.0 Hz, 1H), 6.33 (d, *J =* 5.0 Hz, 1H), 4.63-4.61 (m, 1H), 3.33-3.26 (m, 3H), 3.13-3.11 (m, 1H), 3.05-3.00 (m, 1H), 2.83-2.80 (m, 1H), 2.78 (s, 3H), 2.71-2.64 (m, 2H), 2.45-2.30 (m, 2H), 2.15-2.08 (m, 1H), 1.93-1.88 (m, 1H), 1.82-1.72 (m, 4H). MS m/z (ESI): 364.7 [M+H]⁺.

### Example 32: (6bR,10aS)-3-Methyl-8-(3-(2-(methylthio)phenyl)propyl)-2,3,6b,7,8,9,10,10a-octahydro-1H-pyrido[3',4':4,5]pyrrolo[1,2,3-de]quinoxaline

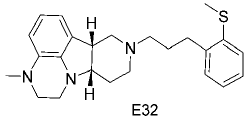

E32 was prepared with reference to the synthesis method of Example 26. ¹H NMR (500 MHz, CDCl₃) δ7.18 (dd, *J =* 4.5,1.0 Hz 2H), 7.14(d, *J =* 7.5, Hz 1H), 7.10~7.06(m, 1H), 6.64(t, *J =* 7.5 Hz 1H), 6.52 (d, *J =* 7.0 Hz 1H), 6.40(d, *J =* 8.0 Hz 1H), 3.62-3.57 (m, 1H), 3.32-3.17 (m, 4H), 32.93-2.80 (m, 5H), 2.71(t, *J* = 7.5 Hz, 3H), 2.46-2.41 (m, 5H), 2.27-2.20(m, 1H), 1.99-1.95 (m, 3H), 1.88-1.82 (m, 2H). MS m/z (ESI): 394.7 [M+H]⁺.

### Example 33: (6bR,10aS)-3-Methyl-8-(3-(2-(methylsulfonyl)phenyl)propyl)-2,3,6b,7,8,9,10,10a-octahydro-1H-pyrido[3',4':4,5]pyrrolo[1,2,3-de]quinoxaline

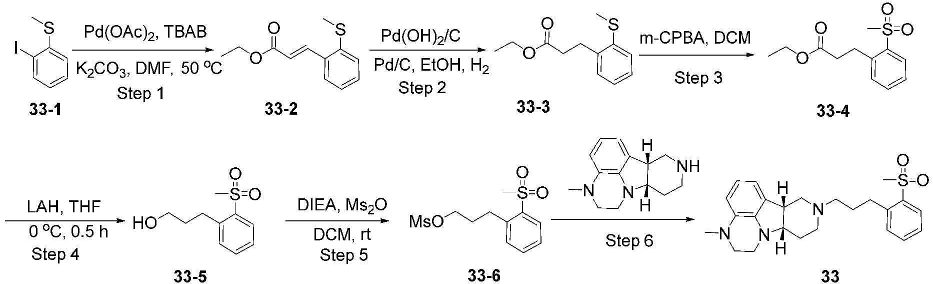

Step 1: The compounds (2-iodophenyl)(methyl)sulfane 33-1 (1 g, 4 mmol), ethyl acrylate (1 g, 10 mmol), Pd(OAc)₂ (50 mg, 0.22 mmol), K₂CO₃ (1.36 g, 9.86 mmol), and TBAB (1.32 g, 4.09 mmol) were added to a three-neck flask containing 10 mL of DMF, and the mixture was purged three times with nitrogen and left to react at 50 °C for 16 h. After TLC analysis showed that the reaction had been complete, water and EA were added, and the aqueous phase was isolated and extracted twice with EA. The organic phases were combined, washed three times with saturated sodium chloride, dried over anhydrous sodium sulfate, filtered, and concentrated, and the resulting residue was purified by normal-phase column chromatography (PE/EA = 100%-5/1) to give the compound ethyl (E)-3-(2-(methylthio)phenyl)acrylate (920 mg, yield: 90%).

Step 2: Ethyl (E)-3-(2-(methylthio)phenyl)acrylate (800 mg, 3.6 mmol), 50 mg of 10% Pd/C, and 50 mg of Pd(OH)₂/C were added to a three-neck flask containing 30 mL of THF and 30 mL of EtOH, and the mixture was purged three times with hydrogen and left to react at room temperature for 16 h. After TLC and LCMS analysis showed that the reaction had been complete, the reaction mixture was filtered and concentrated to give the compound ethyl 3-(2-(methylthio)phenyl)propanoate (crude, 860 mg). MS m/z (ESI): 225.17 [M+H]⁺.

Step 3: Ethyl 3-(2-(methylthio)phenyl)propanoate (300 mg, 1.34 mmol) was added to a single-neck flask containing 5 mL of DCM, and m-CPBA (231 mg, 1.14 mmol) was then added. The mixture was left to react at room temperature for 16 h, and TLC analysis showed that the reaction had been complete. The reaction mixture was washed three times with saturated NaHCO₃. The organic phase was isolated, dried, filtered, and concentrated, and the resulting residue was purified by normal-phase column chromatography (PE/EA = 100%-5/1) to give the compound ethyl 3-(2-(methylsulfonyl)phenyl)propanoate (150 mg, yield: 49%).

Step 4: Ethyl 3-(2-(methylsulfonyl)phenyl)propanoate (116 mg, 0.45 mmol) was added to a three-neck flask containing 4 mL of THF. The mixture was purged three times with nitrogen and cooled to 0 °C, and 0.9 mL of a 1 M solution of LAH in THF was injected. Then, the mixture was left to react for 1 h, and TLC analysis showed that the reaction had been complete. Sodium sulfate decahydrate was added to quench the reaction, and EA and Na₂SO₄ were then added. The mixture was stirred at room temperature for half an hour, filtered, and concentrated to give the compound 3-(2-(methylsulfonyl)phenyl)propan-1-ol (70 mg, yield: 72%).

Step 5: 3-(2-(Methylsulfonyl)phenyl)propyl mesylate was prepared with reference to the synthesis method in step 3 of Example 3.

Step 6: (6bR,10aS)-3-Methyl-8-(3-(2-(methylsulfonyl)phenyl)propyl)-2,3,6b,7,8,9,10,10a-octahydro-1H-pyrido[3',4':4,5]pyrrolo[1,2,3-de]quinoxaline was prepared with reference to the synthesis method in step 4 of Example 3. ¹H NMR (500 MHz, CDCl₃) δ8.03 (dd, *J* = 8.0,1.0 Hz 1H), 7.58~7.55 (m, 1H), 7.42~7.37 (m, 2H), 6.66 (t, *J* = 7.5 Hz 1H), 6.52 (d, *J* = 7.0 Hz 1H), 6.41 (d, *J* = 8.0 Hz 1H), 3.61-3.56 (m, 1H), 3.31-3.25 (m, 4H), 3.10-3.05 (m, 6H), 2.86-2.81 (m, 5H), 2.58 (s, 2H), 2.43 (s, 1H) 2.14-1.97 (m, 5H). MS m/z (ESI): 426.8 [M+H]⁺.

### Example 34: (6bR,10aS)-8-(3-Cyclohexylpropyl)-3-methyl-2,3,6b,7,8,9,10,10a-octahydro-1H-pyrido[3',4':4,5]pyrrolo[1,2,3-de]quinoxaline

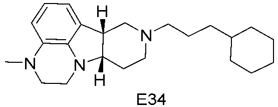

E34 was prepared with reference to the synthesis method of Example 20. ¹H NMR (500 MHz, DMSO-*d*₆) δ 6.63 (t, *J* = 7.6 Hz, 1H), 6.58 (d, *J* = 7.0 Hz, 1H), 6.52 (s, 1H), 3.52-3.47 (m, 2H), 3.47-3.42 (m, 2H), 3.37 (d, *J* = 10.0 Hz, 2H), 3.02-2.94 (m, 2H), 2.85 (d, *J* = 5.8 Hz, 3H), 2.22 (t, *J =* 14.8 Hz, 2H), 1.77-1.57 (m, 9H), 1.33-1.03 (m, 8H), 0.85 (dd, *J =* 21.5, 10.5 Hz, 2H). MS m/z (ESI): 348.6 [M+H]⁺.

### Example 35: (6bR,10aS)-3-Methyl-8-(3-(piperidin-1-yl)propyl)-2,3,6b,7,8,9,10,10a-octahydro-1H-pyrido[3',4':4,5]pyrrolo[1,2,3-de]quinoxaline

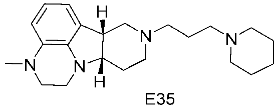

E35 was prepared with reference to the synthesis method of Example 20. ¹H NMR (500 MHz, DMSO-*d*₆) δ 6.51 (t, *J =* 7.5 Hz, 1H), 6.42 (d, *J* = 7.2 Hz, 1H), 6.34 (d, *J* = 7.5 Hz, 1H), 3.32-3.22 (m, 3H), 3.11 (dd, *J =* 4.2, 2.3 Hz, 1H), 3.03-2.97 (m, 1H), 2.82-2.76 (m, 4H), 2.71-2.66 (m, 1H), 2.60 (d, *J* = 11.0 Hz, 1H), 2.52 (t, *J* = 4.0 Hz, 2H), 2.48-2.33 (m, 4H), 2.31-2.20 (m, 2H), 2.11 (t, *J* = 10.5 Hz, 1H), 1.90 (dd, *J* = 14.5, 2.5 Hz, 1H), 1.80-1.72 (m, 2H), 1.61 (s, 2H), 1.56-1.47 (m, 4H), 1.37 (d, *J* = 24.9 Hz, 2H). MS m/z (ESI): 355.7 [M+H]⁺.

### Example 36: 3-(6bR,10aS)-3-Methyl-2,3,6b,9,10,10a-hexahydropyrido[3',4':4,5]pyrrolo[1,2,3-de]quinoxalin-8(7H)-yl)-1-(piperidin-1-yl)propan-1-one

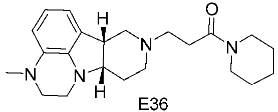

Compound E36 was prepared with reference to the synthesis method of Example 20. ¹H NMR (500 MHz, DMSO) δ 6.53 (t, *J =* 7.6 Hz, 1H), 6.44 (d, *J* = 7.2 Hz, 1H), 6.35 (d, *J* = 7.9 Hz, 1H), 3.50-3.37 (m, 9H), 3.30-3.25 (m, 2H), 3.12 (s, 2H), 2.79 (s, 3H), 2.72-2.63 (m, 2H), 2.57 (s, 2H), 2.13-1.69 (m, 3H), 1.56 (q, *J =* 6.2 Hz, 2H), 1.51-1.45 (m, 2H), 1.42-1.36 (m, 2H). MS m/z (ESI): 369.9 [M+H]⁺.

### Example 37: 2-(((6bR,10aS)-3-Methyl-2,3,6b,9,10,10a-hexahydro-1H-pyrido[3',4':4,5]pyrrolo[1,2,3-de]quinoxalin-8(7H)-yl)methyl)benzo[d]oxazole

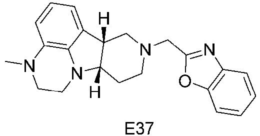

E37 was prepared with reference to the synthesis method of Example 30. ¹H NMR (500 MHz, CDCl₃) δ 7.74-7.68 (m, 1H), 7.58-7.50 (m, 1H), 7.36-7.30 (m, 2H), 6.64 (t, *J =* 7.6 Hz, 1H), 6.50 (d, *J =* 7.3 Hz, 1H), 6.40 (d, *J =* 7.9 Hz, 1H), 3.94-3.81 (m, 2H), 3.60 (td, *J =* 10.8, 2.8 Hz, 1H), 3.33-3.19 (m, 4H), 3.06-2.96 (m, 1H), 2.86 (s, 3H), 2.83-2.75 (m, 2H), 2.50 (td, *J* = 11.5, 3.3 Hz, 1H), 2.19 (t, *J* = 11.0 Hz, 1H), 2.05-1.93 (m, 2H). MS m/z (ESI): 361.6 [M+H]⁺.

### Example 38: 2-(2-((6bR,10aS)-3-Methyl-2,3,6b,9,10,10a-hexahydro-1H-pyrido[3',4':4,5]pyrrolo[1,2,3-de]quinoxalin-8(7H)-yl)ethyl)benzo[d]oxazole

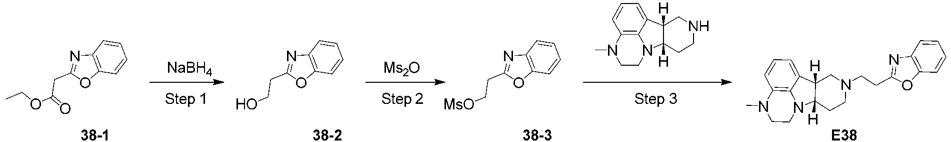

Step 1: The compound ethyl 2-(benzoxazol-2-yl)acetate (60 mg, 0.29 mmol) was dissolved in methanol (3 mL), and sodium borohydride (55 mg, 1.45 mmol) was added at 0 °C. The mixture was stirred at room temperature for 2 h. The reaction solution was added to water (10 mL) at 0 °C and extracted with ethyl acetate (10 mL × 2). The organic phase was dried over anhydrous sodium sulfate, then filtered, and concentrated, and the resulting residue was purified and separated by normal-phase column chromatography (petroleum ether:ethyl acetate = 3:1) to give the title compound 2-(benzo[d]oxazol-2-yl)ethan-1-ol (yellow oil, 30 mg, yield: 64%). MS m/z (ESI): 164.4 [M+H]⁺.

Step 2: 2-(Benzo[d]oxazol-2-yl)ethyl mesylate was prepared with reference to the synthesis method in step 3 of Example 3.

Step 3: 2-(2-((6bR,10aS)-3-Methyl-2,3,6b,9,10,10a-hexahydro-1H-pyrido[3',4':4,5]pyrrolo[1,2,3-de]quinoxalin-8(7H)-yl)ethyl)benzo[d]oxazole was prepared with reference to the synthesis method in step 4 of Example 3. ¹H NMR (500 MHz, DMSO) δ 7.73-7.62 (m, 2H), 7.38-7.30 (m, 2H), 6.51 (t, *J* = 7.6 Hz, 1H), 6.42 (d, *J* = 7.2 Hz, 1H), 6.33 (d, *J* = 7.8 Hz, 1H), 3.47-3.41 (m, 1H), 3.31 (t, *J =* 2.9 Hz, 2H), 3.28-3.24 (m, 1H), 3.18-3.05 (m, 3H), 3.00 (q, *J* = 6.3 Hz, 1H), 2.90-2.80 (m, 2H), 2.78 (s, 3H), 2.74-2.61 (m, 2H), 2.23 (s, 1H), 2.07-1.83 (m, 2H), 1.75 (d, *J =* 13.5 Hz, 1H). MS m/z (ESI): 375.8 [M+H]⁺.

### Example 39: (6bR,10aS)-3-Methyl-8-(3-(thiophen-2-yl)propyl)-2,3,6b,7,8,9,10,10a-octahydro-1H-pyrido[3',4':4,5]pyrrolo[1,2,3-de]quinoxaline

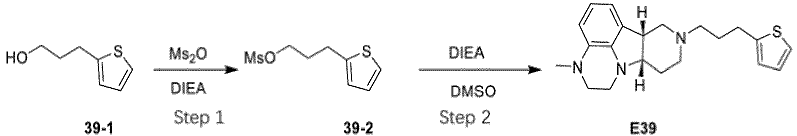

Step 1: 3-(Thiophen-2-yl)propyl mesylate was prepared with reference to the synthesis method in step 3 of Example 3.

Step 2: (6bR,10aS)-3-Methyl-8-(3-(thiophen-2-yl)propyl)-2,3,6b,7,8,9,10,10a-octahydro-1H-pyrido[3',4':4,5]pyrrolo[1,2,3-de]quinoxaline was prepared with reference to the synthesis method in step 4 of Example 3. ¹H NMR (500 MHz, DMSO-*d*₆) δ 7.30 (dd, *J =* 5.0, 1.0 Hz, 1H), 6.95-6.91 (m, 1H), 6.85 (dd, *J* = 3.0, 1.0Hz, 1H), 6.51 (t, *J* = 7.6 Hz, 1H), 6.42 (d, *J* = 7.0 Hz, 1H), 6.33 (d, *J* = 7.6 Hz, 1H), 3.47-3.37 (m, 2H), 3.32-3.23 (m, 2H), 3.11 (d, *J* = 2.0 Hz, 1H), 3.06-2.99 (m, 1H), 2.83-2.79 (m, 2H), 2.78 (s, 3H), 2.67 (ddd, *J =* 20.5, 10.5, 2.5 Hz, 1H), 2.55 (d, *J* = 25.5 Hz, 1H), 2.28 (s, 2H), 2.09 (d, *J* = 16.8 Hz, 1H), 1.90 (d, *J* = 13.1 Hz, 1H), 1.78 (dd, *J =* 18.1, 10.8 Hz, 4H). MS m/z (ESI): 354.7 [M+H]⁺.

Example 40: (6bR,10aS)-8-(3-(3-Methoxythiophen-2-yl)propyl)-3-methyl-2,3,6b,7,8,9,10,10a-octahydro-1H-pyrido[3',4':4,5]pyrrolo[1,2,3-de]quinoxaline

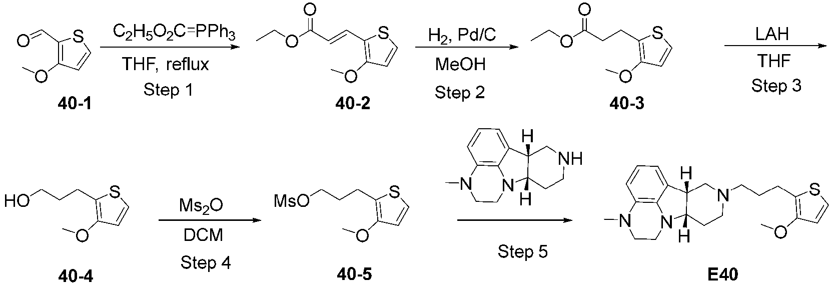

Step 1: The compound 3-methoxythiophene-2-carbaldehyde (350 mg, 2.46 mmol) was dissolved in THF (25.0 mL), and (ethoxycarbonylmethylene)triphenylphosphorane (1.20 g, 3.45 mmol) was added in a nitrogen atmosphere. The mixture was stirred at 75 °C for 18 h. After TLC analysis showed that the reaction had been complete, the reaction mixture was concentrated under reduced pressure, and the resulting residue was purified by normal-phase chromatography to give the compound ethyl (E)-3-(3-methoxythiophen-2-yl)acrylate (yellow oil, 420 mg, yield: 74%). MS m/z (ESI): 213.3 [M+H]⁺.

Step 2: Ethyl (E)-3-(3-methoxythiophen-2-yl)acrylate (270 mg, 1.27 mmol) was dissolved in MeOH (15.0 mL), and 10% Pd/C (135 mg) was added. The compound was hydrogenated at room temperature for 18 h. The reaction mixture was filtered and concentrated to give the compound ethyl 3-(3-methoxythiophen-2-yl)propanoate (colorless oil, 230 mg, yield: 85%). MS m/z (ESI): 215.3 [M+H]⁺.

Step 3: The compound ethyl 3-(3-methoxythiophen-2-yl)propanoate (112 mg, 0.52 mmol) was dissolved in THF (1.0 mL), and the solution was cooled to 0 °C. A 1 M solution of lithium aluminum hydride in tetrahydrofuran (0.5 mL, 0.5 mmol) was added, and the mixture was stirred at room temperature for 1 h. Sodium sulfate decahydrate was added, and the mixture was stirred for 30 min, filtered, and concentrated. The resulting residue was purified by normal-phase chromatography to give the compound 3-(3-methoxythiophen-2-yl)propan-1-ol (colorless oil, 68 mg, yield: 76%). MS m/z (ESI): 173.3 [M+H]⁺.

Step 4: The compound 3-(3-methoxythiophen-2-yl)propan-1-ol (68 mg, 0.40 mmol) was dissolved in DCM (3.0 mL), and DIEA (260 mg, 2.0 mmol) was added. The mixture was cooled to 0 °C. After methanesulfonic anhydride (140 mg, 0.8 mmol) was added, the mixture was heated to 20 °C, stirred for 3 h, quenched with water, and extracted with DCM, and the extract was dried over sodium sulfate and filtered to give the compound 3-(3-methoxythiophen-2-yl)propyl mesylate (yellow oil, 100 mg), which was directly used in the next step.

Step 5: At room temperature, (6bR,10aS)-3-methyl-2,3,6b,7,8,9,10,10a-octahydro-1H-pyrido[3',4':4,5]pyrrolo[1,2,3-de]quinoxaline (55 mg, 0.24 mmol) was dissolved in DMSO (3.0 mL), and DIEA (0.5 mL, 3.0 mmol) and 3-(3-methoxythiophen-2-yl)propyl mesylate (100 mg, 0.4 mmol) were sequentially added. The mixture was heated to 65 °C and stirred for 18 h, cooled to room temperature, filtered, and then purified and separated by prep-HPLC (eluent (v/v): acetonitrile/(water + 0.05% NH₄HCO₃) = 30%-70%) to give the compound (6bR,10aS)-8-(3-(3-methoxythiophen-2-yl)propyl)-3-methyl-2,3,6b,7,8,9,10,10a-octahydro-1H-pyrido[3',4':4,5]pyrrolo[1,2,3-de]quinoxaline (pale yellow oil, 16.0 mg, yield: 17%). 1H NMR (500 MHz, CD₃OD) δ 7.07 (d, *J =* 5.0 Hz, 1H), 6.86 (d, *J =* 5.0 Hz, 1H), 6.60 (t, *J =* 7.5 Hz, 1H), 6.48 (d, *J =* 5.0 Hz, 1H), 6.42 (d, *J =* 5.0 Hz, 1H), 3.79 (s, 3H), 3.53-3.48 (m, 1H), 3.37-3.27 (m, 2H), 3.16-3.10 (m, 2H), 3.00-2.93 (m, 1H), 2.88-2.82 (m, 1H), 2.81 (s, 3H), 2.77-2.65 (m, 3H), 2.53-2.38 (m, 3H), 2.07-1.82 (m, 5H). MS m/z (ESI): 384.9 [M+H]⁺.

### Example 41: (6bR,10aS)-8-(3-(2-Methoxythiophen-3-yl)propyl)-3-methyl-2,3,6b,7,8,9,10,10a-octahydro-1H-pyrido[3',4':4,5]pyrrolo[1,2,3-de]quinoxaline

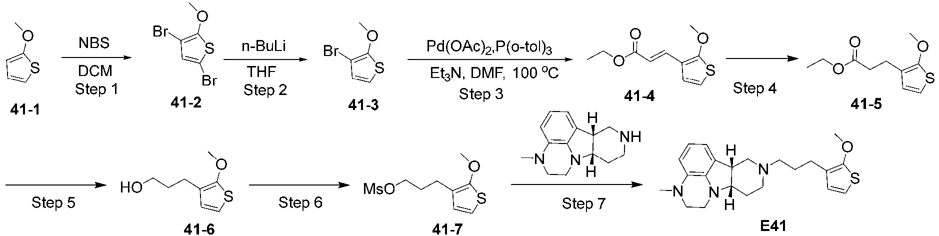

Step 1: The compound 2-methoxythiophene (575 mg, 5.05 mmol) was dissolved in DCM (20.0 mL), and the solution was cooled to 0 °C. NBS (1.78 g, 10.0 mmol) was added, and the mixture was heated to 20 °C, stirred for 3 h, and concentrated under reduced pressure. The residue was purified by normal-phase chromatography to give the compound 3,5-dibromo-2-methoxythiophene (yellow oil, 380 mg, yield: 27%). MS m/z (ESI): 272.4 [M+H]⁺.

Step 2: The compound 3,5-dibromo-2-methoxythiophene (380 mg, 1.40 mmol) was dissolved in THF (5.0 mL), and the solution was cooled to -78 °C. An n-butyllithium solution (0.56 mL, 1.4 mmol) was added, and the mixture was stirred at -78 °C for 30 min. Methanol was added to quench the reaction, and extraction was performed with dichloromethane. The organic phase was washed with saturated brine, dried over anhydrous sodium sulfate, and purified by normal-phase chromatography to give the compound 3-bromo-2-methoxythiophene (yellow oil, 195 mg, yield: 71%). MS m/z (ESI): 194.4 [M+H]⁺.

Step 3: The compound 3-bromo-2-methoxythiophene (190 mg, 1.0 mmol) was dissolved in DMF (5.0 mL), and triethylamine (800 mg, 8.0 mmol), ethyl acrylate (120 mg, 1.2 mmol), tri(o-tolyl)phosphine (60 mg, 0.2 mmol), and palladium acetate (22 mg, 0.1 mmol) were sequentially added in a nitrogen atmosphere. The mixture was stirred at 100 °C for 3 h. After TLC analysis showed that the reaction had been complete, the reaction mixture was quenched with water and extracted with ethyl acetate. The organic phase was washed with saturated brine, dried over anhydrous sodium sulfate, and purified by normal-phase chromatography to give the compound ethyl (E)-3-(2-methoxythiophen-3-yl)acrylate (yellow oil, 48 mg, yield: 23%). MS m/z (ESI): 213.3 [M+H]⁺.

Step 4: The compound ethyl (E)-3-(2-methoxythiophen-3-yl)acrylate (48 mg, 0.23 mmol) was dissolved in MeOH (5.0 mL), and 10% palladium on carbon (30 mg) was added. The mixture was stirred at room temperature for 18 h, filtered, and concentrated to give the compound ethyl 3-(2-methoxythiophen-3-yl)propanoate (yellow oil, 32 mg, yield: 66%). MS m/z (ESI): 215.3 [M+H]⁺.

Step 5: The compound ethyl 3-(2-methoxythiophen-3-yl)propanoate (32 mg, 0.15 mmol) was dissolved in THF (1.5 mL), and the solution was cooled to 0 °C. After a lithium aluminum hydride solution (0.15 mL, 0.15 mmol) was added, the mixture was heated to 20 °C, stirred for 1 h, quenched with water, then dried over sodium sulfate, filtered, and then purified by normal-phase chromatography to give the compound 3-(2-methoxythiophen-3-yl)propan-1-ol (yellow oil, 18 mg, yield: 69%). MS m/z (ESI): 173.3 [M+H]⁺.

Step 6: The compound 3-(2-methoxythiophen-3-yl)propan-1-ol (18 mg, 0.53 mmol) was dissolved in DCM (3.0 mL), and TEA (50 mg, 0.5 mmol) was added. The mixture was cooled to 0 °C. After methanesulfonic anhydride (36 mg, 0.2 mmol) was added, the mixture was heated to 20 °C, stirred for 3 h, quenched with water, and extracted with DCM, and the extract was dried over sodium sulfate and filtered to give the compound 3-(2-methoxythiophen-3-yl)propyl mesylate (colorless oil, 37 mg, yield: 99%), which was directly used in the next step.

Step 7: At room temperature, (6bR,10aS)-3-methyl-2,3,6b,7,8,9,10,10a-octahydro-1H-pyrido[3',4':4,5]pyrrolo[1,2,3-de]quinoxaline (26 mg, 0.11 mmol) was dissolved in DMSO (3.0 mL), and DIEA (195 mg, 1.5 mmol) and the compound 3-(2-methoxythiophen-3-yl)propyl mesylate (37 mg, 0.15 mmol) were sequentially added. The mixture was heated to 65 °C and stirred for 18 h, cooled to room temperature, filtered, and then purified and separated by prep-HPLC (eluent (v/v): acetonitrile/(water + 0.05% NH₄HCO₃) = 30%-70%) to give the compound (6bR,10aS)-8-(3-(2-methoxythiophen-3-yl)propyl)-3-methyl-2,3,6b,7,8,9,10,10a-octahydro-1H-pyrido[3',4':4,5]pyrrolo[1,2,3-de]quinoxaline (yellow solid, 2.0 mg, yield: 5.0%). ¹H NMR (500 MHz, DMSO-*d*₆) δ 7.23 (d, *J =* 5.0 Hz, 1H), 6.95 (d, *J =* 5.0 Hz, 1H), 6.52 (t, *J =* 7.5 Hz, 1H), 6.43 (d, *J =* 10.0 Hz, 1H), 6.33 (d, *J =* 5.0 Hz, 1H), 3.76 (s, 3H), 3.45-3.26 (m, 3H), 3.13-3.01 (m, 2H), 2.80-2.79 (m, 1H), 2.78 (s, 3H), 2.71-2.61 (m, 4H), 2.37-2.03 (m, 3H), 1.96-1.65 (m, 5H). MS m/z (ESI): 384.8 [M+H]⁺.

### Example 42: 8-(3-(2,3-Dihydrobenzofuran-7-yl)propyl)-1,2,6b,7,8,9,10,10a-octahydro-[1,4]oxazino[2,3,4-hi]pyrido[4,3-b]indole

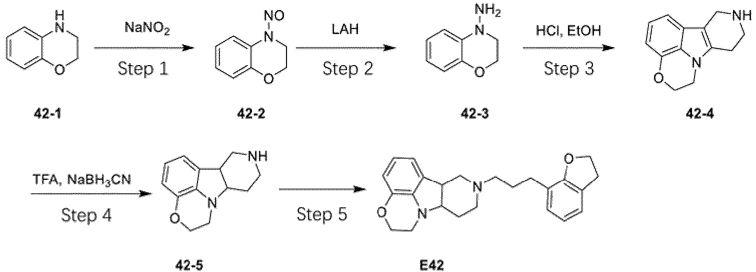

Step 1: The compound benzomorpholine (815 mg, 6.0 mmol) was dissolved in acetic acid (12 mL), and the solution was cooled to 0 °C. An aqueous NaNO₂ solution (414 mg, 6.0 mmol, 4 mL of water) was added with stirring, and the mixture was stirred at 20 °C for 3 h. After TLC analysis showed that the reaction had been complete, the reaction mixture was quenched with water and extracted with DCM. The organic phase was washed with saturated brine, dried over anhydrous sodium sulfate, and concentrated to give the compound 4-nitroso-3,4-dihydro-2H-benzo[b][1,4]oxazine (yellow oil, 680 mg, yield: 69%). MS m/z (ESI): 163.3 [M+H]⁺.

Step 2: Lithium aluminum hydride (158 mg, 4.15 mmol) was suspended in THF (10 mL), and the suspension was cooled to 0 °C. A solution of the compound 4-nitroso-3,4-dihydro-2H-benzo[b][1,4]oxazine (680 mg, 4.15 mmol) in tetrahydrofuran (5 mL) was added with stirring. The mixture was heated to 20 °C, stirred for 18 h, quenched with a 2 M aqueous sodium hydroxide solution, dried over anhydrous sodium sulfate, filtered, and concentrated, and the resulting crude product was purified by normal-phase chromatography to give the compound 4-amino-3,4-dihydro-2H-benzo[b][1,4]oxazine (yellow oil, 413 mg, yield: 67%). MS m/z (ESI): 164.3 [M+H]⁺.

Step 3: The compound 4-amino-3,4-dihydro-2H-benzo[b][1,4]oxazine (413 mg, 2.77 mmol), the compound 4-piperidone hydrochloride (375 mg, 2.78 mmol), and the solvent isopropanol (15 mL) were added to a 25 mL reaction flask, and the mixture was heated to 88 °C and stirred for 2 h. Concentrated HCl (0.23 mL, 2.8 mmol) was added, and the mixture was heated to 88 °C, stirred for 3 h, cooled to room temperature, and filtered under vacuum to give the compound 1,2,7,8,9,10-hexahydro-[1,4]oxazino[2,3,4-hi]pyrido[4,3-b]indole (yellow solid, 335 mg, yield: 55%). MS m/z (ESI): 213.4 [M+H]⁺.

Step 4: The compound 1,2,7,8,9,10-hexahydro-[1,4]oxazino[2,3,4-hi]pyrido[4,3-b]indole (335 mg, 1.55 mmol) was dissolved in TFA (5 mL), and the solution was cooled to 0 °C. NaBH₃CN (296 mg, 4.65 mmol) was added with stirring, and the mixture was stirred at 20 °C for 3 h. After TLC analysis showed that the reaction had been complete, a saturated sodium bicarbonate solution was added, and extraction was performed with DCM. The organic phase was washed with saturated brine, dried over anhydrous sodium sulfate, filtered, and concentrated, and the resulting crude product was purified by normal-phase chromatography to give the compound 1,2,6b,7,8,9,10,10a-octahydro-[1,4]oxazino[2,3,4-hi]pyrido[4,3-b]indole (pale yellow solid, 115 mg, yield: 66%). MS m/z (ESI): 215.3 [M+H]⁺.

Step 5: At room temperature, 1,2,6b,7,8,9,10,10a-octahydro-[1,4]oxazino[2,3,4-hi]pyrido[4,3-b]indole (40 mg, 0.185 mmol) was dissolved in DMSO (1.5 mL), and DIEA (0.5 mL, 3.0 mmol) and 3-(2,3-dihydrobenzofuran-7-yl)propyl mesylate (52 mg, 0.20 mmol) were sequentially added. The mixture was heated to 65 °C, stirred for 18 h, filtered, and then purified and separated by prep-HPLC (eluent (v/v): acetonitrile/(water + 0.05% NH4HCO3) = 30%-70%) to give the compound 8-(3-(2,3-dihydrobenzofuran-7-yl)propyl)-1,2,6b,7,8,9,10,10a-octahydro-[1,4]oxazino[2,3,4-hi]pyrido[4,3-b]indole (white solid, 14.0 mg, yield: 20%). 1H NMR (500 MHz, DMSO-d6) δ 7.05 (d, *J =* 5.0 Hz, 1H), 6.92 (d, *J =* 5.0 Hz, 1H), 6.73 (t, *J =* 7.5 Hz, 1H), 6.67 (d, *J =* 10.0 Hz, 1H), 6.57-6.50 (m, 2H), 4.49 (t, *J =* 7.5 Hz, 2H), 4.42-4.39 (m, 1H), 4.31-4.26 (m, 1H), 3.36-3.34 (m, 1H), 3.19-3.07 (m, 4H), 2.81-2.79 (m, 1H), 2.65-2.60 (m, 2H), 2.49-2.47 (m, 2H), 2.26-2.14 (m, 3H), 1.89-1.70 (m, 5H). MS m/z (ESI): 375.8 [M+H]⁺.

### Example 43: 10-(3-(2,3-Dihydrobenzofuran-7-yl)propyl)-5,6,7a,8,9,10,11,11a-octahydro-4H-pyrido[3',4':4,5]pyrrolo[3,2,1-iJ]quinoline

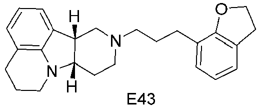

E43 was prepared with reference to the synthesis method of Example 42. ¹H NMR (500 MHz, DMSO-*d*₆) δ 7.04 (d, *J* = 10.0 Hz, 1H), 6.91 (d, *J =* 5.0 Hz, 1H), 6.86 (d, *J* = 5.0 Hz, 1H), 6.76 (d, *J* = 5.0 Hz, 1H), 6.73 (t, *J* = 7.5 Hz, 1H), 6.53 (t, *J* = 7.5 Hz, 1H), 4.49 (t, *J* = 10.0 Hz, 2H), 3.25-3.13 (m, 4H), 3.04-3.00 (m, 1H), 2.74-2.70 (m, 1H), 2.62-2.59 (m, 2H), 2.50-2.46 (m, 2H), 2.26-2.08 (m, 5H), 2.03-1.67 (m, 5H). MS m/z (ESI): 375.8 [M+H]⁺.

### Example 44: (6bR,10aS)-8-(3-(2,3-Dihydrobenzo[b][1,4]dioxin-5-yl)propyl)-3-methyl-2,3,6b,7,8,9,10,10a-octahydro-1H-pyrido[3',4":4,5]pyrrolo[1,2,3-de]quinoxaline

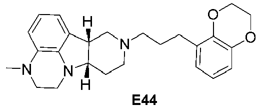

E44 was prepared with reference to the synthesis method of Example 3. ¹H NMR (500 MHz,CD₃OD) δ 6.74-6.66 (m, 3H), 6.62 (t, *J =* 7.6 Hz, 1H), 6.51 (d, *J* = 7.2 Hz, 1H), 6.44 (d, *J* = 8.0 Hz, 1H), 4.24 (d, *J =* 6.0 Hz, 2H), 4.21 (d, *J =* 6.0 Hz, 2H), 3.56-3.48 (m, 1H), 3.37 (d, *J* = 10.0 Hz, 1H), 3.22 (d, *J =* 12.0 Hz, 3H), 3.07 (s, 1H), 2.84 (s, 3H), 2.79-2.70 (m, 4H), 2.62 (dd, *J=* 10.0, 5.0 Hz, 2H), 2.31 (s, 1H), 2.19-2.12 (m, 1H), 2.17-1.99 (m, 2H), 1.96-1.89 (m, 2H). MS m/z (ESI): 406.9 [M+H]⁺.

### Example 45-1: 1-((6bR,10aS)-8-(3-(2,3-Dihydrobenzofuran-7-yl)propyl)-1,2,6b,7,8,9,10,10a-octahydro-3H-pyrido[3',4":4,5]pyrrolo[1,2,3-de]quinoxalin-3-yl)ethan-1-one

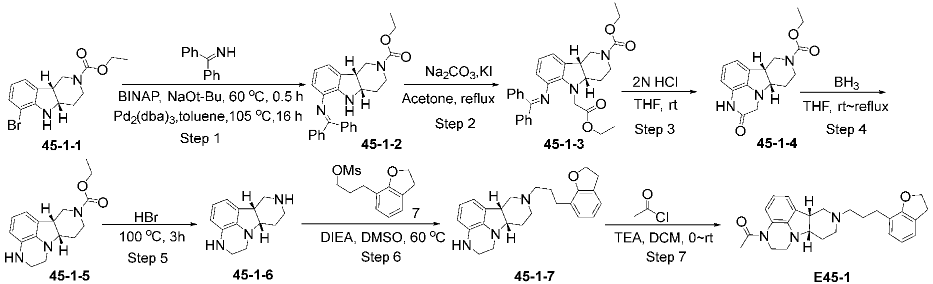

Step 1: Ethyl (4aS,9bR)-6-bromo-1,3,4,4a,5,9b-hexahydro-2H-pyrido[4,3-b]indole-2-carboxylate (2.08 g, 6.4 mmol), benzophenone imine (1.5 g, 7.7 mmol), NaOt-Bu (1.23 g, 12.8 mmol), BINAP (120 mg, 0.19 mmol), and 40 mL of toluene were added to a 250 mL three-neck flask, and the mixture was purged three times with N₂, heated to 60 °C, and left to react for 16 h. Then, the reaction was cooled to room temperature. Pd₂(dba)₃ (59 mg, 0.064 mmol) was added, and the mixture was purged three times with N₂, heated to 105 °C, and left to react for 16 h. LCMS monitoring showed that a small amount of the starting material remained. The reaction solution was cooled to room temperature, and MTBE was added. The mixture was filtered and concentrated to give the compound ethyl (4aS,9bR)-6-((diphenylmethylene)amino)-1,3,4,4a,5,9b-hexahydro-2H-pyrido[4,3-b]indole-2-carboxylate (3.4 g, crude), which was directly used in the next step. MS m/z (ESI): 426.5 [M+H]⁺.

Step 2: Ethyl (4aS,9bR)-6-((diphenylmethylene)amino)-1,3,4,4a,5,9b-hexahydro-2H-pyrido[4,3-b]indole-2-carboxylate (3.4 g, 8.0 mmol), ethyl bromoacetate (2.67 g, 7.7 mmol), Na₂CO₃ (1.7 g, 16.04 mmol), KI (1.99 g, 12.76 mmol), and 40 mL of acetone were added to a 250 mL three-neck flask, and the mixture was purged three times with N2, and heated to 60 °C, and left to react for 16 h. LCMS monitoring showed that a small amount of the starting material remained. The reaction solution was concentrated to dryness, and DCM and water were added. The organic phase was isolated, washed with saturated sodium chloride, dried, filtered, and concentrated to give the compound ethyl (4aS,9bR)-6-((diphenylmethylene)amino)-1,3,4,4a,5,9b-hexahydro-2H-pyrido[4,3-b]indole-2-carboxylate (4 g, crude), which was directly used in the next step. MS m/z (ESI): 513.6 [M+H]⁺.

Step 3: Ethyl (4aS,9bR)-6-((diphenylmethylene)amino)-1,3,4,4a,5,9b-hexahydro-2H-pyrido[4,3-b]indole-2-carboxylate (4 g, crude) was added to a single-neck flask containing 40 mL of THF, and 8 mL of 2 N HCl was added at room temperature. Then, the mixture was stirred at room temperature for half an hour. LCMS monitoring showed that the reaction had been complete. THF was removed by concentration, and DCM was added. The organic phase was isolated, washed three times with saturated sodium chloride, dried, and concentrated, and the residue was mixed with neutral alumina and purified by column chromatography (PE/EA = 100%-1/10 and then MeOH/DCM = 0-3%) to give the compound ethyl (6bR,10aS)-2-oxo-2,3,6b,9,10,10a-hexahydro-1H-pyrido[3',4":4,5]pyrrolo[1,2,3-de]quinoxaline-8(7H)-carboxylate (800 mg). MS m/z (ESI): 302.2 [M+H]⁺.

Step 4: Ethyl (6bR,10aS)-2-oxo-2,3,6b,9,10,10a-hexahydro-1H-pyrido[3',4':4,5]pyrrolo[1,2,3-de]quinoxaline-8(7H)-carboxylate (170 mg, 0.56 mmol) was added to a three-neck flask containing 10 mL of THF, and the mixture was purged three times with N2. 1.7 mL of 1 M BH₃Me₂S was added at 0 °C. After the dropwise addition, the mixture was heated to 70 °C and left to react for 3 h, and LCMS monitoring showed that the reaction had been complete. The reaction solution was cooled to 0 °C, and 6 N HCl was added dropwise until no bubbles were generated. THF was removed by concentration, NaOH (aq) was then added to adjust the pH to 9, and extraction was performed twice with DCM. The extract was dried and concentrated, and the residue was purified by column chromatography (MeOH/DCM = 0-5%) to give the compound ethyl (6bR,10aS)-2,3,6b,9,10,10a-hexahydro-1H-pyrido[3',4':4,5]pyrrolo[1,2,3-de]quinoxaline-8(7H)-carboxylate (100 mg, yellow oil, yield: 62.5%).

Step 5: Ethyl (6bR,10aS)-2,3,6b,9,10,10a-hexahydro-1H-pyrido[3',4':4,5]pyrrolo[1,2,3-de]quinoxaline-8(7H)-carboxylate (100 mg, 0.348 mmol) was left to react with 3 mL of a solution of HBr at 100 °C for 16 h, and LCMS monitoring showed that the reaction had been complete. The reaction mixture was concentrated to give the compound (6bR,10aS)-2,3,6b,7,8,9,10,10a-octahydro-1H-pyrido[3',4':4,5]pyrrolo[1,2,3-de]quinoxaline (70 mg), which was directly used in the next step.

Step 6: (6bR,10aS)-2,3,6b,7,8,9,10,10a-Octahydro-1H-pyrido[3',4':4,5]pyrrolo[1,2,3-de]quinoxaline (70 mg, 0.33 mmol), 7 (110 mg, 0.4 mmol), and 0.1 mL of DIEA were added to a single-neck flask containing 1 mL of DMSO. The mixture was purged three times with nitrogen, heated to 60 °C, and left to react for 2 h, and LCMS monitoring showed that the reaction had been complete. Water and EA were added, and the aqueous phase was isolated and extracted with EA. The organic phases were combined, washed three times with a saturated sodium chloride solution, dried, and concentrated, and the residue was purified by column chromatography (MeOH/DCM = 0-5%) to give the compound (6bR,10aS)-8-(3-(2,3-dihydrobenzofuran-7-yl)propyl)-2,3,6b,7,8,9,10,10a-octahydro-1H-pyrido[3',4':4,5]pyrrolo[1,2,3-de]quinoxaline (40 mg, beige solid, yield: 32%). MS m/z (ESI): 376.6 [M+H]⁺.

Step 7: (6bR,10aS)-8-(3-(2,3-Dihydrobenzofuran-7-yl)propyl)-2,3,6b,7,8,9,10,10a-octahydro-1H-pyrido[3',4':4,5]pyrrolo[1,2,3-de]quinoxaline (40 mg, 0.10 mmol) and TEA (20 mg, 0.20 mmol) were added to a single-neck flask containing 1 mL of DCM, and the mixture was purged three times with nitrogen and cooled to 0 °C. Acetyl chloride (13 mg, 0.15 mmol) was added, and the mixture was left to react at this temperature for 0.5 h. LCMS monitoring showed that the reaction had been complete. Water and DCM were added, and the aqueous phase was isolated and extracted with DCM. The organic phases were combined, washed three times with a saturated sodium chloride solution, dried, and concentrated, and the residue was purified by prep-HPLC (30-70% *CJJ* 30 min, 0.05% NH₄HCO₃ in H₂O) to give the compound 1-((6bR,10aS)-8-(3-(2,3-dihydrobenzofuran-7-yl)propyl)-1,2,6b,7,8,9,10,10a-octahydro-3H-pyrido[3',4':4,5]pyrrolo[1,2,3-de]quinoxalin-3-yl)ethan-1-one (15 mg, yellow solid, yield: 36%). ¹H NMR (500 MHz, CDCl₃) δ7.03 (d, *J =* 8.0 Hz 1H), 6.92(d, *J =* 7.5Hz 1H), 6.88(d, *J* = 7.0 Hz, 1H), 6.76(t, *J =* 7.5Hz, 1H), 6.67(s, 1H), 4.54(t, *J =* 8.5 Hz, 2H), 4.04-3.79 (m, 2H), 3.50-3.29 (m, 3H), 3.20 (t, *J =* 7.5Hz, 2H), 2.91-2.70 (m, 3H), 2.58 (t, *J =* 9.0 Hz, 2H), 2.42-2.26 (m, 6H), 2.06-1.84 (m, 5H). MS m/z (ESI): 418.9 [M+H]⁺.

### Example 45-2: 1-((6bR,10aS)-8-(3-(2,3-Dihydrobenzofuran-7-yl)propyl)-1,2,6b,7,8,9,10,10a-octahydro-3H-pyrido[3',4':4,5]pyrrolo[1,2,3-de]quinoxalin-3-yl)ethan-1-one

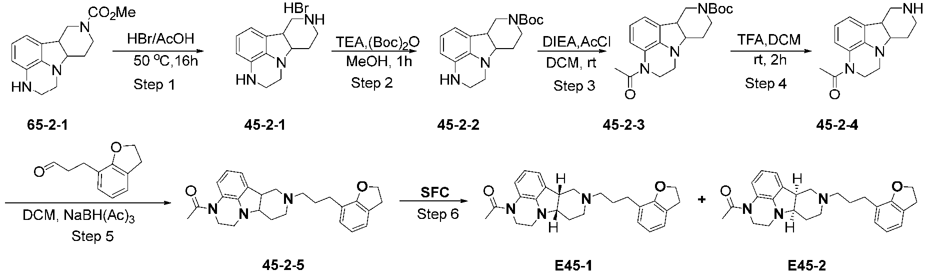

Step 1: 45-2-1 was prepared with reference to the synthesis method in step 2 of E73-1.

Step 2: 45-2-1 (860 mg, 4 mmol), TEA (808 mg, 8 mmol), and 10 mL of MeOH were added to a 100 mL single-neck flask, and Boc₂O (872 mg, 4 mmol) was then added. The mixture was left to react at room temperature for 2 h. After TLC analysis showed that the reaction had been complete, the reaction mixture was concentrated, and the residue was purified by column chromatography (PE/EA = 2/1) to give 45-2-2 (yellow oil, 1.2 g). MS m/z (ESI): 316.5 [M+H]⁺.

Step 3: 45-2-2 (300 mg, 0.95 mmol), TEA (192 mg, 1.9 mmol), and 5 mL of DCM were added to a 50 mL single-neck flask, and acetyl chloride (90 mg, 1.14 mmol) was then added. The mixture was left to react at room temperature for 10 min. LCMS analysis showed that the reaction had been complete. Saturated sodium chloride was added. The organic phase was isolated, dried, and concentrated, and the residue was purified by column chromatography (PE/EA = 1/1) to give 45-2-3 (330 mg, yellow oil). MS m/z (ESI): 358.2 [M+H]⁺.

Step 4: 45-2-3 (330 mg, 0.924 mmol), 1 mL of TFA, and 3 mL of DCM were added to a 50 mL single-neck flask and left to react at room temperature for 2 h. LCMS analysis showed that the reaction had been complete. The reaction mixture was concentrated, and saturated sodium bicarbonate was added to adjust the pH to 7-8. The aqueous phase was isolated and extracted five times with DCM/MeOH = 10/1 (v/v). After drying and concentration, 45-2-4 (210 mg, yellow solid) was obtained. MS m/z (ESI): 258.2 [M+H]⁺.

Step 5: In a nitrogen atmosphere, 45-2-4 (100 mg, 0.389 mmol) was dissolved in MeOH (50 mL) and DCM (30 mL), and 3-(2,3-dihydrobenzofuran-7-yl)propanal (86.7 mg, 0.492 mmol) and NaBH(OAc)₃ (129.1 mg, 2.05 mmol) were added. The mixture was left to react at 25 °C for 1 h. LCMS analysis showed that the reaction had been complete. Water was added, and extraction was performed with EA. The extract was dried over sodium sulfate, filtered, and concentrated, and the residue was purified by silica gel column chromatography (DCM:MeOH = 10:1) to give compound 45-2-5 (yellow solid, 100 mg, yield: 61%). MS m/z (ESI): 418.2 [M+H]⁺.

Step 6: 45-2-5 (100 mg, 0.239 mmol) was resolved by SFC (Daicel ChiralPak IH, 40 mm I.D. × 250 mm, 10 µm; mobile phase: supercritical CO₂/methanol [0.1% NH₃.H₂O (V/V)] = 65/35; flow rate: 140 mL/min) to give E45-2 (PK1: 26.0 mg, purity: 99%, yield: 26%, ee 100%, RT = 1.687, OROT = -97.9) as a white solid and E45-1 (PK2: 29.1 mg, purity: 99%, yield: 29%, ee 98%, RT = 2.267, OROT = +89.1) as a white solid. E45-2: ¹H NMR (400 MHz, DMSO-d6) δ 8.10 (s, 1H), 7.04 (d, *J =* 7.2 Hz, 1H), 6.91 (d, *J* = 7.2 Hz, 1H), 6.84 (d, *J =* 6.4 Hz, 1H), 6.73 (t, *J* = 7.2 Hz, 1H), 6.58 (dd, *J =* 8.0, 7.2 Hz, 1H), 4.49 (t, *J* = 8.8 Hz, 2H), 4.18-3.56 (m, 2H), 3.46-3.20 (m, 3H), 3.20-3.04 (m, 3H), 2.92-2.62 (m, 2H), 2.59-2.41 (m, 2H), 2.36-2.02 (m, 6H), 1.98-1.57 (m, 5H). E45-1: ¹H NMR (400 MHz, DMSO-d6) δ 8.10 (s, 1H), 7.04 (d, *J* = 7.2, 1H), 6.91 (d, *J* = 7.2 Hz, 1H), 6.84 (d, *J* = 6.4 Hz, 1H), 6.73 (t, *J* = 7.2 Hz, 1H), 6.58 (dd, *J* = 8.0, 7.2 Hz, 1H), 4.49 (t, *J* = 8.8 Hz, 2H), 4.15-3.62 (m, 2H), 3.46-3.21 (m, 3H), 3.21-3.05 (m, 3H), 2.93-2.61 (m, 2H), 2.52-2.35 (m, 2H), 2.35-2.01 (m, 6H), 1.99-1.54 (m, 5H).

### Example 46: (6bR,10aS)-8-(3-(3-Chloropyridin-2-yl)propyl)-3-methyl-2,3,6b,7,8,9,10,10a-octahydro-1H-pyrido[3',4':4,5]pyrrolo[1,2,3-de]quinoxaline

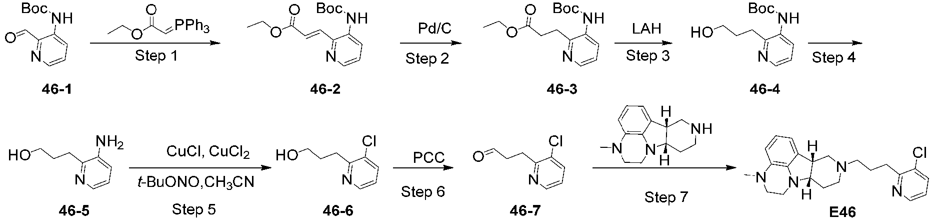

Step 1: The compound tert-butyl (2-formylpyridin-3-yl)carbamate (750 mg, 3.37 mmol) was dissolved in tetrahydrofuran (20 mL). After (ethoxycarbonylmethylene)triphenylphosphorane (1.4 g, 4.04 mmol) was added to the solution, the mixture was stirred at 70 °C for 2 h. After the reaction solution was concentrated, the residue was purified and separated by normal-phase column chromatography (petroleum ether:ethyl acetate = 3:1) to give the compound ethyl (E)-3-(tert-butoxycarbonyl)amino)pyridin-2-ylacrylate (pale yellow oil, 940 mg, yield: 96%). MS m/z (ESI): 293.6 [M+H]⁺.

Step 2: The compound ethyl (E)-3-(tert-butoxycarbonyl)amino)pyridin-2-ylacrylate (940 mg, 3.22 mmol) was dissolved in ethyl acetate (15 mL), and 10% wet palladium on carbon (35 mg, 0.32 mmol) was then added. The mixture was stirred at 28 °C for 15 min in a hydrogen atmosphere. The reaction solution was filtered and then concentrated to give the compound ethyl 3-(3-(((tert-butoxycarbonyl)amino)pyridin-2-yl)propanoate (pale yellow oil, 830 mg, yield: 88%). MS m/z (ESI): 295.7 [M+H]⁺.

Step 3: The compound ethyl 3-(3-(((tert-butoxycarbonyl)amino)pyridin-2-yl)propanoate (830 mg, 2.82 mmol) was dissolved in tetrahydrofuran (15 mL), and LiAlH₄ (4.23 mL, 1 M in THF, 4.23 mmol) was added to the solution at 0 °C in a nitrogen atmosphere. After the mixture was left to react at room temperature for 30 min, sodium sulfate decahydrate was added to the reaction solution until no bubbles were generated during stirring. The mixture was filtered, and the filtrate was concentrated. The resulting residue was purified and separated by normal-phase column chromatography (dichloromethane:methanol = 10:1) to give the compound tert-butyl (2-(3-hydroxypropyl)pyridin-3-yl)carbamate (colorless oil, 460 mg, yield: 65%). MS m/z (ESI): 253.6 [M+H]⁺.

Step 4: The compound tert-butyl (2-(3-hydroxypropyl)pyridin-3-yl)carbamate (460 mg, 1.82 mmol) was dissolved in hydrochloric acid/dioxane (10 mL). After the solution was left to react at room temperature for 30 min, the reaction solution was purified by reversed-phase preparative chromatography (eluent (v/v): acetonitrile/(water + 0.05% NH₄HCO₃)). Acetonitrile was removed under reduced pressure, and the residue was lyophilized to give the compound 3-(3-aminopyridin-2-yl)propanol (white solid, 240 mg, yield: 88%). MS m/z (ESI): 153.2 [M+H]⁺.

Step 5: The compounds 3-(3-aminopyridin-2-yl)propanol (240 mg, 1.58 mmol), cupric chloride (45 mg, 0.32 mmol), cuprous chloride (311 mg, 3.15 mmol), and tert-butyl nitrite (325 mg, 3.15 mmol) were dissolved in acetonitrile (15 mL). In a nitrogen atmosphere, the solution was left to react at 25 °C for 0.5 h and then at 50 °C for 2 h. The reaction solution was filtered and then purified and separated by normal-phase column chromatography (dichloromethane:methanol = 10:1) to give the compound 3-(3-chloropyridin-2-yl)propanol (yellow oil, 50 mg, yield: 19%). MS m/z (ESI): 172.1 [M+H]⁺.

Step 6: The compound 3-(3-chloropyridin-2-yl)propanol (50 mg, 0.29 mmol) was dissolved in dichloromethane (5 mL), and PCC (440 mg, 2.04 mmol) was added to the solution. After the mixture was left to react at room temperature for 0.5 h, the reaction solution was filtered, and the filtrate was washed with a saturated aqueous sodium bicarbonate solution (10 mL × 2). Then, the organic phase was concentrated to give the title compound 3-(3-chloropyridin-2-yl)propanal (brown solid, 45 mg, crude, yield: 92%). MS m/z (ESI): 170.1 [M+H]⁺.

Step 7: The compound 3-(3-chloropyridin-2-yl)propanal (35 mg, 0.31 mmol) was dissolved in dichloromethane (2 mL), and (6bR,10aS)-3-methyl-2,3,6b,7,8,9,10,10a-octahydro-1H-pyrido[3',4':4,5]pyrrolo[1,2,3-de]quinoxaline (49 mg, 0.21 mmol) was added to the solution. After the mixture was left to react at room temperature for 0.5 h, sodium triacetoxyborohydride (135 mg, 0.63 mmol) was added to the mixture. After the mixture was left to react at room temperature for 0.5 h, the reaction solution was filtered and then purified by reversed-phase preparative chromatography (eluent (v/v): acetonitrile/(water + 0.05% NH₄HCO₃)). Acetonitrile was removed under reduced pressure, and the residue was lyophilized to give the compound (6bR,10aS)-8-(3-chloropyridin-2-propyl)-3-methyl-2,3,6b,7,8,9,10,10a-octahydro-1H-pyrido[3',4':4,5]pyrrolo[1,2,3-de]quinoxaline (yellow solid, 23 mg, yield: 19%). ¹H NMR (500 MHz, CDCl₃) δ 8.41 (dd, *J =* 4.7, 1.6 Hz, 1H), 7.62 (dd, *J* = 8.0, 1.6 Hz, 1H), 7.09 (dd, *J =* 8.0, 4.7 Hz, 1H), 6.65 (t, *J* = 7.7 Hz, 1H), 6.51 (dd, *J =* 7.4, 1.0 Hz, 1H), 6.40 (dd, *J =* 7.9, 0.9 Hz, 1H), 3.65-3.56 (m, 1H), 3.32-3.20 (m, 4H), 2.97 (q, *J =* 8.0 Hz, 3H), 2.86 (s, 3H), 2.85-2.77 (m, 2H), 2.52 (d, *J* = 9.4 Hz, 2H), 2.34 (s, 1H), 2.04-1.93 (m, 5H). MS m/z (ESI): 383.7 [M+H]⁺.

### Example 47: 2-(3-(6bR,10aS)-3-Methyl-2,3,6b,9,10,10a-hexahydropyrido[3',4':4,5]pyrrolo[1,2,3-de]quinoxaline-8(7H)-propyl)pyridin-3-amine

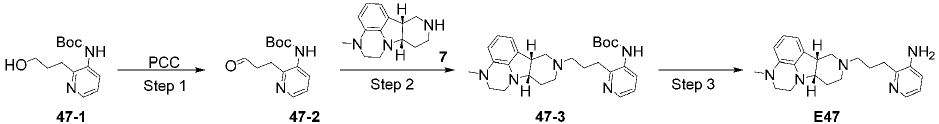

Step 1: The compound tert-butyl (2-(3-hydroxypropyl)pyridin-3-yl)carbamate (20 mg, 0.079 mmol) was dissolved in dichloromethane (2 mL), and PCC (85 mg, 0.40 mmol) was added to the solution. After the mixture was left to react at room temperature for 12 h, the reaction solution was filtered, and the filtrate was washed with a saturated aqueous sodium bicarbonate solution (10 mL × 2). Then, the organic phase was concentrated to give the compound tert-butyl (2-(3-oxopropyl)pyridin-3-yl)carbamate (brown solid, 20 mg, crude, yield: 100%). MS m/z (ESI): 251.5 [M+H]⁺.

Step 2: The compound tert-butyl (2-(3-oxopropyl)pyridin-3-yl)carbamate (20 mg, 0.079 mmol) was dissolved in dichloromethane (2 mL), and (6bR,10aS)-3-methyl-2,3,6b,7,8,9,10,10a-octahydro-1H-pyrido[3',4':4,5]pyrrolo[1,2,3-de]quinoxaline (19 mg, 0.079 mmol) was added to the solution. After the mixture was left to react at room temperature for 0.5 h, sodium triacetoxyborohydride (51 mg, 0.24 mmol) was added to the mixture. After the mixture was left to react at room temperature for 0.5 h, the reaction solution was filtered and then purified by reversed-phase preparative chromatography (eluent (v/v): acetonitrile/(water + 0.05% NH₄HCO₃)). Acetonitrile was removed under reduced pressure, and the residue was lyophilized to give the compound tert-butyl 2-(3-((6bR,10aS)-3-methyl-2,3,6b,9,10,10a-hexahydro-1H-pyrido[3',4':4,5]pyrrolo[1,2,3-de]quinoxaline-8(H)-propyl)pyridine-3-carbamate (yellow solid, 8 mg, yield: 22%). MS m/z (ESI): 464.9 [M+H]⁺.

Step 3: The compound tert-butyl 2-(3-((6bR,10aS)-3-methyl-2,3,6b,9,10,10a-hexahydro-1H-pyrido[3',4':4,5]pyrrolo[1,2,3-de]quinoxaline-8(H)-propyl)pyridine-3-carbamate (8 mg, 0.017 mmol) was dissolved in dichloromethane (3 mL), and hydrochloric acid/dioxane (3 mL) was added to the solution at 0 °C. After the mixture was left to react at 0 °C for 10 min, the reaction solution was filtered and then purified by reversed-phase preparative chromatography (eluent (v/v): acetonitrile/(water + 0.05% NH₄HCO₃)). Acetonitrile was removed under reduced pressure, and the residue was lyophilized to give the compound 2-(3-(6bR,10aS)-3-methyl-2,3,6b,9,10,10a-hexahydropyrido[3',4':4,5]pyrrolo[1,2,3-de]quinoxaline-8(7H)-propyl)pyridin-3-amine (yellow solid, 2 mg, yield: 33%). 1H NMR (500 MHz, DMSO) δ 7.70 (dd, *J* = 4.1, 2.1 Hz, 1H), 6.92-6.85 (m, 2H), 6.50 (t, *J =* 7.6 Hz, 1H), 6.42 (dd, *J =* 7.4, 1.0 Hz, 1H), 6.33 (dd, *J =* 7.9, 1.0 Hz, 1H), 5.13 (s, 2H), 3.45-3.40 (m, 1H), 3.32-3.26 (m, 2H), 3.13-3.09 (m, 1H), 3.05-2.98 (m, 1H), 2.78 (s, 4H), 2.71-2.65 (m, 1H), 2.59 (q, *J* = 7.3, 6.0 Hz, 3H), 2.30-2.18 (m, 2H), 2.12-2.05 (m, 1H), 1.94-1.88 (m, 1H), 1.82-1.72 (m, 4H). MS m/z (ESI): 364.8 [M+H]⁺.

### Example 48: (6bR,10aS)-8-(3-(5-Fluoro-2,3-dihydrobenzofuran-4-yl)propyl)-3-methyl-2,3,6b,7,8,9,10,10a-octahydro-1H-pyrido[3',4':4,5]pyrrolo[1,2,3-de]quinoxaline

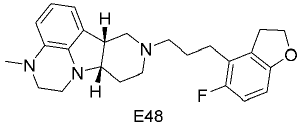

E48 was prepared with reference to the synthesis method of Example 3. ¹H NMR (500 MHz, CDCl₃) δ 6.79-6.70 (m, 1H), 6.69-6.61 (m, 1H), 6.56-6.48 (m, 2H), 6.41 (d, *J* = 7.5 Hz, 1H), 4.57 (t, *J =* 8.8 Hz, 2H), 3.68-3.49 (m, 1H), 3.34-3.11 (m, 6H), 2.97-2.89 (m, 1H), 2.88-2.71 (m, 5H), 2.57 (dd, *J =* 26.0, 18.0 Hz, 2H), 2.47-2.29 (m, 3H), 2.04-1.93 (m, 3H), 1.84 (dt, *J =* 15.1, 7.6 Hz, 2H). MS m/z (ESI): 408.9 [M+H]⁺.

### Example 49: (6bR,10aS)-8-(3-(5-Fluorobenzofuran-4-yl)propyl)-3-methyl-2,3,6b,7,8,9,10,10a-octahydro-1H-pyrido[3',4":4,5]pyrrolo[1,2,3-de]quinoxaline

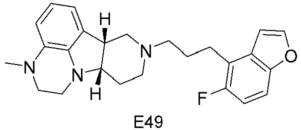

E49 was prepared with reference to the synthesis method of Example 3. ¹H NMR (500 MHz, CDCl₃) δ 7.63 (d, *J =* 2.0 Hz, 1H), 7.28-7.26 (m, 1H), 7.02-6.92 (m, 1H), 6.79 (dd, *J =* 2.0, 1.0 Hz, 1H), 6.69-6.57 (m, 1H), 6.50 (d, *J =* 7.0 Hz, 1H), 6.40 (d, *J =* 7.5 Hz, 1H), 3.58 (ddd, *J =* 13.0, 8.0, 2.5 Hz, 1H), 3.32-3.14 (m, 4H), 2.95-2.75 (m, 8H), 2.70 (d, *J =* 14.6 Hz, 1H), 2.41 (d, *J* = 9.0 Hz, 2H), 2.24 (dd, *J* = 25.5, 9.2 Hz, 1H), 1.94 (dd, *J* = 22.0, 9.0 Hz, 4H). MS m/z (ESI): 406.9 [M+H]⁺.

### Example 50: (6bR,10aS)-8-(2-(1H-Indol-3-yl)ethyl)-3-methyl-2,3,6b,7,8,9,10,10a-octahydro-1H-pyrido[3',4':4,5]pyrrolo[1,2,3-de]quinoxaline

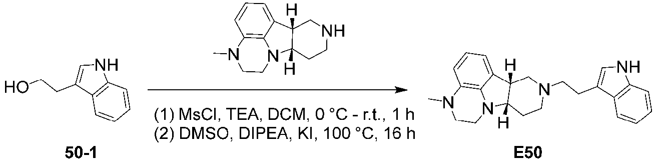

2-(1H-Indol-3-yl)ethan-1-ol (71.2 mg, 0.44 mmol) and triethylamine (220.6 mg, 2.18 mmol) were dissolved in dichloromethane (2 mL) at room temperature, and the solution was cooled to 0 °C. Methanesulfonyl chloride (74.9 mg, 0.65 mmol) was added, and the mixture was warmed to room temperature and stirred for 1 h. Then, the reaction solution was concentrated *in vacuo* at room temperature. The crude product was dissolved in anhydrous dimethyl sulfoxide (3 mL), and (6bR,10aS)-3-methyl-2,3,6b,7,8,9,10,10a-octahydro-1H-pyrido[3',4':4,5]pyrrolo[1,2,3-de]quinoxaline (50.0 mg, 0.22 mmol), potassium iodide (72.3 mg, 0.44 mmol), and DIPEA (0.5 mL) were added. The reaction solution was stirred at 100 °C for 16 h, and LCMS (ENBW230404-026-6-R1, 2023.8.8) showed that a product was generated. The reaction solution was cooled to room temperature, added to 50 mL of water, and extracted with ethyl acetate (40 mL × 3). The organic phases were washed with saturated brine, dried over anhydrous sodium sulfate, combined, and concentrated to give a crude product. The crude product was purified by preparative chromatography to give (6bR,10aS)-8-(2-(1H-indol-3-yl)ethyl)-3-methyl-2,3,6b,7,8,9,10,10a-octahydro-1H-pyrido[3',4':4,5]pyrrolo[1,2,3-de]quinoxaline (7.80 mg, yield: 9.5%) as a white solid. ¹H NMR (400 MHz, CDCl₃) δ 8.09 (s, 1H), 7.59 (d, *J* = 7.9 Hz, 1H), 7.36 (d, *J =* 8.1 Hz, 1H), 7.21-7.16 (m, 1H), 7.13-7.09 (m, 1H), 7.05 (d, *J* = 2.1 Hz, 1H), 6.69 (t, *J =* 7.7 Hz, 1H), 6.54 (d, *J* = 7.2 Hz, 1H), 6.43 (d, *J =* 7.6 Hz, 1H), 3.65-3.57 (m, 1H), 3.48 (s, 1H), 3.31-2.28 (m, 4H), 3.15-3.11 (m, 3H), 2.95-2.82 (m, 6H), 2.72-2.61 (m, 1H), 2.36-2.24 (m, 2H), 2.05 (d, *J =* 15.4 Hz, 1H). MS m/z (ESI): 373.2 [M+H]⁺.

### Example 52: 2-Methyl-1-(2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenoxy)propan-2-ol

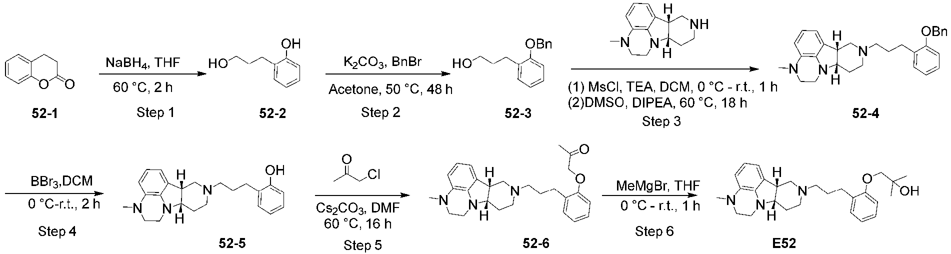

Step 1: At room temperature, chroman-2-one (5.0 g, 33.7 mmol) was dissolved in tetrahydrofuran (50 mL), and sodium borohydride (1.9 g, 50.5 mmol) was added. The reaction mixture was stirred at 60 °C for 2 h. After the reaction was complete, the reaction mixture was cooled to room temperature, quenched with a saturated ammonium chloride solution, and concentrated *in vacuo,* and the resulting residue was triturated with 50 mL of dichloromethane. After filtration, the filtrate was concentrated *in vacuo* to give 2-(3-hydroxypropyl)phenol (5.5 g, yield: 99.1%) as a colorless oil. MS m/z (ESI): 153.2 [M+H]⁺.

Step 2: 2-(3-Hydroxypropyl)phenol (5.5 g, 36.1 mmol) was dissolved in acetone (50 mL), and benzyl bromide (7.4 g, 43.3 mmol) and potassium carbonate (7.5 g, 54.2 mmol) were added. The reaction mixture was stirred at 50 °C for 48 h. The reaction solution was cooled to room temperature, 120 mL of water was added, and extraction was performed with ethyl acetate (100 mL × 3) to give 3-(2-(benzyloxy)phenyl)propan-1-ol (3 g, yield: 34.4%) as a transparent oil. MS m/z (ESI): 243.2 [M+H]⁺.

Step 3: 3-(2-(Benzyloxy)phenyl)propan-1-ol (1.3 g, 5.23 mmol) and triethylamine (1.6 g, 15.7 mmol) were dissolved in dichloromethane (10 mL) at room temperature, and the solution was cooled to 0 °C. Methanesulfonyl chloride (899.1 mg, 7.8 mmol) was added, and the mixture was warmed to room temperature and stirred for 1 h. Then, the reaction solution was concentrated *in vacuo* at room temperature, added to water, and extracted with dichloromethane, and the organic phase was concentrated to dryness by rotary evaporation to give a white solid. (6bR,10aS)-3-Methyl-2,3,6b,7,8,9,10,10a-octahydro-1H-pyrido[3',4':4,5]pyrrolo[1,2,3-de]quinoxaline (600.0 mg, 2.6 mmol) was dissolved in DMSO (9 mL), and the obtained white solid and DIPEA (1.5 mL) were added. The reaction solution was stirred at 60 °C for 18 h. The reaction solution was cooled to room temperature, added to 50 mL of water, and extracted with ethyl acetate (40 mL × 3). The organic phases were washed with saturated brine, dried over anhydrous sodium sulfate, combined, and concentrated to give a crude product. The crude product was purified by silica gel column chromatography (DCM/MeOH = 0/100-8/92) to give (6bR,10aS)-8-(3-(2-(benzyloxy)phenyl)propyl)-3-methyl-2,3,6b,7,8,9,10,10a-octahydro-1H-pyrido[3',4':4,5]pyrrolo[1,2,3-de]quinoxaline (1.0 g, yield: 84.3%) as a yellow oil. MS m/z (ESI): 454.2 [M+H]⁺.

Step 4: A solution of boron tribromide in dichloromethane (22 mL, 22 mmol) was added dropwise to a solution of (6bR,10aS)-8-(3-(2-(benzyloxy)phenyl)propyl)-3-methyl-2,3,6b,7,8,9,10,10a-octahydro-1H-pyrido[3',4':4,5]pyrrolo[1,2,3-de]quinoxaline (1 g, 2.2 mmol) in DCM (30 mL) at 0 °C in a nitrogen atmosphere, and the reaction solution was stirred at room temperature for 2 h. After the reaction was complete, methanol was added under ice bath conditions to quench the reaction. After the mixture was concentrated *in vacuo,* water (30 mL) was added, a 1 M sodium hydroxide solution was added to adjust the pH to 10, and ethyl acetate (40 mL × 3) was added for extraction. The organic phases were combined and concentrated *in vacuo,* and the resulting crude product was purified by silica gel column chromatography (DCM/MeOH = 100/0-90/10) to give 2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenol (600 mg, yield: 77.3%) as a yellow solid. MS m/z (ESI): 364.2 [M+H]⁺.

Step 5: At room temperature, 2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenol (100.0 mg, 0.28 mmol) and 1-chloropropan-2-one (50.9 mg, 0.55 mmol) were dissolved in DMF (1.5 mL), and cesium carbonate (268.9 g, 0.83 mmol) was added. The reaction mixture was stirred at 60 °C for 16 h. The reaction solution was cooled to room temperature, 40 mL of water was added, and extraction was performed with ethyl acetate (30 mL × 3). The organic phases were combined and concentrated *in vacuo,* and the resulting crude product was purified by silica gel column chromatography (PE/EA = 100/0-0/100) to give 1-(2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenoxy)propan-2-one (36 mg, yield: 14.9%) as a yellow oil. MS m/z (ESI): 420.2 [M+H]⁺.

Step 6: At room temperature, 1-(2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenoxy)propan-2-one (50.0 mg, 0.12 mmol) was dissolved in tetrahydrofuran (2 mL), and the system was filled with nitrogen. The reaction solution was cooled to 0 °C, and methylmagnesium bromide (3 M, 0.1 mL, 0.36 mmol) was added dropwise. The reaction solution was stirred at room temperature for 1 h. After the reaction was complete, the reaction solution was quenched with a saturated ammonium chloride solution, diluted with 40 mL of water, and extracted with ethyl acetate (30 mL × 3). The organic phases were combined and concentrated *in vacuo* to give a crude product, and the crude product was purified by preparative chromatography to give 2-methyl-1-(2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenoxy)propan-2-ol (5.77 mg, yield: 10.2%) as a brown solid. ¹H NMR (400 MHz, CDCl₃) δ 7.17-7.10 (m, 2H), 6.89 (t, *J* = 7.3 Hz, 1H), 6.82 (d, *J* = 8.1 Hz, 1H), 6.65 (t, *J* = 7.6 Hz, 1H), 6.50 (d, *J =* 7.3 Hz, 1H), 6.40 (d, *J =* 7.9 Hz, 1H), 3.79 (s, 2H), 3.64-3.56 (m, 1H), 3.30-3.20 (m, 4H), 3.01 (d, *J* = 10.7 Hz, 1H), 2.88-2.78 (m, 5H), 2.68 (t, *J =* 7.5 Hz, 2H), 2.55-2.33 (m, 4H), 2.05-2.01 (m, 2H), 1.94-1.89 (m, 2H), 1.36 (s, 6H). MS m/z (ESI): 436.3 [M+H]⁺.

### Example 53: 2-(2-(3-((6bR,10aS)-3-Methyl-2,3,6b,9,10,10a-hexahydro-1H-pyrido[3',4':4,5]pyrrolo[1,2,3-de]quinoxalin-8(7H)-yl)propyl)phenoxy)ethan-1-ol

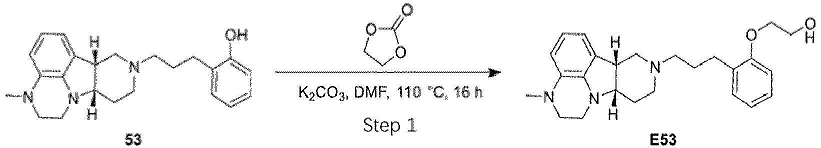

At room temperature, 2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenol (50.0 mg, 0.14 mmol) and 1,3-dioxolan-2-one (24.2 mg, 0.28 mmol) were dissolved in DMF (1 mL), and potassium carbonate (38.0 mg, 0.28 mmol) was added. The reaction mixture was stirred at 110 °C for 16 h. The reaction solution was cooled to room temperature, 40 mL of water was added, and extraction was performed with ethyl acetate (30 mL × 3). The crude product was purified by preparative chromatography to give 2-(2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenoxy)ethan-1-ol (10.82 mg, yield: 18.9%) as a yellow solid. ¹H NMR (400 MHz, CDCl₃) δ 7.19-7.07 (m, 2H), 6.94-6.81 (m, 2H), 6.65 (t, *J* = 7.6 Hz, 1H), 6.51 (d, *J =* 6.8 Hz, 1H), 6.40 (d, *J* = 7.6 Hz, 1H), 4.14-4.03 (m, 2H), 4.00-3.91 (m, 2H), 3.66-3.55 (m, 1H), 3.34-3.16 (m, 4H), 2.95 (d, *J =* 12.5 Hz, 1H), 2.86 (s, 3H), 2.85-2.72 (m, 2H), 2.68 (t, *J =* 7.5 Hz, 2H), 2.34 (d, *J =* 51.1 Hz, 4H), 1.98-1.85 (m, 4H). MS m/z (ESI): 408.2 [M+H]⁺.

### Example 54: (6bR,10aS)-8-(3-(2,2-Difluorobenzo[d][1,3]dioxol-4-yl)propyl)-3-methyl-2,3,6b,7,8,9,10,10a-octahydro-1H-pyrido[3',4':4,5]pyrrolo[1,2,3-de]quinoxaline

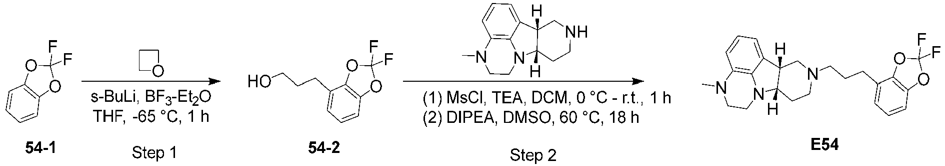

Step 1: At room temperature, 2,2-difluorobenzo[d][1,3]dioxole (500.0 mg, 3.16 mmol) was dissolved in tetrahydrofuran (8 mL), and sec-butyllithium (202.6 mg, 3.16 mmol) was added. The system was filled with nitrogen. The reaction solution was cooled to -65 °C, and oxetane (183.7 mg, 3.16 mmol) and boron trifluoride etherate (189.6 mg, 1.3 mmol) were sequentially added dropwise. The reaction solution was stirred at -65 °C for 1 h. After the reaction was complete, a saturated aqueous ammonium chloride solution was added to quench the reaction, 60 mL of water was added, and extraction was performed with ethyl acetate (50 mL × 3). The organic phase was concentrated *in vacuo* to give a crude product, and the crude product was purified by silica gel column chromatography (DCM/MeOH = 100/0-90/10) to give 3-(2,2-difluorobenzo[d][1,3]dioxol-4-yl)propan-1-ol (470 mg, yield: 68.8%) as a transparent oil. ¹H NMR (400 MHz, CDCl₃) δ 7.02-6.96 (m, 1H), 6.94-6.88 (m, 2H), 3.69 (t, *J* = 6.3 Hz, 2H), 2.84-2.56 (m, 2H), 1.93 (dq, *J =* 13.9, 6.4 Hz, 2H).

Step 2: 3-(2,2-Difluorobenzo[d][1,3]dioxol-4-yl)propan-1-ol (94.3 mg, 0.44 mmol) and triethylamine (220.6 mg, 2.18 mmol) were dissolved in dichloromethane (3 mL), and the solution was cooled to 0 °C. Methanesulfonyl chloride (74.9 mg, 0.65 mmol) was added, and the mixture was warmed to room temperature and stirred for 1 h. Then, the reaction solution was concentrated *in vacuo* at room temperature. The crude product was dissolved in DMSO (3 mL), and (6bR,10aS)-3-methyl-2,3,6b,7,8,9,10,10a-octahydro-1H-pyrido[3',4':4,5]pyrrolo[1,2,3-de]quinoxaline (50 mg, 0.22 mmol) and DIPEA (0.5 mL) were added. The reaction solution was stirred at 60 °C for 18 h. The reaction solution was cooled to room temperature, added to 50 mL of water, and extracted with ethyl acetate (40 mL × 3). The organic phases were washed with saturated brine, dried over anhydrous sodium sulfate, combined, and concentrated to give a crude product. The crude product was purified by preparative chromatography to give (6bR,10aS)-8-(3-(2,2-difluorobenzo[d][1,3]dioxol-4-yl)propyl)-3-methyl-2,3,6b,7,8,9,10,10a-octahydro-1H-pyrido[3',4':4,5]pyrrolo[1,2,3-de]quinoxaline (41.33 mg, yield: 43.72%) as a brown oil. ¹H NMR (400 MHz, CDCl₃) δ 7.01-6.95 (m, 1H), 6.89 (dd, *J =* 7.5, 2.4 Hz, 2H), 6.65 (t, *J =* 7.6 Hz, 1H), 6.51 (d, *J* = 6.9 Hz, 1H), 6.41 (d, *J =* 7.8 Hz, 1H), 3.63-3.56 (m, 1H), 3.34-3.14 (m, 4H), 2.84-2.79 (m, 5H), 2.69 (t, *J =* 7.6 Hz, 3H), 2.45-2.34 (m, 3H), 1.99-1.91 (m, 5H). ¹⁹F NMR (377 MHz, CDCl₃) δ -49.80 (s, 2F). MS m/z (ESI): 428.2 [M+H]⁺.

### Example 55: (6bR,10aS)-3-Methyl-8-(3-(2-(2,2,2-trifluoroethoxy)phenyl)propyl)-2,3,6b,7,8,9,10,10a-octahydro-1H-pyrido[3',4':4,5]pyrrolo[1,2,3-de]quinoxaline

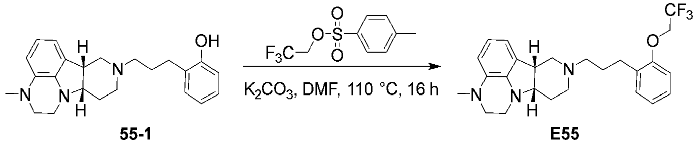

At room temperature, 2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenol (50.0 mg, 0.14 mmol) and 2,2,2-trifluoroethyl 4-methylbenzenesulfonate (52.7 mg, 0.21 mmol) were dissolved in DMF (1 mL), and potassium carbonate (57.1 mg, 0.41 mmol) was added. The reaction mixture was stirred at 110 °C for 16 h. The reaction solution was cooled to room temperature, 40 mL of water was added, and extraction was performed with ethyl acetate (30 mL × 3). The extract was concentrated to give a crude product. The crude product was purified by preparative chromatography to give (6bR,10aS)-3-methyl-8-(3-(2-(2,2,2-trifluoroethoxy)phenyl)propyl)-2,3,6b,7,8,9,10,10a-octahydro-1H-pyrido[3',4':4,5]pyrrolo[1,2,3-de]quinoxaline (5.32 mg, yield: 8.2%) as a brown solid. ¹H NMR (400 MHz, CDCl₃) δ 7.20-7.15 (m, 2H), 6.99-6.95 (m, 1H), 6.77 (d, *J* = 8.3 Hz, 1H), 6.65 (t, *J* = 7.6 Hz, 1H), 6.51 (d, *J* = 7.3 Hz, 1H), 6.40 (d, *J* = 7.6 Hz, 1H), 4.34 (q, *J =* 8.1 Hz, 2H), 3.64-3.55 (m, 1H), 3.35-3.17 (m, 4H), 2.94-2.79 (m, 5H), 2.75-2.64 (m, 3H), 2.41 (s, 2H), 2.27 (s, 1H), 1.96 (s, 2H), 1.73 (s, 3H). ¹⁹F NMR (377 MHz, CDCl₃) δ -73.98 (s, 3F). MS m/z (ESI): 446.2 [M+H]⁺.

### Example 56: (6bR,10aS)-3-Methyl-8-(3-(2-(pyridin-4-yloxy)phenyl)propyl)-2,3,6b,7,8,9,10,10a-octahydro-1H-pyrido[3',4':4,5]pyrrolo[1,2,3-de]quinoxaline

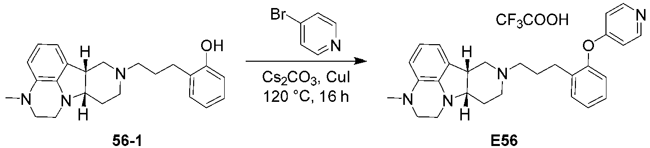

At room temperature, 2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenol (60.0 mg, 0.16 mmol) and 4-bromopyridine (39.1 mg, 0.25 mmol) were dissolved in DMF (2 mL), and cesium carbonate (107.6 mg, 0.33 mmol) and cuprous iodide (6.3 mg, 0.03 mmol) were added. The reaction mixture was stirred at 120 °C for 16 h. The reaction solution was cooled to room temperature, 40 mL of water was added, and extraction was performed with ethyl acetate (30 mL × 3). The crude product was purified by preparative chromatography to give (6bR,10aS)-3-methyl-8-(3-(2-(pyridin-4-yloxy)phenyl)propyl)-2,3,6b,7,8,9,10,10a-octahydro-1H-pyrido[3',4':4,5]pyrrolo[1,2,3-de]quinoxaline 2,2,2-trifluoroacetate (18.42 mg, yield: 25.02%) as a black oil. ¹H NMR (400 MHz, CDCl₃) δ 11.59 (s, 1H), 8.70 (d, *J =* 6.8 Hz, 2H), 7.44-7.34 (m, 3H), 7.24 (d, *J =* 6.7 Hz, 2H), 7.06 (d, *J =* 7.6 Hz, 1H), 6.78 (s, 1H), 6.59 (d, *J =* 8.1 Hz, 2H), 3.58-3.46 (m, 3H), 3.49-3.24 (m, 4H), 3.06-2.85 (m, 7H), 2.54 (t, *J =* 7.4 Hz, 3H), 2.39 (s, 1H), 2.29-1.90 (m, 3H). MS m/z (ESI): 441.1 [M+H]⁺.

### Example 57: Preparation of (6bR,10aS)-8-(3-(2-(Difluoromethoxy)phenyl)propyl)-3-methyl-2,3,6b,7,8,9,10,10a-octahydro-1H-pyrido[3',4':4,5]pyrrolo[1,2,3-de]quinoxaline

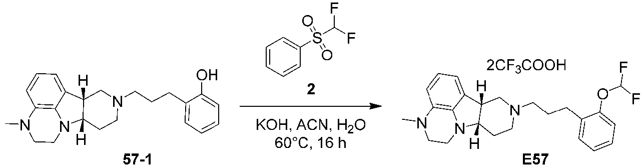

At room temperature, 2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenol (50.0 mg, 0.14 mmol) and ((difluoromethyl)sulfonyl)benzene (60.8 mg, 0.32 mmol) were dissolved in acetonitrile (1 mL), and a 25% solution of potassium hydroxide (84.9 mg, 1.5 mmol) in water (0.255 mL) was added. The reaction system was filled with nitrogen. The reaction mixture was stirred at 60 °C for 16 h. After the reaction was complete, the reaction mixture was diluted with 40 mL of water and extracted with ethyl acetate (30 mL × 3). The organic phases were combined and concentrated *in vacuo* to give a crude product, and the crude product was purified by preparative chromatography to give (6bR,10aS)-8-(3-(2-(difluoromethoxy)phenyl)propyl)-3-methyl-2,3,6b,7,8,9,10,10a-octahydro-1H-pyrido[3',4':4,5]pyrrolo[1,2,3-de]quinoxaline (6.39 mg, yield: 11.0%) as a brown solid. ¹H NMR (400 MHz, CDCl₃) δ 12.03 (s, 1H), 7.24-7.12 (m, 3H), 7.06 (d, *J =* 8.0 Hz, 1H), 6.73 (t, *J* = 7.7 Hz, 1H), 6.58-6.32 (m, 3H), 3.63-3.46 (m, 4H), 3.32-3.29 (m, 3H), 3.03-2.86 (m, 7H), 2.71 (t, *J =* 7.4 Hz, 2H), 2.52-2.48 (m, 2H), 2.17-2.04 (m, 3H). ¹⁹F NMR (377 MHz, CDCl₃) δ -75.78 (s, 6F), -79.97 (d, *J* = 2.7 Hz, 2F). MS m/z (ESI): 414.2 [M+H]⁺.

### Example 58: (6bR,10aS)-3-Methyl-8-(3-(pyridin-2-yl)propyl)-2,3,6b,7,8,9,10,10a-octahydro-1H-pyrido[3',4':4,5]pyrrolo[1,2,3-de]quinoxaline

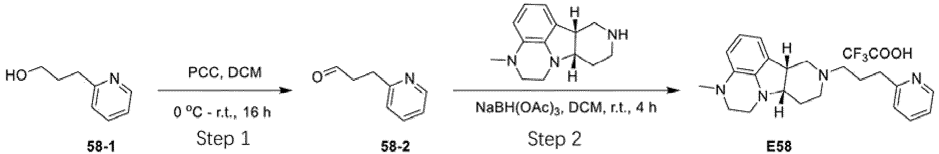

Step 1: 3-(Pyridin-2-yl)propan-1-ol (30.0 mg, 0.22 mmol) was dissolved in dichloromethane (3 mL) at room temperature, and the solution was cooled to 0 °C. PCC (94.3 mg, 0.44 mmol) was added, and the mixture was warmed to room temperature and stirred for 16 h. The reaction solution was filtered through diatomaceous earth and added to 10 mL of water, and the pH was adjusted to 8 with a saturated aqueous sodium bicarbonate solution. The mixture was diluted with 40 mL of water and extracted with dichloromethane (30 mL × 3). The organic phases were dried over anhydrous sodium sulfate, combined, and concentrated to give 3-(pyridin-2-yl)propanal (29 mg, yield: 78.5%) as a transparent oil. The crude product was directly used in the next step. MS m/z (ESI): 136.2 [M+H]⁺.

Step 2: (6bR,10aS)-3-Methyl-2,3,6b,7,8,9,10,10a-octahydro-1H-pyrido[3',4':4,5]pyrrolo[1,2,3-de]quinoxaline (45.0 mg, 0.20 mmol) and 3-(pyridin-2-yl)propanal (29.2 mg, 0.22 mmol) were dissolved in dichloromethane (2 mL) at room temperature, and the mixture was stirred at room temperature for 30 min. Sodium triacetoxyborohydride (83.2 mg, 0.39 mmol) was added, and the reaction solution was stirred at room temperature for 3.5 h. After the reaction was complete, the reaction solution was added to 40 mL of water and extracted with ethyl acetate (30 mL × 3). The organic phases were washed with saturated brine, dried over anhydrous sodium sulfate, combined, and concentrated to give a crude product. The crude product was purified by preparative chromatography to give (6bR,10aS)-3-methyl-8-(3-(pyridin-2-yl)propyl)-2,3,6b,7,8,9,10,10a-octahydro-1H-pyrido[3',4':4,5]pyrrolo[1,2,3-de]quinoxaline 2,2,2-trifluoroacetate (7.24 mg, yield: 10.1%) as a brown oil. ¹H NMR (400 MHz, CDCl₃) δ 8.75 (d, *J =* 5.3 Hz, 1H), 8.25 (s, 1H), 7.79-7.67 (m, 2H), 6.72 (t, *J =* 7.7 Hz, 1H), 6.55-6.48 (m, 2H), 3.63-3.61 (m, 1H), 3.52-3.35 (m, 2H), 3.32 (t, *J* = 11.3 Hz, 3H), 3.23-3.17 (m, 2H), 3.14-3.09 (m, 3H), 2.90-2.78 (m, 5H), 2.42-2.36 (m, 2H), 2.27-2.13 (m, 2H), 2.02-1.99 (m, 1H). ¹⁹F NMR (376 MHz, CDCl₃) δ -75.72 (s, 3F). MS m/z (ESI): 349.2 [M+H]⁺.

### Example 59: 2-(3-((6bR,10aS)-3-Methyl-2,3,6b,9,10,10a-hexahydro-1H-pyrido[3',4':4,5]pyrrolo[1,2,3-de]quinoxalin-8(7H)-yl)propyl)benzonitrile

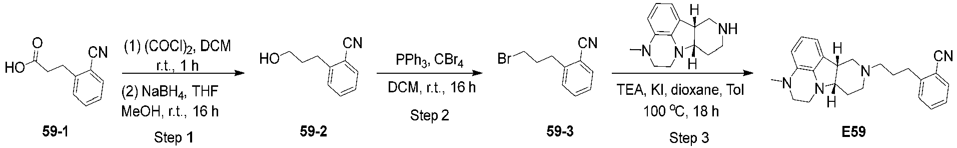

Step 1: At room temperature, 3-(2-cyanophenyl)propanoic acid (500.0 mg, 2.85 mmol) was dissolved in dichloromethane (5 mL), and oxalyl chloride (543.4 mg, 4.28 mmol) was added. The mixture was stirred at room temperature for 1 h. The reaction solution was concentrated *in vacuo* to give a crude product. The crude product was dissolved in THF/MeOH (4 mL/4 mL), and sodium borohydride (539.9 mg, 14.27 mmol) was added. The reaction solution was stirred at room temperature for 16 h. After the reaction was complete, the reaction solution was diluted with methanol, concentrated *in vacuo,* added to 50 mL of water, and extracted with ethyl acetate (40 mL × 3). The organic phases were washed with saturated brine, dried over anhydrous sodium sulfate, combined, and concentrated to give a crude product, and the crude product was purified by silica gel column chromatography (PE/EA = 100/0-60/40) to give 2-(3-hydroxypropyl)benzonitrile (300 mg, yield: 59.2%) as a transparent oil. MS m/z (ESI): 162.1 [M+H]⁺.

Step 2: At room temperature, 2-(3-hydroxypropyl)benzonitrile (300.0 mg, 1.86 mmol) and carbon tetrabromide (925.8 mg, 2.79 mmol) were dissolved in dichloromethane (35 mL), and triphenylphosphine (732.2 mg, 2.79 mmol) was added. The reaction solution was stirred at room temperature for 16 h. After the reaction was complete, the reaction mixture was concentrated *in vacuo* to give a crude product. The crude product was purified by silica gel column chromatography (PE/EA = 100/0-90/10) to give 2-(3-bromopropyl)benzonitrile (250 mg, yield: 59.9%) as a transparent oil. MS m/z (ESI): 226.1 [M+H]⁺.

Step 3: At room temperature, 2-(3-bromopropyl)benzonitrile (48.8 mg, 0.22 mmol) was dissolved in 1,4-dioxane/toluene (1.5 mL/1.5 mL), and (6bR,10aS)-3-methyl-2,3,6b,7,8,9,10,10a-octahydro-1H-pyrido[3',4':4,5]pyrrolo[1,2,3-de]quinoxaline (50.0 mg, 0.22 mmol), potassium iodide (72.4 mg, 0.44 mmol), and triethylamine (66.2 mg, 0.65 mmol) were added. The reaction solution was stirred at 100 °C for 18 h. The reaction solution was cooled to room temperature and concentrated *in vacuo* to give a crude product. The crude product was purified by preparative chromatography to give 2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)benzonitrile (14.34 mg, yield: 16.8%) as a yellow solid. ¹H NMR (400 MHz, CDCl₃) δ 7.61 (d, *J* = 7.7 Hz, 1H), 7.56-7.53 (m, 1H), 7.41 (d, *J* = 7.7 Hz, 1H), 7.32 (t, *J* = 7.6 Hz, 1H), 6.69 (t, *J =* 7.7 Hz, 1H), 6.52 (d, *J* = 7.3 Hz, 1H), 6.43 (d, *J =* 7.9 Hz, 1H), 3.66-3.57 (m, 2H), 3.29-3.24 (m, 4H), 3.22 (d, *J* = 10.8 Hz, 1H), 2.93-2.80 (m, 9H), 2.52 (t, *J* = 14.0 Hz, 1H), 2.38 (t, *J =* 11.8 Hz, 1H), 2.23-2.20 (m, 2H), 2.10 (d, *J =* 15.3 Hz, 1H). MS m/z (ESI): 373.2 [M+H]⁺.

### Example 60: 7-(3-((6bR,10aS)-3-Methyl-2,3,6b,9,10,10a-hexahydro-1H-pyrido[3',4':4,5]pyrrolo[1,2,3-de]quinoxalin-8(7H)-yl)propyl)-2,3-dihydrofuro[2,3-c]pyridine

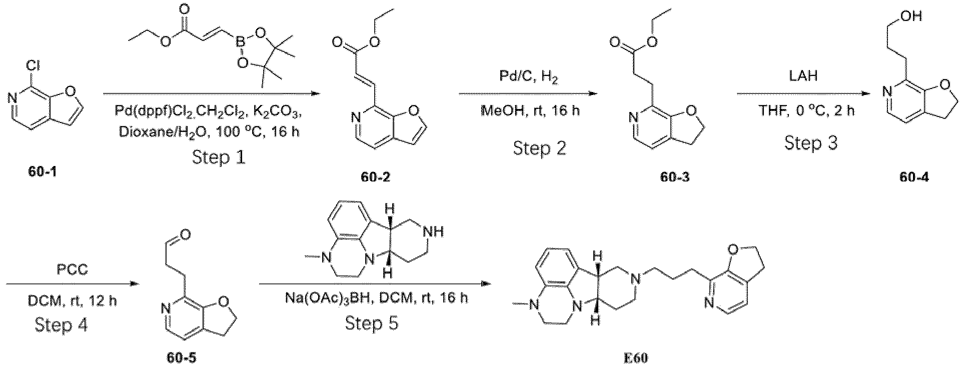

Step 1: The compounds 7-chlorofuro[2,3-c]pyridine (300 mg, 1.96 mmol), ethyl (E)-3-(4,4,5,5-tetramethyl-1,3,2-dioxaborolan-2-yl)acrylate (660 mg, 2.92 mmol), Pd(dppf)Cl₂.CH₂Cl₂ complex (30 mg, 0.04 mmol), and potassium carbonate (540 mg, 3.92 mmol) were added together to a 25 mL single-neck reaction flask, and subsequently, dioxane (4 mL) and water (1 mL) were separately added. After the addition, the mixture was purged 3 times with nitrogen, slowly heated to 100 °C, and stirred for 16 h. After LCMS analysis showed that the reaction had been complete, the reaction solution was filtered. Subsequently, water (30 mL) was added to the reaction solution, and extraction was performed with ethyl acetate (3 × 20 mL). The organic phases were combined, washed with saturated brine (1 × 50 mL), dried over anhydrous sodium sulfate, and concentrated to dryness by rotary evaporation under reduced pressure, and the crude product was purified and separated by normal-phase column chromatography (eluent gradient: 10% EA/PE) to give the compound ethyl (E)-3-(furo[2,3-c]pyridin-7-yl)acrylate (white solid, 180 mg, yield: 42%). MS m/z (ESI): 218.4 [M+H]⁺.

Step 2: The compound ethyl (E)-3-(furo[2,3-c]pyridin-7-yl)acrylate (50 mg, 0.23 mmol) was added to a 25 mL single-neck reaction flask, and subsequently, methanol (10 mL) and 10% Pd/C (wet basis, 10 mg) were separately added. After the addition, the mixture was purged 3 times with hydrogen and stirred at room temperature for 16 h. After LCMS analysis showed that the reaction had been complete, the reaction solution was filtered under vacuum, and the filtrate was concentrated under reduced pressure to give the target compound ethyl 3-(2,3-dihydrofuro[2,3-c]pyridin-7-yl)propanoate (crude, 50 mg, yield: 99%). The crude product was directly used in the next step without further purification. MS m/z (ESI): 222.5 [M+H]⁺.

Step 3: The compound ethyl 3-(2,3-dihydrofuro[2,3-c]pyridin-7-yl)propanoate (50 mg, 0.23 mmol) was added to a 25 mL three-neck reaction flask, and anhydrous tetrahydrofuran (3 mL) was subsequently added. Lithium aluminum hydride (25 mg, 0.69 mmol) was added portionwise under nitrogen and ice-water bath conditions, and the mixture was stirred for 0.5 h in the ice-water bath. After LCMS analysis showed that the reaction had been complete, Na₂SO₄.10H₂O was slowly added to the reaction solution to quench the reaction, and the mixture was filtered. The filter cake was washed with 10% MeOH/DCM (10 mL), and the filtrate was concentrated under reduced pressure. The crude product was purified and separated by normal-phase column chromatography (eluent gradient: 30% EA/PE) to give the compound 3-(2,3-dihydrofuro[2,3-c]pyridin-7-yl)propan-1-ol (pale yellow oil, 25 mg, yield: 62%). MS m/z (ESI): 180.5 [M+H]⁺.

Step 4: The compounds 3-(2,3-dihydrofuro[2,3-c]pyridin-7-yl)propan-1-ol (25 mg, 0.14 mmol) and anhydrous dichloromethane (3 mL) were added together to a 10 mL single-neck reaction flask, and PCC (60 mg, 0.28 mmol) was subsequently added. After the addition, the mixture was stirred at room temperature for 16 h. After LCMS analysis showed that the reaction had been complete, dichloromethane (10 mL) was added to the reaction solution, and the system was subsequently made alkaline with a saturated sodium bicarbonate solution and extracted with dichloromethane (2 × 10 mL). The organic phases were combined, washed with saturated brine (1 × 10 mL), dried over anhydrous sodium sulfate, and concentrated to dryness by rotary evaporation under reduced pressure to give the compound 3-(2,3-dihydrofuro[2,3-c]pyridin-7-yl)propanal (crude, reddish brown oil, 30 mg). The crude product was directly used in the next step without further purification. MS m/z (ESI): 258.5 [M+H]⁺.

Step 5: Preparation of 7-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)-2,3-dihydrofuro[2,3-c]pyridine

The compounds 3-(2,3-dihydrofuro[2,3-c]pyridin-7-yl)propanal (10 mg, 0.06 mmol, crude) and (6bR,10aS)-3-methyl-2,3,6b,7,8,9,10,10a-octahydro-1H-pyrido[3',4':4,5]pyrrolo[1,2,3-de]quinoxaline (13 mg, 0.06 mmol) were added to a 10 mL reaction flask, and DIEA (35 mg, 0.30 mmol) and anhydrous DCM (2 mL) were subsequently added. After the addition, the mixture was stirred at room temperature for 16 h. After LCMS analysis showed that the reaction had been complete, the reaction solution was concentrated to dryness by rotary evaporation under reduced pressure, and the filtrate was directly separated by reversed-phase preparative chromatography (C18 column, eluent gradient: acetonitrile/(water + 0.05% NH₄HCO₃)) to give the title compound 7-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)-2,3-dihydrofuro[2,3-c]pyridine (pale yellow solid, 8.0 mg, yield: 36%). 1H NMR (500 MHz, MeOD) δ 7.92 (d, *J =* 4.9 Hz, 1H), 7.17 (d, *J =* 4.9 Hz, 1H), 6.61-6.55 (m, 1H), 6.47 (d, *J* = 7.2 Hz, 1H), 6.40 (d, *J =* 7.9 Hz, 1H), 4.60 (t, *J =* 8.9 Hz, 2H), 3.52-3.46 (m, 1H), 3.36-3.32 (m, 1H), 3.30-3.24 (m, 3H), 3.14-3.10 (m, 1H), 3.10-3.04 (m, 1H), 2.88-2.81 (m, 4H), 2.76-2.69 (m, 4H), 2.44-2.33 (m, 2H), 2.26 (td, *J =* 12.1, 3.0 Hz, 1H), 2.02-1.97 (m, 1H), 1.94-1.85 (m, 4H). MS m/z (ESI): 391.9 [M+H]⁺.

### Example 61: 4-(2-(3-((6bR,10aS)-3-Methyl-2,3,6b,9,10,10a-hexahydro-1H-pyrido[3',4':4,5]pyrrolo[1,2,3-de]quinoxalin-8(7H)-yl)propyl)phenyl)morpholine

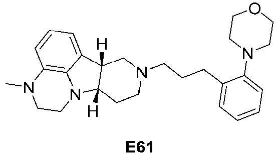

E61 was prepared with reference to the synthesis method of Example 1. ¹H NMR (400 MHz, CDCl₃) δ 7.01 (t, *J =* 7.6 Hz, 1H), 6.92 (d, *J* = 7.1 Hz, 1H), 6.66 (t, *J* = 7.6 Hz, 1H), 6.60-6.47 (m, 3H), 6.41 (d, *J =* 7.9 Hz, 1H), 3.73-3.57 (m, 5H), 3.46-3.42 (m, 2H), 3.34-3.20 (m, 6H), 3.10-3.08 (m, 1H), 2.94 (d, *J =* 10.4 Hz, 1H), 2.87-2.82 (m, 4H), 2.73 (t, *J =* 5.9 Hz, 4H), 2.48 (t, *J =* 11.2 Hz, 1H), 2.18-2.09 (m, 2H), 1.92-1.89 (m, 3H). MS m/z (ESI): 433.2 [M+H]⁺.

### Example 62: (6bR,10aS)-8-(3-(4-Methoxypyridin-3-yl)prop-2-yn-1-yl)-3-methyl-2,3,6b,7,8,9,10,10a-octahydro-1H-pyrido[3',4":4,5]pyrrolo[1,2,3-de]quinoxaline

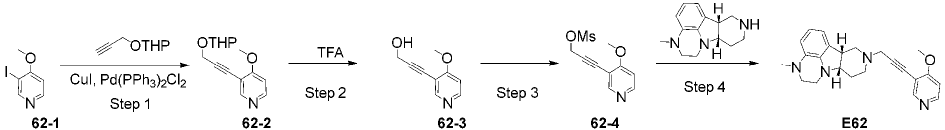

Step 1: 3-Iodo-4-methoxypyridine (1.5 g, 6.38 mmol) was added to a 100 mL reaction flask, and bis(triphenylphosphine)palladium(II) dichloride (0.13 g, 0.19 mmol) and cuprous iodide (0.12 g, 0.64 mmol) were subsequently added. Then, DMF (20 mL), TEA (30 mL), and 2-(prop-2-yn-1-yloxy)tetrahydro-2H-pyran (1.3 g, 9.6 mmol) were added in a nitrogen atmosphere. Then, the mixture was stirred at room temperature for 2 h. The reaction mixture was concentrated. Then, water (50 mL) was added to the reaction mixture, and extraction was performed with ethyl acetate (50 mL × 3). The organic phase was washed with brine (50 mL × 3) and concentrated, and the resulting residue was purified by normal-phase column chromatography (dichloromethane:methanol = 20:1) to give the compound 4-methoxy-3-(3-((tetrahydro-2H-pyran-2-yl)oxy)prop-1-yn-1-yl)pyridine (white solid, 1.2 g, yield: 76%). MS m/z (ESI): 248.5 [M+H]⁺.

Step 2: The compound 4-methoxy-3-(3-((tetrahydro-2H-pyran-2-yl)oxy)prop-1-yn-1-yl)pyridine (400 mg, 1.62 mmol) was added to a 100 mL reaction flask, and dichloromethane (10 mL) and TFA (5 mL) were subsequently added. The mixture was stirred at room temperature for 1 h. The reaction solution was concentrated. Then, saturated sodium bicarbonate (50 mL) was added, and extraction was performed with dichloromethane (2 × 50 mL). The extract was concentrated, and the residue was purified and separated by normal-phase column chromatography (dichloromethane:methanol = 10:1) to give the compound 3-(4-methoxypyridin-3-yl)prop-2-yn-1-ol (colorless liquid, 200 mg, yield: 75.2%). MS m/z (ESI): 164.4 [M+H]⁺.

Step 3: The compound 4-methoxy-3-(3-((tetrahydro-2H-pyran-2-yl)oxy)prop-1-yn-1-yl)pyridine (100 mg, 0.61 mmol) was added to a 25 mL reaction flask, and DCM (50 mL) was subsequently added. Then, DIEA (390 mg, 3 mmol) and Ms₂O (320 mg, 1.84 mmol) were sequentially added. After the addition, the mixture was stirred at room temperature for 1 h. The reaction mixture was extracted with DCM (2 × 20 mL), and the organic phase was washed with saturated brine (2 × 20 mL) and concentrated to give the compound 3-(4-methoxypyridin-3-yl)prop-2-yn-1-yl mesylate (colorless liquid, 120 mg, yield: 81.3%). MS m/z (ESI): 242.5 [M+H]⁺.

Step 4: The compound 3-(4-methoxypyridin-3-yl)prop-2-yn-1-yl mesylate (50 mg, 0.21 mmol) and the starting material (6bR,10aS)-3-methyl-2,3,6b,7,8,9,10,10a-octahydro-1H-pyrido[3',4':4,5]pyrrolo[1,2,3-de]quinoxaline (57 mg, 0.25 mmol) were added to a 25 mL reaction flask, and DMSO (5 mL) was subsequently added. Then, DIPEA (81 mg, 0.63 mmol) was added. After the addition, the mixture was stirred at 60 °C for 16 h. The reaction mixture was added to ethyl acetate (30 mL) and washed with water (30 mL) and brine (50 mL). The organic phase was concentrated, and the resulting residue was preliminarily purified by normal-phase column chromatography (dichloromethane:methanol = 10:1) to give a crude product (50 mg). Then, 20 mg of the crude product was purified by reversed-phase column chromatography (acetonitrile/(water + 0.05% NH₄HCO₃)) to give the compound (6bR,10aS)-8-(3-(4-methoxypyridin-3-yl)prop-2-yn-1-yl)-3-methyl-2,3,6b,7,8,9,10,10a-octahydro-1H-pyrido[3',4':4,5]pyrrolo[1,2,3-de]quinoxaline (gray solid, 10 mg, yield: 31.5%). ¹H NMR (500 MHz, DMSO-*d*₆) δ 8.44 (s, 2H), 7.12 (d, *J =* 5.2 Hz, 1H), 6.55 (d, *J =* 8.4 Hz, 1H), 6.48(d, *J* =6.4 Hz, 1H), 6.38 (d, *J =* 6.4 Hz, 1H), 3.83 (s, 3H), 3.47-3.40 (m, 2H), 3.29 (s, 3H), 3.17 (s, 2H), 2.79 (s, 3H), 2.76-2.61 (m, 2H), 2.51 (s, 2H), 2.25-1.70 (m, 3H). MS m/z (ESI): 375.6 [M+H]⁺.

### Example 63: (6bR,10aS)-8-((E)-3-(Benzofuran-7-yl)allyl)-3-methyl-2,3,6b,7,8,9,10,10a-octahydro-1H-pyrido[3',4':4,5]pyrrolo[1,2,3-de]quinoxaline

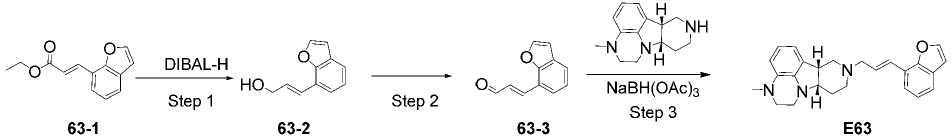

Step 1: Ethyl (E)-3-(benzofuran-7-yl)acrylate (65 mg, 0.3 mmol) was dissolved in THF (2.0 mL), and the solution was cooled to -78 °C. A 1 M solution of DABL-H in tetrahydrofuran (0.45 mL, 0.45 mmol) was added, and the mixture was stirred at -78 °C for 1 h. Sodium sulfate decahydrate was added, and the mixture was stirred for 15 min, filtered, and concentrated. The resulting residue was purified by normal-phase chromatography to give the compound (E)-3-(benzofuran-7-yl)prop-2-en-1-ol (yellow oil, 21 mg, yield: 40%). MS m/z (ESI): 175.3 [M+H]⁺.

Step 2: The compound (E)-3-(benzofuran-7-yl)prop-2-en-1-ol (21 mg, 0.12 mmol) was dissolved in DCM (2.0 mL), and activated MnO₂ (104 mg, 1.2 mmol) was added. The mixture was stirred at 20 °C for 3 h and filtered to give the compound (E)-3-(benzofuran-7-yl)acrylaldehyde (yellow oil, 20 mg), which was directly used in the next step. MS m/z (ESI): 173.3 [M+H]⁺.

Step 3: At room temperature, (6bR,10aS)-3-methyl-2,3,6b,7,8,9,10,10a-octahydro-1H-pyrido[3',4':4,5]pyrrolo[1,2,3-de]quinoxaline (25 mg, 0.10 mmol) and (E)-3-(benzofuran-7-yl)acrylaldehyde (20 mg, 0.12 mmol) were dissolved in DCM (2.5 mL), and triethylamine (5.0 mg, 0.05 mmol) and acetic acid (6.0 mg, 0.1 mmol) were added. After 10 min of stirring, NaBH(OAc)₃ (120 mg, 0.6 mmol) was added, and the mixture was stirred at 20 °C for 16 h, concentrated, filtered, and then purified and separated by prep-HPLC (eluent (v/v): acetonitrile/(water + 0.05% NH₄HCO₃) = 30%-70%) to give the compound (6bR,10aS)-8-((E)-3-(benzofuran-7-yl)allyl)-3-methyl-2,3,6b,7,8,9,10,10a-octahydro-1H-pyrido[3',4':4,5]pyrrolo[1,2,3-de]quinoxaline (pale yellow oil, 10.1 mg, yield: 26%). ¹H NMR (500 MHz, DMSO-*d*₆) δ 8.05 (s, 1H), 7.55 (d, *J* = 5.0 Hz, 1H), 7.36 (d, *J =* 5.0 Hz, 1H), 7.23 (t, *J* = 7.5 Hz, 1H), 7.00 (s, 1H), 6.79-6.71 (m, 2H), 6.50 (t, *J =* 7.5 Hz, 1H), 6.41 (d, *J =* 5.0 Hz, 1H), 6.34 (d, *J =* 5.0 Hz, 1H), 3.47-3.26 (m, 4H), 3.21-3.04 (m, 3H), 2.89-2.85 (m, 1H), 2.71 (s, 3H), 2.69-2.66 (m, 2H), 2.24-2.21 (m, 1H), 1.95-1.82 (m, 3H). MS m/z (ESI): 386.9 [M+H]⁺.

### Example 64: 4-(3-((6bR,10aS)-3-Methyl-2,3,6b,9,10,10a-hexahydro-1H-pyrido[3',4':4,5]pyrrolo[1,2,3-de]quinoxalin-8(7H)-yl)propyl)-2,3-dihydrofuro[3,2-c]pyridine

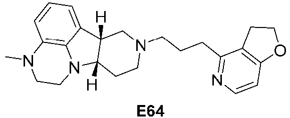

E64 was prepared with reference to the synthesis method of Example 60. ¹H NMR (500 MHz, DMSO-*d*₆) δ 8.13 (d, *J =* 5.4 Hz, 1H), 6.66 (d, *J* = 5.4 Hz, 1H), 6.51 (t, *J* = 7.6 Hz, 1H), 6.42 (d, *J* = 7.2 Hz, 1H), 6.33 (d, *J =* 7.8 Hz, 1H), 4.60 (t, *J =* 8.8 Hz, 2H), 3.33-3.27 (m, 3H), 3.18 (t, *J =* 8.8 Hz, 2H), 3.12-3.08 (m, 1H), 3.01-2.95 (m, 1H), 2.78 (s, 3H), 2.74 (dd, *J =* 11.9, 6.1 Hz, 1H), 2.70-2.66 (m, 1H), 2.63 (dd, *J* = 8.6, 6.6 Hz, 2H), 2.56 (d, *J* = 11.4 Hz, 1H), 2.31-2.21 (m, 2H), 2.10-2.03 (m, 1H), 1.88 (dd, *J* = 14.4, 2.9 Hz, 1H), 1.84-1.71 (m, 4H). MS m/z (ESI): 391.8 [M+H]⁺.

### Example 65-1: (6bR,10aS)-8-(3-(2,3-Dihydrobenzofuran-7-yl)propyl)-3-ethyl-2,3,6b,7,8,9,10,10a-octahydro-1H-pyrido[3',4':4,5]pyrrolo[1,2,3-de]quinoxaline

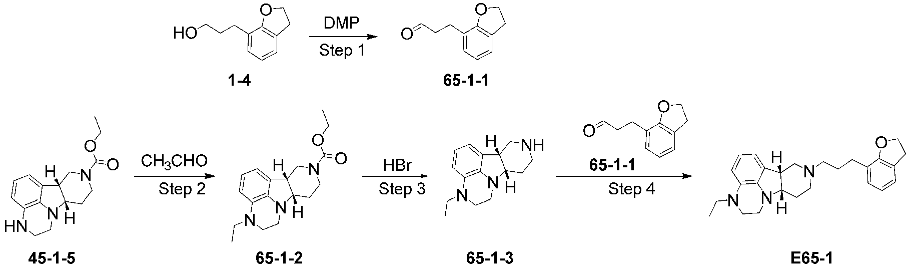

Step 1: At room temperature, the compounds 3-(2,3-dihydrobenzofuran-7-yl)propan-1-ol (3 g, 16.8 mmol) and dichloromethane (50 mL) were added together to a 100 mL single-neck flask, and Dess-Martin reagent (21.4 g, 50.5 mmol) was subsequently added. After the addition, the mixture was stirred at room temperature for 5 h. After TLC analysis showed that the reaction had been complete, the reaction solution was filtered through diatomaceous earth, and the filtrate was made alkaline with a saturated aqueous sodium bicarbonate solution and extracted with DCM (50 mL × 2). The organic phases were combined, washed with saturated brine (50 mL × 1), dried over anhydrous Na₂SO₄, and concentrated to dryness by rotary evaporation under reduced pressure, and the crude product was separated and purified by column chromatography (EtOAc/PE = 0-20%) to give compound 65-1-1 (colorless oil, 2.5 g, yield: 84%).

Step 2: Compound 45-1-5 (2.0 g, 7.0 mmol), an aqueous acetaldehyde solution (40% wt, 10 mL), and methanol (40 mL) were added together to a 100 mL single-neck flask, and Na(OAc)₃BH (8.8 g, 42 mmol) was subsequently added portionwise. After the addition, the mixture was stirred at room temperature for 3 h. After LCMS analysis showed that the reaction had been complete, the reaction solution was concentrated under reduced pressure to remove most of the solvent, and the crude product was subsequently dissolved in EA. The solution was washed with water (50 mL × 1) and with a saturated NaHCO₃ solution (50 mL × 1) and extracted with EA (30 mL × 2). The organic phases were combined, washed with saturated brine (50 mL × 1), dried over anhydrous sodium sulfate, and concentrated to dryness by rotary evaporation under reduced pressure, and the crude product was separated and purified by column chromatography (EtOAc/PE = 0-25%) to give compound 65-1-2 (pale yellow oil, 1.8 g, yield: 82%). ¹H NMR (500 MHz, CDCl₃) δ 6.65 (t, *J* = 7.7 Hz, 1H), 6.51 (d, *J* = 7.3 Hz, 1H), 6.40 (d, *J* = 7.9 Hz, 1H), 4.21-4.03 (m, 3H), 3.88 (s, 1H), 3.73-3.64 (m, 1H), 3.45-3.35 (m, 1H), 3.35-3.27 (m, 3H), 3.26-3.07 (m, 3H), 2.93-2.72 (m, 2H), 1.92-1.80 (m, 2H), 1.28 (t, *J =* 7.1 Hz, 3H), 1.15 (t, *J =* 7.1 Hz, 3H). MS m/z (ESI): 316.4 [M+H]⁺.

Step 3: 65-1-3 was prepared with reference to the synthesis method in step 2 of Example 73-1. MS m/z (ESI): 244.5 [M+H]⁺.

Step 4: E65-1 was prepared with reference to the synthesis method in step 3 of Example 73-1. ¹H NMR (500 MHz, CDCl₃) δ 7.03 (dd, *J =* 7.3, 0.8 Hz, 1H), 6.92 (d, *J* = 7.5 Hz, 1H), 6.76 (t, *J =* 7.4 Hz, 1H), 6.64 (t, *J =* 7.7 Hz, 1H), 6.47 (d, *J =* 7.2 Hz, 1H), 6.39 (d, *J* = 7.9 Hz, 1H), 4.53 (t, *J =* 8.7 Hz, 2H), 3.72-3.64 (m, 1H), 3.44-3.36 (m, 1H), 3.32-3.17 (m, 7H), 3.00-2.90 (m, 1H), 2.83-2.70 (m, 2H), 2.58 (dd, *J =* 14.5, 7.6 Hz, 2H), 2.50-2.28 (m, 3H), 2.07-1.93 (m, 3H), 1.91-1.83 (m, 2H), 1.14 (t, *J =* 7.1 Hz, 3H). MS m/z (ESI): 404.9 [M+H]⁺.

### Example 65-2: (6bS,10aR)-8-(3-(2,3-Dihydrobenzofuran-7-yl)propyl)-3-ethyl-2,3,6b,7,8,9,10,10a-octahydro-1H-pyrido[3',4':4,5]pyrrolo[1,2,3-de]quinoxaline

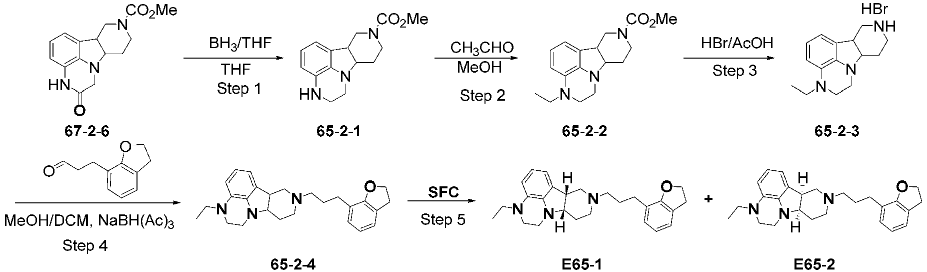

Step 1: 67-2-6 (2.10 g, 7.31 mmol) was dissolved in THF (20.0 mL), and BH₃/THF (21.9 mL, 1 M) was added. The mixture was left to react at 70 °C for 3 h. LCMS analysis showed that the reaction had been complete. Water was added, and extraction was performed with EA. The extract was dried over sodium sulfate, filtered, and concentrated, and the residue was purified by silica gel column chromatography (PE:EA = 5:1) to give compound 65-2-1 (yellow solid, 1.80 g, yield: 90%). MS m/z (ESI): 274.4 [M+H]⁺.

Step 2: Methyl 2,3,6b,7,10,10a-hexahydro-1H-pyrido[3',4':4,5]pyrrolo[1,2,3-de]quinoxaline-8(9H)-carboxylate (1.70 g, 6.22 mmol) was dissolved in MeOH (600.0 mL), and an aqueous acetaldehyde solution (9.12 g, 62.1 mmol) was added. The mixture was left to react at 25 °C for 3 h. LCMS analysis showed that the reaction had been complete. Water was added, and extraction was performed with EA. The extract was dried over sodium sulfate, filtered, and concentrated, and the residue was purified by silica gel column chromatography (PE:EA = 3:1) to give compound 65-2-2 (yellow solid, 1.04 g, yield: 55%). MS m/z (ESI): 302.2 [M+H]⁺.

Step 3: 65-2-2 (1.00 g, 3.31 mmol) was dissolved in a solution of hydrobromic acid in acetic acid (20 mL), and the solution was left to react at 25 °C for 20 h. LCMS analysis showed that the reaction had been complete. The reaction solution was concentrated to give compound 65-2-3 (gray solid, 1.04 g, yield: 96%). MS m/z (ESI): 244.2 [M+H]⁺.

Step 4: In a nitrogen atmosphere, 65-2-3 (400 mg, 1.64 mmol) was dissolved in MeOH (200 mL) and DCM (120 mL), and 3-(2,3-dihydrobenzofuran-7-yl)propanal (347 mg, 1.97 mmol) and NaBH(OAc)₃ (1.73 g, 8.21 mmol) were added. The mixture was left to react at 25 °C for 1 h. LCMS analysis showed that the reaction had been complete. Water was added, and extraction was performed with EA. The extract was dried over sodium sulfate, filtered, and concentrated, and the residue was purified by silica gel column chromatography (DCM:MeOH = 10:1) to give compound 65-2-4 (yellow solid, 1.04 g, yield: 55%). MS m/z (ESI): 404.3 [M+H]⁺.

Step 5: 65-2-4 (100 mg, 0.248 mmol) was resolved by SFC (Daicel ChiralPak IJ, 40 mm I.D. × 250 mm, 10 µm; mobile phase: n-hexane/ethanol [0.1% NH₃.H₂O (V/V)] = 95/5; flow rate: 80 mL/min) to give E65-1 (PK1: 42.8 mg, purity: 98%, yield: 42%, ee 98%, RT = 4.512, OROT = +13.8) as a white solid and E65-2 (PK2: 37.4 mg, purity: 97%, yield: 37%, ee 100%, RT = 5.303, OROT = -10.2)) as a white solid. E65-1: ¹H NMR (400 MHz, DMSO-d6) ¹H NMR (400 MHz, DMSO) δ 7.04 (d, *J* = 7.3 Hz, 1H), 6.91 (d, *J* = 7.4 Hz, 1H), 6.73 (t, *J* = 7.4 Hz, 1H), 6.50 (t, *J* = 7.6 Hz, 1H), 6.37 (d, *J* = 7.2 Hz, 1H), 6.31 (d, *J* = 7.8 Hz, 1H), 4.49 (t, *J* = 8.7 Hz, 2H), 3.51 (d, *J* = 8.6 Hz, 1H), 3.35 (d, *J* = 7.2 Hz, 1H), 3.30-3.22 (m, 2H), 3.21-3.11 (m, 3H), 3.07 (d, *J* = 4.0 Hz, 1H), 3.03-2.94 (m, 1H), 2.75 (d, *J* = 4.3 Hz, 1H), 2.64-2.52 (m, 2H), 2.47 (s, 2H), 2.24 (dd, *J* = 15.9, 7.1 Hz, 2H), 2.08 (s, 1H), 1.89 (d, *J* = 14.4 Hz, 1H), 1.81-1.62 (m, 4H), 1.04 (t, *J* = 7.0 Hz, 3H). MS m/z (ESI): 404.3 [M+H]⁺. E65-2: ¹H NMR (400 MHz, DMSO-d6) δ ¹H NMR (400 MHz, DMSO-d6) δ 7.04 (d, *J* = 7.2 Hz, 1H), 6.92 (d, *J* = 7.6 Hz, 1H), 6.73 (t, *J* = 7.2 Hz, 1H), 6.50 (t, *J* = 7.6 Hz, 1H), 6.37 (d, *J* = 7.2 Hz, 1H), 6.31 (d, *J* = 7.6 Hz, 1H), 4.49 (t, *J* = 8.8 Hz, 2H), 3.60-3.45 (m, 1H), 3.41-3.23 (m, 3H), 3.23-3.11 (m, 3H), 3.10-3.04 (m, 1H), 3.04-2.90 (m, 1H), 2.82-2.71 (m, 1H), 2.63-2.53 (m, 2H), 2.49-2.41 (m, 2H), 2.31-2.17 (m, 2H), 2.15-2.01 (m, 1H), 1.95-1.83 (m, 1H), 1.83-1.60 (m, 4H), 1.05 (t, *J* = 7.2 Hz, 3H). MS m/z (ESI): 404.3 [M+H]⁺.

### Example 66: (6bR,10aS)-8-(3-(2,3-Dihydrobenzofuran-7-yl)propyl)-3-(oxetan-3-yl)-2,3,6b,7,8,9,10,10a-octahydro-1H-pyrido[3',4':4,5]pyrrolo[1,2,3-de]quinoxaline

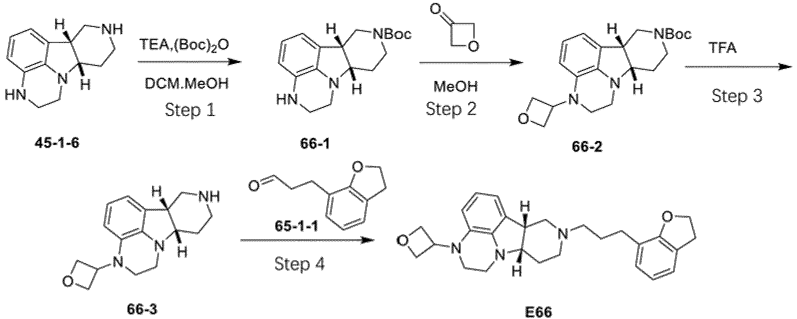

Step 1: 45-1-6 (1 g, 2.67 mmol) and TEA (13.5 g, 13.3 mmol) were dissolved in DCM/MeOH = 10:1 (150 mL), and (Boc)₂O (540 mg, 2.67 mmol) was then added. The mixture was left to react at room temperature for 6 h, and LCMS showed that the reaction had been complete. The reaction mixture was extracted with DCM, and the extract was purified by silica gel column chromatography (PE/EA = 100/0-70/30) to give product 66-1 (600 mg, white solid, yield: 71.3%). MS m/z (ESI): 316.3 [M+H]⁺.

Step 2: 66-1 (70 mg, 0.22 mmol) was dissolved in MeOH (50 mL), oxetan-3-one (80 mg, 1.11 mmol) was then added, and Na(OAc)₃BH (71 mg, 1.11 mmol) was rapidly added. The mixture was left to react for 1 h, and LCMS showed that the reaction had been complete. The reaction mixture was extracted with DCM, and the extract was purified by silica gel column chromatography (PE/EA = 100/0-60/40) to give product 66-2 (70 mg, colorless liquid, yield: 84.9%). MS m/z (ESI): 372.1 [M+H]⁺.

Step 3: Compound 66-2 (60 mg, 0.16 mmol) was added to a 25 mL reaction flask, and dichloromethane (5 mL) and TFA (2 mL) were subsequently added. The mixture was stirred at room temperature for 1 h. The reaction solution was concentrated to give compound 66-3 (colorless liquid, 60 mg, TFA salt, yield: 96.3%). MS m/z (ESI): 272.2 [M+H]⁺.

Step 4: 66-3 (30 mg, 0.11 mmol) was dissolved in MeOH (5 mL) and DCM (5 mL), 65-1-1 (21 mg, 0.12 mmol) was then added, and Na(OAc)₃BH (116 mg, 0.55 mmol) was rapidly added. The mixture was left to react for 1 h, and LCMS showed that the reaction had been complete. The reaction mixture was extracted with DCM, and the extract was concentrated, filtered, and then purified and separated by prep-HPLC (eluent (v/v): acetonitrile/(water + 0.05% NH₄HCO₃) = 30%-70%) to give compound E66 (10 mg, white solid, yield: 21.1%). ¹H NMR (500 MHz, CDCl₃) δ 7.03 (d, *J* = 7.3 Hz, 1H), 6.92 (d, *J* = 7.5 Hz, 1H), 6.76 (t, *J* = 7.4 Hz, 1H), 6.59 (dt, *J* = 14.0, 7.0 Hz, 2H), 6.16 (d, *J* = 7.7 Hz, 1H), 4.86 (tt, *J* = 19.2, 6.5 Hz, 4H), 4.75 (p, *J* = 6.9 Hz, 1H), 4.53 (t, *J* = 8.7 Hz, 2H), 3.55-3.36 (m, 3H), 3.30-3.23 (m, 1H), 3.19 (t, *J* = 8.7 Hz, 3H), 2.90 (s, 1H), 2.82 (td, *J* = 9.8, 3.2 Hz, 1H), 2.72 (s, 1H), 2.60-2.50 (m, 2H), 2.41 (d, *J* = 7.8 Hz, 2H), 2.34-2.22 (m, 1H), 1.97 (s, 3H), 1.85 (dt, *J* = 15.1, 7.5 Hz, 2H). MS m/z (ESI): 432.8 [M+H]⁺.

### Example 67-1: (6bR,10aS)-8-(3-(2,3-Dihydrobenzofuran-7-yl)propyl)-6b,7,8,9,10,10a-hexahydro-1H-pyrido[3',4':4,5]pyrrolo[1,2,3-de]quinoxalin-2(3H)-one

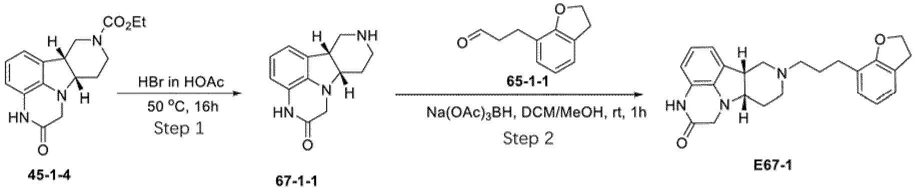

Step 1: 45-1-4 (5 g, 16.6 mmol) and 50 mL of a solution of HBr in HOAc were left to react at 50 °C for 16 h. LCMS showed that the reaction had been complete. The reaction solution was cooled to room temperature and filtered, and the solid was washed with EA and dried to give 67-1-1 (6 g, brown solid). MS m/z (ESI): 230.2 [M+H]⁺.

Step 5: 67-1-1 (2 g, 6.47 mmol) was dissolved in 100 mL of DCM and 100 mL of MeOH, and NaBH(OAc)₃ (6.8 g, 32.2 mmol) was then added. Finally, 65-1-1 (910 mg, 5.18 mmol) was added. The mixture was left to react at room temperature for 0.5 h, and LCMS showed that a small amount of 67-1-1 remained. A saturated sodium bicarbonate solution was added to the reaction solution to adjust the pH to 7-8, and DCM and MeOH were removed by concentration. EA and water were added, and the mixture was filtered. The aqueous phase was extracted twice with EA. The organic phases were combined, dried, and concentrated, and the residue was purified by column chromatography (100% DCM-2% MeOH/DCM) to give compound E67-1 (990 mg). Then, the product was purified by HPLC (0-95% 30 min, 0.05% NH₄HCO₃ in H₂O, basic C18 column) to give compound E67-1 (790 mg, yield: 36%). ¹H NMR (400 MHz, CDCl₃) δ 7.66 (s,1H), 7.04 (d, *J* = 7.0 Hz, 1H), 6.92 (d, *J* = *7.5* Hz, 1H), 6.82 (d, *J* = 7.0 Hz, 1H), 6.76 (t, *J* = 7.5 Hz, 1H), 6.72 (t, *J* = 7.5 Hz, 1H), 6.58 (d, *J* = 7.0 Hz, 1H), 4.53(t, *J* = 9.0 Hz, 2H), 3.95(d, *J* = 14.5 Hz, 1H), 3.39(d, *J* = 14.5 Hz, 1H), 3.34-3.32 (m, 2H), 3.20 (t, *J* = 8.5 Hz, 2H), 2.95-2.90 (m, 1H), 2.78-2.71 (m, 1H), 2.57 (t, *J* = 7.5 Hz, 2H), 2.43-2.33 (m, 2H), 2.26-2.19 (m, 1H), 2.03-1.92 (m, 2H), 1.86-1.80 (m, 3H). MS m/z (ESI): 390.7 [M+H]⁺.

### Example 67-2: (6bS,10aR)-8-(3-(2,3-Dihydrobenzofuran-7-yl)propyl)-6b,7,8,9,10,10a-hexahydro-1H-pyrido[3',4":4,5]pyrrolo[1,2,3-de]quinoxalin-2(3H)-one

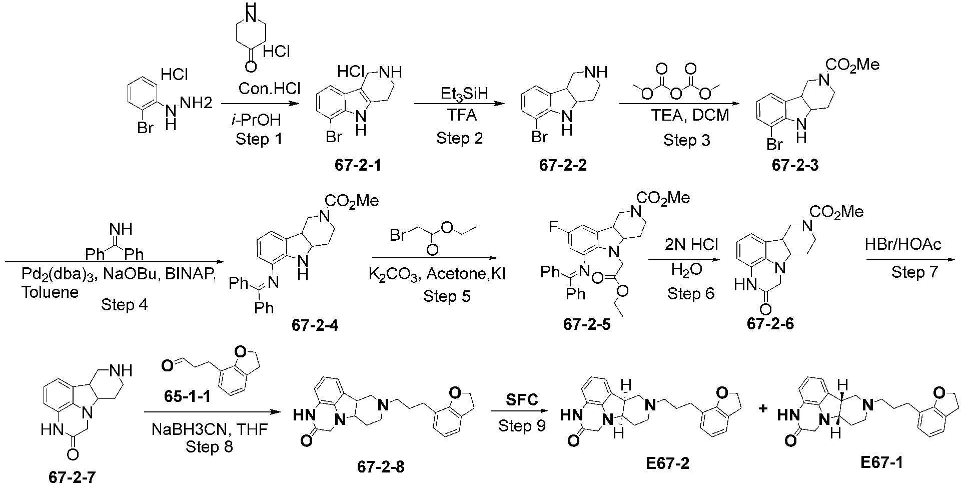

Step 1: (2-Bromophenyl)hydrazine hydrochloride (16.0 g, 71.4 mmol) and piperidin-4-one hydrochloride (12.1 g, 89.8 mmol) were dissolved in isopropanol (200.0 mL), and a concentrated hydrochloric acid solution (16.0 mL) was added. The mixture was left to react at 100 °C for 12 h. LCMS analysis showed that the reaction had been complete. The reaction mixture was filtered and concentrated to give compound 67-2-1 (yellow solid, 20.0 g, yield: 98%). MS m/z (ESI): 251.0, 253.0 [M+H]⁺.

Step 2: 67-2-1 (19.0 g, 60.5 mmol) was dissolved in TFA (20 mL), and triethylsilane (70.3 g, 605 mmol) was added. The mixture was left to react at 40 °C for 48 h. LCMS analysis showed that the reaction had been complete. The reaction solution was concentrated, a saturated sodium bicarbonate solution was added, and extraction was performed with EA. The extract was dried over sodium sulfate, filtered, and concentrated to give compound 67-2-2 (gray solid, 15.0 g, yield: 89%). MS m/z (ESI): 253.0, 255.0 [M+H]⁺.

Step 3: In a nitrogen atmosphere, 67-2-2 (15.0 g, 59.5 mmol) was dissolved in DCM (200 mL), and TEA (13.1 mL, 94.8 mmol) and dimethyl dicarbonate (6.99 g, 52.1 mmol) were added. The mixture was left to react at 25 °C for 3 h. LCMS analysis showed that the reaction had been complete. Water was added, and extraction was performed with EA. The extract was dried over sodium sulfate, filtered, and concentrated, and the residue was purified by silica gel column chromatography (PE:EA = 5:1) to give compound 67-2-3 (white solid, 15.0 g, yield: 81%). MS m/z (ESI): 311.0 [M+H]⁺.

Step 4: In a nitrogen atmosphere, 67-2-3 (14.0 g, 26.9 mmol) was dissolved in toluene (200 mL), and benzophenone imine (5.87 g, 32.3 mmol), NaOt-Bu (5.19 g, 53.9 mmol), and BINAP (0.50 g, 0.810 mmol) were added. The mixture was left to react at 60 °C for 3 h. Pd₂(dba)₃ (0.25 g, 0.270 mmol) was added to the reaction solution. The mixture was left to react at 105 °C for 16 h. LCMS analysis showed that the reaction had been complete. Water was added, and extraction was performed with EA. The extract was dried over sodium sulfate, filtered, and concentrated, and the residue was purified by silica gel column chromatography (PE:EA = 5:1) to give compound 67-2-4 (yellow solid, 8.10 g, yield: 73%). MS m/z (ESI): 412.2 [M+H]⁺.

Step 5: In a nitrogen atmosphere, 67-2-4 (8.10 g, 19.0 mmol) was dissolved in acetone (100 mL), and ethyl 2-bromoacetate (4.27 g, 25.5 mmol), Na₂CO₃ (3.13 g, 29.5 mmol), and KI (4.08 g, 24.6 mmol) were added. The mixture was left to react at 65 °C for 12 h. LCMS analysis showed that the reaction had been complete. Water was added, and extraction was performed with EA. The extract was dried over sodium sulfate, filtered, and concentrated, and the residue was purified by silica gel column chromatography (PE:EA = 5:1) to give compound 67-2-5 (yellow solid, 8.50 g, yield: 86%).

Step 6: In a nitrogen atmosphere, 67-2-5 (3.00 g, 5.86 mmol) was dissolved in THF (10 mL), and hydrochloric acid (3.4 mL, 2 N) was added. The mixture was left to react at 25 °C for 1.5 h. LCMS analysis showed that the reaction had been complete. The reaction mixture was concentrated to give compound 67-2-6 (yellow solid, 1.40 g, yield: 80%). MS m/z (ESI): 288.2 [M+H]⁺.

Step 7: 67-2-6 (660 mg, 2.29 mmol) was dissolved in a solution of hydrobromic acid in acetic acid (5 mL) in a nitrogen atmosphere. The mixture was left to react at 50 °C for 18 h. LCMS analysis showed that the reaction had been complete. The reaction mixture was concentrated to give compound 67-2-7 (yellow solid, 450 mg, yield: 85%). MS m/z (ESI): 230.2 [M+H]⁺.

Step 8: In a nitrogen atmosphere, 67-2-7 (200 mg, 0.872 mmol) was dissolved in THF (5 mL), and 3-(2,3-dihydrobenzofuran-7-yl)propanal (184 mg, 1.05 mmol) and NaBH₃CN (274 mg, 4.36 mmol) were added. The mixture was left to react at 25 °C for 3 h. LCMS analysis showed that the reaction had been complete. Water was added, and extraction was performed with EA. The extract was dried over sodium sulfate, filtered, and concentrated, and the residue was purified by silica gel column chromatography (DCM:MeOH = 10:1) to give compound 67-2-8 (yellow solid, 100 mg, yield: 29%). MS m/z (ESI): 390.2 [M+H]⁺.

Step 9: 67-2-8 (100 mg, 0.257 mmol) was resolved by SFC (Daicel ChiralPak IH, 40 mm I.D. × 250 mm, 10 µm; mobile phase: n-hexane + TFA0.1/ethanol = 60/40; flow rate: 70 mL/min) to give compound E67-2 (PK1: 27.4 mg, purity: 91%, yield: 27%, ee 100%, RT = 3.441, OROT = -219.5) and compound E67-1 (PK2: 30.3 mg, purity: 83%, yield: 30%, ee 99%, RT = 4.936, OROT = +52.6). E67-2: ¹H NMR (400 MHz, DMSO) δ 10.35 (s, 1H), 7.08-7.02 (m, 1H), 6.92 (d, *J* = 7.2 Hz, 1H), 6.80-6.70 (m, 2H), 6.64 (t, *J* = 7.6 Hz, 1H), 6.58 (dd, *J* = 7.6, 0.8 Hz, 1H), 4.49 (t, *J* = 8.8 Hz, 2H), 3.80 (d, *J* = 14.0 Hz, 1H), 3.30-3.03 (m, 7H), 2.90-2.77 (m, 1H), 2.64-2.55 (m, 1H), 2.31-2.15 (m, 2H), 2.10-1.99 (m, 1H), 1.97-1.85 (m, 1H), 1.86-1.75 (m, 1H), 1.73-1.59 (m, 3H). MS m/z (ESI): 390.2 [M+H]⁺. E67-1: ¹H NMR (400 MHz, DMSO) δ 10.36 (s, 1H), 7.04 (d, *J* = 7.2 Hz, 1H), 6.92 (d, *J* = 7.6 Hz, 1H), 6.81-6.70 (m, 2H), 6.64 (t, *J* = 7.6 Hz, 1H), 6.60-6.55 (m, 1H), 4.49 (t, *J* = 8.8 Hz, 2H), 3.80 (d, *J* = 14.4 Hz, 1H), 3.34-3.06 (m, 7H), 2.90-2.77 (m, 1H), 2.66-2.54 (m, 1H), 2.35-2.15 (m, 2H), 2.13-1.98 (m, 1H), 2.00-1.86 (m, 1H), 1.85-1.74 (m, 1H), 1.73-1.58 (m, 3H). MS m/z (ESI): 390.2 [M+H]⁺.

### Example 67-3: trans-8-(3-(2,3-Dihydrobenzofuran-7-yl)propyl)-6b,7,8,9,10,10a-hexahydro-1H-pyrido[3',4':4,5]pyrrolo[1,2,3-de]quinoxalin-2(3H)-one

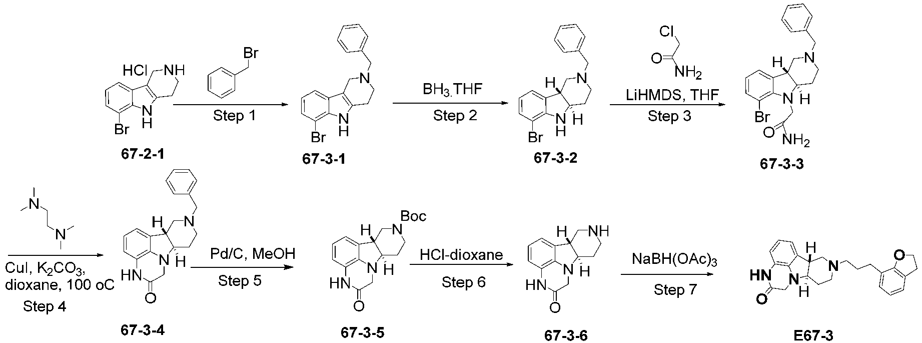

Step 1: 67-2-1 (20.00 g, 79.6 mmol) was dissolved in DMF (250.0 mL), and K₂CO₃ (44.0 g, 318 mmol) was added. The mixture was left to react at 25 °C for 0.5 h. Subsequently, benzyl bromide (0.75 g, 4.380 mmol) was added, and the mixture was then left to react at room temperature for 2 h. LCMS analysis showed that the reaction had been complete. Water was added, and extraction was performed with EA. The extract was dried over sodium sulfate, filtered, and concentrated, and the residue was purified by silica gel column chromatography (PE = 100%) to give compound 67-3-1 (white solid, 11.0 g, yield: 40.7%). MS m/z (ESI): 341.2, 343.2 [M+H]⁺.

Step 2: 67-3-1 (3.0 g, 8.79 mmol) was dissolved in THF (20.0 mL), and borane dimethyl sulfide (90.0 mL, 176 mmol, 2 M) was added in a nitrogen atmosphere. The mixture was left to react at 80 °C for 48 h. LCMS analysis showed that a quarter of the starting material remained in the reaction and that there were 2 peaks corresponding to the desired molecular weight. 6 N HCl was added, and the mixture was left to react at 100 °C for half an hour and then concentrated to dryness using a water pump. The residue was purified by reversed-phase chromatography (ACN:H₂O = 25%) to give compound 67-3-2 (yellow solid, 1.9 g, yield: 63%). MS m/z (ESI): 343.2, 345.0 [M+H]⁺.

Step 3: 67-3-2 (1.9 g, 5.12 mmol) was dissolved in toluene (20.0 mL), and LiHMDS (17 mL, 5.12 mmol) was added at 0 °C in a nitrogen atmosphere. The mixture was left to react for 0.5 h. Subsequently, 2-chloroacetamide (510 mg, 5.448 mmol) was added, and the mixture was left to react overnight at 25 °C. LCMS analysis showed that the reaction had been complete. The reaction mixture was quenched with an aqueous sodium sulfite solution and extracted and washed with DCM and water. After filtration and concentration, the residue was purified by silica gel column chromatography (DCM:MeOH = 10:1) to give compound 67-3-3 (brown solid, 500 mg, yield: 25%). MS m/z (ESI): 400.3, 402.4 [M+H]⁺.

Step 4: 67-3-3 (500 mg, 1.25 mmol) was dissolved in dioxane (5.0 mL), and CuI (70 mg, 0.375 mmol), cesium carbonate (1.9 g, 3.74 mmol), and 2,5-diazahexane (135 mg, 1.5 mmol) were added. The mixture was left to react at 110 °C for 2 h. LCMS analysis showed that the reaction had been complete. The reaction mixture was extracted and washed with EA and water. After filtration and concentration, the residue was purified by silica gel column chromatography (EA:PE = 42%) to give compound 67-3-4 (brown solid, 260 mg, yield: 65%). MS m/z (ESI): 320.2 [M+H]⁺.

Step 5: In a nitrogen atmosphere, 67-3-4 (260 mg, 0.814 mmol) was dissolved in MeOH (3.0 mL), and Pd/C (50 mg) and (Boc)₂O (355 mg, 1.63 mmol) were added. The mixture was left to react at 25 °C for 18 h. LCMS analysis showed that the reaction had been complete. Palladium on carbon was removed by filtration, and the filtrate was mixed with silica gel and concentrated. The residue was purified by silica gel column chromatography (EA:PE = 56%) to give compound 67-3-5 (yellow solid, 130 mg, yield: 48%). MS m/z (ESI): 275.2 [M-56+H]⁺.

Step 6: 67-3-5 (50 mg, 0.152 mmol) was dissolved in DCM (1.0 mL), and HCl-dioxane (0.5 mL) was then added. The mixture was left to react at 25 °C for 3 h. LCMS analysis showed that the reaction had been complete. The reaction solution was concentrated to dryness, and the residue was directly used in the next step to give compound 67-3-6 (yellow solid, 35 mg, yield: 100%). MS m/z (ESI): 230.4 [M+H]⁺.

Step 7: In a nitrogen atmosphere, 67-3-6 (35 mg, 0.153 mmol) was dissolved in MeOH (20 mL) and DCM (12 mL), and 3-(2,3-dihydro-1-benzofuran-7-yl)propanal (60 mg, 0.305 mmol) and sodium triacetoxyborohydride (97 mg, 0.458 mmol) were added. The mixture was left to react overnight at 25 °C. LCMS analysis showed that the reaction had been complete. The reaction solution was concentrated to dryness. After filtration and concentration, the residue was purified by preparative chromatography (eluent (v/v): acetonitrile/(water + 0.05% NH₄HCO₃) = 25%-78%) to give compound E67-3 (white solid, 15.35 mg, yield: 25.8%). ¹H NMR (400 MHz, CD₃OD) δ 7.06 (dd, *J* = 7.4, 1.0 Hz, 1H), 6.94 (d, *J* = 7.6 Hz, 1H), 6.83 (d, *J* = 7.4 Hz, 1H), 6.77 (td, *J* = 7.6, 5.5 Hz, 2H), 6.67 (d, *J* = 7.8 Hz, 1H), 4.54 (t, *J* = 8.6 Hz, 2H), 3.89 (d, *J* = 14 Hz, 1H), 3.78 (d, *J* = 9.6 Hz, 1H), 3.36 (d, *J* = 14.4 Hz, 2H), 3.20 (t, *J* = 8.8 Hz, 2H), 2.95 (dd, *J* = 18.0, 6.1 Hz, 1H), 2.74 (t, *J* = 7.8 Hz, 2H), 2.63 (t, *J* = 7.6 Hz, 3H), 2.56-2.42 (m, 2H), 2.20 (dd, *J* = 12.6, 2.7 Hz, 1H), 1.94 (dd, *J* = 16.0, 12.0, 5.8 Hz, 3H). MS m/z (ESI): 390.2 [M+H]+.

### Example 68: 8-(3-(2,3-Dihydrobenzofuran-7-yl)propyl)-1,2,6b,7,8,9,10,10a-octahydropyrido[4,3-b][1,4]thiazino[2,3,4-hi]indole

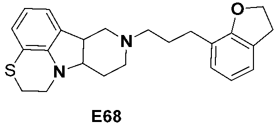

E68 was prepared with reference to the synthesis method of Example 42. ¹H NMR (500 MHz, CD₃OD) δ 7.18-7.04 (m, 2H), 6.98-6.84 (m, 2H), 6.80-6.62 (m, 2H), 4.54-4.49 (m, 2H), 4.29-4.27 (m, 1H), 4.03 (d, *J* = 5.0 Hz, 1H), 3.65-3.61 (m, 1H), 3.48-3.44 (m, 1H), 3.35-3.33 (m, 1H), 3.26-3.16 (m, 5H), 3.10-2.84 (m, 3H), 2.75-2.58 (m, 4H), 2.31-2.18 (m, 1H), 2.08-1.94 (m, 2H). MS m/z (ESI): 392.9 [M+H]⁺.

### Example 69: (6bR,10aS)-8-(3-(2,3-Dihydrobenzofuran-7-yl)-2-fluoropropyl)-3-methyl-2,3,6b,7,8,9,10,10a-octahydro-1H-pyrido[3',4':4,5]pyrrolo[1,2,3-de]quinoxaline

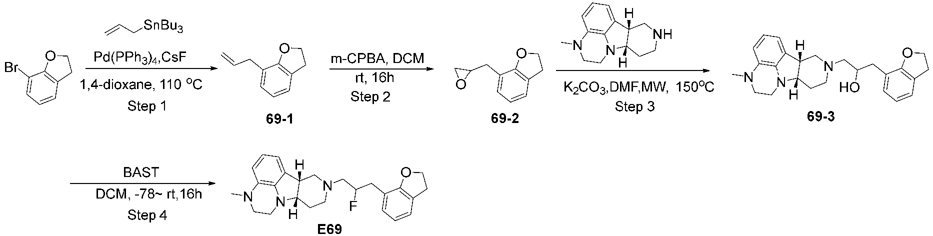

Step 1: 7-Bromo-2,3-dihydrobenzofuran (2 g, 10 mmol), allyltributylstannane (3.5 g, 11 mmol), Pd(PPh₃)₄, CsF (3.04 g, 20 mmol), and 10 mL of 1,4-dioxane were purged three times with N₂, heated to 110 °C, and left to react for 16 h. TLC analysis showed that the reaction had been complete. The reaction mixture was filtered and concentrated, and water and EA were then added. The aqueous phase was isolated and extracted once with EA. The organic phases were combined, dried, and concentrated, and the residue was purified by column chromatography (PE/EA = 50/1) to give 69-1 (1.3 g, colorless liquid).

Step 2: 69-1 (1.1 g, 6.88 mmol) was added to a single-neck flask containing 20 mL of DCM, and m-CPBA (2.79 g, 13.75 mmol) was then added portionwise. The mixture was left to react at room temperature for 16 h, and TLC showed that the reaction had been complete. The reaction mixture was filtered, and the solid was washed with DCM. Then, saturated sodium bicarbonate was added to the filtrate. After liquid separation, drying, and concentration, the residue was purified by column chromatography (PE/EA = 50/1) to give 69-2 (600 mg, colorless liquid).

Step 3: (6bR,10aS)-3-Methyl-2,3,6b,7,8,9,10,10a-octahydro-1H-pyrido[3',4':4,5]pyrrolo[1,2,3-de]quinoxaline (300 mg, 1.3 mmol), 69-2 (276 mg, 1.57 mmol), K₂CO₃ (360 mg, 2.6 mmol), and 5 mL of DMF were left to react under microwave conditions at 150 °C for 1.5 h. LCMS showed that a small amount of the starting material remained. Water and EA were added, and the aqueous phase was isolated and extracted once with EA. The organic phases were combined, dried, and concentrated, and the residue was purified by column chromatography (DCM/MeOH = 50/1) to give 69-3 (430 mg, yellow liquid). MS m/z (ESI): 406.5 [M+H]⁺.

Step 4: BAST (55 mg, 0.24 mmol) was added to a three-neck flask containing 2 mL of DCM, and the mixture was purged three times with N₂ and cooled to -78 °C. A solution of 69-3 (50 mg, 0.12 mmol) in DCM was added, and the mixture was slowly warmed to room temperature and left to react for 16 h. LCMS showed that the main peak was the target product. The reaction was quenched with saturated sodium bicarbonate, and the aqueous phase was isolated and extracted once with DCM. The organic phases were combined, dried, and concentrated, and the residue was purified and separated by prep-HPLC (eluent (v/v): acetonitrile/(water + 0.05% NH₄HCO₃) = 30%-70%) to give compound E69 (7.5 mg, white solid, yield: 15.4%).

¹H NMR (500 MHz, CDCl₃) δ 7.08 (d, *J* = 7.1 Hz, 1H), 6.96 (d, *J* = 6.9 Hz, 1H), 6.78 (t, *J* = 8.2 Hz, 1H), 6.65 (t, *J* = 7.2 Hz, 1H), 6.50 (d, *J* = 7.3 Hz, 1H), 6.41 (d, *J* = 7.8 Hz, 1H), 5.23-4.87 (m, 1H), 4.54 (t, *J* = 8.7 Hz, 2H), 3.63-3.55 (m, 1H), 3.33-3.15 (m, 6H), 3.02-2.66 (m, 11H), 2.17-1.77 (m, 3H). MS m/z (ESI): 408.5 [M+H]⁺.

### Example 70: (6bR,10aS)-8-(3-(3-Chloro-6-methylpyridin-2-yl)propyl)-3-methyl-2,3,6b,7,8,9,10,10a-octahydro-1H-pyrido[3',4":4,5]pyrrolo[1,2,3-de]quinoxaline

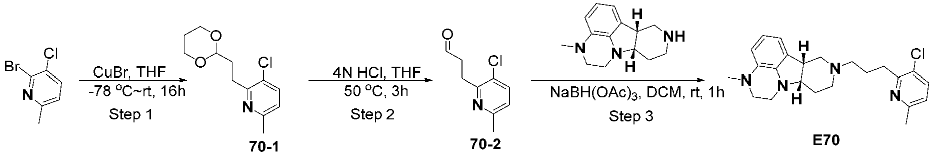

Step 1: CuBr (832 mg, 5.8 mmol) was added to a three-neck flask containing 30 mL of THF, and the mixture was purged three times with N₂ and cooled to -78 °C. 0.5 M (2-(1,3-dioxan-2-yl)ethyl)magnesium bromide (23 mL, 11.6 mmol) was added dropwise, and the mixture was left to react at this temperature for 20 min. 2-Bromo-3-chloro-6-methylpyridine (300 mg, 1.45 mmol) was added. Then, the mixture was left to react at -78 °C for 3 h, warmed to room temperature, and left to react for 16 h, and LCMS analysis showed that the reaction had been complete. Ammonia water was added to adjust the pH to 9-10, and EA and water were then added. The mixture was filtered, and the aqueous phase was isolated and extracted twice with EA. The organic phases were combined, washed once with a saturated sodium chloride solution, dried, and concentrated, and the residue was purified by column chromatography (PE/EA = 3/1) to give 70-1 (300 mg, colorless liquid). MS m/z (ESI): 242.1, 243.9 [M+H]⁺.

Step 2: 70-1 (300 mg, 1.24 mmol) was added to a single-neck flask containing 3 mL of THF, and 4 M HCl (1 mL, 4 mmol) was then added. The mixture was left to react at 50 °C for 3 h, and LCMS showed that the reaction had been complete. THF was removed by concentration, and the pH was adjusted to 8-9 with saturated sodium bicarbonate. Then, EA and water were added, and the aqueous phase was isolated and extracted twice with EA. The organic phases were combined, washed once with a saturated sodium chloride solution, dried, and concentrated, and the residue was purified by column chromatography (PE/EA = 3/1) to give 70-2 (90 mg, brown oil). MS m/z (ESI): 184.0, 185.9 [M+H]⁺.

Step 3: E70 was prepared with reference to the synthesis method in step 7 of E46. 1H NMR (500 MHz, CDCl3) δ 7.47 (d, *J* = 8.0 Hz, 1H), 6.92 (d, *J* = 8.0 Hz, 1H), 6.64 (t, *J* = 7.5 Hz, 1H), 6.51 (d, *J* = 7.0 Hz, 1H), 6.40 (d, *J* = 8.0 Hz, 1H), 3.62-3.57 (m, 1H), 3.32-3.11 (m, 4H), 2.96-2.87 (m, 3H), 2.86 (s, 3H), 2.85-2.65 (m, 2H), 2.54-2.42 (m, 5H), 2.23-2.21 (m, 1H), 2.07-1.88 (s, 5H). MS m/z (ESI): 398.2 [M+H]⁺.

### Example 71: (6bR,10aS)-8-(3-(2,3-Dihydrobenzofuran-7-yl)-3-fluoropropyl)-6b,7,8,9,10,10a-hexahydro-1H-pyrido[3',4":4,5]pyrrolo[1,2,3-de]quinoxalin-2(3H)-one

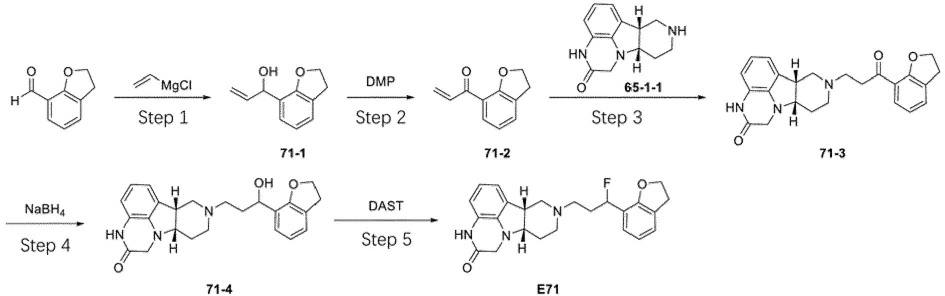

Step 1: The compound 2,3-dihydrobenzofuran-7-carbaldehyde (1.0 g, 6.76 mmol) was dissolved in THF (50 mL), and the solution was cooled to -78 °C. Vinylmagnesium chloride (6.6 mL, 10 mmol) was added, and the mixture was warmed to room temperature and stirred for 2 h. An aqueous ammonium chloride solution (50 mL) was added to quench the reaction, and extraction was performed with ethyl acetate (50 mL × 2). The organic phase was washed with saturated brine, dried over anhydrous sodium sulfate, and concentrated, and the resulting crude product was purified and separated by normal-phase column chromatography to give compound 71-1 (colorless oil, 1.1 g, yield: 92%). MS m/z (ESI): 177.4 [M+H]⁺.

Step 2: 71-2 was prepared with reference to the synthesis method in step 1 of Example E65.

Step 3: 67-1-1 (150 mg, 0.66 mmol), 71-2 (135 mg, 0.79 mmol), and anhydrous DCM (15 mL) were added together to a 50 mL single-neck flask, and TEA (200 mg, 1.98 mmol) was subsequently added. After the addition, the mixture was stirred at room temperature for 16 h. After LCMS analysis showed that the reaction had been complete, the reaction solution was washed with a saturated NH₄Cl solution (50 mL × 1) and extracted with DCM (30 mL × 2). The organic phases were combined, washed with saturated brine (50 mL × 1), dried over anhydrous Na₂SO₄, and concentrated to dryness by rotary evaporation under reduced pressure, and the crude product was separated and purified by column chromatography (MeOH/DCM = 0-5%) to give 71-3 (pale yellow oil, 150 mg, yield: 57%). ¹H NMR (500 MHz, CDCl₃) δ 7.78 (s, 1H), 7.67 (d, *J* = 7.6 Hz, 1H), 7.36 (dd, *J* = 7.2, 1.1 Hz, 1H), 6.92-6.87 (m, 1H), 6.85 (d, *J* = 7.4 Hz, 1H), 6.75 (t, *J* = 7.6 Hz, 1H), 6.62 (d, *J* = 7.7 Hz, 1H), 4.71 (t, *J* = 8.8 Hz, 2H), 3.95 (d, *J* = 14.5 Hz, 1H), 3.71-3.51 (m, 1H), 3.45-3.31 (m, 4H), 3.25 (t, *J* = 8.8 Hz, 2H), 3.21-2.93 (m, 4H), 2.67-2.49 (m, 1H), 2.37-2.07 (m, 2H), 2.05-1.97 (m, 1H). MS m/z (ESI): 404.5 [M+H]⁺.

Step 4: Compound 71-3 (150 mg, 0.37 mmol) and absolute MeOH (10 mL) were added together to a 50 mL single-neck flask, and NaBH₄ (45 mg, 1.11 mmol) was subsequently added portionwise at room temperature. After the addition, the mixture was stirred at room temperature for 1 h. After LCMS analysis showed that the reaction had been complete, the reaction solution was washed with a saturated NH₄Cl solution (20 mL × 1) and extracted with DCM (20 mL × 2). The organic phases were combined, washed with saturated brine (30 mL × 1), dried over anhydrous Na₂SO₄, and concentrated to dryness by rotary evaporation under reduced pressure, and the crude product was separated and purified by column chromatography (MeOH/DCM = 0-10%) to give 71-4 (white solid, 120 mg, yield: 80%). ¹H NMR (500 MHz, MeOD) δ 7.18 (dd, *J* = 7.4, 3.6 Hz, 1H), 7.10 (dd, *J* = 7.3, 1.0 Hz, 1H), 6.87-6.79 (m, 2H), 6.72 (td, *J* = 7.6, 1.9 Hz, 1H), 6.65 (d, *J* = 7.8 Hz, 1H), 4.93-4.90 (m, 1H), 4.63-4.47 (m, 3H), 3.89 (d, *J* = 14.6 Hz, 1H), 3.34 (dd, *J* = 11.9, 9.0 Hz, 2H), 3.18 (t, *J* = 8.6 Hz, 2H), 3.08-2.98 (m, 1H), 2.92-2.82 (m, 1H), 2.67-2.45 (m, 2H), 2.44-2.33 (m, 1H), 2.09-1.86 (m, 5H). MS m/z (ESI): 406.6 [M+H]⁺.

Step 5: Compound 71-4 (20 mg, 0.05 mmol) and anhydrous DCM (3 mL) were added together to a 25 mL three-neck flask, and a solution of DAST (55 mg, 0.25 mmol) in dichloromethane was slowly added dropwise under nitrogen and dry ice-ethanol bath conditions. After the dropwise addition, the mixture was stirred at room temperature for 3 h. After LCMS analysis showed that the reaction had been complete, the reaction solution was washed with a saturated NH₄Cl solution (10 mL × 1) to quench the reaction and subsequently extracted with DCM (10 mL × 2). The organic phases were combined, washed with saturated brine (30 mL × 1), dried over anhydrous Na₂SO₄, and concentrated to dryness by rotary evaporation under reduced pressure, and the crude product was purified and separated by prep-HPLC (eluent (v/v): acetonitrile/(water + 0.05% NH₄HCO₃) = 30%-70%) to give the target compound E71 (white solid, 6 mg, yield: 30%). ¹H NMR (500 MHz, MeOD) δ 7.18 (t, *J* = 6.8 Hz, 1H), 7.12 (t, *J* = 8.4 Hz, 1H), 6.84 (dt, *J* = 15.0, 7.4 Hz, 2H), 6.72 (dd, *J* = 15.7, 7.8 Hz, 1H), 6.66 (t, *J* = 8.0 Hz, 1H), 4.95-4.88 (m, 1H), 4.61-4.51 (m, 3H), 3.89 (dd, *J* = 14.5, 8.5 Hz, 1H), 3.49-3.33 (m, 4H), 3.21-3.13 (m, 3H), 3.06-2.94 (m, 1H), 2.82-2.54 (m, 3H), 2.16-2.03 (m, 3H). MS m/z (ESI): 408.8 [M+H]⁺.

### Example 72: (6bR,10aS)-8-(3-(2,3-Dihydrobenzofuran-7-yl)-2-fluoropropyl)-6b,7,8,9,10,10a-hexahydro-1H-pyrido[3',4":4,5]pyrrolo[1,2,3-de]quinoxalin-2(3H)-one

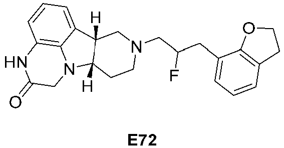

E72 was prepared with reference to the synthesis method of Example 69. ¹H NMR (500 MHz, CDCl₃) δ 7.41 (s, 1H), 7.08 (d, *J* = 7.3 Hz, 1H), 6.97 (d, *J* = 7.6 Hz, 1H), 6.84-6.68 (m, 3H), 6.57 (d, *J* = 7.8 Hz, 1H), 4.99 (d, *J* = 50.2 Hz, 1H), 4.57-4.51 (m, 2H), 3.94 (dd, *J* = 14.6, 2.6 Hz, 1H), 3.54-3.28 (m, 3H), 3.21 (t, *J* = 8.7 Hz, 2H), 2.99-2.85 (m, 3H), 2.58 (t, *J* = 102.0 Hz, 4H), 1.97 (d, *J* = 33.6 Hz, 3H). MS m/z (ESI): 408.6 [M+H]⁺.

### Example 73-1: (6bR,10aS)-8-(3-(2,3-Dihydrobenzofuran-7-yl)propyl)-3-methyl-6b,7,8,9,10,10a-hexahydro-1H-pyrido[3',4":4,5]pyrrolo[1,2,3-de]quinoxalin-2(3H)-one

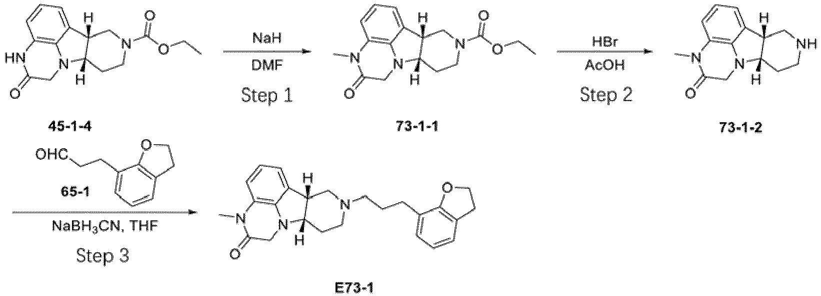

Step 1: In a nitrogen atmosphere, compound 45-1-4 (210 mg, 0.69 mmol) was dissolved in dry tetrahydrofuran (5.0 mL), and sodium hydride (55.7 mg, 1.39 mmol, 60% content, dispersed in mineral oil) was added. After the mixture was left to react at 0 °C for 0.5 h, iodomethane (55.7 mg, 1.39 mmol) was added. Then, the mixture was left to react at room temperature for 2 h. After LCMS analysis showed that the reaction had been complete, the reaction mixture was quenched with 10.0 mL of iced water and extracted with dichloromethane (10.0 mL × 2). The organic phase was concentrated to dryness by rotary evaporation, and the crude product was purified by normal-phase silica gel column chromatography to give 73-1-1 (brown solid, 190 mg, yield: 96%). MS m/z (ESI): 316.2 [M+H]⁺.

Step 2: Compound 73-1-1 (190 mg, 0.60 mmol) was dissolved in a 30% solution of hydrobromic acid in acetic acid (3.0 mL), and the solution was stirred at 50 °C for 16 h. LCMS analysis showed that the starting material disappeared. The reaction mixture was cooled to room temperature and then filtered to give compound 73-1-2 (brown solid, 45 mg, yield: 20%). MS m/z (ESI): 244.2 [M+H]⁺.

Step 3: In a nitrogen atmosphere, compound 73-1-2 (45.0 mg, 0.19 mmol), 3-(2,3-dihydrobenzofuran-7-yl)propanal (31.0 mg, 0.19 mmol), and acetic acid (0.05 mL, 0.93 mmol) were dissolved in THF (3.0 mL), and sodium cyanoborohydride (24.7 mg, 0.39 mmol) was added. The mixture was left to react at 0 °C for 2 h. LCMS analysis showed that the reaction had been complete. The reaction mixture was filtered and concentrated to dryness by rotary evaporation, and the residue was purified by reversed-phase chromatography (eluent (v/v): acetonitrile/(water + 0.05% NH₄HCO₃) = 30%-70%) to give compound E73-1 (white solid, 10.1 mg, yield: 14%). 1H NMR (400 MHz, CDCl₃) δ 7.01 (d, *J* = 7.2 Hz, 1H), 6.91 (d, *J* = 7.6 Hz, 1H), 6.86-6.84 (m, 1H), 6.79-6.72 (m, 3H), 4.55 (t, *J* = 8.4 Hz, 2H), 4.01 (t, *J* = 14.0 Hz, 1H), 3.38-3.35 (m, 2H), 3.32 (s, 3H), 3.30-3.29 (m, 1H), 3.19 (t, *J* = 8.8 Hz, 2H), 2.94-2.72 (m, 2H), 2.57 (t, *J* = 7.6 Hz, 2H), 2.39-2.16 (m, 3H), 2.02-1.77 (m, 5H). MS m/z (ESI): 404.4 [M+H]+.

### Example 73-2: (6bS,10aR)-8-(3-(2,3-Dihydrobenzofuran-7-yl)propyl)-3-methyl-6b,7,8,9,10,10a-hexahydro-1H-pyrido[3',4":4,5]pyrrolo[1,2,3-de]quinoxalin-2(3H)-one

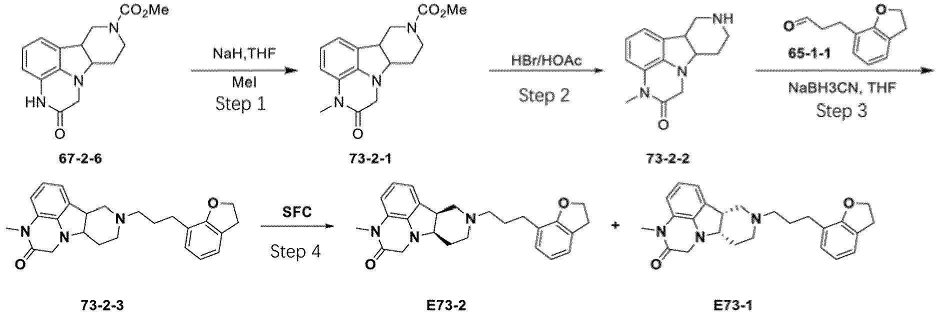

Step 1: 67-2-6 (840 mg, 2.92 mmol) was dissolved in THF (5.0 mL), and NaH (9.12 g, 62.1 mmol) and iodomethane (77.3 mg, 0.636 mmol) were added under ice bath conditions. The mixture was left to react at 0 °C for 2 h. LCMS analysis showed that the reaction had been complete. Water was added, and extraction was performed with EA. The extract was dried over sodium sulfate, filtered, and concentrated, and the residue was purified by silica gel column chromatography (DCM:MeOH = 20:1) to give compound 73-2-1 (yellow solid, 770 mg, yield: 87%). MS m/z (ESI): 302.1 [M+H]⁺.

Step 2: 73-2-1 (770 mg, 2.55 mmol) was dissolved in a solution of hydrobromic acid in acetic acid (5 mL), and the solution was left to react at 50 °C for 18 h. LCMS analysis showed that the reaction had been complete. The reaction solution was concentrated to give compound 73-2-2 (gray solid, 526 mg, yield: 84%). MS m/z (ESI): 244.2 [M+H]⁺.

Step 3: In a nitrogen atmosphere, 73-2-2 (300 mg, 1.23 mmol) was dissolved in THF (5.0 mL), and 3-(2,3-dihydrobenzofuran-7-yl)propanal (260 mg, 1.48 mmol) and NaBH₃CN (387 mg, 6.16 mmol) were added. The mixture was left to react at 25 °C for 3 h. LCMS analysis showed that the reaction had been complete. Water was added, and extraction was performed with EA. The extract was dried over sodium sulfate, filtered, and concentrated, and the residue was purified by silica gel column chromatography (DCM:MeOH = 10:1) to give compound 73-2-3 (yellow solid, 30.0 mg, yield: 6%). MS m/z (ESI): 404.2 [M+H]⁺.

Step 4: 73-2-3 (30.0 mg, 0.0744 mmol) was resolved by SFC (Daicel ChiralCel OD, 40 mm I.D. × 250 mm, 10 µm; mobile phase: n-hexane/ethanol [0.1% NH₃.H₂O (V/V)] = 70/30; flow rate: 80 mL/min) to give E73-2 (PK1: 2.20 mg, purity: 96%, yield: 7%, ee 100%, RT = 3.543, OROT = -205.3) as a white solid and E73-1 (PK2: 6.00 mg, purity: 72%, yield: 20%, ee 99%, RT = 4.604, OROT = +163.3) as a white solid. E73-2: ¹H NMR (400 MHz, CDCl₃) δ 6.97 (d, *J* = 7.2 Hz, 1H), 6.85-6.74 (m, 3H), 6.72-6.65 (m, 2H), 4.45 (t, *J* = 8.8 Hz, 2H), 3.91 (d, *J* = 14.0 Hz, 1H), 3.42-3.27 (m, 2H), 3.26 (s, 3H), 3.20-3.00 (m, 3H), 2.75-2.40 (m, 4H), 2.15-1.82 (m, 4H), 1.61-1.37 (m, 3H), 1.26-1.07 (m, 1H). E73-1: ¹H NMR (400 MHz, CDCl₃) δ 7.01 (d, *J* = 7.2 Hz, 1H), 6.91 (d, *J* = 7.6 Hz, 1H), 6.86-6.84 (m, 1H), 6.79-6.72 (m, 3H), 4.55 (t, *J* = 8.4 Hz, 2H), 4.01 (t, *J* = 14.0 Hz, 1H), 3.38-3.35 (m, 2H), 3.32 (s, 3H), 3.30-3.29 (m, 1H), 3.19 (t, *J* = 8.8 Hz, 2H), 2.94-2.72 (m, 2H), 2.57 (t, *J* = 7.6 Hz, 2H), 2.39-2.16 (m, 3H), 2.02-1.77 (m, 5H).

### Example 74: (6bR,10aS)-8-(3-(2,3-Dihydrobenzofuran-7-yl)propyl)-3-ethyl-6b,7,8,9,10,10a-hexahydro-1H-pyrido[3',4":4,5]pyrrolo[1,2,3-de]quinoxalin-2(3H)-one

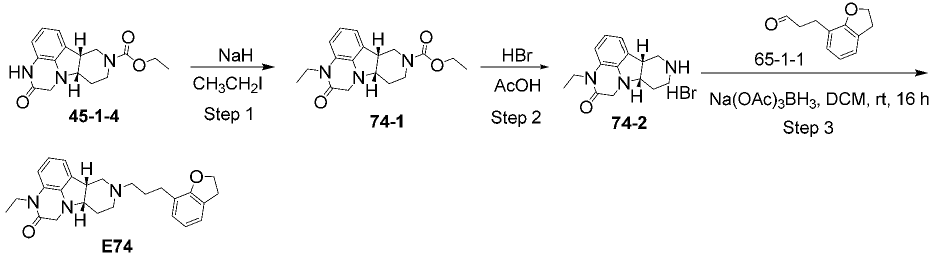

Step 1: 45-1-4 (70 mg, 0.23 mmol) was added to a 50 mL three-neck reaction flask, and DMF (5 mL) was subsequently added. Then, in a nitrogen atmosphere, after 5 min of stirring at 0 °C, sodium hydride (9 mg, 0.35 mmol) was added to the mixture. Then, iodoethane (43 mg, 0.28 mmol) was added dropwise, and the mixture was left to react at room temperature for 5 h. After LCMS analysis showed that the reaction had been complete, the reaction mixture was quenched with a saturated aqueous NH₄Cl solution. Then, water (10 mL) was added to the reaction solution, and extraction was performed with ethyl acetate (10 mL × 2). The organic phase was washed twice with saturated brine (10 mL), dried over anhydrous sodium sulfate, and then concentrated to give compound 74-1 (blackish brown oil, 50 mg, yield: 66%). MS m/z (ESI): 330.5 [M+H]⁺.

Step 2: 74-2 was prepared with reference to the synthesis method in step 1 of Example 72.

Step 3: E74 was prepared with reference to the synthesis method in step 5 of Example 8. ¹H NMR (500 MHz, CDCl₃) δ 7.06-7.01 (m, 1H), 6.91 (d, *J* = 7.5 Hz, 1H), 6.88-6.69 (m, 4H), 4.52 (t, *J* = 8.7 Hz, 2H), 4.03-3.84 (m, 3H), 3.59-3.24 (m, 3H), 3.19 (t, *J* = 8.7 Hz, 4H), 2.59 (t, *J* = 7.6 Hz, 5H), 1.99 (d, *J* = 22.6 Hz, 5H), 1.26 (t, *J* = 7.1 Hz, 3H). MS m/z (ESI): 418.9 [M+H]⁺.

### Example 75: (6bR,10aS)-8-(2-((2,3-Dihydrobenzofuran-7-yl)oxy)ethyl)-6b,7,8,9,10,10a-hexahydro-1H-pyrido[3',4":4,5]pyrrolo[1,2,3-de]quinoxalin-2(3H)-one

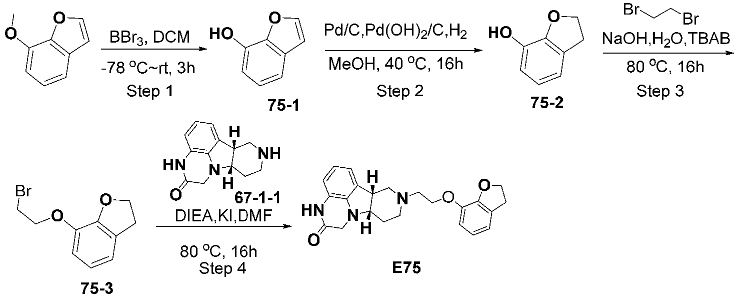

Step 1: 7-Methoxybenzofuran (500 mg, 3.37 mmol) and 10 mL of DCM were purged three times with N₂ and cooled to -78 °C, and 2 M BBr3 (2.5 mL, 5 mmol) was added dropwise. The mixture was naturally warmed to room temperature and left to react for 3 h. TLC showed that the starting material had completely reacted. Sat. NH₄Cl was added to the reaction solution, and the aqueous phase was isolated and then extracted once with DCM. The organic phases were combined, dried, and concentrated, and the residue was purified by column chromatography (PE/EA = 10/1) to give 75-1 (280 mg, colorless liquid). MS m/z (ESI): 133.2 [M-H]⁻.

Step 2: Compound 75-1 (280 mg, 2.09 mmol) was added to a 100 mL single-neck reaction flask, and methanol (10 mL), 10% Pd/C (wet basis, 50 mg), and 20% Pd(OH)₂/C (wet basis, 50 mg) were subsequently added. After the addition, the mixture was purged 3 times with hydrogen and stirred at 60 °C for 16 h. After LCMS analysis showed that the reaction had been complete, the reaction solution was filtered under vacuum, and the filtrate was concentrated under reduced pressure to give the target compound 75-2 (crude, 260 mg, yield: 92%). The crude product was directly used in the next step without further purification. MS m/z (ESI): 135.2 [M-H]⁻.

Step 3: 75-2 (200 mg, 1.47 mmol), 1,2-dibromoethane (1.37 g, 7.35 mmol), NaOH (176 mg, 4.41 mmol), TBAB (142 mg, 0.44 mmol), and 10 mL of water were purged three times with N₂, heated to 90 °C, and left to react for 16 h. TLC showed that a small amount of the starting material remained. EA was added to the reaction solution, and the aqueous phase was isolated and then extracted twice with EA. The organic phases were combined, dried, and concentrated, and the residue was purified by column chromatography (PE/EA = 10/1) to give 75-3 (130 mg, yellow oil).

Step 4: 67-1-1 (20 mg, 0.087 mmol), 75-3 (32 mg, 0.131 mmol), DIEA (23 mg, 0.174 mmol), KI (29 mg, 0.174 mmol), and 2 mL of DMF were purged three times with N₂, heated to 80 °C, and left to react for 16 h, and LCMS showed that the reaction had been complete. The reaction solution was cooled to room temperature, filtered, and purified and separated by prep-HPLC (eluent (v/v): acetonitrile/(water + 0.05% NH₄HCO₃) = 30%-70%) to give compound E75 (8 mg, pink solid, yield: 23.5%). ¹H NMR (500 MHz, CDCl₃) δ 7.74 (s, 1H), 6.83 (d, *J* = 7.0 Hz, 2H), 6.81-6.69 (m, 3H), 6.59 (d, *J* = 7.5 Hz, 1H), 4.60 (t, *J* = 9.0 Hz, 2H), 4.19 (t, *J* = 6.0 Hz, 2H), 3.96 (d, *J* = 15.0 Hz, 1H), 3.50-3.30 (m, 3H), 3.22 (t, *J* = 8.5 Hz, 2H), 3.07-2.99 (m, 1H), 2.94-2.73 (m, 3H), 2.51-2.38 (m, 1H), 2.15-1.94 (m, 3H). MS m/z (ESI): 392.2 [M+H]⁺.

### Example 76: (6bR,10aS)-8-(3-(2,3-Dihydrobenzofuran-7-yl)-3-hydroxypropyl)-3-methyl-6b,7,8,9,10,10a-hexahydro-1H-pyrido[3',4':4,5]pyrrolo[1,2,3-de]quinoxalin-2(3H)-one (HJM-2119, CJJ120-093)

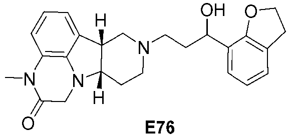

E76 was prepared with reference to the synthesis method of Example 71. ¹H NMR (500 MHz, CD3OD) δ 7.18 (t, *J* = 6.8 Hz, 1H), 7.11 (dd, *J* = 7.3, 0.9 Hz, 1H), 6.94 (d, *J* = 7.2 Hz, 1H), 6.90 (d, *J* = 8.0 Hz, 1H), 6.84 (ddd, *J* = 8.7, 7.7, 1.5 Hz, 2H), 4.94-4.90 (m, 1H), 4.63-4.49 (m, 3H), 4.00 (d, *J* = 14.4 Hz, 1H), 3.45-3.38 (m, 2H), 3.37-3.31 (m, 3H), 3.24-3.14 (m, 3H), 3.08-3.00 (m, 1H), 2.85-2.55 (m, 3H), 2.17-1.99 (m, 5H). MS m/z (ESI): 420.6 [M+H]⁺.

### Example 77: (6bR,10aS)-8-(3-(2,3-Dihydrofuro[2,3-c]pyridin-7-yl)propyl)-3-methyl-6b,7,8,9,10,10a-hexahydro-1H-pyrido[3',4':4,5]pyrrolo[1,2,3-de]quinoxalin-2(3H)-one

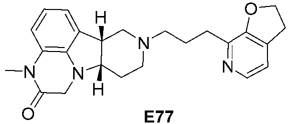

E77 was prepared with reference to the synthesis method of Example 60. ¹H NMR (500 MHz, MeOD) δ 7.92 (d, *J* = 4.9 Hz, 1H), 7.17 (d, *J* = 4.9 Hz, 1H), 6.91-6.79 (m, 3H), 4.60 (t, *J* = 8.9 Hz, 2H), 3.97 (d, *J* = 14.4 Hz, 1H), 3.35 (t, *J=* 10.9 Hz, 1H), 3.31 (d, *J* = 1.3 Hz, 3H), 3.29-3.25 (m, 4H), 2.96-2.89 (m, 1H), 2.80-2.71 (m, 3H), 2.44-2.32 (m, 2H), 2.22 (td, *J* = 12.0, 3.3 Hz, 1H), 2.03-1.87 (m, 4H), 1.74 (t, *J* = 11.1 Hz, 1H). MS m/z (ESI): 405.7 [M+H]⁺.

### Example 78: (6bR,10aS)-8-(3-(3-Chloropyridin-2-yl)propyl)-3-methyl-6b,7,8,9,10,10a-hexahydro-1H-pyrido[3',4':4,5]pyrrolo[1,2,3-de]quinoxalin-2(3H)-one

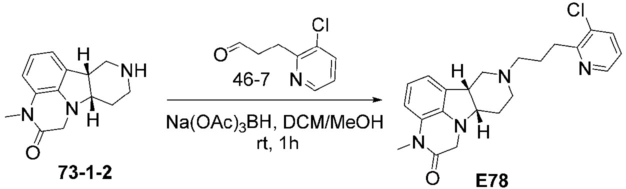

E78 was prepared with reference to the synthesis method in step 5 of E67-1. 1H NMR (500 MHz, CD₃OD) δ 8.54 (d, *J* = 4.5 Hz, 1H), 8.06 (d, *J* = 8.1 Hz, 1H), 7.50-7.41 (m, 1H), 7.04-6.99 (m, 1H), 6.96 (d, *J* = 7.4 Hz, 1H), 6.90 (t, *J* = 7.7 Hz, 1H), 4.07 (d, *J* = 14.5 Hz, 1H), 3.78-3.63 (m, 2H), 3.58-3.53 (m, 1H), 3.49-3.43 (m, 2H), 3.32 (s, 3H), 3.28-3.19 (m, 3H), 3.12 (t, *J* = 7.5 Hz, 2H), 2.70 -2.56 (m,1H), 2.40 (d, *J* =16.0 Hz, 1H), 2.30-2.22 (m, 3H). MS m/z (ESI): 397.9, 399.9 [M+H]⁺.

### Example 79: (6bR,10aS)-3-Cyclobutyl-8-(3-(2,3-dihydrobenzofuran-7-yl)propyl)-6b,7,8,9,10,10a-hexahydro-1H-pyrido[3',4":4,5]pyrrolo[1,2,3-de]quinoxalin-2(3H)-one

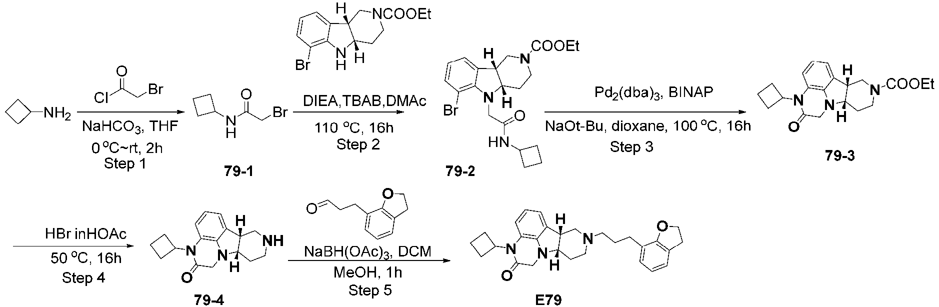

Step 1: 2-Bromoacetyl chloride (1 g, 6.36 mmol) and NaHCO₃ (1.07 g, 12.72 mmol) were added to a three-neck flask containing 10 mL of THF, and the mixture was purged three times with N₂ and cooled to 0 °C. Cyclobutylamine (544 mg, 7.63 mmol) was added, and the mixture was slowly warmed to room temperature and left to react for 2 h. EA and a saturated sodium chloride solution were added, and the aqueous phase was isolated and extracted once with EA. The organic phases were combined, dried, and concentrated to give 79-1 (800 mg, white solid), which was directly used in the next step.

Step 2: Ethyl (4aS,9bR)-6-bromo-1,3,4,4a,5,9b-hexahydro-2H-pyrido[4,3-b]indole-2-carboxylate (200 mg, 0.6 mmol), 79-1 (370 mg, 0.9 mmol), DIEA (156 mg, 1.2 mmol), and TBAB (200 mg, 0.6 mmol) were added to a single-neck flask containing 5 mL of DMAc, and the mixture was purged three times with N₂, heated to 110 °C, and left to react for 48 h. EA and water were added, and the aqueous phase was isolated and extracted three times with EA. The organic phases were combined, washed three times with saturated sodium chloride, dried, and concentrated, and the residue was purified by column chromatography (PE/EA = 3/1) to give 79-2 (180 mg, brown oil). MS m/z (ESI): 436.6, 438.6 [M+H]⁺.

Step 3: 79-2 (180 mg, 0.414 mmol), Pd₂(dba)₃ (38 mg, 0.0414 mmol), NaOt-Bu (80 mg, 0.828 mmol), BINAP (77 mg, 0.124 mmol), and 10 mL of dioxane were added to a 100 mL single-neck flask, and the mixture was purged three times with N₂, heated to 110 °C, and left to react for 16 h. LCMS monitoring showed that the starting material had completely reacted. The reaction solution was cooled to room temperature, and water and EA were added. The aqueous phase was isolated and extracted twice with EA, and the organic phases were combined, dried, and concentrated to give compound 79-3 (150 mg, crude), which was directly used in the next step. MS m/z (ESI): 356.2 [M+H]⁺.

Step 4: 79-3 (150 mg, 0.422 mmol) and 2 mL of a solution of HBr in HOAc were left to react at 50 °C for 16 h. LCMS showed that the reaction had been complete. The reaction solution was cooled to room temperature and concentrated to remove most of HOAc, and the residue was purified by HPLC (0.05% HCl in H₂O, 0-95% ACN 30 min) to give 79-4 (45 mg, brown solid). MS m/z (ESI): 230.2 [M+H]⁺.

Step 5: E79 was prepared with reference to the synthesis method in step 5 of E67-1.

¹H NMR (500 MHz, CDCl₃) δ 7.03 (d, *J* = 8.5 Hz, 1H), 6.92 (d, *J* = 7.5 Hz, 1H), 6.84 (dd, *J* = 6.5, 1.0 Hz, 1H), 6.80-6.73 (m, 3H), 4.66 (m, 1H), 4.53 (t, *J* = 8.5 Hz, 2H), 3.92 (d, *J* = 15.0 Hz, 1H), 3.42-3.30 (m, 1H), 3.26-3.15 (m, 4H), 3.01-2.92 (m, 1H), 2.82-2.75 (m, 1H), 2.72-2.63 (m, 1H), 2.61-2.53 (m, 4H), 2.49-2.34 (m, 3H), 2.33-2.21 (m, 1H), 2.00-1.77 (m, 7H). MS m/z (ESI): 444.8 [M+H]⁺.

### Example 80: (6bR,10aS)-8-(2-((2,3-Dihydrobenzofuran-7-yl)amino)ethyl)-6b,7,8,9,10,10a-hexahydro-1H-pyrido[3',4':4,5]pyrrolo[1,2,3-de]quinoxalin-2(3H)-one

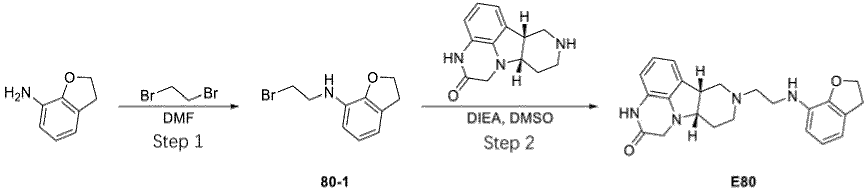

Step 1: 2,3-Dihydrobenzofuran-7-amine (900 mg, 6.66 mmol) was dissolved in DMF (10.0 mL), and 1,2-dibromoethane (3.75 g, 19.9 mmol) was added. The mixture was left to react at 60 °C for 2 h. TLC analysis showed that the reaction had been complete. The reaction mixture was quenched with water and extracted with EA. The extract was dried over sodium sulfate, filtered, and concentrated, and the resulting residue was purified (PE:EA = 10:1) to give compound 80-1 (white solid, 300 mg, yield: 18%). MS m/z (ESI): 242.0, 244.0 [M+H]⁺.

Step 2: Compound 80-1 (135 mg, 0.591 mmol) was dissolved in DMSO (10.0 mL), and DIEA (208 mg, 1.61 mmol) and (6bR,10aS)-6b,7,8,9,10,10a-hexahydro-1H-pyrido[3',4':4,5]pyrrolo[1,2,3-de]quinoxalin-2(3H)-one (130 mg, 0.537 mmol) were added. The mixture was stirred at 65 °C for 16 h. TLC analysis showed that the reaction had been complete. The reaction mixture was quenched with water and extracted with EA. The extract was dried over sodium sulfate, filtered, and concentrated, and the residue was purified by preparative chromatography (eluent (v/v): acetonitrile/(water + 0.05% NH₄HCO₃) = 30%-70%) to give compound E80 (white solid, 29.0 mg, yield: 13%). ¹H NMR (400 MHz, DMSO-d6) δ 10.36 (s, 1H), 6.78 (d, *J* = 6.4 Hz, 1H), 6.65 (dd, *J* = 15.6, 7.6 Hz, 2H), 6.58 (dd, *J* = 7.6, 0.8 Hz, 1H), 6.51 (d, *J* = 6.8 Hz, 1H), 6.41 (d, *J* = 7.6 Hz, 1H), 4.65-4.38 (m, 3H), 3.81 (d, *J* = 14.5 Hz, 1H), 3.35-3.17 (m, 4H), 3.18-3.04 (m, 4H), 2.96-2.83 (m, 1H), 2.71-2.59 (m, 1H), 2.49-2.38 (m, 1H), 2.22-2.07 (m, 1H), 2.01-1.87 (m, 1H), 1.87-1.75 (m, 1H), 1.72 (t, *J* = 11.2 Hz, 1H). MS m/z (ESI): 391.2 [M+H]⁺.

### Example 81: (8aS,12aR)-11-(3-(2,3-Dihydrobenzofuran-7-yl)propyl)-6,7,8a,9,10,11,12,12a-octahydro-[1,4]diazepino[3,2,1-hi]pyrido[4,3-b]indol-5(4H)-one

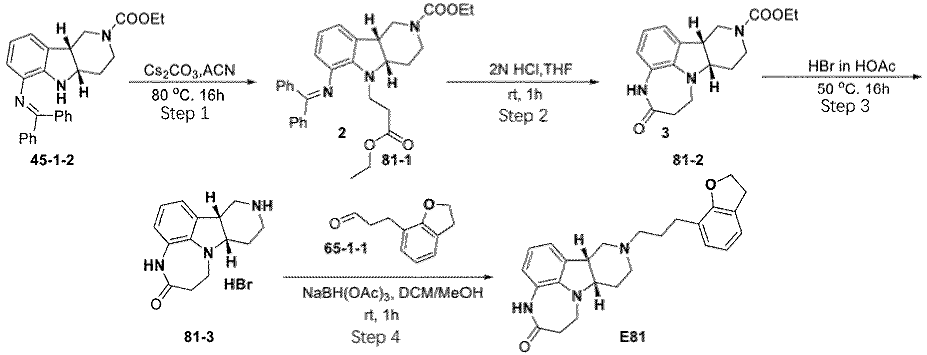

Step 1: 81-1 was prepared with reference to the synthesis method in step 5 of E83.

Step 2: 81-2 was prepared with reference to the synthesis method in step 3 of E45-1.

Step 3: 81-3 was prepared with reference to the synthesis method in step 4 of E67-1.

Step 4: E81 was prepared with reference to the synthesis method in step 5 of E67-1. δ 1H NMR (500 MHz, CDCl₃) δ 7.48 (s, 1H), 7.04 (d, *J* = 7.5 Hz, 1H), 6.91 (d, *J* = 8.0 Hz, 1H), 6.85 (d, *J* = 7.0 Hz, 1H), 6.77-6.71 (m, 2H), 6.60 (d, *J* = 8.0 Hz, 1H), 4.53 (t, *J* = 9.0 Hz, 2H), 3.62-3.31 (m, 3H), 3.24-3.09 (m, 3H), 3.05 -2.75 (m, 4H), 2.61-2.44 (m, 5H), 2.30-1.88 (m, 5H). MS m/z (ESI): 404.5 [M+H]⁺.

### Example 82: (8aS,12aR)-11-(3-(2,3-Dihydrobenzofuran-7-yl)propyl)-4-methyl-6,7,8a,9,10,11,12,12a-octahydro-[1,4]diazepino[3,2,1-hi]pyrido[4,3-b]indol-5(4H)-one

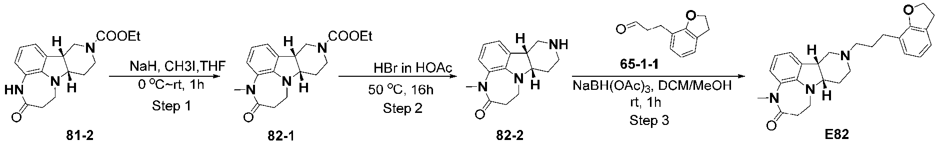

Step 1: 82-1 was prepared with reference to the synthesis method in step 1 of E73-1.

Step 2: 82-2 was prepared with reference to the synthesis method in step 2 of E73-1.

Step 3: E82 was prepared with reference to the synthesis method in step 3 of E73-1. ¹H NMR (500 MHz, CDCl₃) δ 7.03 (d, *J* = 8.5 Hz, 1H), 6.94-6.89 (m, 3H), 6.76 (t, *J* = 7.5 Hz, 2H), 4.53 (t, *J* = 9.0 Hz, 2H), 3.74 (t, *J* = 12.5 Hz, 1H), 3.45-3.42 (m, 1H), 3.38-3.33 (m, 5H), 3.19 (t, *J* = 8.5 Hz, 2H), 2.84-2.77 (m, 1H), 2.75 -2.62 (m, 2H), 2.60 -2.52 (m, 3H), 2.45-2.34 (m, 2H), 2.32-2.21 (m, 1H), 2.06-1.94 (m, 2H), 1.88-1.81 (m, 3H). MS m/z (ESI): 418.5 [M+H]⁺.

### Example 83: 8-(3-(2,3-Dihydrobenzofuran-7-yl)propyl)-5-fluoro-6b,7,8,9,10,10a-hexahydro-1H-pyrido[3',4':4,5]pyrrolo[1,2,3-de]quinoxalin-2(3H)-one

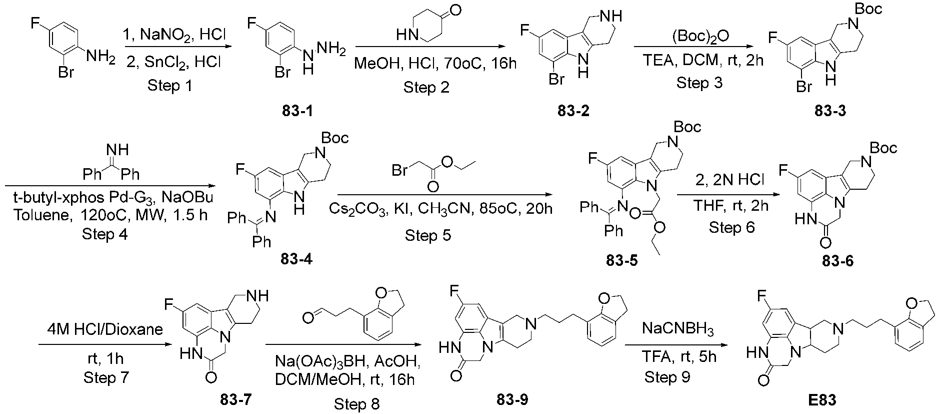

Step 1: 4-Fluoro-2-bromoaniline (5.0 g, 26.3 mmol) and a 5 M HCl solution (50 mL) were added together to a 250 mL three-neck flask and cooled to 0 °C in an ice-water bath, and subsequently, 20 mL of a NaNO₂ (2.8 g, 39.4 mmol) solution was slowly added dropwise. After the dropwise addition, the mixture was stirred at 0 °C for 2 h, and subsequently, a solution of SnCl₂ (10 g, 52.6 mmol) in hydrochloric acid (5.5 mL) was slowly added dropwise under ice-water bath conditions. After the dropwise addition, the mixture was stirred overnight at room temperature. After TLC analysis showed that the reaction had been complete, the reaction solution was directly filtered, and the filter cake was washed with a small amount of concentrated HCl. Water was added, and lyophilization was performed to give compound 83-1 (crude, off-white solid, 4.4 g, yield: 82%). The crude product was directly used in the next step without further purification.

Step 2: Compound 83-1 (2.0 g, 9.8 mmol) was dissolved in isopropanol (30 mL), and subsequently, 4-oxopiperidone hydrochloride (1.5 g, 10.7 mmol) and concentrated HCl (2 mL) were separately added. After the addition, the mixture was heated to 90 °C and stirred overnight in a nitrogen atmosphere. After LCMS analysis showed that the reaction had been complete, the reaction solution was cooled to room temperature, and a large amount of solid precipitated. The mixture was filtered, and the filter cake was washed with isopropanol. After complete concentration by rotary evaporation under reduced pressure, compound 83-2 (crude, off-white solid, 2.0 g, yield: 76%) was obtained. The crude product was directly used in the next step without further purification. MS m/z (ESI): 269.2, 271.2 [M+H]⁺.

Step 3: Compound 83-2 (crude, 200 mg, 0.74 mmol), TEA (225 mg, 2.22 mmol), and anhydrous DCM (10 mL) were added together to a 25 mL three-neck flask, and subsequently, (Boc)₂O (200 mg, 0.90 mmol) was slowly added dropwise in a N₂ atmosphere. After the dropwise addition, the mixture was stirred at room temperature for 2 h. After LCMS analysis showed that the reaction had been complete, water (20 mL) was added to the reaction solution, and extraction was performed with DCM (2 × 20 mL). The organic phases were combined, washed with saturated brine (1 × 30 mL), dried over anhydrous sodium sulfate, and concentrated to dryness by rotary evaporation under reduced pressure, and the crude product was purified and separated by normal-phase column chromatography (eluent gradient: 2% MeOH/DCM) to give compound 83-3 (white solid, 190 mg, yield: 69%).

Step 4: Compound 83-3 (100 mg, 0.27 mmol), benzophenone imine (60 mg, 0.32 mmol), t-BuONa (1.23 g, 12.8 mmol), t-BuXPhos-Pd-G₃ (11 mg, 0.01 mmol), and toluene (5 mL) were added together to a 35 mL microwave tube, and argon was bubbled through the mixture for 2 min. Subsequently, the mixture was microwaved to 120 °C and stirred for 1 h. After LCMS analysis showed that the reaction had been complete, water (30 mL) was added to the reaction solution, and extraction was performed with EA (20 mL × 2). The organic phases were combined, washed with saturated brine (30 mL × 1), dried over anhydrous Na₂SO₄, and concentrated to dryness by rotary evaporation under reduced pressure, and the crude product was separated and purified by column chromatography (EA/PE = 0-20%) to give compound 83-4 (pale yellow solid, 110 mg, yield: 70%). MS m/z (ESI): 470.9 [M+H]⁺.

Step 5: Compound 83-4 (100 mg, 0.21 mmol), ethyl bromoacetate (70 mg, 0.42 mmol), Cs₂CO₃ (240 mg, 0.63 mmol), KI (1.99 g, 12.76 mmol), and anhydrous CH₃CN (10 mL) were added together to a 25 mL single-neck flask and refluxed overnight in a N₂ atmosphere. After LCMS analysis showed that the reaction had been complete, the reaction solution was filtered, water (20 mL) was added to the filtrate, and extraction was performed with EA (30 mL × 2). The organic phases were combined, washed with saturated brine (30 mL × 1), dried over anhydrous Na₂SO₄, and concentrated to dryness by rotary evaporation under reduced pressure, and the crude product was separated and purified by column chromatography (EA/PE = 0-15%) to give compound 83-5 (yellow oil, 100 mg, yield: 85%). MS m/z (ESI): 556.6 [M+H]⁺.

Step 6: Compound 83-5 (100 mg, 0.18 mmol) and THF (10 mL) were added together to a 25 mL single-neck flask, and a 2 N HCl solution (3 mL) was subsequently added. After the addition, the mixture was stirred at room temperature for 2 h. After LCMS monitoring showed that the reaction had been complete, a saturated NaHCO₃ solution was added to the reaction solution to make the system alkaline, and extraction was performed with EA (20 mL × 3). The organic phases were combined, washed with saturated brine (30 mL × 1), dried over anhydrous Na₂SO₄, and concentrated to dryness by rotary evaporation under reduced pressure, and the crude product was separated and purified by column chromatography (MeOH/DCM = 0-2%) to give compound 83-6 (yellow oil, 40 mg, yield: 90%). ¹H NMR (500 MHz, CDCl₃) *δ* 7.85 (s, 1H), 6.78 (d, *J* = 8.5 Hz, 1H), 6.33 (s, 1H), 4.85 (s, 2H), 4.63-4.53 (m, 2H), 3.86-3.76 (m, 2H), 2.76 (s, 2H), 1.50 (s, 9H). MS m/z (ESI): 246.5 [M-Boc]⁺.

Step 7: Compound 83-6 (40 mg, 0.12 mmol) and anhydrous DCM (5 mL) were added together to a 25 mL single-neck flask, and TFA (1 mL) was subsequently added. After the addition, the mixture was stirred at room temperature for 1 h. After LCMS analysis showed that the reaction had been complete, the reaction solvent was removed under reduced pressure. Subsequently, the crude product was dissolved in 10% MeOH/DCM (20 mL), a saturated NaHCO₃ solution was added to make the system alkaline, and extraction was performed with 10% MeOH/DCM (10 mL × 3). The organic phases were combined, washed with saturated brine (10 mL × 1), dried over anhydrous Na₂SO₄, and concentrated to dryness by rotary evaporation under reduced pressure to give compound 83-7 (crude, brown oil, 30 mg). The crude product was directly used in the next step without further purification. MS m/z (ESI): 246.7 [M+H]⁺.

Step 8: Compound 83-8 was prepared with reference to the synthesis method in step 3 of Example E73-1. MS m/z (ESI): 406.7 [M+H]⁺.

Step 9: Compound 83-8 (crude, 20 mg, 0.05 mmol) and TFA (5 mL) were added together to a 25 mL single-neck flask, and Na(CN)BH₃ (30 mg, 0.50 mmol) was subsequently added. After the addition, the mixture was stirred overnight at room temperature. After LCMS analysis showed that the reaction had been complete, the reaction solvent was removed under reduced pressure, EA and water were subsequently added to the crude product, and extraction was performed with EA (10 mL × 3). The organic phases were combined, washed with saturated brine (10 mL × 1), dried over anhydrous Na₂SO₄, and concentrated to dryness by rotary evaporation under reduced pressure, and the crude product was purified and separated by prep-HPLC (eluent (v/v): acetonitrile/(water + 0.05% NH₄HCO₃) = 30%-70%) to give compound E83 (white solid, 4 mg, yield: 20%). ¹H NMR (500 MHz, CDCl₃) δ 7.49 (s, 1H), 7.07 (t, *J* = 8.2 Hz, 1H), 6.86 (d, *J* = 7.4 Hz, 1H), 6.77 (t, *J* = 7.4 Hz, 1H), 6.65 (dd, *J* = 8.0, 2.1 Hz, 1H), 6.45 (dd, *J* = 9.2, 2.1 Hz, 1H), 4.52 (t, *J* = 9.0 Hz, 2H), 3.90 (d, *J* = 14.3 Hz, 1H), 3.85-3.77 (m, 1H), 3.59-3.53 (m, 1H), 3.52-3.44 (m, 2H), 3.40 (d, *J* = 14.3 Hz, 1H), 3.20 (t, *J* = 8.8 Hz, 2H), 3.06-3.00 (m, 3H), 2.65 (t, *J* = 7.2 Hz, 2H), 2.52-2.41 (m, 2H), 2.21-2.15 (m, 1H), 2.13-2.07 (m, 2H). MS m/z (ESI): 408.7 [M+H]⁺.

### Example 84: (6bR,10aS)-8-(3-(2,2-Dimethyl-2,3-dihydrobenzofuran-7-yl)propyl)-6b,7,8,9,10,10a-hexahydro-1H-pyrido[3',4':4,5]pyrrolo[1,2,3-de]quinoxalin-2(3H)-one

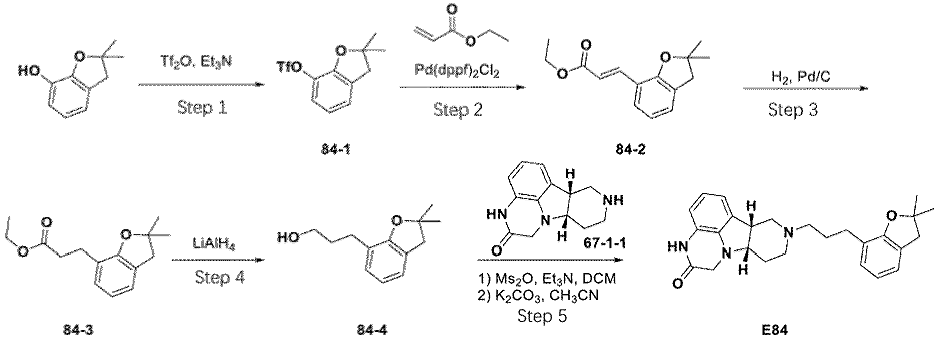

Step 1: At room temperature, 2,2-dimethyl-2,3-dihydrobenzofuran-7-ol (1.8 g, 11 mmol) was dissolved in dichloromethane (50 mL), and pyridine (1.8 mL, 22.8 mmol) was added. The mixture was cooled to 0 °C, and Tf₂O (2.3 mL, 13.5 mmol) was then added. The mixture was warmed to room temperature and stirred for 1 h. The reaction solution was washed with 1 M HCl (50 mL), water (50 mL), and a saturated aqueous sodium bicarbonate solution (50 mL) and dried over anhydrous sodium sulfate, and the organic phase was concentrated to give compound 84-1 (yellow oil, 3.1 g). The crude product was directly used in the next step. MS m/z (ESI): 296.6 [M+H]⁺.

Step 2: At room temperature, compound 84-1 (3.1 g, 10.5 mmol) was dissolved in DMF (24 mL), Et₃N (6 mL) and ethyl acrylate (2.0 g, 20 mmol) were added, and Pd(dppf)₂Cl₂ (770 mg, 1.05 mmol) was then added. The mixture was heated to 98 °C and left to react for 18 h in a N₂ atmosphere. The reaction mixture was concentrated to remove triethylamine and DMF, added to 50 mL of water, and extracted with ethyl acetate (50 mL). The organic phase was washed with saturated brine, dried over anhydrous sodium sulfate, and concentrated, and the resulting crude product was purified and separated by normal-phase column chromatography to give compound 84-2 (yellow oil, 760 mg, yield: 31%). MS m/z (ESI): 247.3 [M+H]⁺.

Step 3: Preparation was performed with reference to the synthesis method in step 2 of Example 1. MS m/z (ESI): 249.1 [M+H]⁺.

Step 4: Preparation was performed with reference to the synthesis method in step 3 of Example 1. MS m/z (ESI): 207.1 [M+H]⁺.

Step 5: Compound 84-4 (40 mg, 0.194 mmol) and triethylamine (78 mg, 0.78 mmol) were dissolved in dichloromethane (4 mL) at room temperature, and the solution was cooled to 0 °C. Methanesulfonic anhydride (67 mg, 0.39 mmol) was added, and the mixture was warmed to room temperature and stirred for 1.5 h. DCM (15 mL) and water (15 mL) were added, and layers formed. The organic phase was washed with water (15 mL), then dried over anhydrous sodium sulfate, filtered, and concentrated. The resulting crude product was dissolved in acetonitrile (5 mL), and compound 67-1-1 (30 mg, 0.194 mmol) and potassium carbonate (32 mg, 0.23 mmol) were added. The mixture was heated to 80 °C and left to react for 16 h. After the reaction was complete, the reaction mixture was cooled to room temperature, filtered, and concentrated, and the resulting residue was purified and separated by prep-HPLC (eluent (v/v): acetonitrile/(water + 0.05% NH₄HCO₃) = 30%-70%) to give compound E84 (white solid, 9.0 mg, yield: 10%). ¹H NMR (500 MHz, CD₃OD) δ 6.96 (d, *J* = 10.0 Hz, 1H), 6.88 (d, *J* = 10.0 Hz, 1H), 6.82 (d, *J* = 5.0 Hz, 1H), 6.72-6.68 (m, 2H), 6.64 (d, *J* = 10.0 Hz, 1H), 3.87 (d, *J* = 10.0 Hz, 1H), 3.35 (d, *J* = 10.0 Hz, 1H), 3.03-2.94 (m, 4H), 2.86-2.77 (m, 1H), 2.66-2.63 (m, 1H), 2.55-2.52 (m, 2H), 2.43-2.26 (m, 3H), 2.05-1.82 (m, 5H), 1.42 (s, 6H). MS m/z (ESI): 418.6 [M+H]⁺.

### Example 85: (6bR,10aS)-8-(3-(2H-Spiro[benzofuran-3,1'-cyclopropan]-7-yl)propyl)-6b,7,8,9,10,10a-hexahydro-1H-pyrido[3',4":4,5]pyrrolo[1,2,3-de]quinoxalin-2(3H)-one

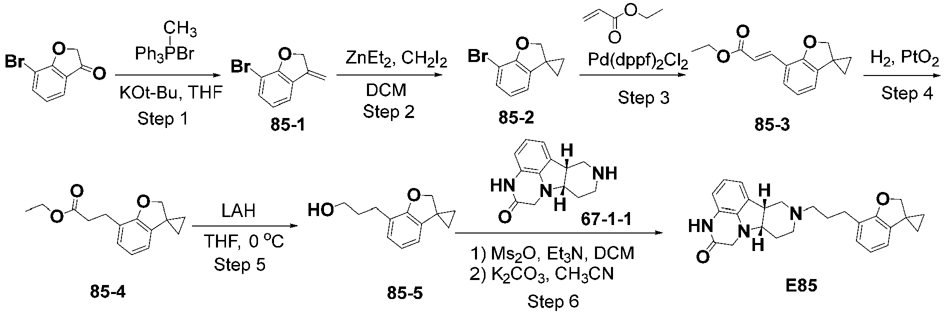

Step 1: Methyltriphenylphosphonium bromide (5.06 g, 14.2 mmol) was dissolved in THF (150 mL), and the solution was cooled to 0 °C. Potassium tert-butoxide (1.67 g, 14.9 mmol) was added, and the mixture was heated to 25 °C and stirred for 1 h. 7-Bromo-3-benzofuranone (2.0 g, 9.39 mmol, dissolved in 150 mL of THF) was added, and the mixture was stirred at 25 °C for 16 h. The reaction mixture was quenched with 100 mL of water and extracted with ethyl acetate (150 mL × 2). The organic phase was washed with saturated brine, dried over anhydrous sodium sulfate, and concentrated, and the resulting crude product was purified and separated by normal-phase column chromatography to give compound 85-1 (colorless oil, 1.58 g, yield: 79%). MS m/z (ESI): 212.4 [M+H]⁺.

Step 2: Diethylzinc (27 mL, 27 mmol) was dissolved in DCM (150 mL), and the solution was cooled to 0 °C. TFA (2.32 mL, 31.4 mmol) was added, and the mixture was stirred at 0 °C for 15 min. After diiodomethane (8.0 g, 30 mmol) was added, the mixture was stirred at 0 °C for 15 min, and compound 85-1 (1.58 g, 7.49 mmol, dissolved in DCM (150 mL)) was then added. The mixture was stirred at 0 °C for 15 min, heated to 25 °C, and stirred for 16 h. After the reaction was quenched with 2 M HCl (150 mL), layers formed. The organic phase was washed with saturated brine, dried over anhydrous sodium sulfate, and concentrated, and the resulting crude product was purified and separated by normal-phase column chromatography to give compound 85-2 (colorless oil, 390 mg, yield: 23%). MS m/z (ESI): 226.4 [M+H]⁺.

Step 3: Compound 85-2 (350 mg, 1.54 mmol) was dissolved in DMF (15 mL), triethylamine (3.5 mL) was added, and then, ethyl acrylate (308 mg, 3.08 mmol) and Pd(dppf)Cl₂ (224 mg, 0.308 mmol) were sequentially added. The reaction was heated to 95 °C and stirred for 18 h in a nitrogen atmosphere. The solvent was removed by rotary evaporation, and ethyl acetate (50 mL) was added. The mixture was filtered, and the filtrate was washed with water (50 mL), dried over anhydrous sodium sulfate, and concentrated. The resulting crude product was purified and separated by normal-phase column chromatography to give compound 85-3 (colorless oil, 300 mg, yield: 79%). MS m/z (ESI): 245.3 [M+H]⁺.

Step 4: Compound 85-3 (290 mg, 1.18 mmol) was dissolved in ethyl acetate (30 mL), and platinum dioxide (80 mg) was added. The compound was hydrogenated at 25 °C for 16 h. The reaction mixture was filtered, and the filtrate was concentrated to give the product (colorless oil, 220 mg, yield: 76%). MS m/z (ESI): 247.1 [M+H]⁺.

Step 5: Preparation was performed with reference to the synthesis method in step 3 of Example 1. MS m/z (ESI): 205.3 [M+H]⁺.

Step 6: Preparation was performed with reference to the synthesis method in step 5 of Example 84. Compound E85 was obtained. ¹H NMR (500 MHz, CD₃OD) δ 6.88-6.83 (m, 2H), 6.73-6.71 (m, 2H), 6.65 (d, *J* = 5.0 Hz, 1H), 6.55 (d, *J* = 5.0 Hz, 1H), 4.44 (s, 2H), 3.87 (d, *J* = 15.0 Hz, 1H), 3.38-3.34 (m, 2H), 3.09-3.06 (m, 1H), 2.94-2.92 (m, 1H), 2.58-2.44 (m, 6H), 2.10-1.87 (m, 5H), 1.00 (s, 4H). MS m/z (ESI): 416.6 [M+H]⁺.

### Example 86: (6bR,10aS)-8-(3-(6-Fluoro-2,3-dihydrobenzofuran-7-yl)propyl)-6b,7,8,9,10,10a-hexahydro-1H-pyrido[3',4':4,5]pyrrolo[1,2,3-de]quinoxalin-2(3H)-one

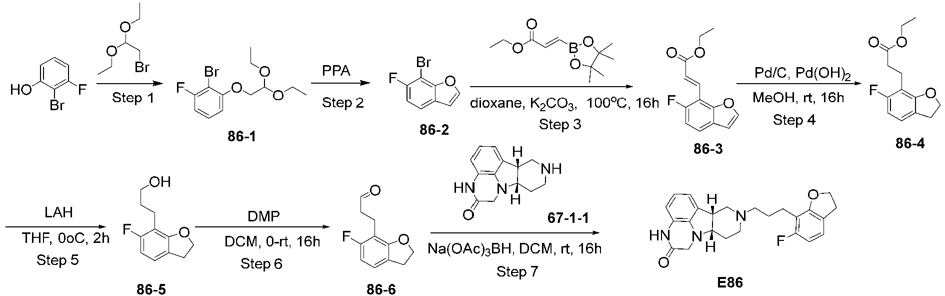

Step 1: In a nitrogen atmosphere, the compounds 2-bromo-3-fluorophenol (2 g, 10.5 mmol) and 2-bromo-1,1-diethoxyethane (2.47 g, 12.6 mmol) were dissolved in dry DMF (50 mL), and K₂CO₃ (4.3 g, 31.4 mmol) was then added. The mixture was left to react at 145 °C for 3 h. After LCMS analysis showed that the reaction had been complete, the reaction mixture was quenched with 100.0 mL of iced water and extracted with ethyl acetate (100 mL × 3). The organic phase was washed with saturated brine (100 mL × 3) and concentrated to dryness by rotary evaporation, and the crude product was purified by normal-phase silica gel column chromatography to give 86-1 (colorless liquid, 3 g, yield: 93%).

Step 2: Compound 86-1 (2 g, 9.77 mmol) was dissolved in toluene (30 mL), and PPA (3 mL) was then added. The mixture was stirred at 90 °C for 3 h. After the reaction mixture was cooled to room temperature, the solvent was removed by rotary evaporation. The reaction was quenched with 100.0 mL of iced water, and extraction was performed with ethyl acetate (100 mL × 3). The organic phase was washed with saturated brine (100 mL × 3) and concentrated to dryness by rotary evaporation, and the crude product was purified by normal-phase silica gel column chromatography to give 86-2 (colorless liquid, 1 g, yield: 47.6%).

Step 3: Compound E86 was prepared with reference to the synthesis method in steps 1-5 of Example 60. ¹H NMR(400 MHz, CDCl₃) δ 7.48 (d, *J* = 15.5 Hz, 1H), 6.92 (dd, *J* = 8.0, 5.5 Hz, 1H), 6.83 (d, *J* = 7.5 Hz, 1H), 6.73 (t, *J* = 7.5 Hz, 1H), 6.58 (d, *J* = 7.5 Hz, 1H), 6.49 (dd, *J* = 10.0, 8.0 Hz, 1H), 4.61-4.54 (m, 2H), 3.94 (d, *J* = 14.5 Hz, 1H), 3.44-3.27 (m, 3H), 3.15 (t, *J* = 8.7 Hz, 2H), 3.08-2.76 (m, 2H), 2.60 (t, *J* = 7.4 Hz, 2H), 2.26 (d, *J* = 194.5 Hz, 4H), 2.06-1.79 (m, 4H). MS m/z (ESI): 408.6 [M+H]⁺.

### Example 87: (6bR,10aS)-8-(3-(4-Fluoro-2,3-dihydrobenzofuran-7-yl)propyl)-6b,7,8,9,10,10a-hexahydro-1H-pyrido[3',4':4,5]pyrrolo[1,2,3-de]quinoxalin-2(3H)-one

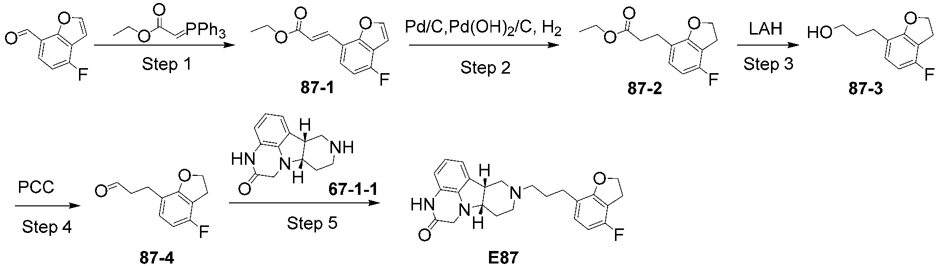

Step 1: 87-1 was prepared with reference to the synthesis method in step 1 of Example 3.

Step 2: 87-2 was prepared with reference to the synthesis method in step 2 of Example 3.

Step 3: 87-3 was prepared with reference to the synthesis method in step 3 of Example 3.

Step 4: 87-4 was prepared with reference to the synthesis method in step 4 of Example 8.

Step 5: E87 was prepared with reference to the synthesis method in step 5 of Example 8. ¹H NMR (500 MHz, CDCl₃) δ 7.55 (s, 1H), 6.89-6.80 (m, 2H), 6.72 (t, *J* = 7.6 Hz, 1H), 6.57 (dd, *J* = 7.9, 0.9 Hz, 1H), 6.48 (t, *J* = 8.4 Hz, 1H), 4.59 (t, *J* = 8.7 Hz, 2H), 3.95 (d, *J* = 14.5 Hz, 1H), 3.45-3.30 (m, 3H), 3.23 (t, *J* = 8.7 Hz, 2H), 2.94 (s, 1H), 2.84-2.68 (m, 1H), 2.53 (t, *J* = 7.6 Hz, 2H), 2.33 (d, *J* = 61.8 Hz, 3H), 2.06-1.79 (m, 5H). MS m/z (ESI): 408.6 [M+H]⁺.

### Example 88: (6bR,10aS)-8-(3-(5-Fluoro-2,3-dihydrobenzofuran-7-yl)propyl)-6b,7,8,9,10,10a-hexahydro-1H-pyrido[3',4":4,5]pyrrolo[1,2,3-de]quinoxalin-2(3H)-one

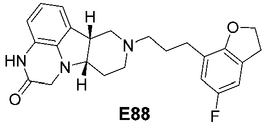

E88 was prepared with reference to the synthesis method of Example 60. ¹H NMR (400 MHz, CDCl₃) δ 7.59 (s, 1H), 6.83 (d, *J* = 7.5 Hz, 1H), 6.73 (t, *J* = 7.5 Hz, 2H), 6.63 (dd, *J* = 10.0, 2.5 Hz, 1H), 6.58 (d, *J* = 7.5 Hz, 1H), 4.54 (t, *J* = 8.5 Hz, 2H), 3.95 (d, *J* = 14.5 Hz, 1H), 3.44-3.27 (m, 3H), 3.17 (t, *J* = 8.6 Hz, 2H), 2.96 (s, 1H), 2.78 (s, 1H), 2.60-2.47 (m, 2H), 2.35 (d, *J* = 60.3 Hz, 3H), 2.04 (s, 1H), 1.97-1.77 (m, 4H). MS m/z (ESI): 408.6 [M+H]⁺.

### Example 89: (6bR,10aS)-8-(3-(6-Chloro-2,3-dihydrobenzofuran-7-yl)propyl)-6b,7,8,9,10,10a-hexahydro-1H-pyrido[3',4':4,5]pyrrolo[1,2,3-de]quinoxalin-2(3H)-one

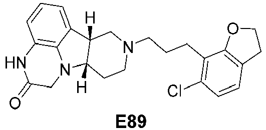

E89 was prepared with reference to the synthesis method of Example 60. ¹H NMR (400 MHz, CDCl₃) δ 7.42 (s, 1H), 6.94 (d, *J* = 8.0 Hz, 1H), 6.83 (dd, *J* = 12.5, 7.5 Hz, 2H), 6.76 (t, *J* = 7.5 Hz, 1H), 6.60 (d, *J* = 8.0 Hz, 1H), 4.55 (dt, *J* = 15.0, 8.5 Hz, 3H), 3.92 (s, 1H), 3.40 (d, *J* = 14.5 Hz, 3H), 3.17 (t, *J* = 8.7 Hz, 4H), 2.72 (d, *J* = 7.5 Hz, 3H), 2.67-2.43 (m, 2H), 2.11 (d, *J* = 94.6 Hz, 2H), 1.97-1.73 (m, 2H). MS m/z (ESI): 424.6 [M+H]⁺.

### Example 90: (6bR,10aS)-8-(3-(2,3-Dihydrobenzofuran-7-yl)propyl)-2-carbonyl-2,3,6b,7,8,9,10,10a-octahydro-1H-pyrido[3',4':4,5]pyrrolo[1,2,3-de]quinoxaline 8-oxide

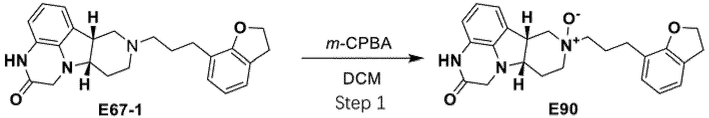

Step 1: E67-1 (10 mg, 2.67 mmol) was dissolved in DCM (5 mL), and m-CPBA (540 mg, 2.67 mmol) was then added. The mixture was left to react at room temperature for 2 h, and LCMS showed that the reaction had been complete. The reaction mixture was extracted with DCM, and the extract was concentrated, filtered, and then purified and separated by prep-HPLC (eluent (v/v): acetonitrile/(water + 0.05% NH₄HCO₃) = 30%-70%) to give compound E90 (5 mg, white solid, yield: 21.1%). ¹H NMR (400 MHz, CDCl₃) δ 8.61 (s, 1H), 7.07-7.00 (m, 1H), 6.89 (t, *J* = 10.5 Hz, 1H), 6.84 (d, *J* = 7.5 Hz, 1H), 6.76 (dq, *J* = 9.5, 7.5 Hz, 2H), 6.66 (d, *J* = 7.5 Hz, 1H), 4.49 (q, *J* = 9.0 Hz, 2H), 4.05-3.98 (m, 1H), 3.91 (d, *J* = 14.5 Hz, 1H), 3.41 (t, *J* = 14.6 Hz, 3H), 3.33-3.26 (m, 3H), 3.18 (q, *J* = 8.4 Hz, 2H), 2.96-2.86 (m, 1H), 2.73 (t, *J* = 11.6 Hz, 1H), 2.62 (t, *J* = 7.3 Hz, 2H), 2.25-2.17 (m, 2H), 1.92 (d, *J* = 15.0 Hz, 2H). MS m/z (ESI): 406.6 [M+H]⁺.

### Example 91: 8-(3-(2,3-Dihydrobenzofuran-7-yl)propyl)-6-fluoro-6b,7,8,9,10,10a-hexahydro-1H-pyrido[3',4':4,5]pyrrolo[1,2,3-de]quinoxalin-2(3H)-one

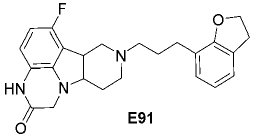

E91 was prepared with reference to the synthesis method of Example 83. ¹H NMR (400 MHz, CDCl₃) δ 7.70 (s,1H), 7.04 (d, *J* = 8.0 Hz, 1H), 6.93 (d, *J* = 7.5 Hz, 1H), 6.76 (t, *J* = 7.5 Hz, 1H), 6.50 (dd, *J* = 8.5, 3.5 Hz, 1H), 6.40 (t, *J* = 8.5 Hz, 1H), 4.54(t, *J* = 9.0 Hz, 2H), 3.94(d, *J* = 14.5 Hz, 1H), 3.62-3.50(m, 1H), 3.38 (d, *J* = 14.5 Hz, 2H), 3.20 (t, *J* = 8.5 Hz, 2H), 3.11-3.02 (m, 1H), 2.82-2.70 (m, 1H), 2.62-2.53 (m, 2H), 2.47-2.33 (m, 2H), 2.27-2.17 (m, 1H), 2.07-1.80 (m, 5H). MS m/z (ESI): 408.9 [M+H]⁺.

### Example 92: (6bR,10aS)-8-(3-(2,3-Dihydrofuro[3,2-c]pyridin-7-yl)propyl)-6b,7,8,9,10,10a-hexahydro-1H-pyrido[3',4':4,5]pyrrolo[1,2,3-de]quinoxalin-2(3H)-one

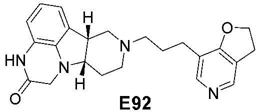

E92 was prepared with reference to the synthesis method of Example 60. ¹H NMR (500 MHz, CD₃OD) δ 8.13 (s, 1H), 8.05 (d, *J* = 10.5 Hz, 1H), 6.83 (d, *J* = 6.7 Hz, 1H), 6.70 (t, *J* = 7.6 Hz, 1H), 6.64 (dd, *J* = 7.8, 0.8 Hz, 1H), 4.72-4.65 (m, 2H), 3.87 (d, *J* = 14.6 Hz, 1H), 3.34 (d, *J* = 14.5 Hz, 1H), 3.28-3.24 (m, 2H), 3.21-3.08 (m, 1H), 2.94-2.90 (m, 1H), 2.80-2.74 (m, 1H), 2.60 (t, *J* = 7.6 Hz, 2H), 2.40-2.32 (m, 2H), 2.28-2.22 (m, 1H), 2.04-1.96 (m, 2H), 1.94-1.75 (m, 4H). MS m/z (ESI): 391.6 [M+H]⁺.

### Example 93: (6bR,10aS)-8-(3-(3-Methoxythiophen-2-yl)propyl)-6b,7,8,9,10,10a-hexahydro-1H-pyrido[3',4':4,5]pyrrolo[1,2,3-de]quinoxalin-2(3H)-one

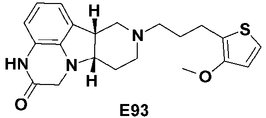

E93 was prepared with reference to the preparation method of Example 40. ¹H NMR (500 MHz, CD₃OD) δ 7.09 (d, *J* = 5.0 Hz, 1H), 6.89-6.87 (m, 2H), 6.75 (d, *J* = 10.0 Hz, 1H), 6.69 (d, *J* = 10.0 Hz, 1H), 3.91 (d, *J* = 15.0 Hz, 1H), 3.79 (s, 3H), 3.51-3.46 (m, 1H), 3.40-3.35 (m, 2H), 3.19-3.13 (m, 1H), 2.84-2.77 (m, 3H), 2.75-2.71 (m, 2H), 2.31-2.08 (m, 3H), 1.95-1.82 (m, 3H). MS m/z (ESI): 384.6 [M+H]⁺.

### Example 94: (6bR,10aS)-8-(4-(2,3-Dihydrobenzofuran-7-yl)butan-2-yl)-6b,7,8,9,10,10a-hexahydro-1H-pyrido[3',4':4,5]pyrrolo[1,2,3-de]quinoxalin-2(3H)-one

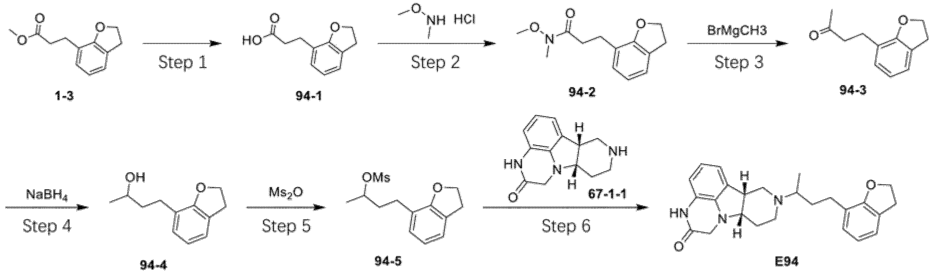

Step 1: 1-3 (320 mg, 1.47 mmol) was dissolved in methanol (5 mL) and a saturated aqueous sodium hydroxide solution (2 mL) at room temperature. The reaction solution was stirred at room temperature for 12 h. After the reaction solution was extracted with water and ethyl acetate, the pH of the aqueous phase was adjusted to 3-4 with dilute hydrochloric acid, and the aqueous phase was then extracted with water and ethyl acetate. The organic phase was dried over anhydrous sodium sulfate and then concentrated to dryness by rotary evaporation to give 94-1 (260 mg, yield: 94%) as a white solid.

Step 2: 94-1 (260 mg, 1.35 mmol), N,O-dimethylhydroxylamine hydrochloride (158 mg, 1.62 mmol), HATU (772 mg, 2.03 mmol), and DIEA (525 mg, 4.06 mmol) were dissolved in DMF (5 mL) at room temperature. The reaction solution was stirred at room temperature for 2 h. The reaction solution was extracted with water and ethyl acetate, and the organic phase was dried over anhydrous sodium sulfate and concentrated. The resulting residue was purified by column chromatography (PE:EA = 3:1) to give the title compound 94-2 (white solid, 300 mg, yield: 94%). MS m/z (ESI): 236.4 [M+H]⁺.

Step 3: At -78 °C, in a nitrogen atmosphere, 94-2 (250 mg, 1.06 mmol) was dissolved in THF (5 mL), and a solution of methylmagnesium bromide in tetrahydrofuran (3 M) (0.7 mL, 2.13 mmol) was added dropwise to the solution. Then, the reaction solution was gradually warmed and stirred at room temperature for 2 h. A saturated aqueous ammonium chloride solution (5 mL) was added to the reaction solution, and the reaction solution was extracted with water and ethyl acetate. The organic phase was dried over anhydrous sodium sulfate and concentrated, and the resulting residue was purified by column chromatography (PE:EA = 3:1) to give the title compound 94-3 (white solid, 180 mg, yield: 90%).

Step 4: 94-3 (50 mg, 0.26 mmol) and sodium borohydride (30 mg, 0.79 mmol) were dissolved in methanol (3 mL) at room temperature. The reaction solution was stirred at room temperature for 20 min. The reaction solution was extracted with water and ethyl acetate, and the organic phase was dried over anhydrous sodium sulfate and then concentrated to dryness by rotary evaporation to give the title compound 94-4 (colorless oil, 40 mg, yield: 82%).

Step 5: Ester 94-5 was prepared with reference to the synthesis method in step 4 of Example 3.

Step 6: At room temperature, 94-5 (15 mg, 0.055 mmol), 67-1-1 (13 mg, 0.055 mmol), and sodium iodide (3 mg, 0.11 mmol) were dissolved in DMF (2 mL), and potassium carbonate (23 mg, 0.17 mmol) was then added. The reaction solution was stirred at 80 °C for 12 h. The reaction solution was filtered under vacuum, and the filtrate was directly separated by reversed-phase preparative chromatography (C18 column, eluent gradient: acetonitrile/(water + 0.05% NH₄HCO₃)) to give compound E94 (2 mg, yield: 9%) as a white solid. ¹H NMR (500 MHz, CDCl₃) δ 7.53 (s, 1H), 7.07-7.01 (m, 1H), 6.94 (d, *J* = 7.5 Hz, 1H), 6.87-6.81 (m, 1H), 6.80-6.69 (m, 2H), 6.58 (d, *J* = 7.8 Hz, 1H), 4.58-4.47 (m, 2H), 3.95 (dd, *J* = 14.5, 1.7 Hz, 1H), 3.40-3.33 (m, 2H), 3.23-3.17 (m, 2H), 2.87-2.57 (m, 5H), 2.22 (t, *J* = 7.6 Hz, 1H), 2.11-1.72 (m, 4H), 1.43-1.21 (m, 3H), 1.01 (s, 2H). MS m/z (ESI): 404.8 [M+H]⁺.

### Example 95: (6bR,10aS)-8-(3-(6-Fluoro-2,3-dihydrobenzofuran-7-yl)propyl)-3-methyl-6b,7,8,9,10,10a-hexahydro-1H-pyrido[3',4':4,5]pyrrolo[1,2,3-de]quinoxalin-2(3H)-one

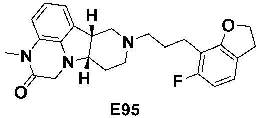

E95 was prepared with reference to the preparation method of Example 86. ¹H NMR (500 MHz, CDCl₃) δ 6.95-6.89 (m, 1H), 6.86 (d, *J* = 7.1 Hz, 1H), 6.80 (t, *J* = 7.6 Hz, 1H), 6.76-6.70 (m, 1H), 6.49 (dd, *J* = 10.0, 8.1 Hz, 1H), 4.58 (t, *J* = 8.7 Hz, 2H), 4.01 (d, *J* = 14.2 Hz, 1H), 3.39-3.25 (m, 6H), 3.21-3.10 (m, 2H), 2.93 (dd, *J* = 11.0, 6.2 Hz, 1H), 2.75 (d, *J* = 10.7 Hz, 1H), 2.58 (t, *J* = 7.5 Hz, 2H), 2.44-2.31 (m, 2H), 2.21 (t, *J* = 10.3 Hz, 1H), 2.05-1.90 (m, 2H), 1.79 (dd, *J* = 15.5, 7.3 Hz, 3H). MS m/z (ESI): 422.7 [M+H]⁺.

### Example 96: (6bR,10aS)-8-(2-((2,3-Dihydrobenzofuran-7-yl)oxy)ethyl)-3-methyl-6b,7,8,9,10,10a-hexahydro-1H-pyrido[3',4':4,5]pyrrolo[1,2,3-de]quinoxalin-2(3H)-one

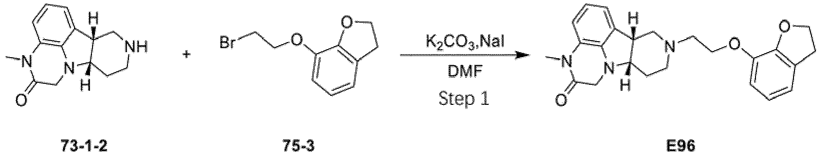

Step 1: At room temperature, 73-1-2 (10 mg, 0.041 mmol) was dissolved in DMF (2 mL), and 7-(2-bromoethoxy)-2,3-dihydrobenzofuran (10 mg, 0.041 mmol), sodium iodide (13 mg, 0.082 mmol), and DIEA (16 mg, 0.12 mmol) were added. The reaction solution was stirred at 80 °C for 2 h. The reaction solution was cooled to room temperature and concentrated *in vacuo* to give a crude product. The crude product was concentrated, filtered, and then purified and separated by prep-HPLC (eluent (v/v): acetonitrile/(water + 0.05% NH₄HCO₃) = 30%-70%) to give compound E96 (4.7 mg, yield: 28%) as a white solid. ¹H NMR (500 MHz, CDCl₃) δ 6.89-6.72 (m, 6H), 4.62 (q, *J* = 8.9 Hz, 3H), 4.38-4.33 (m, 1H), 4.18 (t, *J* = 6.0 Hz, 2H), 4.02 (d, *J* = 14.3 Hz, 1H), 3.68-3.60 (m, 1H), 3.36 (d, *J* = 30.0 Hz, 5H), 3.26-3.20 (m, 3H), 3.03 (s, 1H), 2.83 (s, 3H), 2.39 (s, 1H). MS m/z (ESI): 406.8 [M+H]⁺.

### Example 97: (6bR,10aS)-8-(3-(2,3-Dihydrofuro[2,3-c]pyridin-7-yl)propyl)-3-(methyl-d₃)-6b,7,8,9,10,10a-hexahydro-1H-pyrido[3',4':4,5]pyrrolo[1,2,3-de]quinoxalin-2(3H)-one

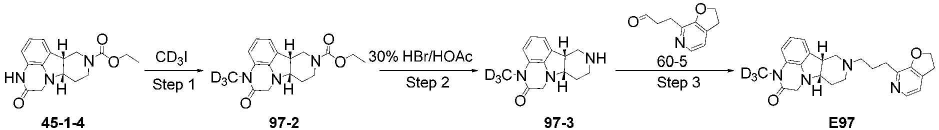

Step 1: Compound 45-1-4 (120 mg, 0.40 mmol) and anhydrous THF (5 mL) were added together to a 25 mL three-neck reaction flask, and NaH (30 mg, 0.80 mmol, 60% in oil) was subsequently added. After the addition, the mixture was stirred at room temperature for 0.5 h in a nitrogen atmosphere, and CD₃I (170 mg, 1.20 mmol) was subsequently added. After the addition, the mixture was stirred at room temperature for 3 h. After LCMS analysis showed that the reaction had been complete, water (30 mL) was added to the reaction solution, and extraction was performed with EtOAc (20 mL × 2). The organic phases were combined, washed with saturated brine (1 × 20 mL), dried over anhydrous sodium sulfate, and concentrated to dryness by rotary evaporation under reduced pressure to give compound 97-2 (crude, pale yellow oil, 120 mg, yield: 95%). The crude product was directly used in the next step without further purification. MS m/z (ESI): 319.5 [M+H]⁺.

Step 2: 97-3 was prepared with reference to the synthesis method in step 5 of Example 45-1. MS m/z (ESI): 247.6 [M+H]⁺.

Step 3: E97 was prepared with reference to the synthesis method in step 5 of Example E60. ¹H NMR (500 MHz, CD₃OD) δ 7.83 (d, *J* = 4.9 Hz, 1H), 7.09 (d, *J* = 4.9 Hz, 1H), 6.81 (d, *J* = 7.2 Hz, 1H), 6.77 (dd, *J* = 7.9, 0.9 Hz, 1H), 6.75-6.70 (m, 1H), 4.51 (t, *J* = 8.9 Hz, 2H), 3.88 (d, *J* = 14.4 Hz, 1H), 3.28 (d, *J* = 14.3 Hz, 1H), 3.25-3.21 (m, 2H), 3.20-3.15 (m, 2H), 2.97-2.87 (m, 1H), 2.75 (d, *J* = 9.5 Hz, 1H), 2.65 (t, *J* = 7.6 Hz, 2H), 2.42-2.30 (m, 2H), 2.28-2.18 (m, 1H), 1.99-1.81 (m, 4H), 1.78-1.68 (m, 1H). MS m/z (ESI): 409.0 [M+H]⁺.

### Example 98: (6bR,10aS)-8-(3-(3-Methoxypyridin-2-yl)propyl)-3-methyl-6b,7,8,9,10,10a-hexahydro-1H-pyrido[3',4':4,5]pyrrolo[1,2,3-de]quinoxalin-2(3H)-one

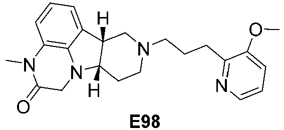

E98 was prepared with reference to the synthesis method of Example 16. ¹H NMR (400 MHz, CDCl₃) δ 7.42 (s, 1H), 6.94 (d, *J* = 8.0 Hz, 1H), 6.83 (dd, *J* = 12.5, 7.5 Hz, 2H), 6.76 (t, *J* = 7.5 Hz, 1H), 6.60 (d, *J* = 8.0 Hz, 1H), 4.55 (dt, *J* = 15.0, 8.5 Hz, 3H), 3.92 (s, 1H), 3.40 (d, *J* = 14.5 Hz, 3H), 3.17 (t, *J* = 8.7 Hz, 4H), 2.72 (d, *J* = 7.5 Hz, 3H), 2.67-2.43 (m, 2H), 2.11 (d, *J* = 94.6 Hz, 2H), 1.97-1.73 (m, 2H). MS m/z (ESI): 393.6 [M+H]⁺.

### Example 99: (6bR,10aS)-8-(3-(2-Methoxyphenyl)propyl)-3-methyl-6b,7,8,9,10,10a-hexahydro-1H-pyrido[3',4':4,5]pyrrolo[1,2,3-de]quinoxalin-2(3H)-one

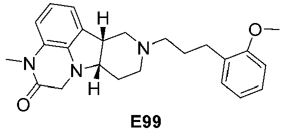

Preparation was performed with reference to the synthesis method of Example 3. ¹H NMR (500 MHz, CD₃OD) δ 7.23-7.15 (m, 2H), 7.00-6.87 (m, 5H), 4.05 (d, *J* = 15.0 Hz, 1H), 3.84 (s, 3H), 3.60-3.47 (m, 5H), 3.32 (s, 3H), 3.09-2.98 (m, 3H), 2.70 (t, *J* = 7.5 Hz, 2H), 2.50 (t, *J* = 10.0 Hz, 1H), 2.33-2.16 (m, 2H), 2.06-2.00 (m, 2H). MS m/z (ESI): 393.9 [M+H]⁺.

### Example 100: (6bR,10aS)-8-(3-(3-Methoxypyridin-2-yl)propyl)-6b,7,8,9,10,10a-hexahydro-1H-pyrido[3',4':4,5]pyrrolo[1,2,3-de]quinoxalin-2(3H)-one

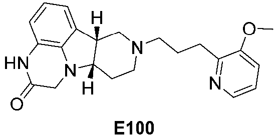

E100 was prepared with reference to the synthesis method of Example 16. ¹H NMR (400 MHz, CDCl₃) δ 8.09 (dd, *J* = 4.1, 1.9 Hz, 1H), 7.55 (s, 1H), 7.11-7.08 (m, 2H), 6.81 (dd, *J* = 12.9, 4.8 Hz, 1H), 6.73 (t, *J* = 7.6 Hz, 1H), 6.58 (d, *J* = 7.8 Hz, 1H), 3.93 (t, *J* = 14.6 Hz, 1H), 3.82 (s, 3H), 3.47-3.31 (m, 3H), 3.01 (s, 1H), 2.91-2.77 (m, 3H), 2.64-2.18 (m, 4H), 1.95 (d, *J* = 12.1 Hz, 4H). MS m/z (ESI): 379.6 [M+H]⁺.

### Example 101: (6bR,10aS)-8-(3-(3-Methoxythiophen-2-yl)propyl)-3-methyl-6b,7,8,9,10,10a-hexahydro-1H-pyrido[3',4':4,5]pyrrolo[1,2,3-de]quinoxalin-2(3H)-one

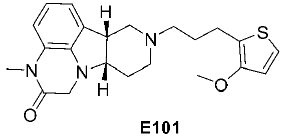

Preparation was performed with reference to the synthesis method of Example 3. ¹H NMR (500 MHz, CD₃OD) δ 6.88 (d, *J* = 5.0 Hz, 1H), 6.82-6.71 (m, 4H), 3.88 (d, *J* = 15.0 Hz, 1H), 3.73 (s, 3H), 3.29-3.24 (m, 3H), 3.22 (s, 3H), 2.94-2.90 (m, 1H), 2.77-2.75 (m, 1H), 2.60 (t, *J* = 7.5 Hz, 2H), 2.41-2.23 (m, 3H), 2.01-1.71 (m, 5H). MS m/z (ESI): 399.0 [M+H]⁺.

### Example 102: (6bR,10aS)-8-(3-(2,3-Dihydrofuro[3,2-c]pyridin-7-yl)propyl)-6b,7,8,9,10,10a-hexahydro-1H-pyrido[3',4':4,5]pyrrolo[1,2,3-de]quinoxalin-2(3H)-one

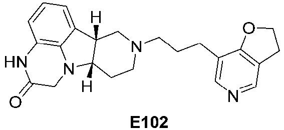

E102 was prepared with reference to the synthesis method of Example E60. ¹H NMR (500 MHz, CD₃OD) δ 8.13 (s, 1H), 8.05 (d, *J* = 10.5 Hz, 1H), 6.83 (d, *J* = 6.7 Hz, 1H), 6.70 (t, *J* = 7.6 Hz, 1H), 6.64 (dd, *J* = 7.8, 0.8 Hz, 1H), 4.72-4.65 (m, 2H), 3.87 (d, *J* = 14.6 Hz, 1H), 3.34 (d, *J* = 14.5 Hz, 1H), 3.28-3.24 (m, 2H), 3.21-3.08 (m, 1H), 2.94-2.90 (m, 1H), 2.80-2.74 (m, 1H), 2.60 (t, *J* = 7.6 Hz, 2H), 2.40-2.32 (m, 2H), 2.28-2.22 (m, 1H), 2.04-1.96 (m, 2H), 1.94-1.75 (m, 4H). MS m/z (ESI): 391.7 [M+H]⁺.

### Example 103: 8-(3-(2,3-Dihydrobenzofuran-7-yl)propyl)-4-fluoro-6b,7,8,9,10,10a-hexahydro-1H-pyrido[3',4':4,5]pyrrolo[1,2,3-de]quinoxalin-2(3H)-one

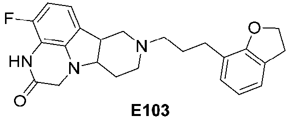

Preparation was performed with reference to the synthesis method of Example 83. ¹H NMR (500 MHz, CD₃OD) δ 6.83-6.80 (m, 1H), 6.73 (d, *J* = 10.0 Hz, 1H), 6.61 (t, *J* = 10.0 Hz, 1H), 6.54 (d, *J* = 5.0 Hz, 1H), 6.38 (t, *J* = 7.5 Hz, 1H), 4.49 (t, *J* = 7.5 Hz, 2H), 3.77 (d, *J* = 15.0 Hz, 1H), 3.26-3.23 (m, 3H), 3.12 (t, *J* = 7.5 Hz, 2H), 2.85-2.82 (m, 1H), 2.70-2.68 (m, 1H), 2.43 (t, *J* = 7.5 Hz, 2H), 2.30-2.15 (m, 3H), 1.95-1.68 (m, 5H). MS m/z (ESI): 408.9 [M+H]⁺.

### Example 104: (5bR,9aS)-7-(3-(2,3-Dihydrobenzofuran-7-yl)propyl)-5b,6,7,8,9,9a-hexahydroimidazo[4,5,1-hi]pyrido[4,3-b]indol-1(2H)-one

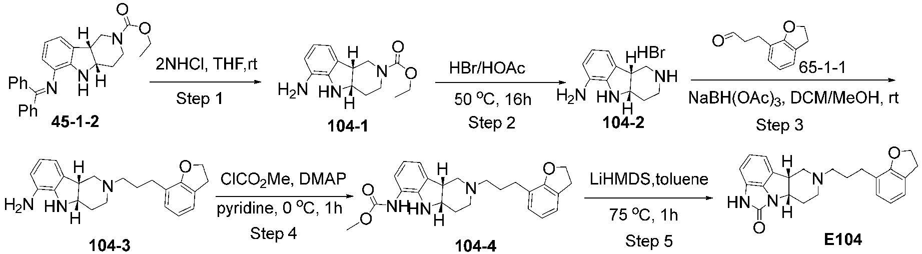

Step 1: 104-1 was prepared with reference to the synthesis method in step 3 of E45-1. MS m/z (ESI): 262.2 [M+H]⁺.

Step 2: 104-2 was prepared with reference to the synthesis method in step 5 of E45-1. MS m/z (ESI): 190.3 [M+H]⁺.

Step 3: 104-3 was prepared with reference to the synthesis method in step 2 of E67-1. MS m/z (ESI): 350.2 [M+H]⁺.

Step 4: 104-3 (140 mg, 0.4 mmol), DMAP (38 mg, 0.4 mmol), and pyridine (2 mL) were added to a 25 mL three-neck flask, and the mixture was purged three times with N₂ and cooled to 0 °C. Methyl chloroformate (38 mg, 0.4 mmol) was added, and the mixture was left to react at 0 °C for 1 h. Water and DCM were added, and the aqueous phase was isolated and extracted once with DCM. The organic phases were combined, washed three times with saturated sodium chloride, dried, and concentrated, and the residue was purified by column chromatography (DCM/MeOH = 20/1) to give 104-4 (90 mg, white solid, yield: 55%). MS m/z (ESI): 408.6 [M+H]⁺.

Step 5: 104-4 (90 mg, 0.22 mmol) and toluene (2 mL) were added to a 25 mL three-neck flask, and the mixture was purged three times with N₂. 1 M LiHMDS (0.9 mL, 0.9 mmol) was added, and the mixture was left to react at 75 °C for 1 h. Water and EA were added, and the aqueous phase was isolated and extracted once with EA. The organic phases were combined, washed three times with saturated sodium chloride, dried, and concentrated, and the residue was purified and separated by prep-HPLC (eluent (v/v): acetonitrile/(water + 0.05% NH₄HCO₃) = 30%-70%) to give E104 (13 mg, yellow solid, yield: 15.7%).

¹H NMR (500 MHz, CDCl₃) δ 8.13 (s, 1H), 7.04 (d, *J* = 8.2 Hz, 1H), 6.97-6.84 (m, 3H), 6.82-6.73 (m, 2H), 4.64 (q, *J* = 5.2 Hz, 1H), 4.54 (t, *J* = 8.5 Hz, 2H), 3.92 (q, *J* = 6.5 Hz, 1H), 3.20 (t, *J* = 8.5 Hz, 2H), 2.98 (dd, *J* = 11.9, 5.8 Hz, 1H), 2.66-2.47(m, 4H), 2.42 (m, 3H), 2.36-2.22 (m, 2H), 1.85-1.78 (m, 2H). MS m/z (ESI): 376.6 [M+H]⁺.

### Example 105: (6bR,10aS)-8-(2-((2,3-Dihydrofuro[2,3-c]pyridin-7-yl)oxy)ethyl)-3-methyl-6b,7,8,9,10,10a-hexahydro-1H-pyrido[3',4':4,5]pyrrolo[1,2,3-de]quinoxalin-2(3H)-one

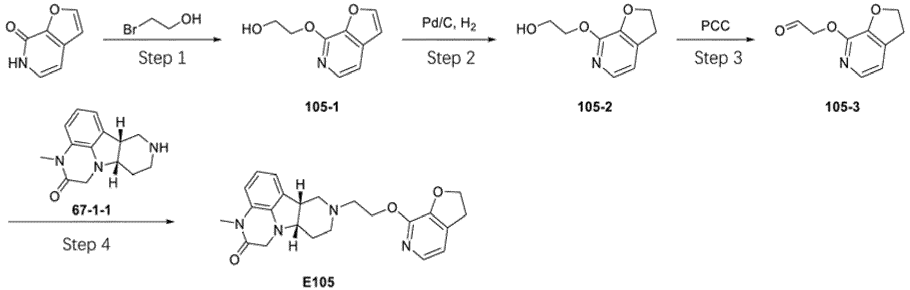

Step 1: The compounds furo[2,3-c]pyridin-7(6H)-one (120 mg, 0.89 mmol), bromoethanol (330 mg, 2.64 mmol), and anhydrous toluene (10 mL) were added together to a 25 mL single-neck reaction flask, and subsequently, Ag₂CO₃ (730 mg, 2.6 mmol) and NaI (330 mg, 2.6 mmol) were separately added. After the addition, the mixture was purged 3 times with nitrogen, slowly heated to 115 °C, and stirred for 48 h. After LCMS analysis showed that the reaction had been complete, the reaction solution was filtered, and the filtrate was concentrated to dryness by rotary evaporation under reduced pressure. The crude product was purified and separated by normal-phase column chromatography (eluent gradient: 5% MeOH/DCM) to give compound 105-2 (pale yellow oil, 60 mg, yield: 42%). ¹H NMR (500 MHz, CD₃OD) δ 7.93 (d, *J* = 2.1 Hz, 1H), 7.86 (d, *J* = 5.5 Hz, 1H), 7.26 (d, *J* = 5.5 Hz, 1H), 6.92 (d, *J* = 2.1 Hz, 1H), 4.57 (dd, *J* = 5.4, 4.3 Hz, 2H), 4.01-3.95 (m, 2H). MS m/z (ESI): 180.4 [M+H]⁺.

Step 2: 105-3 was prepared with reference to the synthesis method in step 2 of Example E60. MS m/z (ESI): 182.4 [M+H]⁺.

Step 3: 105-4 was prepared with reference to the synthesis method in step 3 of Example E60. MS m/z (ESI): 180.6 [M+H]⁺.

Step 4: E105 was prepared with reference to the synthesis method in step 4 of Example E60. ¹H NMR (500 MHz, CD₃OD) δ 7.62 (d, *J* = 5.0 Hz, 1H), 6.95-6.91 (m, 2H), 6.90 (d, *J* = 7.1 Hz, 1H), 6.86-6.83 (m, 1H), 4.62 (t, *J* = 9.3 Hz, 2H), 4.51 (t, *J* = 5.7 Hz, 2H), 4.01 (d, *J* = 14.4 Hz, 1H), 3.42-3.36 (m, 3H), 3.37-3.34 (m, 3H), 3.26 (t, *J* = 9.0 Hz, 2H), 3.12-3.08 (m, 1H), 2.96-2.90 (m, 1H), 2.86-2.78 (m, 2H), 2.46-2.40 (m, 1H), 2.07-2.02 (m, 2H), 1.98-1.94 (m, 1H). MS m/z (ESI): 407.6 [M+H]⁺.

### Example 106: (6bR,10aS)-8-(3-(2,3-Dihydrobenzofuran-4-yl)propyl)-6b,7,8,9,10,10a-hexahydro-1H-pyrido[3',4':4,5]pyrrolo[1,2,3-de]quinoxalin-2(3H)-one

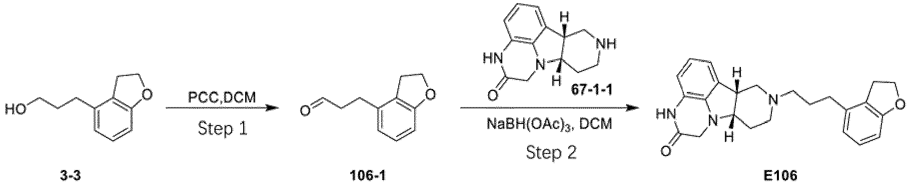

Step 1: 106-1 was prepared with reference to the synthesis method in step 4 of Example E8.

Step 2: E106 was prepared with reference to the synthesis method in step 5 of Example E8. ¹H NMR (500 MHz, CDCl₃) δ 7.53 (s, 1H), 7.04 (t, *J* = 7.8 Hz, 1H), 6.87-6.80 (m, 1H), 6.74 (t, *J* = 7.6 Hz, 1H), 6.65 (dd, *J* = 7.7, 6.0 Hz, 2H), 6.59 (d, *J* = 7.7 Hz, 1H), 4.56 (t, *J* = 8.7 Hz, 2H), 3.95 (d, *J* = 14.5 Hz, 1H), 3.56-3.29 (m, 3H), 3.15 (t, *J* = 8.6 Hz, 2H), 3.01 (s, 1H), 2.81 (d, *J* = 29.9 Hz, 1H), 2.66-2.26 (m, 5H), 2.17-1.82 (m, 5H). MS m/z (ESI): 390.9 [M+H]⁺.

### Example 107: (6bR,10aS)-8-(3-(5-Hydroxy-2,3-dihydrobenzofuran-7-yl)propyl)-6b,7,8,9,10,10a-hexahydro-1H-pyrido[3',4':4,5]pyrrolo[1,2,3-de]quinoxalin-2(3H)-one

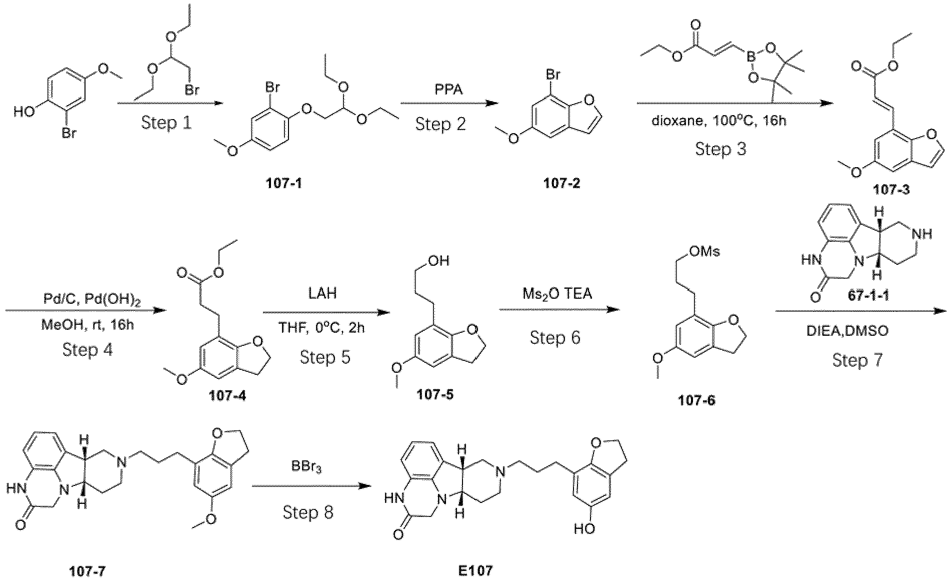

Preparation in steps 1-5 was performed with reference to the synthesis method in steps 1-5 of Example 86.

Step 6: 107-6 was prepared with reference to the synthesis method in step 6 of Example 13.

Step 7: 107-7 was prepared with reference to the synthesis method in step 6 of Example 7.

Step 8: Compound 107-7 (30 mg, 0.07 mmol) and anhydrous dichloromethane (3 mL) were added together to a 25 mL three-neck reaction flask, and BBr₃ (2.1 mL, 2.1 mmol) was added at -78 °C. After the addition, the mixture was slowly warmed to -10 °C and then stirred at this temperature for 3 h. After LCMS analysis showed that the reaction had been complete, ammonia water (1 mL) was added to the reaction solution, and the mixture was concentrated, filtered, and then purified and separated by prep-HPLC (eluent (v/v): acetonitrile/(water + 0.05% NH₄HCO₃) = 30%-70%) to give compound E107 (5 mg, white solid, yield: 16.9%).

¹H NMR(400 MHz, CDCl₃) δ 7.49 (d, *J* = 16.3 Hz, 1H), 6.83 (d, *J* = 7.3 Hz, 1H), 6.74 (t, *J* = 7.6 Hz, 1H), 6.58 (dd, *J* = 12.8, 4.9 Hz, 2H), 6.43 (d, *J* = 2.4 Hz, 1H), 4.49 (t, *J* = 8.6 Hz, 2H), 3.94 (d, *J* = 14.5 Hz, 1H), 3.50 (s, 1H), 3.42-3.31 (m, 2H), 3.13 (dd, *J* = 19.9, 11.2 Hz, 2H), 3.07 (s, 1H), 2.90 (d, *J* = 12.4 Hz, 2H), 2.50 (dd, *J* = 33.2, 25.7 Hz, 5H), 2.18 (s, 1H), 1.98 (dd, *J* = 31.1, 16.2 Hz, 4H). MS m/z (ESI): 406.8 [M+H]⁺.

### Example 108: (6bR,10aS)-8-(3-(6-Hydroxy-2,3-dihydrobenzofuran-7-yl)propyl)-6b,7,8,9,10,10a-hexahydro-1H-pyrido[3',4':4,5]pyrrolo[1,2,3-de]quinoxalin-2(3H)-one hydrochloride

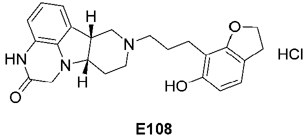

Preparation was performed with reference to the synthesis method of Example 107. ¹H NMR (500 MHz, DMSO-*d*₆) δ 10.61 (s, 1H), 10.50 (s, 1H), 9.33 (s, 1H), 6.86 (d, *J* = 7.2 Hz, 1H), 6.82 (d, *J* = 7.9 Hz, 1H), 6.70 (d, *J* = 7.5 Hz, 1H), 6.66 (d, *J* = 7.8 Hz, 1H), 6.32 (d, *J* = 8.0 Hz, 1H), 5.33 (t, *J* = 5.1 Hz, 1H), 4.47 (t, *J* = 8.5 Hz, 2H), 3.90 (d, *J* = 14.5 Hz, 2H), 3.04 (t, *J* = 8.5 Hz, 4H), 2.48-2.35 (m, 4H), 2.24 (s, 2H), 2.03-1.97 (m, 3H), 1.90 (s, 2H). MS m/z (ESI): 406.6 [M+H]⁺.

### Example 109: (6bR,10aS)-8-(3-(2,3-Dihydrobenzofuran-7-yl)propyl)-1,1-dimethyl-6b,7,8,9,10,10a-hexahydro-1H-pyrido[3',4':4,5]pyrrolo[1,2,3-de]quinoxalin-2(3H)-one

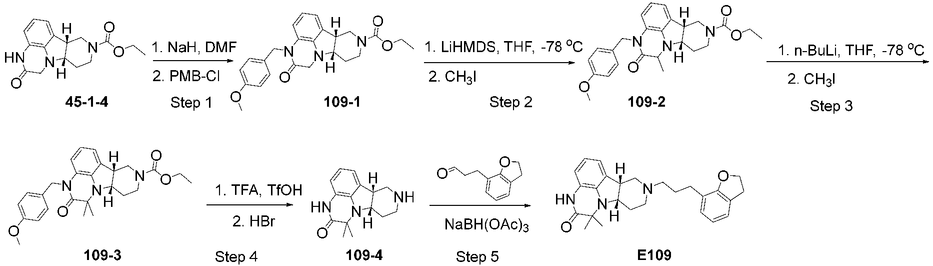

Step 1: Compound 45-1-4 (300 mg, 1.0 mmol) was dissolved in DMF (10 mL), and the solution was cooled to 0 °C in an ice-water bath. Sodium hydride (120 mg, 3.0 mmol) was added, and the mixture was stirred at 0 °C for 30 min. Then, p-methoxybenzyl chloride (310 mg, 2.0 mmol) was added. The mixture was stirred at room temperature for 16 h. After LCMS analysis showed that the reaction had been complete, water (20 mL) was added to the reaction solution, and extraction was performed with EtOAc (2 × 25 mL). The organic phases were combined, washed with saturated brine (1 × 25 mL), dried over anhydrous sodium sulfate, and concentrated to dryness by rotary evaporation under reduced pressure, and the crude product was triturated with methyl tert-butyl ether to give compound 109-1 (white solid, 185 mg, yield: 44%). MS m/z (ESI): 422.5 [M+H]⁺.

Step 2: Compound 109-1 (150 mg, 0.36 mmol) was dissolved in THF (5 mL), and the solution was cooled to -78 °C. LiHMDS (1.45 mL, 1.45 mmol) was added. After 1 h of stirring at -78 °C, iodomethane (200 mg, 1.42 mmol) was added, and the mixture was warmed to room temperature and stirred for 18 h. After LCMS analysis showed that the reaction had been complete, water (15 mL) was added to the reaction solution, and extraction was performed with EtOAc (2 × 25 mL). The organic phases were combined, washed with saturated brine (1 × 25 mL), dried over anhydrous sodium sulfate, and concentrated to dryness by rotary evaporation under reduced pressure, and the crude product was purified and separated by normal-phase column chromatography (eluent gradient: 0-50% EtOAc/petroleum ether) to give compound 109-2 (yellow oil, 130 mg, yield: 83%). MS m/z (ESI): 437.0 [M+H]⁺.

Step 3: Compound 109-2 (240 mg, 0.55 mmol) was dissolved in THF (5 mL), and the solution was cooled to -78 °C. n-BuLi (1 mL, 1.6 mmol) was added. After 1 h of stirring at - 78 °C, iodomethane (235 mg, 1.6 mmol) was added, and the mixture was warmed to room temperature and stirred for 16 h. After LCMS analysis showed that the reaction had been complete, water (15 mL) was added to the reaction solution, and extraction was performed with EtOAc (2 × 25 mL). The organic phases were combined, washed with saturated brine (1 × 25 mL), dried over anhydrous sodium sulfate, and concentrated to dryness by rotary evaporation under reduced pressure, and the crude product was purified and separated by normal-phase column chromatography (eluent gradient: 0-50% EtOAc/petroleum ether) to give compound 109-3 (yellow oil, 66 mg, yield: 26%). MS m/z (ESI): 450.5 [M+H]⁺.

Step 4: Compound 109-3 (66 mg, 0.15 mmol) was dissolved in TFA (205 µL), and TfOH (90 mg, 0.6 mmol) was added. The mixture was stirred at room temperature for 3 h. After LCMS analysis showed that the reaction had been complete, the reaction mixture was diluted with DCM (20 mL), washed with a saturated aqueous sodium bicarbonate solution (20 mL) and saturated brine (20 mL), dried over anhydrous sodium sulfate, and concentrated to dryness by rotary evaporation under reduced pressure, and a solution of HBr in acetic acid (1.5 mL) was added. The mixture was heated to 50 °C, stirred for 3 h, and concentrated, and the crude product was purified and separated by prep-HPLC (eluent (v/v): acetonitrile/(water + 0.05% NH₄HCO3) = 30%-70%) to give compound 109-4 (colorless oil, 18 mg, yield: 46%). MS m/z (ESI): 258.4 [M+H]⁺.

Step 5: Preparation was performed with reference to the synthesis method in step 5 of Example 8. ¹H NMR (500 MHz, CD₃OD) δ 6.94 (d, *J* = 5.0 Hz, 1H), 6.79 (d, *J* = 5.0 Hz, 1H), 6.70 (d, *J* = 10.0 Hz, 1H), 6.65-6.59 (m, 2H), 6.51 (d, *J* = 5.0 Hz, 1H), 4.40 (t, *J* = 7.5 Hz, 2H), 3.84 (d, *J* = 10.0 Hz, 1H), 3.31-3.26 (m, 1H), 3.07 (d, *J* = 10.0 Hz, 2H), 2.89 (d, *J* = 10.0 Hz, 1H), 2.78 (d, *J* = 10.0 Hz, 1H), 2.47-2.35 (m, 5H), 2.24-2.21 (m, 1H), 2.00-1.87 (m, 2H), 1.80-1.73 (m, 2H), 1.54 (s, 3H), 1.07 (s, 3H). MS m/z (ESI): 418.8 [M+H]⁺.

### Example 110: (6bR,10aS)-8-(3-(2,3-Dihydrofuro[3,2-b]pyridin-7-yl)propyl)-3-methyl-6b,7,8,9,10,10a-hexahydro-1H-pyrido[3',4':4,5]pyrrolo[1,2,3-de]quinoxalin-2(3H)-one

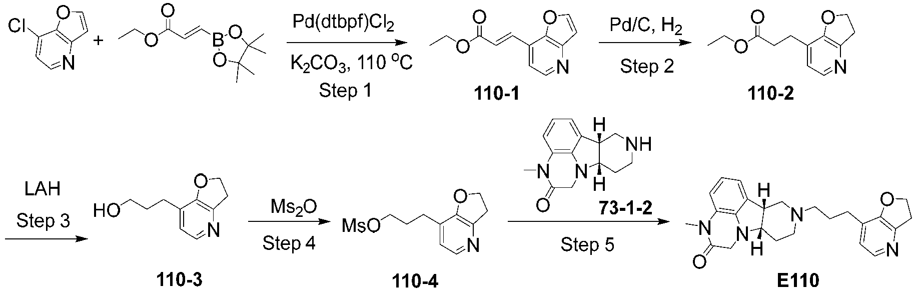

Step 1: The compounds 7-chlorofuro[3,2-b]pyridine (500 mg, 3.3 mmol), ethyl (E)-3-(4,4,5,5-tetramethyl-1,3,2-dioxaborolan-2-yl)acrylate (1.47 g, 6.5 mmol), Pd(dtbpf)Cl₂ (425 mg, 0.65 mmol), and K₂CO₃ (1.4 g, 9.8 mmol) were added to a three-neck flask containing dioxane (6 mL) and water (1 mL), and the mixture was purged three times with nitrogen and left to react at 110 °C for 16 h. After LCMS analysis showed that the reaction had been complete, water and EA were added, and the aqueous phase was isolated and extracted twice with EA. The organic phases were combined, washed twice with saturated sodium chloride, dried over anhydrous sodium sulfate, filtered, and concentrated, and the resulting residue was purified by normal-phase column chromatography (petroleum ether:ethyl acetate = 5:1) to give 110-1 (colorless oil, 600 mg, yield: 84%).

Step 2: 110-2 was prepared with reference to the synthesis method in step 2 of Example 1.

Step 3: 110-3 was prepared with reference to the synthesis method in step 3 of Example 1.

Step 4: 110-4 was prepared with reference to the synthesis method in step 4 of Example 3.

Step 5: E110 was prepared with reference to the synthesis method in step 5 of Example 3. ¹H NMR (500 MHz, CDCl₃) δ 7.93 (d, *J* = 5.0 Hz, 1H), 6.88-6.79 (m, 3H), 6.77-6.72 (m, 1H), 4.63 (t, *J* = 8.9 Hz, 2H), 4.01 (d, *J* = 14.2 Hz, 1H), 3.40-3.28 (m, 8H), 2.92 (s, 1H), 2.73 (s, 1H), 2.57 (t, *J* = 7.6 Hz, 2H), 2.36 (s, 2H), 2.11-1.73 (m, 6H). MS m/z (ESI): 405.8 [M+H]⁺.

### Example 111: (6bR,10aS)-8-(3-(2,3-Dihydrofuro[3,2-b]pyridin-7-yl)propyl)-6b,7.,8,9,10,10a-hexahydro-1H-pyrido[3',4':4,5]pyrrolo[1,2,3-de]quinoxalin-2(3H)-one

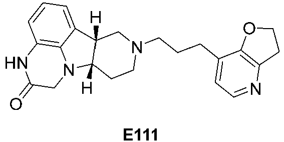

Preparation was performed with reference to the synthesis method of Example 110. ¹H NMR (500 MHz, CDCl₃) δ 7.94 (d, *J* = 5.0 Hz, 1H), 7.59 (s, 1H), 6.86-6.81 (m, 2H), 6.74 (t, *J* = 7.6 Hz, 1H), 6.59 (d, *J* = 7.7 Hz, 1H), 4.63 (t, *J* = 8.9 Hz, 2H), 3.95 (d, *J* = 14.5 Hz, 1H), 3.46-3.26 (m, 5H), 2.96 (s, 1H), 2.79 (s, 1H), 2.58 (t, *J* = 7.6 Hz, 2H), 2.42 (s, 2H), 2.14-1.81 (m, 6H). MS m/z (ESI): 391.9 [M+H]⁺.

### Example 112: (6bR,10aS)-8-(3-(4-Hydroxy-2,3-dihydrobenzofuran-7-yl)propyl)-6b,7,8,9,10,10a-hexahydro-1H-pyrido[3',4':4,5]pyrrolo[1,2,3-de]quinoxalin-2(3H)-one

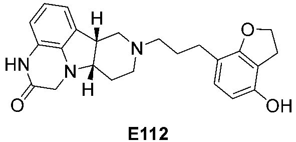

Preparation was performed with reference to the synthesis method of Example 107. ¹H NMR (500 MHz, CDCl₃) δ 7.47 (d, *J* = 4.2 Hz, 1H), 6.83 (d, *J* = 7.4 Hz, 1H), 6.75 (t, *J* = 7.9 Hz, 2H), 6.60 (d, *J* = 7.8 Hz, 1H), 6.22 (d, *J* = 8.1 Hz, 1H), 4.57 (t, *J* = 8.7 Hz, 2H), 3.93 (d, *J* = 14.5 Hz, 1H), 3.58 (s, 1H), 3.40 (d, *J* = 14.5 Hz, 2H), 3.14 (t, *J* = 8.7 Hz, 4H), 2.52 (t, *J* = 7.4 Hz, 5H), 2.15-1.84 (m, 5H). MS m/z (ESI): 406.8 [M+H]⁺.

### Example 114: (6bR,10aS)-8-(3-(2,3-Dihydrobenzofuran-7-yl)propyl)-6b,7,8,9,10,10a-hexahydro-1H-pyrido[3',4':4,5]pyrrolo[1,2,3-de]quinoxalin-2(3H)-one-1,1-d₂

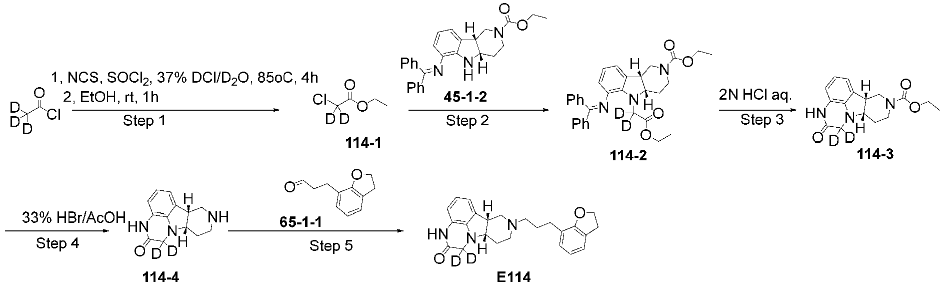

Step 1: Deuterated acetyl chloride (1.4 g, 17.2 mmol), thionyl chloride (5 mL), and NCS (3.5 g, 25.7 mmol) were added together to a 25 mL single-neck flask, and 2 drops of deuterated hydrochloric acid (35% wt in D₂O) were subsequently added. After the addition, the mixture was slowly heated to 85 °C and stirred for 4 h in a nitrogen atmosphere. Subsequently, the reaction solution was cooled to 0 °C, and absolute ethanol (4 mL) was slowly added dropwise. After the dropwise addition, the mixture was stirred at 0 °C for 0.5 h. After the reaction was complete, water (20 mL) was added to the reaction solution, and extraction was performed with n-heptane (20 mL × 3). The organic phases were combined, washed with saturated NaHCO₃ (20 mL × 1) and then with saturated brine (50 mL × 1), and dried over anhydrous sodium sulfate, and n-heptane was removed under reduced pressure to give compound 114-1 (yellow oil, 600 mg, yield: 28%). The crude product was directly used in the next step without further purification.

Step 2: Compound 114-2 was prepared with reference to the synthesis method in step 2 of Example 45-1. MS m/z (ESI): 514.8 [M+H]⁺.

Step 3: Compound 114-3 was prepared with reference to the synthesis method in step 3 of Example 45-1. MS m/z (ESI): 304.4 [M+H]⁺.

Step 4: Compound 114-4 was prepared with reference to the synthesis method in step 5 of Example 45-1. MS m/z (ESI): 232.5 [M+H]⁺.

Step 5: Compound E114 was prepared with reference to the synthesis method in step 5 of Example E60. ¹H NMR (500 MHz, CDCl₃) *δ* 7.55 (s, 1H), 7.04 (dd, *J* = 7.3, 0.9 Hz, 1H), 6.92 (d, *J* = 7.5 Hz, 1H), 6.83 (d, *J* = 7.3 Hz, 1H), 6.74 (dt, *J* = 19.5, 7.5 Hz, 2H), 6.57 (d, *J* = 7.6 Hz, 1H), 4.53 (t, *J* = 8.7 Hz, 2H), 3.45-3.31 (m, 2H), 3.20 (t, *J* = 8.7 Hz, 2H), 3.00-2.90 (m, 1H), 2.86-2.74 (m, 1H), 2.60-2.54 (m, 2H), 2.48-2.20 (m, 3H), 2.10-1.98 (m, 1H), 1.97-1.81 (m, 4H). MS m/z (ESI): 392.5 [M+H]⁺.

### Example 116: (6bR,10aS)-8-(3-(2,3-Dihydrothieno[3,4-b]furan-6-yl)propyl)-3-methyl-6b,7,8,9,10,10a-hexahydro-1H-pyrido[3',4':4,5]pyrrolo[1,2,3-de]quinoxalin-2(3H)-one

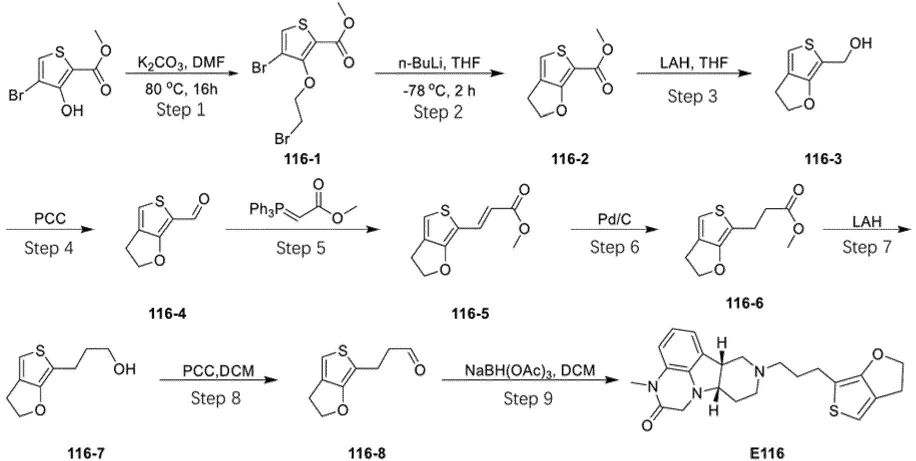

Step 1: Methyl 4-bromo-3-hydroxythiophene-2-carboxylate (5 g, 21.09 mmol), 1,2-dibromoethane (31.7 g, 168.7 mmol), potassium carbonate (11.6 g, 84.36 mmol), and anhydrous DMF (50 mL) were added together to a 250 mL three-neck flask, and the mixture was stirred at 80 °C for 16 h. After TLC analysis showed that the reaction had been complete, water (80 mL) was added to the reaction solution, and extraction was performed with ethyl acetate (80 mL × 3). The organic phase was washed with brine (50 mL × 3) and concentrated, and the resulting residue was purified by normal-phase column chromatography (petroleum ether:ethyl acetate = 5:1) to give 116-1 (white solid, 6.2 g, yield: 85%).

Step 2: 116-1 (3.5 g, 10.17 mmol) and anhydrous THF (35 mL) were added together to a 250 mL three-neck flask and cooled to -78 °C, and subsequently, n-BuLi (2.5 M in hexanes, 4.5 mL, 11.19 mmol) was slowly added dropwise. After the dropwise addition, the mixture was stirred at -78 °C for 2 h. After LCMS analysis showed that the reaction had been complete, a saturated aqueous ammonium chloride solution (40 mL) was added to the reaction solution, and extraction was performed with ethyl acetate (60 mL × 3). The organic phase was washed with brine (50 mL × 3) and concentrated, and the resulting residue was purified by normal-phase column chromatography (petroleum ether:ethyl acetate = 5:1) to give 116-2 (white solid, 400 mg, yield: 21%). MS m/z (ESI): 185.2 [M+H]⁺.

Step 3: 116-3 was prepared with reference to the synthesis method in step 3 of Example 1.

Step 4: 116-4 was prepared with reference to the synthesis method in step 4 of Example 8.

Step 5: 116-5 was prepared with reference to the synthesis method in step 1 of Example 1.

Step 6: 116-6 was prepared with reference to the synthesis method in step 2 of Example 1.

Step 7: 116-7 was prepared with reference to the synthesis method in step 3 of Example 1.

Step 8: 116-8 was prepared with reference to the synthesis method in step 4 of Example 8.

Step 9: E116 was prepared with reference to the synthesis method in step 5 of Example 8. ¹H NMR (500 MHz, CDCl₃) δ 6.89-6.85 (m, 1H), 6.82 (t, *J* = 7.6 Hz, 1H), 6.75 (dd, *J* = 7.9, 1.1 Hz, 1H), 6.52 (t, *J =* 1.5 Hz, 1H), 4.82 (t, *J* = 7.9 Hz, 2H), 4.01 (d, *J* = 14.2 Hz, 1H), 3.46 (d, *J* = 9.0 Hz, 1H), 3.38 (d, *J* = 14.3 Hz, 1H), 3.33 (s, 3H), 3.31 (s, 1H), 3.01 (s, 1H), 2.94 (td, *J* = 7.9, 1.5 Hz, 2H), 2.85 (s, 1H), 2.63 (t, *J* = 7.3 Hz, 2H), 2.47 (s, 2H), 2.32 (s, 1H), 2.10 (s, 1H), 1.91 (d, *J* = 36.5 Hz, 4H). MS m/z (ESI): 410.8 [M+H]⁺.

### Example 117: (6bR,10aS)-8-(3-(2,3-Dihydrobenzofuran-7-yl)propyl)-6b,7,8,9,10,1.a-hexahydro-1H-pyrido[3',4':4,5]pyrrolo[1,2,3-de]quinoxaline-1,2(3H)-dione

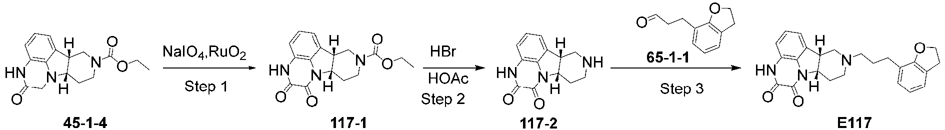

Step 1: 45-1-4 (60 mg, 0.2 mmol), NaIO₄ (120 mg, 0.56 mmol), RuO₂ (15 mg, 0.11 mmol), and CCl₄ (5 mL) were added together to a 50 mL single-neck flask and stirred at room temperature for 80 h. After LCMS analysis showed that the reaction had been complete, water and EA were added, and the aqueous phase was isolated and extracted twice with EA. The organic phases were combined, washed three times with saturated sodium chloride, and dried over anhydrous sodium sulfate to give 117-1 (gray solid, 40 mg, yield: 63%). The crude product was directly used in the next step without further purification. MS m/z (ESI): 316.5 [M+H]⁺.

Step 2: E117-2 was prepared with reference to the synthesis method in step 5 of Example 45-1.

Step 3: E117 was prepared with reference to the synthesis method in step 5 of Example 8. ¹H NMR (500 MHz, CDCl₃) δ 10.05 (s, 1H), 7.16 (t, *J* = 7.7 Hz, 1H), 7.10 (dt, *J* = 7.5, 1.1 Hz, 1H), 7.06-7.01 (m, 2H), 6.88 (d, *J* = 7.5 Hz, 1H), 6.76 (t, *J* = 7.4 Hz, 1H), 4.88 (td, *J* = 8.2, 6.2 Hz, 1H), 4.55 (t, *J* = 8.7 Hz, 2H), 3.71 (s, 1H), 3.21 (t, *J* = 8.7 Hz, 2H), 3.15 (d, *J* = 12.4 Hz, 1H), 2.72-2.60 (m, 2H), 2.59-2.35 (m, 5H), 2.24 (dd, *J* = 20.1, 12.5 Hz, 1H), 1.98 (s, 1H), 1.85-1.75 (m, 2H). MS m/z (ESI): 404.9 [M+H]⁺.

### Example 118: (6bR,10aS)-8-(3-(2,3-Dihydrobenzofuran-7-yl)propyl)-1-methyl-6b,7,8,9,10,10a-hexahydro-1H-pyrido[3',4':4,5]pyrrolo[1,2,3-de]quinoxalin-2(3H)-one

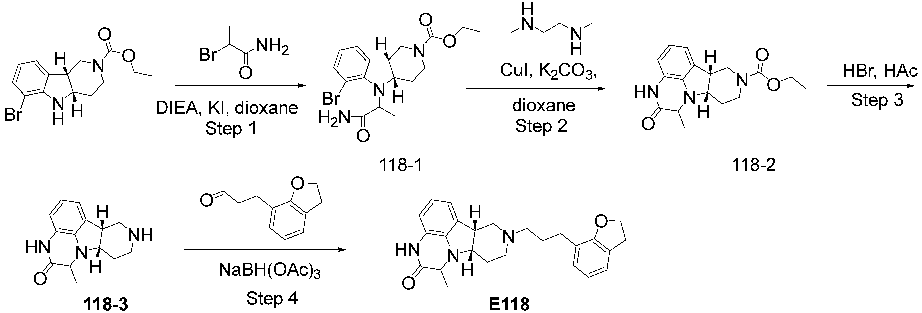

Step 1: The compound ethyl (4aS,9bR)-6-bromo-1,3,4,4a,5,9b-hexahydro-2H-pyrido[4,3-b]indole-2-carboxylate (320 mg, 1.0 mmol) was dissolved in dioxane (5 mL), and 2-bromopropanamide (228 mg, 1.5 mmol), KI (165 mg, 1.0 mmol), and DIEA (260 mg, 2.0 mmol) were sequentially added. The mixture was heated to 105 °C and stirred for 16 h. The reaction solution was cooled to room temperature, and EtOAc (50 mL) was added. The mixture was filtered and concentrated to dryness by rotary evaporation under reduced pressure, and the crude product was purified and separated by normal-phase column chromatography (eluent gradient: 20-100% EtOAc/petroleum ether) to give compound 118-1 (colorless oil, 180 mg, yield: 45%). MS m/z (ESI): 398.5 [M+H]⁺.

Step 2: Compound 118-1 (145 mg, 0.37 mmol) was dissolved in dioxane (3 mL), and CuI (18 mg, 0.092 mmol), K₂CO₃ (112 mg, 0.82 mmol), and N,N-dimethylethylenediamine (24 µL, 0.22 mmol) were sequentially added. The reaction mixture was purged with nitrogen, heated to 110 °C, and stirred for 18 h. The reaction solution was cooled to room temperature, filtered, and concentrated to dryness by rotary evaporation under reduced pressure, and the crude product was purified and separated by normal-phase column chromatography (eluent gradient: 20-100% EtOAc/petroleum ether) to give compound 118-2 (yellow oil, 86 mg, yield: 73%). MS m/z (ESI): 317.8 [M+H]⁺.

Step 3: Compound 118-2 (86 mg, 0.273 mmol) was added to a solution of HBr in acetic acid (2.0 mL), and the mixture was heated to 50 °C, stirred for 3 h, and concentrated to give compound 118-3 (yellow oil, 80 mg, crude). MS m/z (ESI): 244.4 [M+H]⁺.

Step 4: Preparation was performed with reference to the synthesis method in step 5 of Example 8. ¹H NMR (500 MHz, CD₃OD) δ 7.14-7.10 (m, 1H), 6.90-6.70 (m, 5H), 4.58 (t, *J* = 7.5 Hz, 2H), 3.60-3.55 (m, 3H), 3.27 (t, *J* = 7.5 Hz, 2H), 3.11-2.98 (m, 2H), 2.65-2.14 (m, 7H), 2.05-1.91 (m, 3H), 1.65 (d, *J* = 7.5 Hz, 3H). MS m/z (ESI): 404.8 [M+H]⁺.

### Example 119: (6bR,10aS)-8-(2-((2,3-Dihydrofuro[2,3-c]pyridin-7-yl)oxy)ethyl)-6b,7,8,9,10,10a-hexahydro-1H-pyrido[3',4':4,5]pyrrolo[1,2,3-de]quinoxalin-2(3H)-one

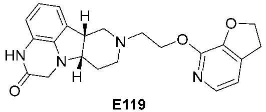

E119 was prepared with reference to the synthesis method of Example E105. ¹H NMR (500 MHz, CD₃OD) *δ* 7.77 (d, *J* = 5.0 Hz, 1H), 7.07 (d, *J* = 5.0 Hz, 1H), 7.00 (d, *J* = 7.3 Hz, 1H), 6.88 (t, *J* = 7.6 Hz, 1H), 6.81 (d, *J* = 7.2 Hz, 1H), 4.76 (t, *J* = 8.9 Hz, 2H), 4.65 (t, *J* = 5.7 Hz, 2H), 4.05 (d, *J* = 14.6 Hz, 1H), 3.53-3.48 (m, 3H), 3.43-3.37 (m, 2H), 3.23 (dd, *J* = 11.8, 6.2 Hz, 1H), 3.10-3.03 (m, 1H), 3.02-2.92 (m, 2H), 2.63-2.55 (m, 1H), 2.22-2.09 (m, 3H). MS m/z (ESI): 393.9 [M+H]⁺.

### Example 120: (6bR,10aS)-8-(3-(2,3-Dihydrobenzofuran-4-yl)propyl)-3-methyl-6b,7,8,9,10,10a-hexahydro-1H-pyrido[3',4':4,5]pyrrolo[1,2,3-de]quinoxalin-2(3H)-one

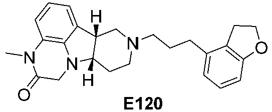

Preparation was performed with reference to the synthesis method of Example 106. ¹H NMR (500 MHz, CDCl₃) δ 7.04 (t, *J* = 7.8 Hz, 1H), 6.86 (dd, *J* = 7.3, 1.1 Hz, 1H), 6.81 (t, *J* = 7.6 Hz, 1H), 6.75 (dd, *J* = 7.9, 1.1 Hz, 1H), 6.65 (dd, *J* = 9.2, 7.8 Hz, 2H), 4.56 (t, *J* = 8.7 Hz, 2H), 4.01 (d, *J* = 14.2 Hz, 1H), 3.38 (d, *J* = 14.3 Hz, 2H), 3.33 (s, 4H), 3.14 (t, *J* = 8.7 Hz, 2H), 2.98 (s, 1H), 2.80 (s, 1H), 2.61-2.53 (m, 2H), 2.42 (d, *J* = 16.7 Hz, 2H), 2.26 (d, *J* = 21.4 Hz, 1H), 2.03-1.93 (m, 2H), 1.86 (q, *J* = 8.5, 7.3 Hz, 3H). MS m/z (ESI): 404.6 [M+H]⁺.

### Example 121: 8-(3-(2,3-Dihydrobenzofuran-7-yl)propyl)-7,8,9,10-tetrahydro-1H-pyrido[3',4':4,5]pyrrolo[1,2,3-de]quinoxalin-2(3H)-one

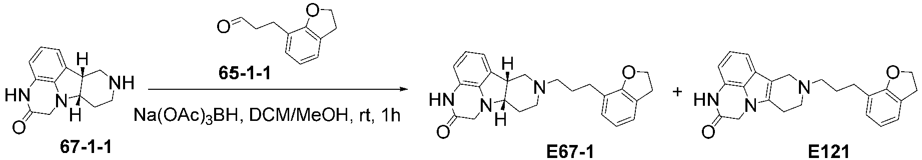

E121 was prepared with reference to the synthesis method in step 2 of Example E67-1. ¹H NMR (500 MHz, CDCl₃) *δ* 7.80 (s, 1H), 7.07 (t, *J* = 7.8 Hz, 2H), 6.99-6.90 (m, 2H), 6.79 (t, *J* = 7.4 Hz, 1H), 6.49 (d, *J* = 7.3 Hz, 1H), 4.83 (s, 2H), 4.56 (t, *J* = 8.7 Hz, 2H), 3.72 (s, 2H), 3.22 (t, *J* = 8.7 Hz, 2H), 2.94-2.88 (m, 2H), 2.84-2.80 (m, 2H), 2.74-2.63 (m, 4H), 2.01-1.94 (m, 2H). MS m/z (ESI): 388.7 [M+H]⁺.

### Example 122: (6bR,10aS)-8-(2-(Benzofuran-7-yl)ethyl)-6b,7,8,9,10,10a-hexahydro-1H-pyrido[3',4':4,5]pyrrolo[1,2,3-de]quinoxalin-2(3H)-one

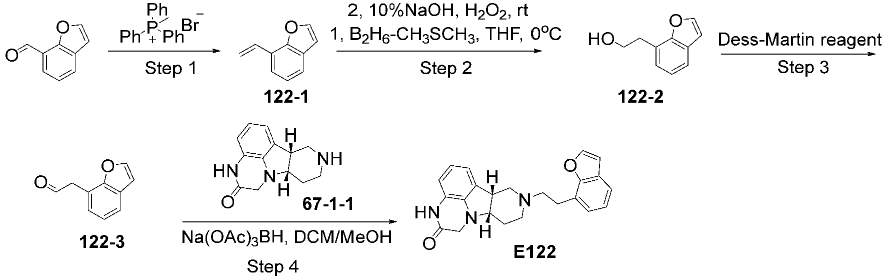

Step 1: The compounds benzofuran-7-carbaldehyde (1.0 g, 6.85 mmol), methyltriphenylphosphonium bromide (3.0 g, 8.22 mmol), and anhydrous THF (20 mL) were added together to a 100 mL three-neck flask, and anhydrous *t*-BuOK (1.15 g, 10.28 mmol) was added portionwise under N₂ and ice-water bath conditions. After the addition, the mixture was stirred overnight at room temperature. After TLC analysis showed that the reaction had been complete, a saturated aqueous NH₄Cl solution (50 mL) was added to the reaction solution, and extraction was performed with ethyl acetate (50 mL × 2). The organic phases were combined, washed with saturated brine (50 mL × 1), dried over anhydrous sodium sulfate, and concentrated to dryness by rotary evaporation under reduced pressure, and the crude product was separated and purified by column chromatography (EtOAc/PE = 0-5%) to give 122-1 (0.70 g, yield: 71%) as a pale yellow oil.

Step 2: Compound 122-1 (700 mg, 4.86 mmol) and anhydrous THF (15 mL) were added together to a 100 mL three-neck flask, and a 2 M B₂H₆/dimethyl sulfide solution (4 mL) was slowly added dropwise under N₂ and ice-water bath conditions. After the dropwise addition, the mixture was stirred at room temperature for 5 h. Subsequently, a 10% aqueous NaOH solution (5 mL) and a 30% H₂O₂ solution (0.5 mL) were separately slowly added dropwise under ice-water bath conditions. After the dropwise addition, the mixture was stirred overnight at room temperature. After TLC analysis showed that the reaction had been complete, water (30 mL) was added to the reaction solution, and extraction was performed with ethyl acetate (30 mL × 2). The organic phases were combined, washed with saturated brine (30 mL × 1), dried over anhydrous sodium sulfate, and concentrated to dryness by rotary evaporation under reduced pressure, and the crude product was separated and purified by column chromatography (EtOAc/PE = 0-10%) to give compound 122-2 (pale yellow oil, 400 mg, yield: 50%). ¹H NMR (500 MHz, CDCl₃) *δ* 7.62 (d, *J* = 2.2 Hz, 1H), 7.48 (dd, *J* = 7.5, 1.3 Hz, 1H), 7.17 (dt, *J* = 14.2, 6.9 Hz, 2H), 6.77 (d, *J* = 2.2 Hz, 1H), 3.99 (t, *J* = 6.5 Hz, 2H), 3.18 (t, *J* = 6.5 Hz, 2H).

Step 3: At room temperature, 122-2 (100 mg, 0.62 mmol) and anhydrous DCM (10 mL) were added together to a 50 mL single-neck flask, and Dess-Martin reagent (0.32 g, 0.74 mmol) was subsequently added. After the addition, the mixture was stirred at room temperature for 1 h. After TLC analysis showed that the reaction had been complete, the reaction solution was filtered, a saturated NaHCO₃ solution was slowly added dropwise to the filtrate to make the system alkaline, and extraction was performed with DCM (20 mL × 2). The organic phases were combined, washed with saturated brine (20 mL × 1), dried over anhydrous sodium sulfate, and concentrated to dryness by rotary evaporation under reduced pressure to give compound 112-3 (crude, 90 mg, yield: 90%). The crude product was directly used in the next step without further purification.

Step 4: Compound E122 was prepared with reference to the synthesis method in step 5 of Example E60. ¹H NMR (500 MHz, CDCl₃) 6 7.68 (s, 1H), 7.60 (d, *J* = 2.2 Hz, 1H), 7.48-7.44 (m, 1H), 7.16 (t, *J* = 7.5 Hz, 1H), 7.12 (d, *J* = 6.7 Hz, 1H), 6.88 (d, *J* = 7.4 Hz, 1H), 6.78-6.73 (m, 2H), 6.61 (d, *J* = 7.7 Hz, 1H), 3.97 (d, *J* = 14.5 Hz, 1H), 3.60-3.46 (m, 1H), 3.45-3.37 (m, 2H), 3.24-3.12 (s, 3H), 3.06-2.94 (m, 1H), 2.91-2.76 (m, 2H), 2.58-2.42 (m, 1H), 2.20-2.06 (m, 2H), 2.04-2.00 (m, 1H). MS m/z (ESI): 374.6 [M+H]⁺.

### Example 123: (6bR,10aS)-8-(2-(Benzofuran-7-yl)ethyl)-3-methyl-2,3,6b,7,8,9,10,10a-octahydro-1H-pyrido[3',4':4,5]pyrrolo[1,2,3-de]quinoxaline

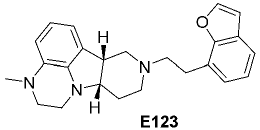

E123 was prepared with reference to the synthesis method of Example E122. ¹H NMR (500 MHz, CDCl3) δ 7.60 (d, *J* = 2.2 Hz, 1H), 7.45 (dd, *J* = 7.6, 1.2 Hz, 1H), 7.16 (t, *J* = 7.5 Hz, 1H), 7.11 (d, *J* = 6.6 Hz, 1H), 6.75 (d, *J* = 2.2 Hz, 1H), 6.68 (t, *J* = 7.6 Hz, 1H), 6.56 (d, *J* = 6.8 Hz, 1H), 6.43 (d, *J* = 7.6 Hz, 1H), 3.64-3.59 (m, 1H), 3.35-3.25 (m, 4H), 3.20-3.06 (m, 3H), 2.90-2.82 (m, 5H), 2.81-2.71 (m, 2H), 2.51-2.39 (m, 1H), 2.18-2.10 (m, 1H), 2.07-1.97 (m, 2H). MS m/z (ESI): 374.6 [M+H]⁺.

### Example 124: (6bR,10aS)-8-(3-(Benzofuran-7-yl)propyl)-6b,7,8,9,10,10a-hexahydro-1H-pyrido[3',4':4,5]pyrrolo[1,2,3-de]quinoxalin-2(3H)-one

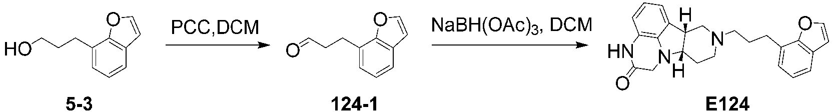

Step 1: 124-1 was prepared with reference to the synthesis method in step 4 of Example E8.

Step 2: E124 was prepared with reference to the synthesis method in step 5 of Example E8. ¹H NMR (500 MHz, CDCl₃) δ 7.61 (d, *J* = 2.1 Hz, 1H), 7.52 (s, 1H), 7.44 (dd, *J* = 7.8, 1.3 Hz, 1H), 7.15 (t, *J* = 7.5 Hz, 1H), 7.09 (dd, *J* = 7.3, 1.4 Hz, 1H), 6.83 (dd, *J* = 7.5, 0.9 Hz, 1H), 6.76 (d, *J* = 2.2 Hz, 1H), 6.72 (t, *J* = 7.6 Hz, 1H), 6.57 (dd, *J* = 7.8, 0.9 Hz, 1H), 3.95 (d, *J* = 14.6 Hz, 1H), 3.45-3.27 (m, 3H), 2.93 (t, *J* = 7.7 Hz, 3H), 2.79 (d, *J* = 7.7 Hz, 1H), 2.45 (s, 2H), 2.25 (d, *J* = 15.2 Hz, 1H), 2.03-1.83 (m, 5H). MS m/z (ESI): 388.7 [M+H]⁺.

### Example 125: (6bR,10aS)-8-(3-(2,3-Dihydrobenzofuran-7-yl)propyl)-6b,7,8,9,10,10a-hexahydro-1H-pyrido[3',4':4,5]pyrrolo[1,2,3-de]quinoxalin-2(3H)-thione

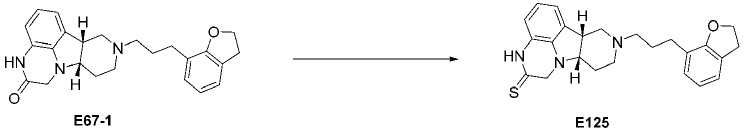

Compound E67-1 (40 mg, 0.103 mmol) was dissolved in THF (3 mL), and Lawesson's reagent (62 mg, 0.15 mmol) was added. The reaction solution was stirred at 70 °C for 1 h and concentrated *in vacuo.* Then, ethyl acetate (25 mL) and water (25 mL) were added, and layers formed. The organic phase was washed with water and concentrated to give a crude product. The crude product was concentrated, filtered, and then purified and separated by prep-HPLC (eluent (v/v): acetonitrile/(water + 0.05% NH4HCO3) = 30%-70%) to give compound E125 (7.8 mg, white solid, yield: 18%). 1H NMR (500 MHz, CD3OD) δ 6.91 (d, J = 5.0 Hz, 1H), 6.81-6.78 (m, 2H), 6.65-6.60 (m, 3H), 4.40 (t, J = 10.0 Hz, 2H), 4.23 (t, J = 15.0 Hz, 1H), 3.46 (t, J = 15.0 Hz, 1H), 3.19 (s, 2H), 3.07 (t, J = 10.0 Hz, 2H), 2.88-2.84 (m, 1H), 2.72-2.70 (m, 1H), 2.45 (t, J = 10.0 Hz, 2H), 2.34-2.12 (m, 3H), 1.96-1.69 (m, 5H). MS m/z (ESI): 406.9 [M+H]+.

### Example 126: (6bR,10aS)-8-(3-(2,3-Dihydrobenzofuran-7-yl)propyl)-2,3,6b,7,8,9,10,10a-octahydro-1H-pyrido[3',4':4,5]pyrrolo[1,2,3-de]quinoxaline

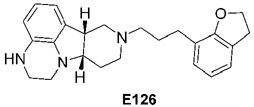

E126 was prepared with reference to the synthesis method of Example E45-1. ¹H NMR (500 MHz, CDCl₃) δ 7.03 (d, *J* = 7.0 Hz 1H), 6.93(d, *J* = 7.5Hz 1H), 6.76(t, *J* = 7.5Hz, 1H), 6.58(t, *J* = 7.5 Hz 1H), 6.54 (d, *J* = 7.0 Hz, 1H), 6.38(d, *J* = 7.5 Hz 1H), 4.53(t, *J* = 8.5 Hz, 2H), 3.71-3.66 (m, 1H), 3.49-3.45 (m, 1H), 3.31-3.28 (m, 1H), 3.24-3.18 (m, 4H), 2.94-2.91 (m, 1H), 2.74-2.72 (m, 1H), 2.68-2.63 (m, 1H), 2.59-2.56 (m, 2H), 2.47-2.29 (m, 3H), 2.05-1.83 (m, 6H). MS m/z (ESI): 376.6 [M+H]⁺.

### Example 127: 2-((6bR,10aS)-8-(3-(2,3-Dihydrobenzofuran-7-yl)propyl)-1,2,6b,7,8,9,10, 0a-octahydro-3H-pyrido[3',4':4,5]pyrrolo[1,2,3-de]quinoxalin-3-yl)ethan-1-ol

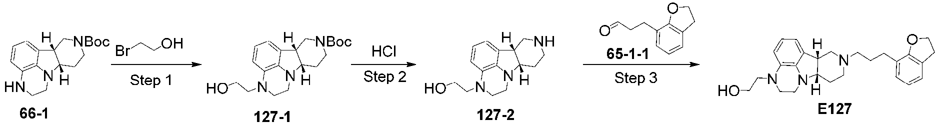

Step 1: Compound 66-1 (50 mg, 0.16 mmol) was dissolved in CH₃CN (5 mL), and bromoethanol (98 mg, 0.8 mmol) and cesium carbonate (204 mg, 0.63 mmol) were sequentially added. The mixture was heated to 80 °C and stirred for 16 h. The reaction solution was cooled to room temperature and filtered, and the crude product was purified and separated using a reversed-phase C18 column to give compound 127-1 (colorless oil, 18 mg, yield: 31.6%). MS m/z (ESI): 360.1 [M+H]⁺.

Step 2: Compound 127-1 (25 mg, 0.07 mmol) was dissolved in methanol (3 mL), and HCl/dioxane (3 mL, 12 mmol) was then added. The mixture was stirred at room temperature for 2 h. The reaction solution was concentrated to dryness to give compound 127-2 (brown solid, 20 mg, hydrochloride). MS m/z (ESI): 259.8 [M+H]⁺.

Step 3: E127 was prepared with reference to the synthesis method in step 2 of Example E67-1. ¹H NMR (500 MHz, CD₃OD) δ 7.06-6.96 (m, 1H), 6.89 (d, *J* = 7.5 Hz, 1H), 6.73 (t, *J* = 7.4 Hz, 1H), 6.58 (t, *J* = 7.7 Hz, 1H), 6.41 (dd, *J* = 16.5, 7.6 Hz, 2H), 4.50 (t, *J* = 8.7 Hz, 2H), 3.85-3.55 (m, 3H), 3.49-3.35 (m, 2H), 3.34-3.31 (m, 2H), 3.17 (t, *J* = 8.7 Hz, 2H), 3.08 (dd, *J* = 11.0, 4.8 Hz, 2H), 2.95-2.83 (m, 1H), 2.77 (d, *J* = 11.5 Hz, 1H), 2.66-2.57 (m, 1H), 2.55 (t, *J* = 7.5 Hz, 2H), 2.46-2.29 (m, 3H), 2.06-1.89 (m, 3H), 1.85 (dt, *J* = 14.8, 7.3 Hz, 2H). MS m/z (ESI): 420.6 [M+H]⁺.

### Example 128: (6bR,10aS)-8-(3-(2,3-Dihydrobenzofuran-7-yl)propyl)-3-methyl-2,3,6b,7,8,9,10,10a-octahydro-1H-pyrido[3',4':4,5]pyrrolo[1,2,3-de]quinoxaline-5-carbonitrile

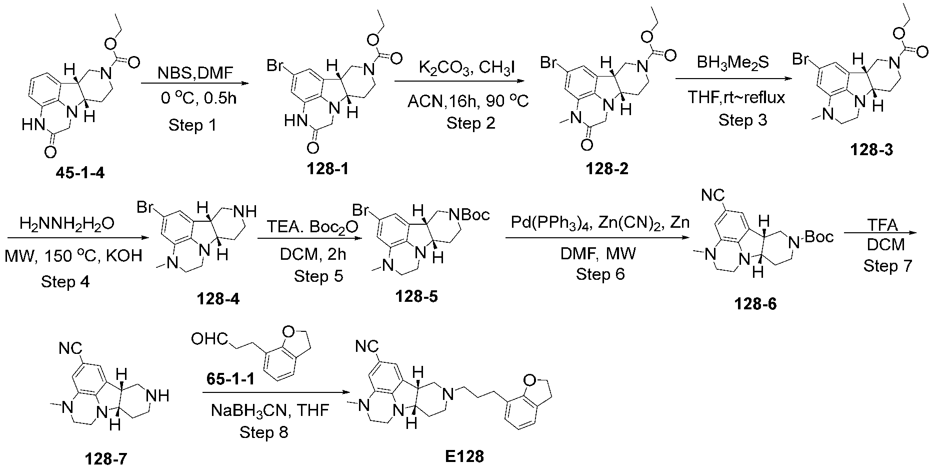

Step 1: 45-1-4 (600 mg, 2.0 mmol) and 6 mL of DMF were added to a 25 mL single-neck flask, and the mixture was purged three times with N₂ and cooled to 0 °C. NBS (356 mg, 2.0 mmol) was added. The mixture was left to react at 0 °C for 0.5 h, and LC-MS showed that the reaction had been complete. A saturated NaHCO₃ solution and EA were added, and the aqueous phase was isolated and extracted three times with EA. The organic phases were combined, washed three times with saturated sodium chloride, dried, and concentrated to give 128-1 (750 mg, crude). MS m/z (ESI): 380.2, 382.2 [M+H]⁺.

Step 2: 128-1 (750 mg, 2.0 mmol), CH₃I (5.1 g, 36.0 mmol), K₂CO₃ (552 mg, 4.0 mmol), and acetonitrile (20 mL) were added to a 100 mL single-neck flask, and the mixture was purged three times with N₂, heated to 80 °C, and left to react for 16 h. LC-MS showed that the reaction had been complete. The solvent was removed by concentration, water and EA were added, and the aqueous phase was isolated and extracted once with EA. The organic phases were combined, washed three times with saturated sodium chloride, dried, and concentrated to give 128-2 (750 mg, crude). MS m/z (ESI): 394.3, 396.4 [M+H]⁺.

Step 3: 128-3 was prepared with reference to the synthesis method in step 4 of E45-1. MS m/z (ESI): 380.2, 382.2 [M+H]⁺.

Step 4: 128-3 (200 mg, 0.52 mmol), hydrazine hydrate (60 mg, 1.04 mmol), KOH (112 mg, 2.08 mmol), and ethylene glycol (8 mL) were added to a 25 mL microwave tube, and the mixture was heated to 150 °C and left to react for 1.5 h. Water and EA were added, and the aqueous phase was isolated and extracted three times with EA. The organic phases were combined, washed three times with saturated sodium chloride, dried, and concentrated to give 128-4 (140 mg, crude). MS m/z (ESI): 308.2, 310.2 [M+H]⁺.

Step 5: 128-5 was prepared with reference to the synthesis method in step 2 of E45-2. MS m/z (ESI): 408.2, 410.2 [M+H]⁺.

Step 6: Compound 128-5 (18.0 mg, 0.04 mmol) was dissolved in DMF (2.0 mL), and tetrakis(triphenylphosphine)palladium(0) (1.20 g, 3.45 mmol), zinc cyanide (5.18 mg, 0.044 mmol), and zinc powder (0.860 mg, 0.013 mmol) were added in a nitrogen atmosphere. The mixture was stirred at 150 °C for 3 h under microwave conditions. After TLC analysis showed that the reaction had been complete, the reaction mixture was concentrated under reduced pressure, and the residue was purified by normal-phase chromatography to give compound 128-6 (yellow solid, 150 mg, yield: 74%). MS m/z (ESI): 355.2 [M+H]⁺.

Step 7: 128-6 (15.0 mg, 0.04 mmol) was dissolved in dichloromethane (3.0 mL), and trifluoroacetic acid (1.0 mL) was added. The mixture was left to react at room temperature for 1 h. The reaction mixture was filtered and concentrated to give compound 128-7 (brown oil, 12.0 mg, yield: 85%). MS m/z (ESI): 255.2 [M+H]⁺.

Step 8: In a nitrogen atmosphere, compound 128-7 (15.0 mg, 0.05 mmol), 2-(2,3-dihydro-1-benzofuran-6-yl)acetaldehyde (9.57 mg, 0.05 mmol), and acetic acid (0.052 mL, 0.93 mmol) were dissolved in THF (2 mL), and sodium cyanoborohydride (12.3 mg, 0.15 mmol) was added. The mixture was left to react at 0 °C for 2 h. After TLC analysis showed that the reaction had been complete, the reaction mixture was filtered and purified by reversed-phase preparative chromatography (eluent (v/v): acetonitrile/(water + 0.05% NH₄HCO₃) = 30%-70%) to give E128 (white solid, 1.0 mg, yield: 4%). ¹H NMR (400 MHz, CDCl₃) δ 7.04 (d, *J* = 7.2 Hz, 1H), 6.91-6.88 (m, 1H), 6.79-6.74 (m, 2H), 6.56 (s, 1H), 4.55-4.50 (t, *J* = 8.8 Hz, 2H), 3.61-3.56 (m, 1H), 3.43-3.17 (m, 4H), 3.19 (t, *J* = 8.8 Hz, 2H), 3.02-2.91 (m, 2H), 2.87 (s, 3H), 2.75-2.60 (m, 3H), 2.05-1.76 (m, 4H), 1.32-1.25 (m, 4H). MS m/z (ESI): 415.4 [M+H]⁺.

### Example 129: 8-(3-(2,3-Dihydrobenzofuran-7-yl)propyl)-5-methoxy-3-methyl-2,3,6b,7,8,9,10,10a-octahydro-1H-pyrido[3',4':4,5]pyrrolo[1,2,3-de]quinoxaline

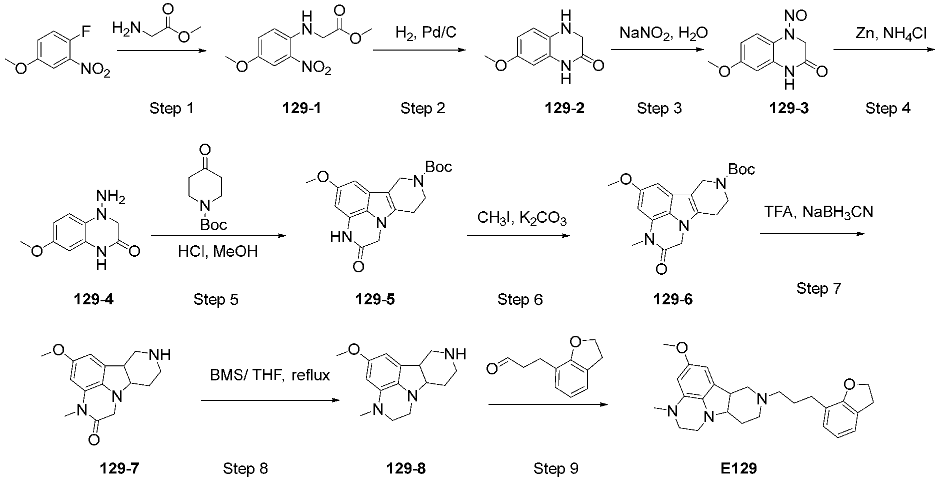

Step 1: Glycine methyl ester hydrochloride (7.2 g, 57.9 mmol) and K₂CO₃ (4.72 g, 34.2 mmol) were dissolved in 90 mL of NMP, and 1-fluoro-4-methoxy-2-nitrobenzene (9.0 g, 52.6 mmol) was then added. The mixture was heated to 80 °C and left to react for 16 h. TLC analysis showed that the reaction had been complete. Water and EtOAc were added, and layers formed. The aqueous phase was extracted with EtOAc. The organic phases were combined, washed three times with a saturated sodium chloride solution, dried, and concentrated, and the residue was purified by normal-phase silica gel column chromatography (PE/EtOAc = 5/1) to give compound 129-1 (2.2 g, yellow solid, yield: 17%). MS m/z (ESI): 240.4 [M+H]⁺.

Step 2: 129-1 (2.2 g, 9.17 mmol) was dissolved in 30 mL of methanol, and 1 g of 10% Pd/C was added. The mixture was stirred at room temperature for 16 h, filtered, and concentrated to give 129-2 (1.6 g, off-white solid, yield: 98%), which was directly used in the next step. MS m/z (ESI): 179.2 [M+H]⁺.

Step 3: 129-3 was prepared with reference to the synthesis method in step 1 of E42. MS m/z (ESI): 208.3 [M+H]⁺.

Step 4: Compound 129-3 (320 mg, 1.54 mmol) was dissolved in 10 mL of methanol, ammonium chloride (447 mg, 7.7 mmol) was added at room temperature, and zinc powder (492 mg, 7.7 mmol) was then added. The mixture was stirred at room temperature for 3 h, filtered, and concentrated to give 129-4 (220 mg, off-white solid, yield: 74%), which was directly used in the next step. MS m/z (ESI): 194.4 [M+H]⁺.

Step 5: 129-5 was prepared with reference to the synthesis method in step 3 of E42. MS m/z (ESI): 357.4 [M+H]⁺.

Step 6: 129-6 was prepared with reference to the synthesis method in step 1 of E73-1. MS m/z (ESI): 371.5 [M+H]⁺.

Step 7: 129-7 was prepared with reference to the synthesis method in step 4 of E42. MS m/z (ESI): 273.3 [M+H]⁺.

Step 8: 129-8 was prepared with reference to the synthesis method in step 4 of E45. MS m/z (ESI): 259.4 [M+H]⁺.

Step 9: E129 was prepared with reference to the synthesis method in step 5 of E42. ¹H NMR (500 MHz, CD₃OD) δ 7.03 (d, *J* = 5.0 Hz, 1H), 6.90 (d, *J* = 5.0 Hz, 1H), 6.74 (t, *J* = 7.5 Hz, 1H), 6.08-6.02 (m, 2H), 4.51 (t, *J* = 10.0 Hz, 2H), 3.76-3.68 (m, 4H), 3.22-3.19 (m, 1H), 3.16 (s, 3H), 3.07-2.91 (m, 6H), 2.66-2.56 (m, 6H), 2.19-1.92 (m, 6H). MS m/z (ESI): 419.9 [M+H]⁺.

### Example 130: 8-(3-(2,3-Dihydrobenzofuran-7-yl)propyl)-1,2,6b,7,8,9,10,10a-octahydropyrido[4,3-b][1,4]thiazino[2,3,4-hi]indole 3,3-dioxide

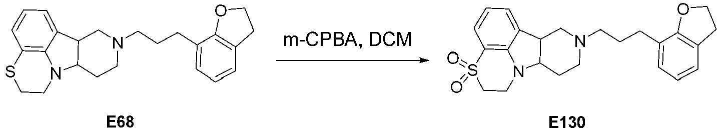

Step 1: Compound E68 (12 mg, 0.031 mmol) was dissolved in DCM (3 mL), and the solution was cooled to 0 °C. m-CPBA (12 mg, 0.067 mmol) was added, and the reaction solution was stirred at room temperature for 2 h and concentrated *in vacuo* to give a crude product. The crude product was concentrated, filtered, and then purified and separated by prep-HPLC (eluent (v/v): acetonitrile/(water + 0.05% NH₄HCO₃) = 30%-70%) to give compound E130 (5.0 mg, white solid, yield: 38%). ¹H NMR (500 MHz, CD₃OD) δ 7.03 (d, *J* = 5.0 Hz, 1H), 6.90 (d, *J* = 5.0 Hz, 1H), 6.73 (t, *J* = 7.5 Hz, 1H), 6.66 (d, *J* = 5.0 Hz, 1H), 6.59 (d, *J* = 7.5 Hz, 1H), 6.51 (d, *J* = 10.0 Hz, 1H), 4.50 (t, *J* = 10.0 Hz, 2H), 4.43-4.32 (m, 2H), 3.36-3.32 (m, 2H), 3.19-3.10 (m, 4H), 2.93-2.80 (m, 1H), 2.63-2.54 (m, 2H), 2.56 (t, *J* = 10.0 Hz, 2H), 2.42-2.26 (m, 3H), 2.19 (t, *J* = 10.0 Hz, 1H), 2.05-1.81 (m, 3H). MS m/z (ESI): 424.8 [M+H]⁺.

### Example 131: 1-((6bR,10aS)-8-((2-(2,3-Dihydrobenzofuran-7-yl)cyclopropyl)methyl)-1,2,6b,7,8,9,10,10a-octahydro-3H-pyrido[3',4':4,5]pyrrolo[1,2,3-de]quinoxalin-3-yl)ethan-1-one

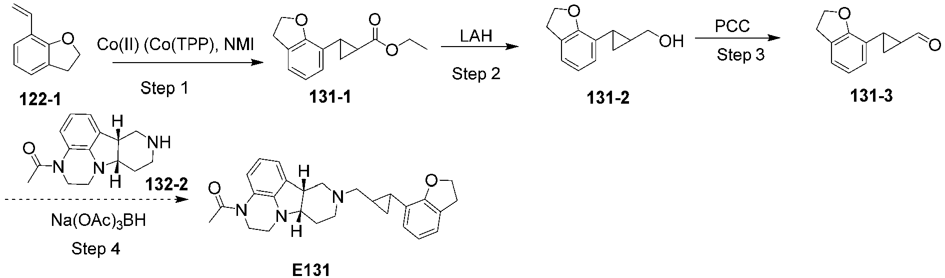

Step 1: Compound 122-2 (1.6 g, 12.5 mmol) was dissolved in toluene (20 mL), and ethyl diazoacetate (2.2 g, 18.75 mmol), NMI (3.08 g, 37.5 mmol), and Co(II) (Co(TPP)) (84 mg, 0.125 mmol) were added in a nitrogen atmosphere. The mixture was stirred at 80 °C for 48 h. After TLC analysis showed that the reaction had been complete, the reaction mixture was concentrated under reduced pressure, and the residue was purified by normal-phase chromatography to give compound 131-1 (purple solid, 1 g, yield: 34.5%). MS m/z (ESI): 233.1 [M+H]⁺.

Step 2: 131-2 was prepared with reference to the synthesis method in step 3 of E60.

Step 3: 131-3 was prepared with reference to the synthesis method in step 4 of E60.

Step 4: E131 was prepared with reference to the synthesis method in step 45 of E60. ¹H NMR (500 MHz, CDCl₃) δ 7.01 (d, *J* = 7.2 Hz, 1H), 6.88 (d, *J* = 7.3 Hz, 2H), 6.76 (t, *J* = 7.5 Hz, 1H), 6.69 (d, *J* = 7.5 Hz, 2H), 4.52 (t, *J* = 8.4 Hz, 2H), 3.92 (d, *J* = 85.9 Hz, 3H), 3.61-3.24 (m, 3H), 3.20 (dd, *J* = 15.7, 8.3 Hz, 3H), 2.78 (d, *J* = 165.2 Hz, 3H), 2.39 (d, *J* = 50.9 Hz, 5H), 1.99 (d, *J* = 18.0 Hz, 2H), 1.78 (s, 1H), 1.33 (s, 1H), 1.05 (s, 1H), 0.79 (s, 1H). MS m/z (ESI): 430.8 [M+H]⁺.

### Example 132: 1-((6bR,10aS)-8-(3-(2,3-Dihydrobenzofuran-7-yl)-2-hydroxypropyl)-1,2,6b,7,8,9,10,10a-octahydro-3H-pyrido[3',4':4,5]pyrrolo[1,2,3-de]quinoxalin-3-yl)ethan-1-one

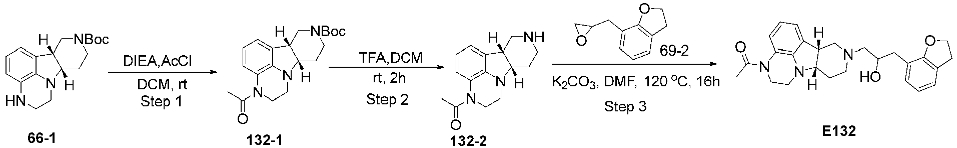

Step 1: 132-1 was prepared with reference to the synthesis method in step 3 of E45-2. MS m/z (ESI): 358.2 [M+H]⁺.

Step 2: 132-2 was prepared with reference to the synthesis method in step 4 of E45-2. MS m/z (ESI): 258.2 [M+H]⁺.

Step 3: 132-2 (50 mg, 0.19 mmol), 69-2 (69 mg, 0.4 mmol), K₂CO₃ (52 mg, 0.4 mmol), and 2 mL of DMF were added to a 25 mL single-neck flask, and the mixture was purged three times with N₂, heated to 120 °C, and left to react for 16 h. Water and EA were added, and the aqueous phase was isolated and extracted three times with EA. The organic phases were combined, washed three times with saturated sodium chloride, dried, and concentrated, and the residue was purified by column chromatography (DCM/MeOH = 20/1) to give E132 (30 mg, crude). 15 mg of the crude product was purified and separated by prep-HPLC (eluent (v/v): acetonitrile/(water + 0.05% NH4HCO3) = 30%-70%) to give E132 (7.9 mg, white solid, yield: 19.3%). ¹H NMR (500 MHz, CDCl₃)δ 7.06 (t, *J* = 6.5 Hz, 1H), 6.99 (t, *J* = 7.3 Hz, 1H), 6.95-6.81 (m, 2H), 6.81-6.74 (m, 1H), 6.67 (s, 1H), 4.53 (t, *J* = 8.7 Hz, 2H), 4.15 0-3.76 (m, 3H), 3.66-3.09 (m, 6H), 3.06 -2.52 (m, 6H), 2.43-2.27 (m, 6H), 2.08-1.86 (m, 2H). MS m/z (ESI): 434.6 [M+H]⁺.

### Example 133: (6bR,10aS)-8-((2,3-Dihydrobenzofuran-7-yl)methyl)-6b,7,8,9,10,10a-hexahydro-1H-pyrido[3',4':4,5]pyrrolo[1,2,3-de]quinoxalin-2(3H)-one

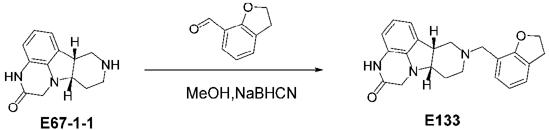

E67-1-1 (50 mg, 0.16 mmol) was dissolved in MeOH (2.00 mL), and sodium cyanoborohydride (30 mg, 0.484 mmol) was added. After 20 min of stirring at room temperature, 2,3-dihydrobenzofuran-7-carbaldehyde (47 mg, 0.322 mmol) was added, and the mixture was then left to react at room temperature for 2 h. LCMS analysis showed that the reaction had been complete. The reaction mixture was purified by HPLC (NH3.H2O system) to give compound E133 (white solid, 22 mg, yield: 37.7%). 1H NMR (400 MHz, CDCl₃) δ 7.41 (s, 1H), 7.11 (d, J = 7.4 Hz, 2H), 6.80-6.78 (m, 2H), 6.70 (t, J = 7.6 Hz, 1H), 6.55 (d, J = 7.2 Hz, 1H), 4.54 (t, J = 8.8 Hz, 2H), 3.95 (d, J = 14.6 Hz, 1H), 3.49 (d, J = 2.0 Hz, 2H), 3.41-3.29 (m, 4H), 3.19 (d, J = 8.6 Hz, 2H), 2.96-2.90 (m, 1H), 2.79-2.72 (m, 1H), 2.29-2.22 (m, 1H), 1.94-1.86 (m, 2H). MS m/z (ESI): 362.4 [M+H]+.

### Example 134: (6bR,10aS)-8-(2-(2,3-Dihydrobenzofuran-7-yl)ethyl)-6b,7,8,9,10,10a-hexahydro-1H-pyrido[3',4':4,5]pyrrolo[1,2,3-de]quinoxalin-2(3H)-one

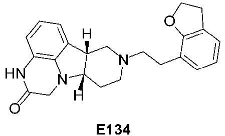

E134 was prepared with reference to the synthesis method in step 2 of Example E67-1. 1H NMR (400 MHz, CDCl3) δ 7.39 (s, 1H), 7.04 (d, J = 7.2 Hz, 1H), 6.94 (d, J = 7.3 Hz, 1H), 6.85 (d, J = 7.3 Hz, 1H), 6.79-6.70 (m, 2H), 6.57 (d, J = 7.8 Hz, 1H), 4.52 (t, J = 8.7 Hz, 2H), 3.97 (d, J = 14.6 Hz, 1H), 3.38 (dd, J = 15.2, 8.0 Hz, 3H), 3.19 (t, J = 8.7 Hz, 2H), 3.04-2.98 (m, 1H), 2.79 (dd, J = 21.4, 12.6 Hz, 3H), 2.59 (dd, J = 16.4, 8.0 Hz, 2H), 2.37-2.21 (m, 1H), 1.98 (dd, J = 22.3, 8.5 Hz, 3H). MS m/z (ESI): 376.4 [M+H]+.

### Example 135: (6bR,10aS)-8-(4-(2,3-Dihydrobenzofuran-7-yl)butyl)-6b,7,8,9,10,10a-hexahydro-1H-pyrido[3',4':4,5]pyrrolo[1,2,3-de]quinoxalin-2(3H)-one

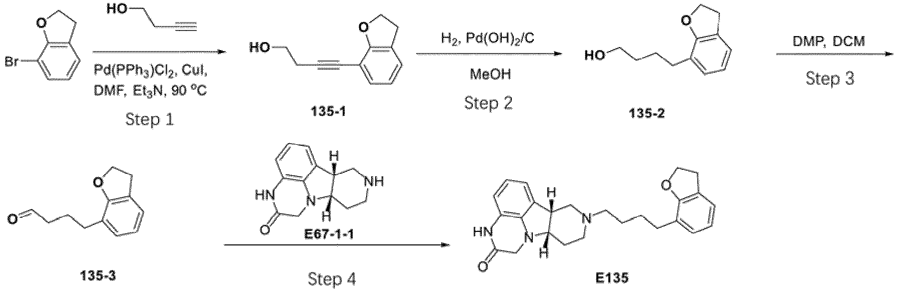

Step 1: 7-Bromobenzofuran (600 mg, 3 mmol) and 3-butyn-1-ol (420 mg, 6 mmol) were dissolved in DMF (5 mL), and Et3N (5 mL), CuI (114 mg, 0.6 mmol), and Pd(PPh3)2Cl2 (210 mg, 0.3 mmol) were sequentially added with stirring. The mixture was stirred at 90 °C for 16 h in a nitrogen atmosphere, and the reaction was complete. The reaction solution was poured into iced water (30 mL), and the aqueous phase was extracted with ethyl acetate (30 mL × 2). The organic layer was washed with brine (30 mL × 2), dried over anhydrous sodium sulfate, then filtered, and concentrated, and the residue was purified by column chromatography (ethyl acetate/petroleum ether: 0%-50%) to give compound 135-1 (white solid, 520 mg, yield: 92%). MS m/z (ESI): 189.2 [M+H]+.

Step 2: Compound 135-1 (360 mg, 2 mmol) was dissolved in MeOH (10 mL), and wet 20% Pd(OH)2/C (50 mg) was added with stirring in a nitrogen atmosphere. The mixture was purged three times with hydrogen and stirred at 25 °C for 16 h in a hydrogen atmosphere, and the reaction was complete. The reaction system was filtered and concentrated to give product 135-2 (colorless oil, 190 mg, yield: 50%). MS m/z (ESI): 193.3 [M+H]+.

Step 3: 135-2 was dissolved in DCM (5.0 mL), and Dess-Martin periodinane (390 mg, 0.91 mmol) was added. The mixture was left to react at 25 °C for 1 h, and LCMS analysis showed that the reaction had been complete. Water was added, and extraction was performed with EA. The extract was dried over sodium sulfate, filtered, and concentrated, and the residue was purified by silica gel column chromatography (EA/PE = 5%) to give compound 135-3 (white solid, 35 mg, yield: 35.4%). MS m/z (ESI): 162.2 [M+H]+.

Step 4: E135 was prepared with reference to the synthesis method in step 2 of Example E67-1. ¹H NMR (400 MHz, CDCl3) δ 7.40 (s, 1H), 7.03 (d, J = 7.2 Hz, 1H), 6.92 (d, J = 7.6 Hz, 1H), 6.83 (d, J = 7.6 Hz, 1H), 6.74-6.68 (m, 2H), 6.56 (d, J = 7.6 Hz, 1H), 4.53 (t, J = 8.8 Hz, 2H), 3.95 (d, J = 14.6 Hz, 1H), 3.41-3.30 (m, 3H), 3.20 (t, J = 8.6 Hz, 2H), 2.92-2.67 (m, 2H), 2.57 (t, J = 7.2 Hz, 2H), 2.39-2.15 (m, 3H), 1.92-1.88 (m, 4H), 1.66-1.58 (m, 3H). MS m/z (ESI): 404.5 [M+H]+.

### Example 136: (6bR,10aS)-8-(3-(3-Hydroxy-2,3-dihydrobenzofuran-7-yl)propyl)-6b,7,8,9,10,10a-hexahydro-1H-pyrido[3',4':4,5]pyrrolo[1,2,3-de]quinoxalin-2(3H)-one

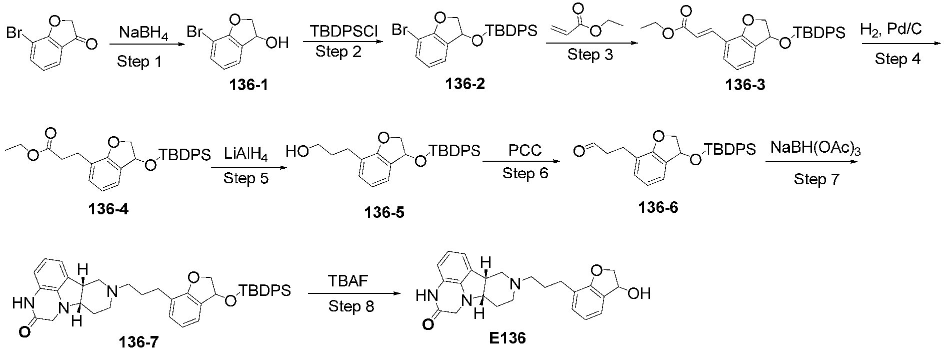

Step 1: 7-Bromo-3-benzofuranone (1.28 g, 6.0 mmol) was dissolved in methanol (10 mL), and the solution was cooled to 0 °C. Sodium borohydride (295 mg, 7.8 mmol) was added, and the reaction solution was stirred at room temperature for 2 h. The solvent was removed by concentration under reduced pressure. Ethyl acetate (30 mL) and water (30 mL) were added, and layers formed. Then, the organic phase was washed with saturated brine (30 mL), dried over anhydrous sodium sulfate, and concentrated to give compound 137-1 (crude, 980 mg, yield: 76.1%) as a colorless oil, which was directly used in the next step. MS m/z (ESI): 216.2 [M+H]⁺.

Step 2: At room temperature, compound 136-1 (980 mg, 4.56 mmol) was dissolved in DCM (10 mL), imidazole (930 mg, 13.7 mmol) was added, and TBDPSCl (1.88 g, 6.84 mmol) was then added. The reaction solution was stirred at room temperature for 16 h. After LCMS monitoring showed that the reaction had been complete, water (25 mL) was added to quench the reaction, and extraction was performed with DCM (25 mL). The organic phase was washed with water (25 mL), dried over anhydrous sodium sulfate, and concentrated, and the resulting crude product was purified and separated by normal-phase column chromatography (EtOAc/PE = 0-10%) to give compound 136-2 (1.36 g, yield: 66%) as a colorless oil. MS m/z (ESI): 481.4 [M+H]⁺.

Step 3: Compound 136-2 (1.36 g, 3.0 mmol) was dissolved in DMF (15 mL), triethylamine (3.5 mL) was added, and then, ethyl acrylate (600 mg, 6.0 mmol) and Pd(dtbpf)Cl₂ (19 mg, 0.3 mmol) were sequentially added. The reaction was heated to 98 °C and stirred for 18 h in a nitrogen atmosphere. The solvent was removed by rotary evaporation, and ethyl acetate (50 mL) was added. The mixture was filtered, and the filtrate was washed with water (50 mL), dried over anhydrous sodium sulfate, and concentrated. The resulting crude product was purified and separated by normal-phase column chromatography (EtOAc/PE = 0-10%) to give compound 136-3 (325 mg, yield: 23%) as a colorless oil. MS m/z (ESI): 473.6 [M+H]⁺.

Step 4: 136-4 was prepared with reference to the synthesis method in step 2 of Example 3.

Step 5: 136-5 was prepared with reference to the synthesis method in step 3 of Example 3.

Step 6: 136-6 was prepared with reference to the synthesis method in step 4 of Example 8.

Step 7: 136-7 was prepared with reference to the synthesis method in step 5 of Example 8.

Step 8: Compound 136-7 (33 mg, 0.1 mmol) was dissolved in THF (2 mL), and TBAF (0.15 mL, 0.15 mmol) was added. The reaction solution was stirred at room temperature for 2 h. The solvent was removed by concentration under reduced pressure. Ethyl acetate (10 mL) and water (10 mL) were added, and layers formed. Then, the organic phase was washed with saturated brine (10 mL), concentrated, filtered, and then purified and separated by prep-HPLC (eluent (v/v): acetonitrile/(water + 0.05% NH₄HCO₃) = 30%-70%) to give compound E136 (6.6 mg, white solid, yield: 16%). ¹H NMR (500 MHz, CD₃OD) δ 7.23 (d, *J* = 5.0 Hz, 1H), 7.05 (d, *J* = 5.0 Hz, 1H), 6.85-6.81 (m, 2H), 6.70 (t, *J* = 7.5 Hz, 1H), 6.63 (d, *J* = 10.0 Hz, 1H), 5.31-5.29 (m, 1H), 4.50-4.47 (m, 1H), 4.34-4.31 (m, 1H), 3.86 (d, *J* = 15.0 Hz, 1H), 3.33-3.26 (m, 3H), 2.93-2.90 (m, 1H), 2.78-2.76 (m, 1H), 2.59 (t, *J* = 7.5 Hz, 2H), 2.39-2.32 (m, 2H), 2.26-2.21 (m, 1H), 2.05-1.75 (m, 5H). MS m/z (ESI): 406.8 [M+H]⁺.

### Example 137: (6bR,10aS)-8-(3-(2,3-Dihydrobenzofuran-7-yl)propyl)-1-hydroxy-6b,7,8,9,10,10a-hexahydro-1H-pyrido[3',4':4,5]pyrrolo[1,2,3-de]quinoxalin-2(3H)-one

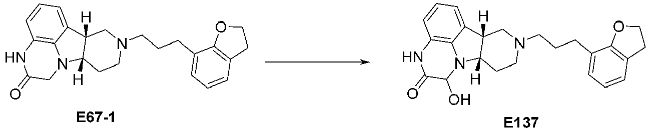

E67-1 (500 mg, 1.24 mmol) was dissolved in HCl (pH = 2, 10.0 mL), and the solution was left to react at 25 °C for 0.5 h (the reaction would gradually deteriorate over time in the reaction solution). After LCMS analysis showed that the reaction had been complete, the reaction mixture was filtered and purified by HPLC (HCOOH system) to give compound E137 (gray solid, 7.5 mg, yield: 2.36%). ¹H NMR (400 MHz, CDCl₃) δ 8.50 (s, 1H), 7.03 (d, *J* = 7.3 Hz, 1H), 6.87 (dd, *J* = 21.4, 9.8 Hz, 2H), 6.81-6.71 (m, 2H), 6.66 (t, *J* = 7.8 Hz, 1H), 4.58-4.47 (m, 2H), 3.60 (dd, *J* = 14.0, 7.7 Hz, 2H), 3.13 (dt, *J* = 87.9, 33.5 Hz, 4H), 2.76-2.57 (m, 3H), 2.14 (d, *J* = 103.4 Hz, 8H).

### Example 138: (6bR,10aS)-8-(3-(3-Hydroxy-2,3-dihydrobenzofuran-7-yl)propyl)-6b,7,8,9,10,10a-hexahydro-1H-pyrido[3',4':4,5]pyrrolo[1,2,3-de]quinoxaline-1,2(3H)-dione

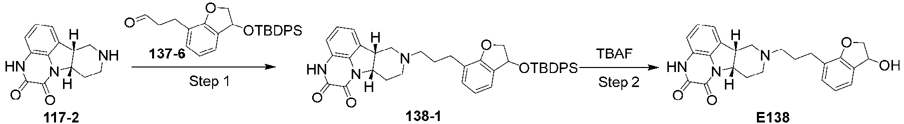

E138 was prepared with reference to the synthesis method of Example E137. ¹H NMR (500 MHz, CDCl₃) δ 11.95 (s, 1H), 7.21 (d, *J* = 40.4 Hz, 3H), 7.01 (d, *J* = 19.1 Hz, 2H), 6.86 (s, 1H), 5.63 (s, 1H), 5.31 (s, 1H), 4.81 (s, 1H), 4.57-4.50 (m, 1H), 4.28 (d, *J* = 10.1 Hz, 1H), 3.73 (s, 1H), 3.13 (s, 1H), 2.66 (d, *J* = 42.7 Hz, 2H), 2.43 (s, 2H), 2.28 (d, *J* = 65.2 Hz, 3H), 2.21 (s, 1H), 1.97 (s, 1H), 1.74 (s, 2H). MS m/z (ESI): 420.6 [M+H]⁺.

In the following effect examples, the structures of the control compounds used are as follows:
control compound 1: 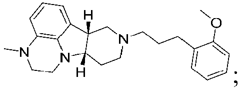
control compound 2: lumateperone 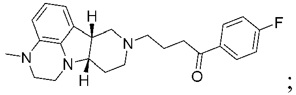
control compound 3:

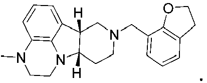

Control compound 1: prepared with reference to CN116444520A.

Control compound 2: The preparation method for 1-(4-fluorophenyl)-4-((6bR,10aS)-3-methyl-2,3,6b,9,10,10a-hexahydro-1H-pyrido[3',4':4,5]pyrrolo[1,2,3-de]quinoxalin-8(7H)-yl)butan-1-one was as follows:

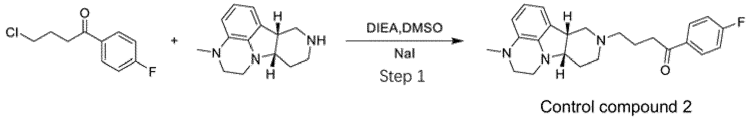

Step 1: (6bR,10aS)-3-Methyl-2,3,6b,7,8,9,10,10a-octahydro-1H-pyrido[3',4':4,5]pyrrolo[1,2,3-de]quinoxaline (2 g, 8.72 mmol), 4-chloro-1-(4-fluorophenyl)-1-butanone (2.6 g, 13.08 mmol), DIEA (3.4 g, 26.16 mmol), and sodium iodide (1.96 g, 13.08 mmol) were dissolved in DMSO (20 mL) at room temperature. The reaction solution was stirred at 60 °C for 16 h. The reaction solution was cooled to room temperature. After water (80 mL) was added, the aqueous phase was extracted with EA (80 mL × 2), and the organic phases were combined, washed with saturated sodium chloride (80 mL × 3), dried over anhydrous sodium sulfate, and concentrated *in vacuo* to give a crude product. The crude product was concentrated, filtered, and then purified and separated by prep-HPLC (eluent (v/v): acetonitrile/(water + 0.05% NH₄HCO₃) = 30%-70%) to give control compound 2 (1 g, yield: 29%, yellow oil). ¹H NMR (500 MHz, CDCl₃) δ 8.05-7.94 (m, 2H), 7.16-7.09 (m, 2H), 6.65 (t, *J* = 7.6 Hz, 1H), 6.51 (dd, *J* = 7.4, 0.9 Hz, 1H), 6.40 (dd, *J* = 8.0, 0.9 Hz, 1H), 3.63-3.54 (m, 1H), 3.35-3.24 (m, 2H), 3.23-3.17 (m, 1H), 3.10 (s, 1H), 2.99 (t, *J* = 7.1 Hz, 2H), 2.92-2.77 (m, 5H), 2.66 (s, 1H), 2.40 (s, 2H), 2.25 (s, 1H), 2.06-1.78 (m, 5H). MS m/z (ESI): 394.9 [M+H]⁺.

### Control compound 3: (6bR,10aS)-8-((2,3-Dihydrobenzofuran-7-yl)methyl)-3-methyl-2,3,6b,7,8,9,10,10a-octahydro-1H-pyrido[3',4':4,5]pyrrolo[1,2,3-de]quinoxaline

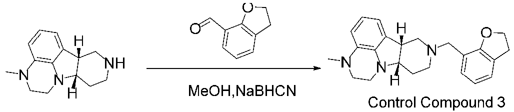

Control compound 3 was prepared with reference to the preparation method of Example E133. 1H NMR (400 MHz, CDCl3) δ 7.16-7.05 (m, 2H), 6.81 (t, J = 7.4 Hz, 1H), 6.69-6.59 (m, 1H), 6.49 (d, J = 6.8 Hz, 1H), 6.39 (d, J = 7.4 Hz, 1H), 4.54 (dd, J = 13.4, 4.9 Hz, 2H), 3.66-3.55 (m, 1H), 3.48 (d, J = 2.1 Hz, 2H), 3.34-3.14 (m, 6H), 2.90 (dd, J = 10.7, 5.3 Hz, 1H), 2.86 (s, 3H), 2.84-2.77 (m, 1H), 2.71 (d, J = 12.1 Hz, 1H), 2.30 (td, J = 10.8, 5.1 Hz, 1H), 2.08-1.98 (m, 1H), 1.97-1.87 (m, 2H). MS m/z (ESI): 362.3 [M+H]+.

### Effect Example 1: Affinity Assay of Compounds for 5-HT2A Receptor

Affinity was determined using a radioligand competition experiment. A plasmid vector containing the 5-HT2A receptor sequence was transfected into HEK293 cells (ATCC, CRL-1573). After screening, HEK293 cells stably expressing the 5-HT2A receptor were obtained. The cells were seeded into a 10 cm culture dish. After overnight culture, the culture medium was removed using a pipette, 3 mL of lysis buffer was added, and the mixture was left to stand at 4 °C for 10 min. After the cells fell off, the mixture was centrifuged at 4 °C at 1500 rpm for 5 min, and the supernatant was discarded. The cell pellet was transferred to a tissue homogenizer, and 3 mL of lysis buffer was then added thereto. The cells were well ground until they were broken. Then, the mixture was centrifuged at 4 °C at 12,000 rpm for 5 min, and the supernatant was discarded. The pellet, which was cell membrane components containing the 5-HT2A receptor, was resuspended in 10 mL of binding buffer. A 96-well plate was prepared. The test compounds, serially diluted, were added to the plate, and 100 µL of membrane stock solution and 100 µL of [3H]-ketanserin radioligand were added. The plate was incubated at a rotational speed of 300 rpm for 1 h. Meanwhile, a UniFilter^{™} plate (GF/B) was prepared. The UniFilter^{™} plate was soaked in 50 µL of 0.3% PEI for 0.5 h. Then, the aforementioned membrane and ligand incubation mixture was filtered through the UniFilter^{™} plate, and the plate was washed 4 times with washing solution. The filter plate was dried at 50 °C for 1 h. After drying, the bottom of the filter plate was sealed with a plate-sealing film, and 50 µL of liquid scintillation buffer was added. The amount of [3H]-ketanserin bound to the filter plate was read from the instrument and compared to that in the control wells to calculate the inhibition rate of binding. The data were analyzed using Prism software. Curves were fit to the data using a "log(inhibitor) vs. response-variable slope" model to obtain the IC₅₀ values, and after further processing, the affinity Ki values of the different compounds for the 5-HT2A receptor were obtained. The compounds of the present disclosure exhibited relatively good affinity for the 5-HT2A receptor. The data for some of the compounds are shown in Table 1.

**Table 1**

| Compound | [3H]Ketanserin Ki (nM) | Compound | [3H]Ketanserin Ki (nM) |
|---|---|---|---|
| Example 1 (E1) | 13.42 | Example 41 (E41) | 14.41 |
| Example 2 (E2) | 18.73 | Example 42 (E42) | 8.00 |
| Example 3 (E3) | 4.21 | Example 43 (E43) | 6.11 |
| Example 4 (E4) | 225.18 | Example 44 (E44) | 18.59 |
| Example 5 (E5) | 36.87 | Example 45-1 (E45-1) | 5.54 |
| Example 6 (E6) | 5.44 | Example 46 (E46) | 12.23 |
| Example 7 (E7) | 12.51 | Example 47 (E47) | 187.22 |
| Example 8 (E8) | 39.09 | Example 48 (E48) | 8.03 |
| Example 9 (E9) | 61.85 | Example 49 (E49) | 3.90 |
| Example 10 (E10) | 42.31 | Example 50 (E50) | 169.96 |
| Example 11 (E11) | 2.43 | Example 52 (E52) | 359.01 |
| Example 12 (E12) | 2.99 | Example 53 (E53) | 308.48 |
| Example 14 (E14) | 85.39 | Example 54 (E54) | 31.12 |
| Example 16 (E16) | 60.73 | Example 55 (E55) | 37.92 |
| Example 17 (E17) | 44.59 | Example 57 (E57) | 29.89 |
| Example 18 (E18) | 239.26 | Example 58 (E58) | 204.90 |
| Example 19 (E19) | 352.72 | Example 59 (E59) | 37.38 |
| Example 22 (E22) | 193.89 | Example 60 (E60) | 50.92 |
| Example 23 (E23) | 49.60 | Example 61 (E61) | 247.44 |
| Example 24 (E24) | 175.59 | Example 63 (E63) | 22.36 |
| Example 25 (E25) | 72.01 | Example 64 (E64) | 127.31 |
| Example 26 (E26) | 56.77 | Example 65-1 (E65-1) | 26.42 |
| Example 27 (E27) | 7.04 | Example 66 (E66) | 3.28 |
| Example 29 (E29) | 77.28 | Example 67-1 (E67-1) | 14.9 |
| Example 30 (E30) | 99.27 | Example 68 (E68) | 3.58 |
| Example 31 (E31) | 416.09 | Example 69 (E69) | 6.07 |
| Example 32 (E32) | 50.57 | Example 70 (E70) | 12.16 |
| Example 33 (E33) | 288.28 | Example 73-1 (E73-1) | 2.24 |
| Example 35 (E35) | 358.04 | Example 74 (E74-1) | 8.86 |
| Example 39 (E39) | 110.13 | Control compound 1 | 19.54 |
| Example 40 (E40) | 17.24 | | |

### Effect Example 2: Downstream Beta-Arrestin Functional Activity Assays for 5-HT2A Receptor

2.1. Method 1: An assay was mainly performed using a NanoBit kit from Promega. The method was specifically as follows: A 5HT2A receptor plasmid with LgBiT at the C-terminus and a beta-arrestin plasmid with SmBiT at the N-terminus were separately constructed. Before transfection, 4 × 10e6 HEK293 cells (ATCC, CRL-1573) were seeded into a 10 cm culture dish. After overnight culture, 10 µg of 5HT2AR-LgBiT, 10 µg of pBiT2.1-N-SmBiT-beta arrestin plasmid, and a transfection reagent were mixed and then added to the culture dish for transfection. After 24 h of culture, the cells were transferred to a 384-well plate at 20,000 cells per well, and a test compound or 5-HT, as a positive control, was added. Then, the plate was incubated overnight at 37 °C in a 5% CO₂ environment. The next day, the plate was taken out, 5 µL of diluted NanoBit detection solution was added, and the luminescence values were read from a microplate reader. The reading of the highest concentration of 5-HT was taken as 100% of the activation degree of the downstream pathway, and the data were analyzed using Prism software. Curves were fit to the data using a "log(agonist) vs. response -- variable slope (four parameters)" model to obtain the EC₅₀ and Eₘₐₓ values.
2.2. Method 2: An assay was mainly performed using a NanoBret kit from Promega. The method was specifically as follows: A 5HT2A receptor plasmid with Nanoluc at the C-terminus, a beta-arrestin plasmid with a Halo-tag at the C-terminus, and a pcDNA3.1 plasmid expressing GRK2 were separately constructed. Before transfection, HEK293 cells (ATCC, CRL-1573) were seeded into a 6-well plate. After overnight culture, the Nanoluc-5HT2AR, GRK2, and beta-arrestin-halotag plasmids were mixed in a ratio of 1:1:10 and then added to the 6-well plate together with a transfection reagent, with a transfection amount of 2 µg per well. After 4 h, the cells were digested and then transferred to a 384-well plate at 20,000 cells per well, and a test compound or 5-HT, as a positive control, was added. Then, the plate was incubated overnight at 37 °C in a 5% CO₂ environment. The next day, after the plate was taken out, a diluted NanoBRET^{™} Nano-Glo substrate was added, and the readings at 618 nm/460 nm were read from a microplate reader. The reading of the highest concentration of 5-HT was taken as 100% of the activation degree of the downstream pathway, and the data were analyzed using Prism software. Curves were fit to the data using a "log(agonist) vs. response -- variable slope (four parameters)" model to obtain the EC₅₀ and Eₘₐₓ values. The following data were obtained using method 1. The compounds of the present disclosure well activated the downstream signaling activity of the 5HT2A receptor. The data for some of the compounds are shown in Table 2:

**Table 2**

| Example No. | β-arrestin | |
|---|---|---|
| | EC₅₀ (nM) | Eₘₐₓ% |
| The following data were obtained from the assay using method 1 | | |
| Example 1 (E1) | 20.31 | 28.99 |
| Example 4 (E4) | 15.57 | 44.38 |
| Example 8 (E8) | 27.78 | 43.71 |
| Example 9 (E9) | 38.93 | 43.87 |
| Example 15 (E15) | 1273.00 | 20.54 |
| Example 16 (E16) | 64.53 | 16.34 |
| Example 17 (E17) | 31.12 | 17.35 |
| Example 18 (E18) | 443.70 | 64.34 |
| Example 21 (E21) | 281.70 | 86.37 |
| Example 22 (E22) | 79.38 | 118.20 |
| Example 23 (E23) | 38.35 | 93.15 |
| Example 24 (E24) | 170.00 | 49.46 |
| Example 26 (E26) | 83.33 | 23.62 |
| Example 30 (E30) | 437.80 | 54.17 |
| Example 33 (E33) | 144.30 | 35.01 |
| Example 38 (E38) | 717.30 | 46.87 |
| Example 40 (E40) | 19.93 | 32.86 |
| Example 41 (E41) | 47.25 | 13.87 |
| Example 42 (E42) | 101.00 | 25.65 |
| Example 43 (E43) | 30.89 | 39.41 |
| Example 45-1 (E45-1) | 83.64 | 28.85 |
| Example 46 (E46) | 29.34 | 14.38 |
| Example 54 (E54) | 32.21 | 49.91 |
| Example 57 (E57) | 35.89 | 23.62 |
| Example 58 (E58) | 29.14 | 37.21 |
| Example 60 (E60) | 186.30 | 40.02 |
| Example 62 (E62) | 854.00 | 36.02 |
| Control compound 1 | >100000 | -6.09 |

| The following data were obtained from the assay using method 2 | | |
|---|---|---|
| Example 65-1 (E65-1) | 333 | 74.33 |
| Example 65-2 (E65-2) | 1.6 | 44.3 |
| Example 66 (E66) | 95.4 | 56.2 |
| Example 67-1 (E67-1) | 163.1 | 30.2 |
| Example 67-2 (E67-2) | 60.52 | 41.87 |
| Example 67-3 (E67-3) | 155.4 | 32.67 |
| Example 68 (E68) | 138.8 | 130.9 |
| Example 69 (E69) | 235.5 | 59.0 |
| Example 70 (E70) | 233.7 | 122.1 |
| Example 71 (E71) | 2665.0 | 62.4 |
| Example 73-1 (E73-1) | 62.8 | 97.7 |
| Example 74 (E74) | 391.3 | 107.0 |
| Example 77 (E77) | 188.7 | 178.3 |
| Example 78 (E78) | 1020.0 | 80.4 |
| Example 79 (E79) | ~ 174.0 | 11.4 |
| Example 80 (E80) | 913.7 | 100.2 |
| Example 81 (E81) | 169.8 | 55.7 |
| Example 82 (E82) | ~ 643.3 | 36.7 |
| Example 84 (E84) | 170.5 | 43.44 |
| Example 85 (E85) | 875.5 | 75.5 |
| Example 86 (E86) | 97.6 | 53.0 |
| Example 87 (E87) | 524.1 | 59.1 |
| Example 88 (E88) | 658.7 | 77.9 |
| Example 89 (E89) | 96.3 | 34.4 |
| Example 91 (E91) | 493.5 | 88.5 |
| Example 94 (E94) | 362.5 | 70.8 |
| Example 95 (E95) | 57.3 | 83.6 |
| Example 96 (E96) | 32.8 | 12.8 |
| Example 97 (E97) | 654.7 | 81.2 |
| Example 98 (E98) | 753.1 | 54.3 |
| Example 101 (E101) | 157.5 | 90.1 |
| Example 102 (E102) | 2395.0 | 84.9 |
| Example 104 (E104) | 34.7 | 27.5 |
| Example 105 (E105) | 469.2 | 36.3 |
| Example 107 (E107) | 335.7 | 37.9 |
| Example 108 (E108) | 2594.0 | 34.0 |
| Example 109 (E109) | 585.1 | 45.4 |
| Example 112 (E112) | 328.7 | 44.9 |
| Example 116 (E116) | 46.1 | 68.1 |
| Example 118 (E118) | 372 | 46.5 |
| Example 122 (E122) | 3936 | 51.7 |
| Example 123 (E123) | 67.5 | 50.4 |
| Example 125 (E125) | 542.3 | 14.6 |
| Example 126 (E126) | 29.5 | 18.5 |
| Example 127 (E127) | 147.3 | 64.2 |
| Example 133 (E133) | 3144 | 79.7 |
| Example 134 (E134) | 784.3 | 11.8 |
| Example 135 (E135) | 3374 | 61.8 |
| Example 137 (E137) | 453.1 | 28.2 |
| Example 138 (E138) | 1024 | 31.8 |
| Control compound 3 | 1932 | 130.4 |

### Effect Example 3: Downstream Calcium Flux Functional Activity Assays for 5-HT2A Receptor

3.1. Method 1: A downstream calcium flux activity assay of the compounds for the 5-HT2A receptor was performed using HEK293 cells (ATCC, CRL-1573) stably expressing the 5-HT2A receptor. The 5-HT2A receptor was stably expressed in HEK293 cells by lentivirus infection to form HEK293-5HT2AR cells. The cells were seeded into 384-well plates at 25,000 cells/well and incubated overnight at 37 °C in a 5% CO₂ environment. The next day, the cell culture medium was removed using a pipette, a buffer containing FLIPR Calcium 6 dye was added, and the cells were incubated at room temperature for 30 min. Meanwhile, 384-well plates with added compounds were prepared. The plates were all placed into an MD FLIPR instrument, and the calcium flux signal values were read. When the data were analyzed, the reading of the highest concentration of 5-HT was taken as 100% of the activation degree of the downstream pathway, and the data were analyzed using Prism software. Curves were fit to the data using a "log(agonist) vs. response -- variable slope (four parameters)" model to obtain the EC₅₀ and Eₘₐₓ values.
3.2. Method 2: A downstream calcium flux activity assay of the compounds for the 5-HT2A receptor was performed using HEK293 cells (ATCC, CRL-1573) transiently transfected with the 5-HT2A receptor. The full-length gene of 5-HT2A was inserted into a pcDNA3.1 vector, and the vector was transfected into HEK293 cells using a lipofectamine 3000 reagent. After 4-6 h, the cells were seeded into a 384-well plate at 20,000 cells/well and incubated overnight at 37 °C in a 5% CO₂ environment. The next day, the cell culture medium was removed using a pipette, a buffer containing FLIPR Calcium 6 dye was added, and the cells were incubated at room temperature for 30 min. Meanwhile, 384-well plates of compounds were prepared. The plates were placed into an MD FLIPR instrument, and the calcium flux signal values were read. When the data were analyzed, the reading of the highest concentration of 5-HT was taken as 100% of the activation degree of the downstream pathway, and the data were analyzed using Prism software. Curves were fit to the data using a "log(agonist) vs. response -- variable slope (four parameters)" model to obtain the EC₅₀ and Eₘₐₓ values. The compounds of the present disclosure generally have no hallucinogenic effect while combating depression. The data for some of the compounds are shown in Table 3:

**Table 3**

| | Ca²⁺ flux Assay | | | Ca²⁺ flux Assay | |
|---|---|---|---|---|---|
| | EC₅₀ (nM) | Eₘₐₓ% | | EC₅₀ (nM) | Eₘₐₓ% |
| Example 1 (E1) | >100000 | 0.75 | Example 53 (E53) | ND | ND |
| Example 2 (E2) | >100000 | 0.88 | Example 54 (E54) | 6145.00 | 69.50 |
| Example 3 (E3) | >100000 | 0.40 | Example 55 (E55) | >100000 | 1.15 |
| Example 4 (E4) | >100000 | 25.06 | Example 56 (E56) | >100000 | 1.35 |
| Example 5 (E5) | >100000 | -0.22 | Example 57 (E57) | >100000 | 6.42 |
| Example 6 (E6) | >100000 | -0.05 | Example 58 (E58) | >100000 | 31.89 |
| Example 7 (E7) | >100000 | -0.04 | Example 59 (E59) | >100000 | 5.10 |
| Example 8 (E8) | >100000 | 33.85 | Example 60 (E60) | >100000 | 2.65 |
| Example 9 (E9) | >100000 | 7.11 | Example 61 (E61) | >100000 | 6.29 |
| Example 10 (E10) | >100000 | 5.58 | Example 62 (E62) | ND | ND |
| Example 11 (E11) | 46010.00 | 21.06 | Example 63 (E63) | 41800.00 | 43.89 |
| Example 12 (E12) | >100000 | 6.06 | Example 64 (E64) | >100000 | 1.44 |
| Example 13 (E13) | ND | ND | Example 65-1 (E65-1)^{a} | 91619 | 61.4 |
| Example 14 (E14) | >100000 | 3.72 | Example 66 (E66)^{a} | >100000 | 4.91 |
| Example 15 (E15) | >100000 | 1.24 | Example 67-1 (E67-1)^{a} | >100000 | 1.12 |
| Example 16 (E16) | >100000 | -0.07 | Example 67-3 (E67-3)^{a} | 35997 | 27.36 |
| Example 17 (E17) | >100000 | 0.61 | Example 68 (E68)^{a} | 4415 | 74.77 |
| Example 18 (E18) | >100000 | -0.44 | Example 69 (E69)^{a} | >100000 | 8.64 |
| Example 19 (E19) | ND | ND | Example 70 (E70)^{a} | 1233.00 | 21.25 |
| Example 20 (E20) | ND | ND | Example 72 (E72)^{a} | >100000 | 4.72 |
| Example 21 (E21) | ND | ND | Example 73-1 (E73-1)^{a} | 952.00 | 12.07 |
| Example 22 (E22) | ND | ND | Example 74 (E74)^{a} | 16881.00 | 21.00 |
| Example 23 (E23) | 563.30 | 20.41 | Example 75 (E75)^{a} | 658.90 | 12.89 |
| Example 24 (E24) | ND | ND | Example 76 (E76)^{a} | 427.7 | 17.38 |
| Example 25 (E25) | >100000 | 5.58 | Example 77 (E77)^{a} | 412.2 | 18.46 |
| Example 26 (E26) | >100000 | 1.31 | Example 79 (E79)^{a} | >100000 | 25.39 |
| Example 27 (E27) | >100000 | 5.13 | Example 81 (E81)^{a} | >100000 | 3.69 |
| Example 28 (E28) | ND | ND | Example 83 (E83)^{a} | 758.8 | 18.00 |
| Example 29 (E29) | ND | ND | Example 84 (E84)^{a} | 1113 | 26.72 |
| Example 30 (E30) | 5114.00 | 33.47 | Example 86 (E86)^{a} | >100000 | 12.92 |
| Example 31 (E31) | ND | ND | Example 87 (E87)^{a} | >100000 | 2.23 |
| Example 32 (E32) | >100000 | 7.01 | Example 89 (E89)^{a} | >100000 | 46.83 |
| Example 33 (E33) | >100000 | 2.19 | Example 91 (E91)^{a} | 2295 | 23.19 |
| Example 34 (E34) | ND | ND | Example 94 (E94)^{a} | 230.5 | 73.85 |
| Example 35 (E35) | ND | ND | Example 95 (E95)^{a} | 1182 | 34.79 |
| Example 36 (E36) | ND | ND | Example 96 (E96)^{a} | 993.7 | 14.8 |
| Example 37 (E37) | ND | ND | Example 101 (E101)^{a} | 132.5 | 14.23 |
| Example 38 (E38) | >100000 | 0.88 | Example 104 (E104)^{a} | >100000 | 0 |
| Example 39 (E39) | >100000 | 10.40 | Example 105 (E105)^{a} | 159.2 | 38.41 |
| Example 40 (E40) | >100000 | 6.79 | Example 107 (E107)^{a} | >100000 | 5.83 |
| Example 41 (E41) | >100000 | 4.07 | Example 112 (E112)^{a} | 22.27 | 38.55 |
| Example 42 (E42) | ND | ND | Example 114 (E114)^{a} | >100000 | 3.316 |
| Example 43 (E43) | >100000 | 0.81 | Example 116 (E116)^{a} | 61.04 | 34.01 |
| Example 44 (E44) | >100000 | 18.52 | Example 118 (E118)^{a} | >100000 | 9.139 |
| Example 45-1 (E45-1) | >100000 | 5.04 | Example 123 (E123)^{a} | 156.3 | 12.74 |
| Example 45-2 (E45-2)^{a} | 46299.00 | 10.22 | Example 126 (E126)^{a} | >100000 | 1.35 |
| Example 46 (E46)^{a} | >100000 | 18.2 | Example 127 (E127)^{a} | >100000 | 4.231 |
| Example 47 (E47)^{a} | >100000 | 1.06 | Example 128 (E128)^{a} | >100000 | 9.991 |
| Example 48 (E48) | >100000 | 9.23 | Example 129 (E129)^{a} | >100000 | 5.425 |
| Example 49 (E49) | 10094.00 | 14.52 | Example 130 (E130)^{a} | >100000 | 8.883 |
| Example 50 (E50) | >100000 | 9.60 | Example 131 (E131)^{a} | >100000 | 3.884 |
| Example 52 (E52) | >100000 | 6.52 | Example 132 (E132)^{a} | >100000 | 1.638 |

| | | | | | |
|---|---|---|---|---|---|
| ND means "not detected". a: The data were derived from method 2, and the rest were derived from method 1. | | | | | |

### Effect Example 4: Animal Depression-Like Behavior Test (FST)

The forced swimming test is commonly used to assess depression-like moods of mice. The animals in this experiment were C57BL/6J male mice aged 7-8 weeks, with 12 mice in each group. The mice were allowed to swim for 15 min in a 5 L glass beaker with a water surface height of 15 cm, 24 h before the test. On the day of the test, the mice were intraperitoneally injected with a blank vehicle (5% DMSO, 10% polyethylene glycol-15-hydroxystearate, and 85% normal saline), ketamine (20 mpk), or a test substance (15 mpk) for 30 min and then placed in the same beaker for 6 min. Software (Shanghai Jiliang, JLBehv-FSG-4) was used to analyze the last four-minute immobility time of the mice. The percentage of the immobility time of each treatment group to the immobility time of the solvent control group of the same batch was calculated and statistically analyzed. If the data followed a normal distribution, a parametric test, specifically an independent samples t-test, was used for statistical analysis. If the data did not follow a normal distribution, a non-parametric test was used for statistical analysis. The compounds of the present disclosure exhibited relatively good antidepressant effects. The data for some of the compounds are shown in Tables 4-1 and 4-2.

**Table 4-1**

| Group | Ratio to immobility time of solvent control group (%) | | | |
|---|---|---|---|---|
| | Median | Lower limit of 95% confidence interval | Upper limit of 95% confidence interval | P value (vs solvent control) |
| Solvent control^{b} | 103 | 98.98 | 107.7 | NA |
| Ketamine^{b} | 90.9 | 80.84 | 96.87 | <0.001 |
| Control compound 1 | 94.9 | 83.55 | 103.7 | 0.09 |
| Example 2 (E1) | 90.3 | 83.47 | 96.64 | 0.01 |
| Example 40 (E40) | 73.2 | 56.87 | 85.77 | <0.001 |
| Example 8 (E8) | 66.2 | 19.27 | 104.2 | 0.01 |
| Example 45-1 (E45-1) | 64.5 | 40.44 | 76.5 | <0.001 |
| Example 46 (E46) | 56.3 | 17.44 | 78.87 | <0.001 |

**Table 4-2**

| Group | Ratio to immobility time of solvent control group (%) | | | |
|---|---|---|---|---|
| | Median | Lower limit of 95% confidence interval | Upper limit of 95% confidence interval | P value (vs solvent control) |
| Solvent control^{b} | 102.6 | 95.6 | 105.8 | NA |
| Ketamine^{b} | 81.4 | 74.1 | 93.0 | <0.0001 |
| Example 65-1 (E65-1) | 76.4 | 61.8 | 95.2 | <0.05 |
| Example 67-1 (E67-1) | 23.6 | 3.1 | 63.7 | <0.0001 |
| Example 68 (E68) | 94.2 | 86.7 | 103.1 | <0.01 |
| Example 73-1 (E73-1) | 36.6 | 3.9 | 80.4 | <0.0001 |
| Example 86 (E86) | 53.7 | 5.9 | 70.4 | <0.0001 |

| | | | | |
|---|---|---|---|---|
| NA = Not Available b: The data were the averages of multiple experiments. | | | | |

The experimental results show that the example compounds of the present disclosure significantly reduced the immobility time (not shown) and the ratio to the immobility time of the solvent control, suggesting that these compounds can ameliorate depression-like moods of mice. Moreover, under the experimental conditions, the positive control compound ketamine significantly reduced the immobility time and the ratio to the immobility time of the solvent control, while control compound 1 exhibited no significant effect on the depression-like behavior of mice.

### Effect Example 5: Metabolic Property Test

5.1. Experimental animals: Nine C57 mice were divided into 3 groups, alternate blood collection was performed, and each mouse was administered a single intraperitoneal injection of a compound at 15 mg/kg at a volume of 10 mL/kg. Blood samples of the test substance were collected at 5 min, 15 min, 30 min, 1 h, 2 h, 4 h, 6 h, 8 h, and 24 h post-dose. Biological sample collection and storage: 0.08 mL of blood was collected and placed in an EDTA-2K anticoagulation tube containing a marker. Immediately after the tube was gently inverted so that the anticoagulant (EDTA-2K) was well mixed with the blood, the tube was placed in wet ice, and the plasma was isolated by centrifugation (4 °C, 6800 g, 6 min) within 30 min of collecting the blood. After centrifugation, the isolated plasma was placed in a labeled EP tube and stored in a freezer at no higher than -20 °C within 30 min, and the plasma was stored until the sample was analyzed. Sample analysis: An LC-MS/MS analysis method for determining the concentration of the drug substance in mouse plasma was established and used to determine the concentration in the biological samples obtained in this experiment. The pretreatment process of the biological samples should be carried out under yellow light and ice-water bath conditions. PK parameters were calculated using WinNonlin software. The compounds of the present disclosure exhibited relatively good metabolic properties. The data for some of the compounds are shown in Table 5-1:

**Table 5-1**

| PK parameter | Control compound 1 | Example 2 E2 | Example 40 E40 | Example 8 E8 | Example 45-1 E45-1 | Example 46 E46 |
|---|---|---|---|---|---|---|
| T_{1/2} (h) | 1.6 | 3.0 | 5.3 | 1.82 | 9.2 | 1.27 |
| Tₘₐₓ (h) | 0.25 | 0.033 | 0.25 | 0.083 | 0.083 | 0.083 |
| Cₘₐₓ (ng/mL) | 346 | 344 | 88 | 339 | 1047 | 334 |
| AUCₗₐₛₜ (h*ng/mL) | 1031 | 1422 | 422 | 653 | 1140 | 763 |
| AUC_{INF} | 1071 | 1427 | 646 | 676 | 1173 | 774 |
| (h*ng/mL) | | | | | | |
| Mouse PPB Fu (%) | 2.48 | 1.96 | 2.47 | 2.18 | 3.29 | 11.3 |

In comparison, at similar plasma exposure levels, compounds 45-1 and 46 achieved higher plasma free exposure levels due to their higher plasma free fractions (PPB, Fu%).

### 5.2. Experimental animals: C57/6J mice, SPF, male, 9 mice/group.

Experimental design: The animals in the intravenous injection group (i.v.) were not fasted before administration, and the animals in the oral gavage group (P.O.) were fasted. The fasted animals were fasted overnight (10-14 h) before the experiment, given free access to water, dosed on an empty stomach, and fed 4 h after administration. Administration doses: intravenous injection group: 2 mg/kg, administration concentration: 0.4 mg/mL, administration volume: 5 mL/kg; oral gavage group: 10 mg/kg, administration concentration: 1.0 mg/mL, administration volume: 10 mL/kg. Method of administration: The weight was measured before administration, and the amount of administration was calculated based on the body weight. Single administration; plasma samples were cross-collected. Administration vehicle: 5% DMSO + 10% solutol + 85% saline.

Blood collection time points for the intravenous injection group: 0.083 h, 0.25 h, 0.5 h, 1 h, 2 h, 4 h, 6 h, 8 h, and 24 h; blood collection time points for the oral gavage group: 0.25 h, 0.5 h, 1 h, 2 h, 4 h, 6 h, 8 h, and 24 h; blood collection was performed via a jugular vein or other suitable veins, about 0.08 mL was collected for each sample, K2-EDTA was used for anticoagulation, and the collected blood samples were placed on ice.

Treatment of plasma samples: The plasma was isolated by centrifugation (4 °C, 6800 g, 6 min) within 30 min of collecting the blood. After centrifugation, the isolated plasma was placed in a labeled EP tube and stored in a freezer at no higher than -20 °C within 30 min, and the plasma was stored until the sample was analyzed. An LC-MS/MS analysis method for determining the concentration of the drug substance in mouse plasma was established and used to determine the concentration in the biological samples obtained in this experiment. The pretreatment process of the biological samples should be carried out under yellow light and ice-water bath conditions.

Analysis of results: Pharmacokinetic parameters were calculated using Phoenix WinNonlin 7.0 based on the plasma concentration data of the different time points. The following parameters are provided: AUC₀₋ₜ, MRT₀₋ₜ, Cₘₐₓ, Tₘₐₓ, T_{1/2}, etc. The pharmacokinetic parameters in mice are shown in Table 5-2. The compounds of the present disclosure exhibited relatively good metabolic properties. The data for some of the compounds are shown in Table 5-2.

**Table 5-2**

| P.O (10 mg/kg) i.v (2 mg/kg) | | Example 45-1 E45-1 | | Example 65-1 E65-1 | | Example 86 E86 | | Control compound 2 | |
|---|---|---|---|---|---|---|---|---|---|
| Route of administration | | i.v | P.O | i.v | P.O | i.v | P.O | i.v | P.O |
| t_{1/2} | h | 0.37 | 7.93 | 1.9 | 2.51 | 0.67 | 1.8 | 1.98 | 2.4 |
| Tₘₐₓ | h | | 0.25 | | 0.25 | | 0.25 | | 0.25 |
| Cₘₐₓ | ng/mL | | 37.4 | | 49.6 | | 242 | | 12.8 |
| C1 | mL/min/kg | 143 | | 67.3 | | 62.7 | | 72 | |
| Vd | L/kg | 3.6 | | 6.3 | | 1.96 | | 11.2 | |
| AUC₀₋ₜ | h*ng/mL | 229 | 58.4 | 482 | 127 | 530 | 616 | 439 | 46 |
| AUC _{0-inf} | h*ng/mL | 233 | 85 | 495 | 146 | 532 | 648 | 463 | 54 |
| F | % | 7.3 | | 5.9 | | 24 | | 2.3 | |

The compounds of the present disclosure exhibited better *in vivo* bioavailability than control compound 2. F(%) = (AUC_{0-inf}-PO/AUC_{0-inf}-IV)/(DosePO/DoseIV) × 100

### Effect Example 6: Drug Interaction Test

Through incubation in human liver microsomes, the *in vitro* inhibitory effects of drugs on the CYP2D6 enzyme were studied, and their potential metabolic drug-drug interactions were assessed. After a test substance and a dextromethorphan hydrobromide (DM) probe substrate were co-incubated in a human liver microsomal (source: BIOIVT, Lot No. CDN) incubation system for a certain period of time, the percentage content of the remaining enzyme activity at each administration concentration was calculated. The liver microsomal incubation system medium was phosphate-buffered saline (PBS) containing the test substance or a positive inhibitor, the probe substrate, liver microsomes (final concentration: 0.1 mg/mL), and NADPH (1 mM). The amount of metabolite generated by the probe substrate was determined by LC-MS/MS. The amount of metabolite generated was used to reflect the enzyme activity. The remaining percentage (%) of CYP2D6 at different concentration levels of the test substance was calculated. A semi-log inhibition graph was plotted with the remaining percentage as the ordinate and the concentration of the test substance as the abscissa, and the IC₅₀ value was calculated using Prism software. At different concentration points of the test compound or the positive inhibitor, the remaining activity percentage was obtained from the ratio of the amount of the characteristic metabolite of the probe substrate generated to the amount of the metabolite generated in the absence of the test compound or the positive inhibitor. If a significant decrease in metabolite generation was observed at the highest concentration set point, the half maximal inhibitory concentration (IC50) would be calculated using the log(inhibitor) vs. responsevariable slope formula of GraphPad Prism software:
$Y = Bottom + \left(Top-Bottom\right) / \left(1+{10}^{∧}\left(\left(LogIC50-X\right)\times Hillslope\right)\right)$
X: Log(concentration of test compound or positive inhibitor);
Y: the remaining activity percentage;
Top and Bottom refer to the theoretical maximum and minimum remaining activity percentages, respectively;
Hillslope: the slope coefficient or slope.

If there was no significant decrease in metabolite generation at the highest concentration set point (enzyme remaining activity percentage > 50%), the half maximal inhibitory concentration (IC50) could not be accurately calculated, and the reported IC50 was greater than the highest concentration tested.

It is generally considered that a compound has relatively strong inhibitory activity when its IC₅₀ value for CYP enzyme inhibition < 1 µM, has moderate inhibitory activity when 1 µM < IC₅₀ < 10 µM, and has relatively weak inhibitory activity when IC₅₀ > 10 µM.

The compounds of the present disclosure exhibited relatively small drug interaction effects. The results for some of the compounds are shown in Table 6 below.

**Table 6**

| Test parameter | Control compound 1 | Example 2 (E2) | Example 40 (E40) | Example 8 (E8) | Example 45-1 (E45-1) | Example 46 (E46) |
|---|---|---|---|---|---|---|
| CYP2D6 inhibition IC50 (µM) | 0.346 | 0.604 | 1.77 | 0.0806 | > 20 | 1.30 |

The CYP2D6 gene encodes the CYP2D6 enzyme. The CYP2D6 enzyme is an important member of the cytochrome P450 family and a key enzyme in multiple types of drug metabolism. The smaller the inhibitory effect of a drug on the CYP2D6 enzyme, the smaller the effect on the metabolism of other drugs. Generally, a CYP2D6 inhibition IC50 of <1 indicates a relatively strong inhibitory effect. As can be seen from the data in the table, the inhibitory effects of the test compounds on the CYP2D6 enzyme were all smaller than that of the control compound. Particularly, compound 45-1 exhibited a relatively weak inhibitory effect on other drugs, resulting in a relatively small drug interaction effect.

### Effect Example 7: Head-Twitch Response (HTR) Experiment on Mice

The experiment used C57BL/6J mice aged 6-8 weeks, with 10 mice in each group. On D1, the animals in each group were first acclimatized in a test chamber for 20 min and then intraperitoneally injected with a blank vehicle (5% DMSO, 10% polyethylene glycol-15-hydroxystearate, and 85% normal saline), the positive compound DOI (1-(4-iodo-2,5-dimethoxyphenyl)-2-aminopropan) (1 mg/kg), or a test substance (50 mg/kg), videos were recorded, and the number of mouse head twitches within 70 min (0-70 min post-dose) was observed. On D2, 24 h ± 5 min after administration, videos were recorded, and the number of mouse head twitches within 30 min was observed.

Analysis of data: The number of head twitches was described using the mean ± standard error. For a quantitative indicator, when the data followed a normal distribution and had homogeneous variance, ONE-WAY ANOVA was used for a statistical test; when they did not have homogeneous variance, a Kruskal-Wallis H rank sum test (K-W method) was used for statistical analysis; all statistical analyses were completed using GraphPad Prism 9.0 software.

The compounds of the present disclosure exhibited no significant hallucinogenic effects. The results for some of the compounds are shown in Table 7 below.

**Table 7**

| | Number of mouse head twitches/times | | | | | | | |
|---|---|---|---|---|---|---|---|---|
| Group | Observed immediately after administration (for 70 min) | | | | Observed 24 h after administration (for 30 min) | | | |
| | Median | Lower limit of 95% confidence interval | Upper limit of 95% confidence interval | P value (vs blank vehicle) | Median | Lower limit of 95% confidence interval | Upper limit of 95% confidence interval | P value (vs blank vehicle) |
| Blank vehicle | 4.5 | 3 | 10 | NA | 2.5 | 1 | 5 | NA |
| DOI | 108.5 | 75 | 143 | <0.0001 | 5.5 | 3 | 8 | 0.0285 |
| Example 73-1 (E73-1) | 6.5 | 0 | 10 | >0.9999 | 1.5 | 0 | 3 | 0.4133 |
| Example 65-1 (E65-1) | 8.5 | 0 | 18 | 0.9889 | 2 | 2 | 5 | 0.9961 |
| Example 45-1 (E45-1) | 19.5 | 7 | 32 | 0.1074 | 2.5 | 1 | 4 | 0.9303 |

The results show that within 70 min of performing administration, the positive compound DOI produced a significant hallucinogenic effect, while the compounds of the present disclosure, consistent with the blank group, did not produce significant hallucinogenic effects, and that hallucinogenic effects were still not observed 24 h after administration of the compounds of the present disclosure.

## Claims

1. A tetracyclic compound represented by formula I or a pharmaceutically acceptable salt thereof: 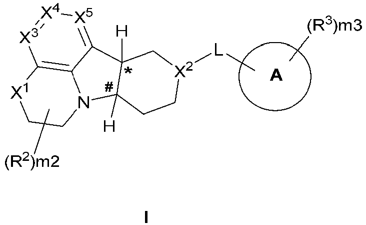
wherein the carbon atom with "*" represents a chiral carbon atom that has an S configuration, an R configuration, or a mixture thereof;
the carbon atom with "#" represents a chiral carbon atom that has an S configuration, an R configuration, or a mixture thereof;
X¹ is -NR^{X1}-, -O-, -CR^{X2}R^{X3}-, -S-, -S(O)-, or -S(O)₂-;
R^{X1} is H, C₁-C₆ alkyl, C₁-C₆ alkyl substituted with one or more R^{X1-1}, -C(O)-C₁-C₆ alkyl, or "3-12 membered heterocycloalkyl containing 1, 2, or 3 heteroatoms selected from 1, 2, or 3 of N, O, and S";
R^{X1-1} is independently OH;
R^{X2} and R^{X3} are independently H or C₁-C₆ alkyl;
X² is N or CR^{X4};
R^{X4} is H or C₁-C₆ alkyl;
X³ is N or CR¹⁻¹;
X⁴ is N or CR¹⁻²;
X⁵ is N or CR¹⁻³;
R¹⁻¹, R¹⁻², and R¹⁻³ are independently H, CN, halogen, C₁-C₆ alkyl, C₁-C₆ haloalkyl, C₁-C₆ alkoxy, or C₁-C₆ haloalkoxy;
L is -(CR^{L1}R^{L2})n1-, -(CR^{L1}R^{L2})n2-Y¹-(CR^{L1}R^{L2})n3-, or -(CR^{L1}R^{L2})n4-Y²-(CR^{L1}R^{L2})n5-,
R^{L1} and R^{L2} are independently H or C₁-C₆ alkyl;
n1 is 1, 2, 3, or 4;
Y¹ is -O-, -NR^{L3}- , -C(O)-, -CR^{L4}R^{L5}-, 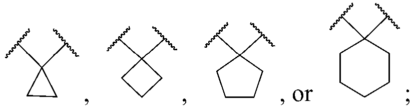
n2 is 0, 1, 2, or 3;
n3 is 0, 1, 2, or 3;
n2 + n3 = 1, 2, or 3;
Y² is -C≡C-, -C(O)-NR^{L3}-, -CR^{L6}=CR^{L7}-, 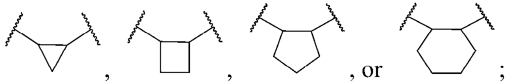
n4 is 0, 1, or 2;
n5 is 0, 1, or 2;
n4 + n5 = 1 or 2;
R^{L3} is independently H or C₁-C₆ alkyl;
R^{L4} is H or halogen;
R^{L5} is halogen, OH, or C₁-C₆ alkoxy;
R^{L6} and R^{L7} are independently H or halogen;
R² is independently C₁-C₆ alkyl, -C(O)-C₁-C₆ alkyl, or oxo (=O);
m2 is 0, 1, 2, 3, or 4;
ring A is"9-12 membered bicyclic heterocyclyl containing 1, 2, or 3 heteroatoms selected from 1, 2, or 3 of N, O, and S", "5-6 membered monocyclic heterocyclyl containing 1, 2, or 3 heteroatoms selected from 1, 2, or 3 of N, O, and S", C₆-C₁₀ aryl, or C₃-C₆ cycloalkyl;
and when L is -(CR^{L1}R^{L2})n1-, -(CR^{L1}R^{L2})n2-O-, or -(CR^{L1}R^{L2})n2-C(O)-, 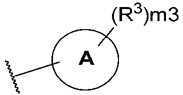 is 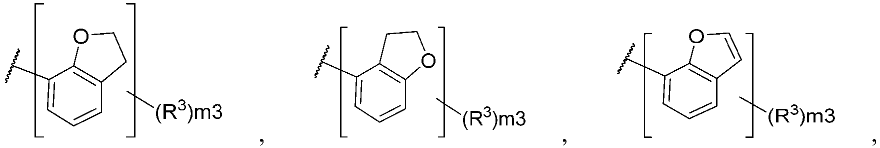 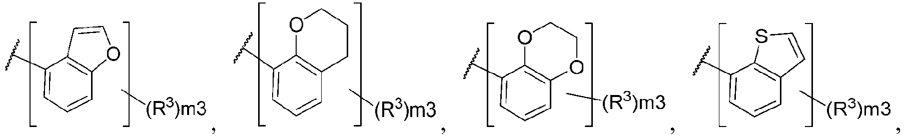 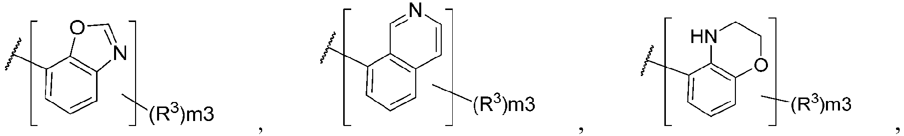 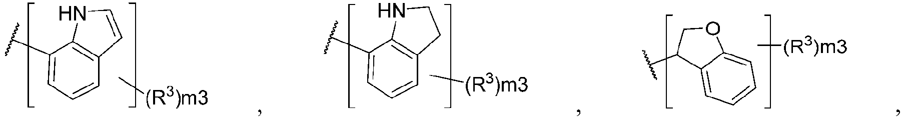 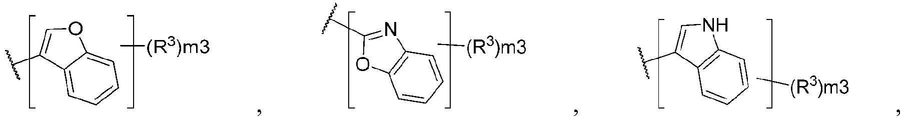 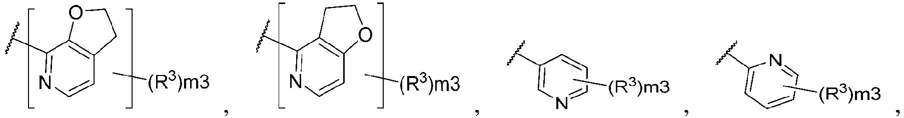 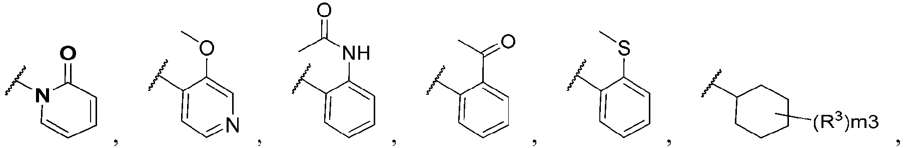 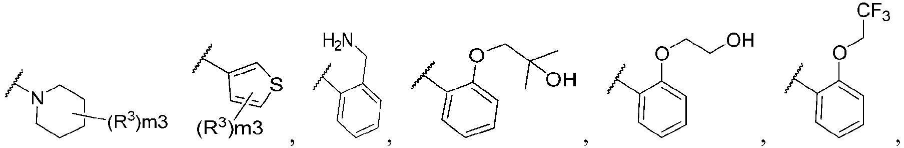 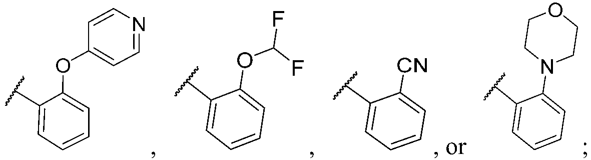
R³ is independently oxo (=O), C₁-C₆ alkyl, C₁-C₆ alkoxy, -NR⁴R⁵, -C(O)R⁶, -SR⁷, -S(O)₂R⁸, halogen, C₁-C₆ alkoxy substituted with one or more R⁹, C₁-C₆ alkyl substituted with one or more R¹⁰, CN, "3-12 membered heterocycloalkyl containing 1, 2, or 3 heteroatoms selected from 1, 2, or 3 of N, O, and S", or -OR¹¹;
R⁴ is independently H or C₁-C₆ alkyl;
R⁵ is independently H, C₁-C₆ alkyl, or -C(O)R⁵⁻¹.
R⁶, R⁵⁻¹, R⁷, and R⁸ are independently C₁-C₆ alkyl;
R⁹ and R¹⁰ are independently OH, -NR⁴R⁵, or halogen;
R¹¹ is H or "5-10 membered heteroaryl containing 1, 2, or 3 heteroatoms selected from 1, 2, or 3 of N, O, and S";
m3 is 0, 1, 2, 3, or 4.

2. The tetracyclic compound represented by formula I or the pharmaceutically acceptable salt thereof as defined in claim 1, meeting one or more of the following conditions:
(1) R^{X2} and R^{X3} are independently H;
(2) R^{X4} is H;
(3) X⁵ is CR¹⁻³;
(4) R¹⁻¹, R¹⁻², and R¹⁻³ are independently H, CN, or C₁-C₆ alkoxy;
(5) R^{L1} and R^{L2} are independently H;
(6) n1 is 1, 2, or 3;
(7) Y¹ is -O-, -NR^{L3}-, -C(O)-, -CR^{L4}R^{L5}-, or 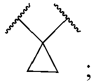
(8) n2 is 0, 1, or 2;
(9) n3 is 0, 1, or 2;
(10) n2 + n3 = 2;
(11) Y² is -C=C-, -C(O)-NR^{L3}-, -CR^{L6}=CR^{L7}-, or 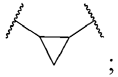
(12) n4 is 0 or 1;
(13) n5 is 0 or 1;
(14) n4 + n5 = 1;
(15) R^{L4} is H;
(16) R^{L5} is halogen;
(17) R^{L6} and R^{L7} are independently H;
(18) R² is independently oxo;
(19) m2 is 0 or 1;
(20) when L is -(CR^{L1}R^{L2})n1-, -(CR^{L1}R^{L2})n2-O-, or -(CR^{L1}R^{L2})n2-C(O)-, 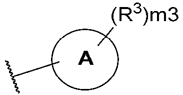 is 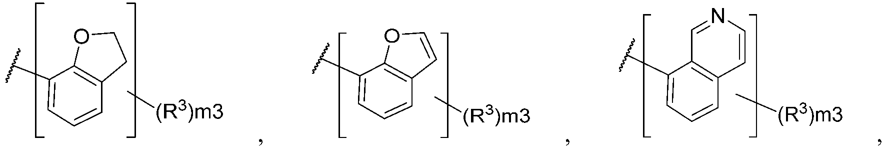 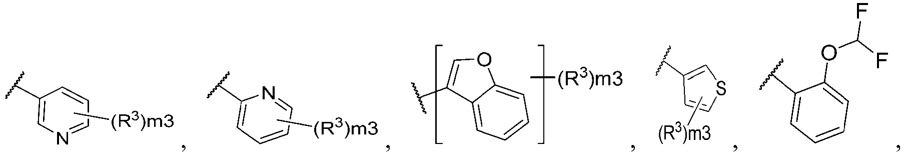 or 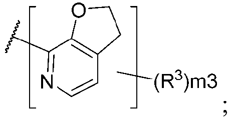
(21) R³ is independently oxo (=O), C₁-C₆ alkoxy, -NR⁴R⁵, -C(O)R⁶, -SR⁷, -S(O)₂R⁸, halogen, C₁-C₆ alkoxy substituted with one or more R⁹, C₁-C₆ alkyl substituted with one or more R¹⁰, CN, "3-12 membered heterocycloalkyl containing 1, 2, or 3 heteroatoms selected from 1, 2, or 3 of N, O, and S", or -OR¹¹;
(22) R⁴ is independently H;
(23) R³ is independently H or -C(O)R⁵⁻¹;
(24) R⁹ is independently halogen; and
(25) m3 is 0 or 1.

3. The tetracyclic compound represented by formula I or the pharmaceutically acceptable salt thereof as defined in claim 1, meeting one or more of the following conditions:
(1) R¹⁻¹ and R¹⁻³ are H, and R¹⁻² is H, CN, or C₁-C₆ alkoxy;
(2) n1 is 3;
(3) Y¹ is -C(O)- or -CR^{L4}R^{L5}-;
(4) n2 is 2;
(5) n3 is 0;
(6) Y² is -CR^{L6}=CR^{L7}-;
(7) n4 is 1;
(8) n5 is 0;
(9) m2 is 0;
(10) R³ is independently C₁-C₆ alkoxy, C₁-C₆ alkoxy substituted with one or more R⁹, or halogen;
(11) R⁵ is independently H; and
(12) m3 is 0.

4. The tetracyclic compound represented by formula I or the pharmaceutically acceptable salt thereof as defined in claim 1, meeting one or more of the following conditions:
(1) the "C₁-C₆ alkyl" in each "C₁-C₆ alkyl", "substituted C₁-C₆ alkyl", and "-C(O)-C₁-C₆ alkyl" is independently methyl, ethyl, n-propyl, isopropyl, n-butyl, isobutyl, sec-butyl, or tert-butyl;
(2) the "C₁-C₆ alkoxy" in each "C₁-C₆ alkoxy" and "substituted C₁-C₆ alkoxy" is independently methoxy, ethoxy, n-propoxy, isopropoxy, n-butoxy, isobutoxy, sec-butoxy, or tert-butoxy;
(3) each "3-12 membered heterocycloalkyl containing 1, 2, or 3 heteroatoms selected from 1, 2, or 3 of N, O, and S" is independently "5-6 membered heterocycloalkyl containing 1 or 2 heteroatoms selected from 1 or 2 of N and O";
(4) each halogen is independently F, Cl, Br, or I;
(5) in ring A, the "9-12 membered bicyclic heterocyclyl containing 1, 2, or 3 heteroatoms selected from 1, 2, or 3 of N, O, and S" is 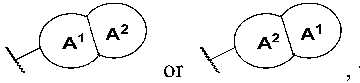 wherein ring A¹ is independently phenyl or pyridyl, and ring A² is independently "5-6 membered heterocycloalkyl containing 1 or 2 heteroatoms selected from 1 or 2 of N and O" or "5-6 membered heteroaryl containing 1 or 2 heteroatoms selected from 1 or 2 of N and O"; for example, ring A² is independently "5-membered heterocycloalkyl containing 1 or 2 heteroatoms selected from O" or "5-6 membered heteroaryl containing 1 or 2 heteroatoms selected from 1 or 2 of N and O";
(6) in ring A, the "5-6 membered monocyclic heterocyclyl containing 1, 2, or 3 heteroatoms selected from 1, 2, or 3 of N, O, and S" is "5-6 membered monocyclic heteroaryl containing 1 or 2 heteroatoms selected from 1 or 2 of N and S" or "5-6 membered monocyclic heterocycloalkyl containing 1 or 2 heteroatoms selected from N", e.g., pyridyl (e.g., 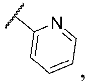 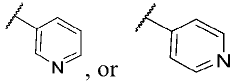 ), thienyl (e.g., 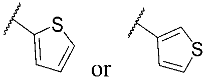 ), or piperidinyl (e.g., 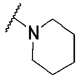 );
(7) in ring A, the C₆-C₁₀ aryl is phenyl or naphthyl;
(8) in ring A, the C₃-C₆ cycloalkyl is cyclopropyl, cyclobutyl, cyclopentyl, or cyclohexyl;
(9) each "5-10 membered heteroaryl containing 1, 2, or 3 heteroatoms selected from 1, 2, or 3 of N, O, and S" is independently "5-6 membered heteroaryl containing 1 or 2 heteroatoms selected from N"; and
(10) 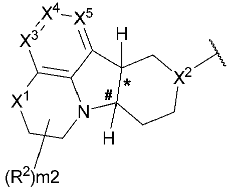 is

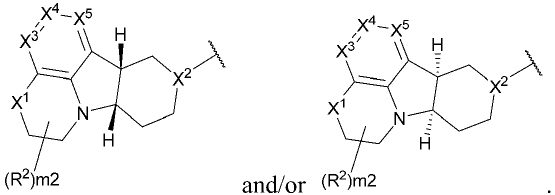

5. The tetracyclic compound represented by formula I or the pharmaceutically acceptable salt thereof as defined in claim 1, meeting one or more of the following conditions:
(1) the "C₁-C₆ alkyl" in each "C₁-C₆ alkyl", "substituted C₁-C₆ alkyl", and "-C(O)-C₁-C₆ alkyl" is independently methyl or ethyl;
(2) the "C₁-C₆ alkoxy" in each "C₁-C₆ alkoxy" and "substituted C₁-C₆ alkoxy" is independently methoxy or ethoxy;
(3) each "3-12 membered heterocycloalkyl containing 1, 2, or 3 heteroatoms selected from 1, 2, or 3 of N, O, and S" is independently piperidinyl (e.g., 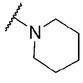 ), morpholinyl ( 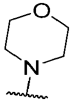 ), or oxetanyl (e.g., 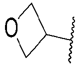 );
(4) each halogen is independently F or Cl;
(5) in ring A, the "9-12 membered bicyclic heterocyclyl containing 1, 2, or 3 heteroatoms selected from 1, 2, or 3 of N, O, and S" is 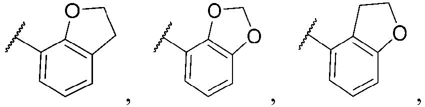 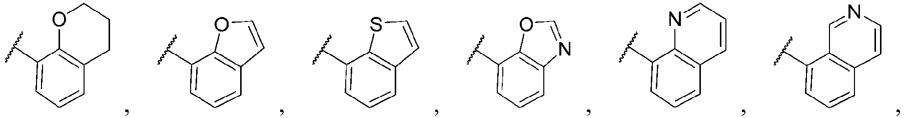 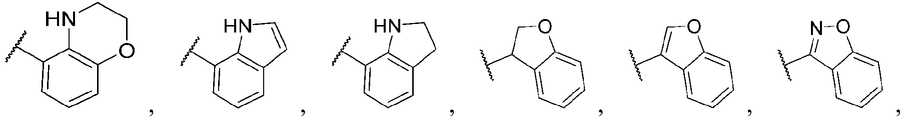 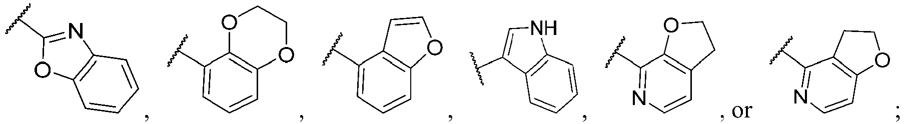
(6) in ring A, the "5-6 membered monocyclic heterocyclyl containing 1, 2, or 3 heteroatoms selected from 1, 2, or 3 of N, O, and S" is 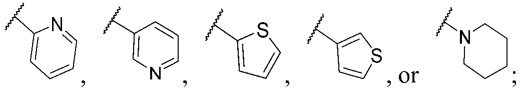
(7) in ring A, the C₆-C₁₀ aryl is phenyl;
(8) in ring A, the C₃-C₆ cycloalkyl is cyclohexyl;
(9) each "5-10 membered heteroaryl containing 1, 2, or 3 heteroatoms selected from 1, 2, or 3 of N, O, and S" is independently pyridyl (e.g., 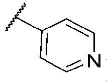 ); and
(10) 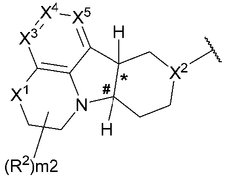 is

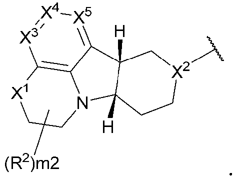

6. The tetracyclic compound represented by formula I or the pharmaceutically acceptable salt thereof as defined in claim 1, meeting one or more of the following conditions:
(1) L is 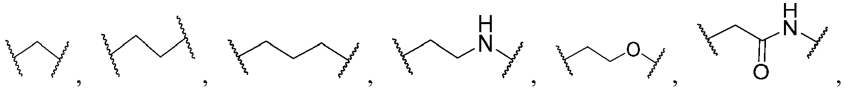 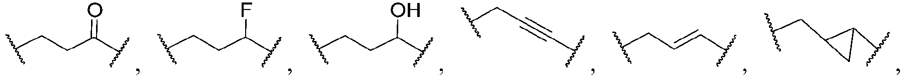 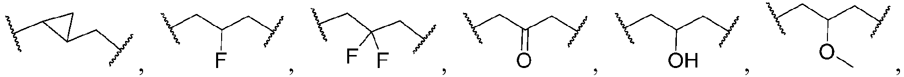 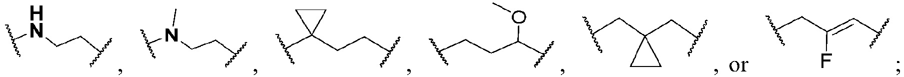 and
(2) 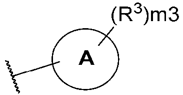 is

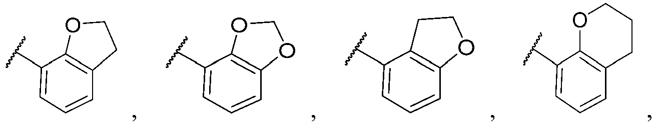

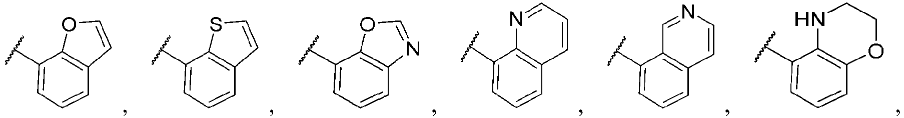

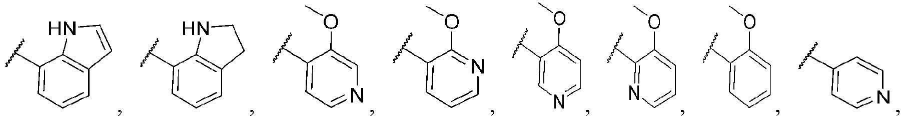

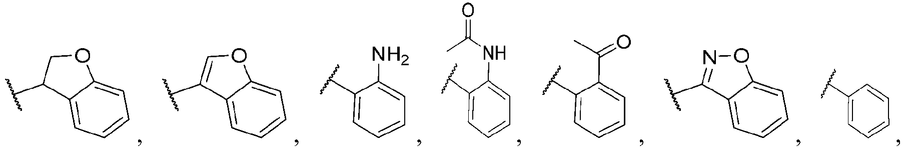

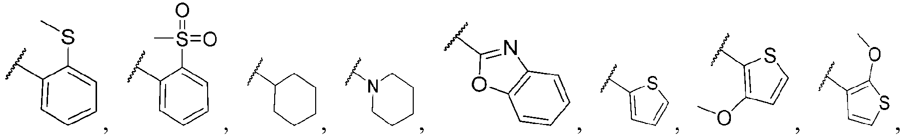

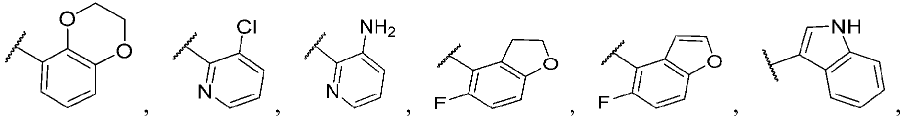

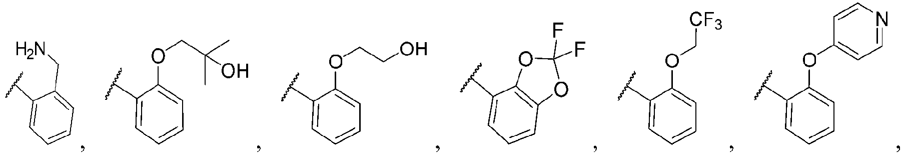

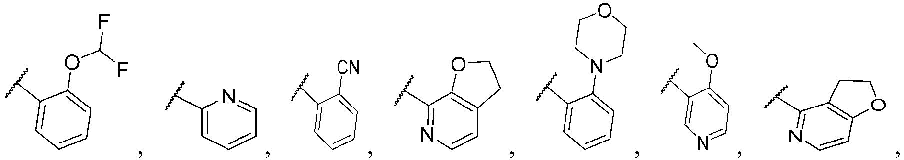

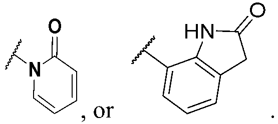

7. The tetracyclic compound represented by formula I or the pharmaceutically acceptable salt thereof as defined in claim 1, meeting one or more of the following conditions:
(1) 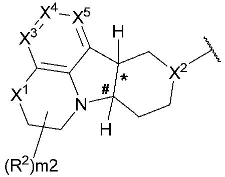 is 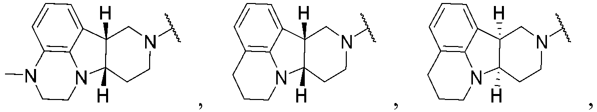 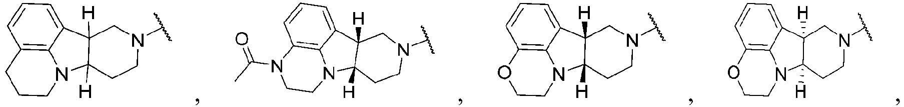 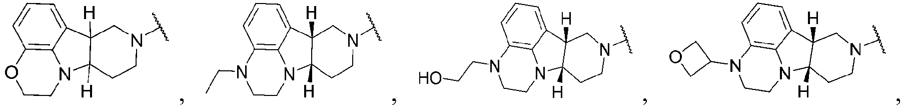 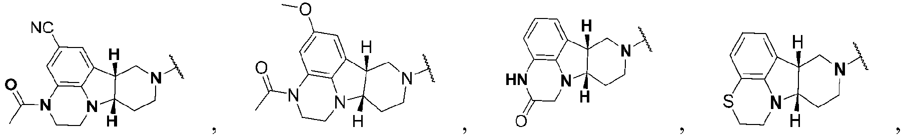 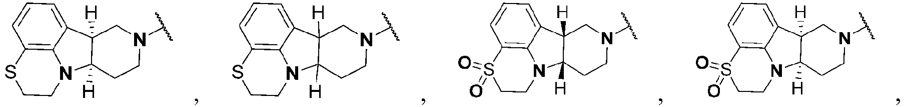 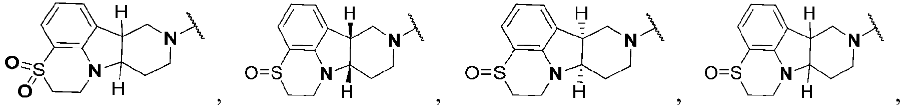 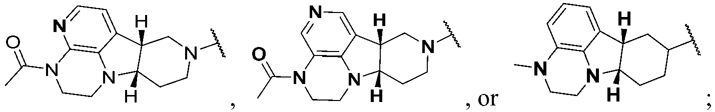 and
(2)

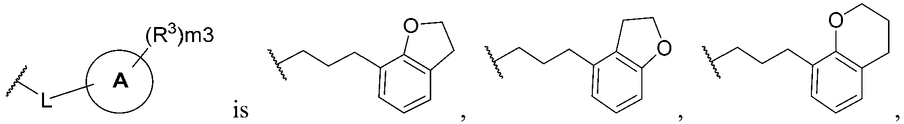

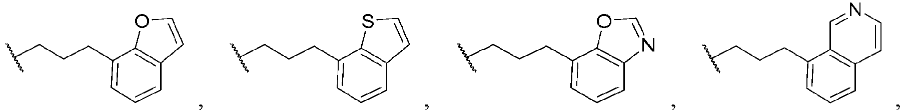

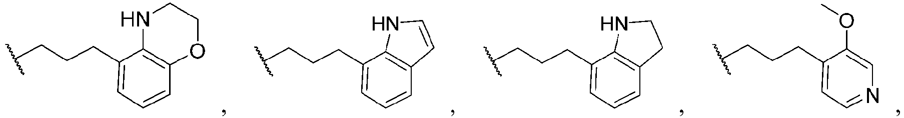

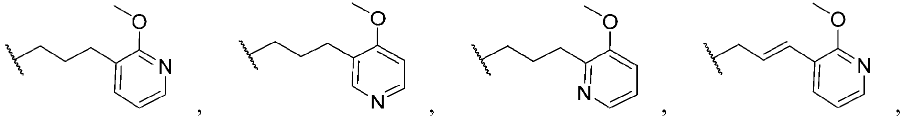

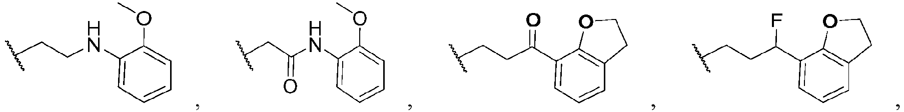

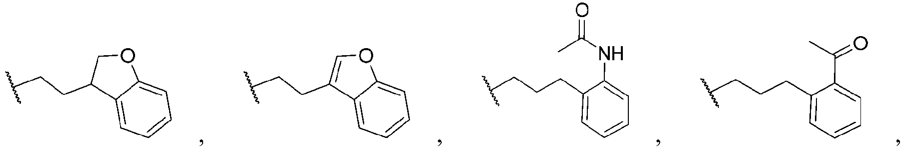

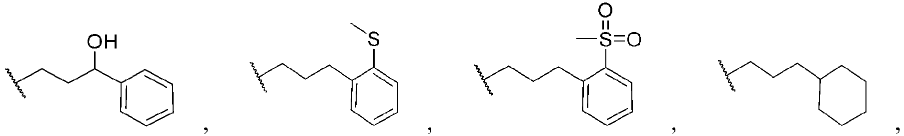

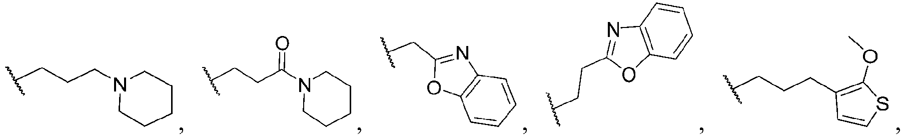

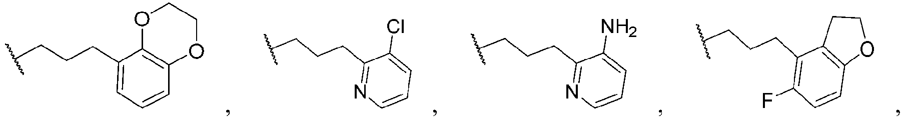

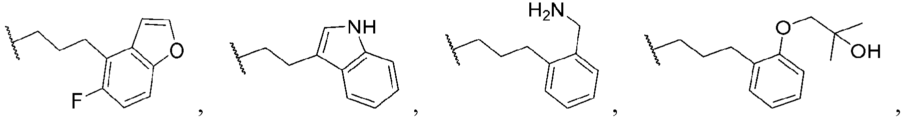

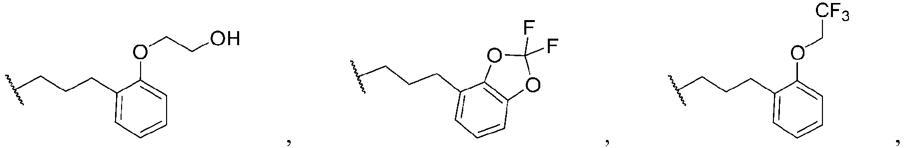

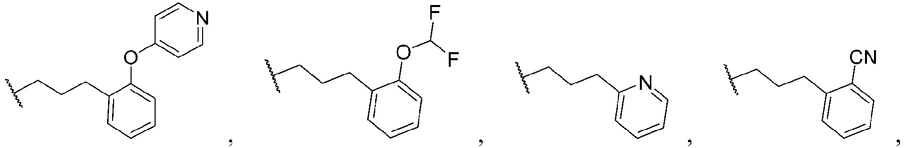

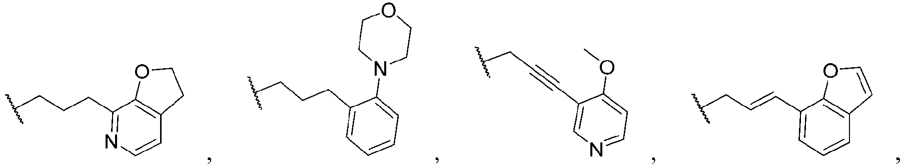

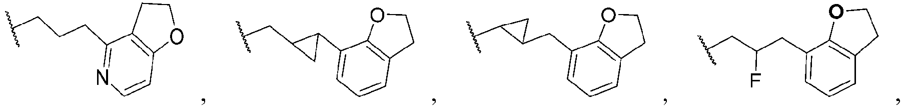

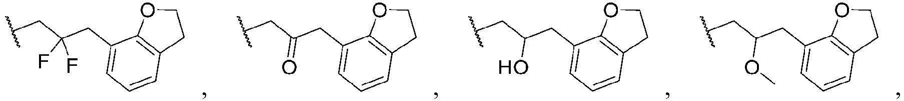

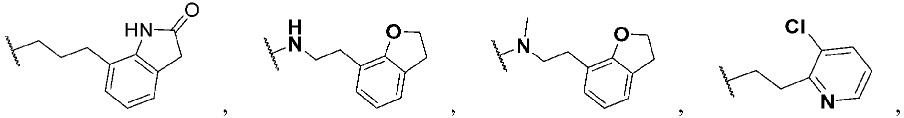

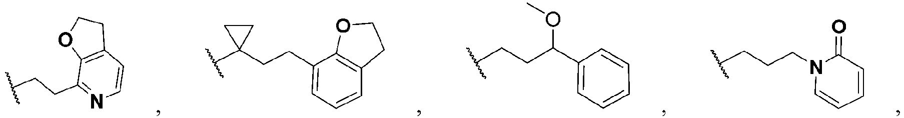

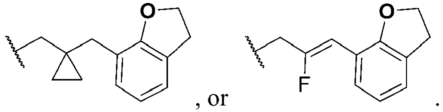

8. The tetracyclic compound represented by formula I or the pharmaceutically acceptable salt thereof as defined in claim 1, wherein the tetracyclic compound represented by formula I is a compound represented by formula I-1, I-2, or I-3: 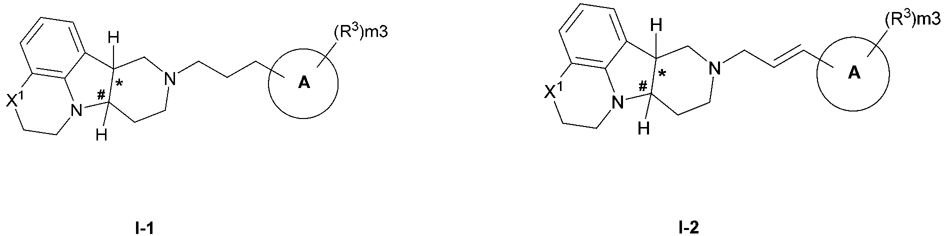 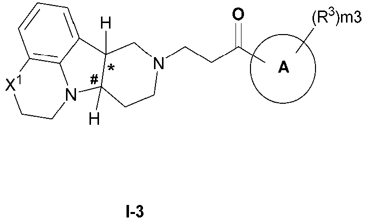 wherein *, #, X¹, ring A, R³, and m3 are as defined in any one of claims 1-7.

9. The tetracyclic compound represented by formula I or the pharmaceutically acceptable salt thereof as defined in claim 1, wherein 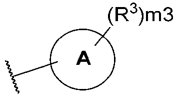 is

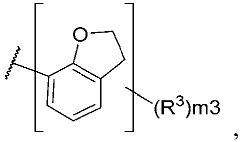

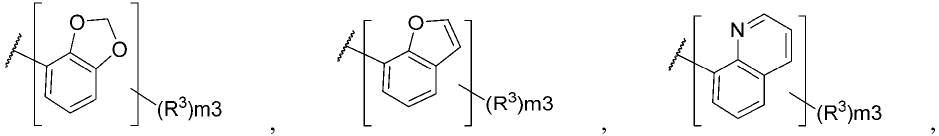

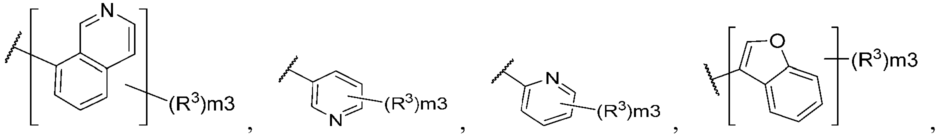

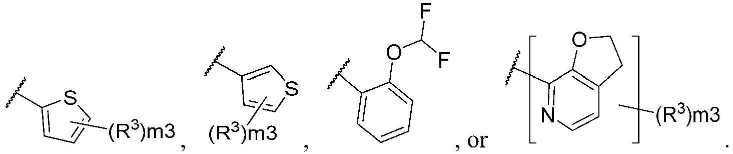

10. The tetracyclic compound represented by formula I or the pharmaceutically acceptable salt thereof as defined in claim 1, wherein the tetracyclic compound represented by formula I is not the following compounds: 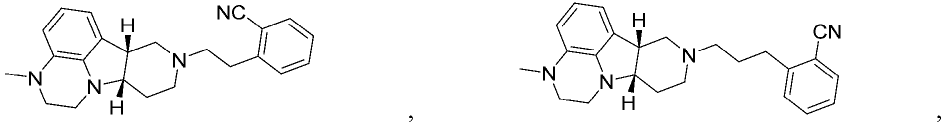 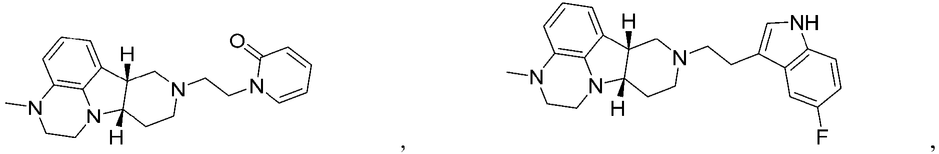 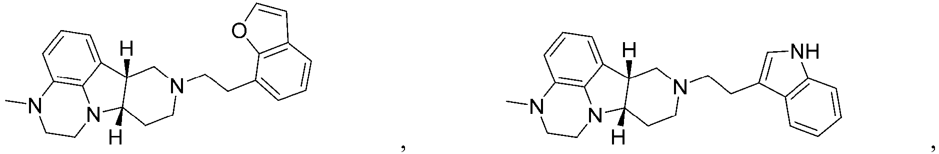 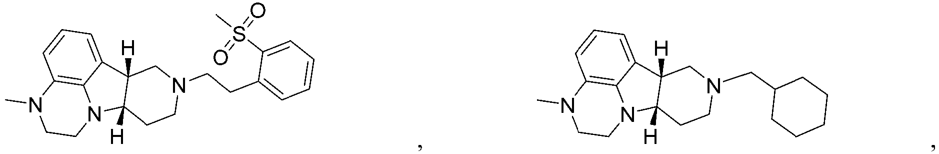 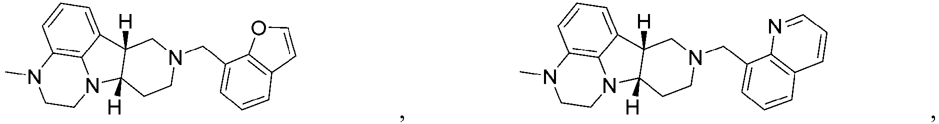 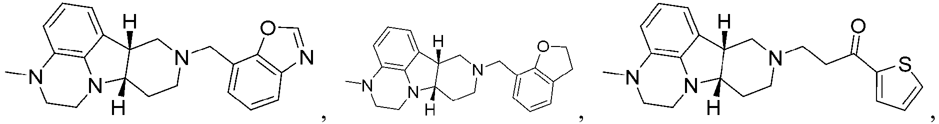 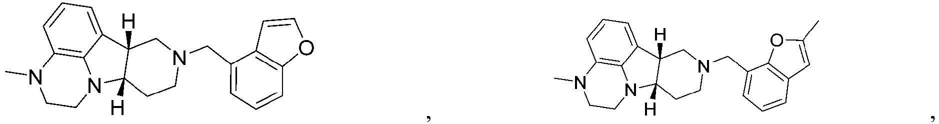 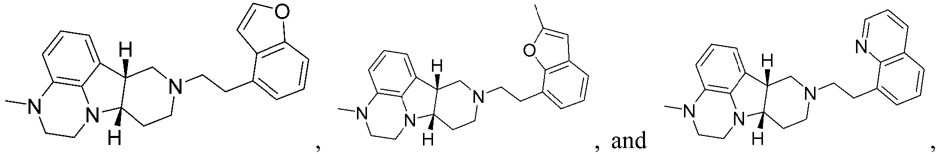 and stereoisomers thereof.

11. The tetracyclic compound represented by formula I or the pharmaceutically acceptable salt thereof as defined in any one of claims 1-10, wherein the tetracyclic compound represented by formula I is replaced with a tetracyclic compound represented by formula I-0: 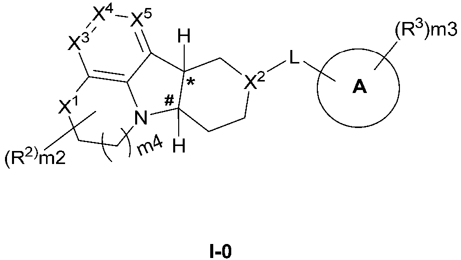
wherein the carbon atom with "*" represents a chiral carbon atom that has an S configuration, an R configuration, or a mixture thereof;
the carbon atom with "#" represents a chiral carbon atom that has an S configuration, an R configuration, or a mixture thereof;
X¹ is -NR^{X1}-, -O-, -CR^{X2}R^{X3}-, -S-, -S(O)-, or -S(O)₂-;
R^{X1} is H, C₁-C₆ alkyl, C₁-C₆ alkyl substituted with one or more R^{X1-1}, -C(O)-C₁-C₆ alkyl, C₃-C₆ cycloalkyl, or "3-12 membered heterocycloalkyl containing 1, 2, or 3 heteroatoms selected from 1, 2, or 3 of N, O, and S";
R^{X1-1} is independently deuterium or OH;
R^{X2} and R^{X3} are independently H, deuterium, C₁-C₆ alkyl, or C₁-C₆ deuterated alkyl;
X² is N, N⁺O⁻, or CR^{X4};
R^{X4} is H or C₁-C₆ alkyl;
X³ is N or CR¹⁻¹;
X⁴ is N or CR¹⁻²;
X⁵ is N or CR¹⁻³;
R¹⁻¹, R¹⁻², and R¹⁻³ are independently H, CN, halogen, C₁-C₆ alkyl, C₁-C₆ haloalkyl, C₁-C₆ alkoxy, or C₁-C₆ haloalkoxy;
L is -(CR^{L1}R^{L2})n1-, -(CR^{L1}R^{L2})n2-Y¹-(CR^{L1}R^{L2})n3-, or -(CR^{L1}R^{L2})n4-Y²-(CR^{L1}R^{L2})n5-;
R^{L1} and R^{L2} are independently H or C₁-C₆ alkyl;
n1 is 3 or 4;
Y¹ is -O-, -NR^{L3}-, -C(O)-, -CR^{L4}R^{L5}-, 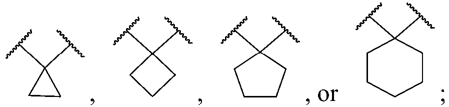
n2 is 0, 1, 2, or 3;
n3 is 0, 1, 2, or 3;
n2 + n3 = 1, 2, or 3;
Y² is -C≡C-, -C(O)-NR^{L3}-, -CR^{L6}=CR^{L7}-, 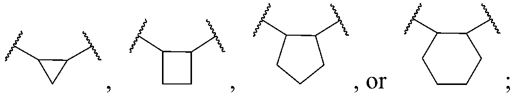
n4 is 0, 1, or 2;
n5 is 0, 1, or 2;
n4 + n5 = 1 or 2;
R^{L3} is independently H or C₁-C₆ alkyl;
R^{L4} is H or halogen;
R^{L5} is halogen, OH, or C₁-C₆ alkoxy;
R^{L6} and R^{L7} are independently H or halogen;
R² is independently deuterium, C₁-C₆ alkyl, C₁-C₆ deuterated alkyl, -C(O)-C₁-C₆ alkyl, oxo (=O), thio (=S), or hydroxyl;
m2 is 0, 1, 2, 3, or 4;
ring A is "8-12 membered bicyclic heterocyclyl containing 1, 2, or 3 heteroatoms selected from 1, 2, or 3 of N, O, and S", "5-6 membered monocyclic heterocyclyl containing 1, 2, or 3 heteroatoms selected from 1, 2, or 3 of N, O, and S", "9-16 membered polycyclic heterocyclyl containing 1, 2, or 3 heteroatoms selected from 1, 2, or 3 of N, O, and S", or C₃-C₆ cycloalkyl;
and when L is -(CR^{L1}R^{L2})n1-, -(CR^{L1}R^{L2})n2-O-, or -(CR^{L1}R^{L2})n2-C(O)-, 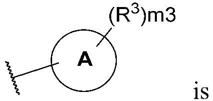 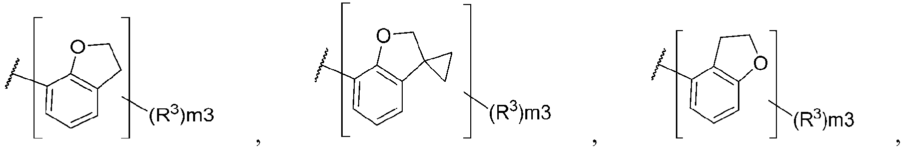 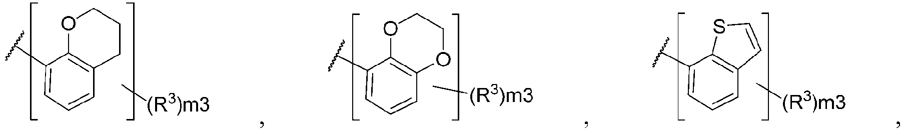 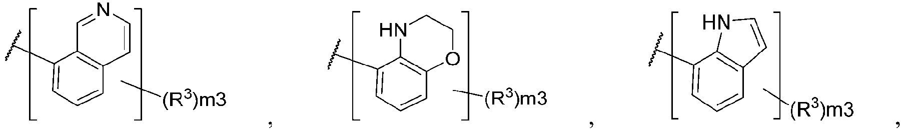 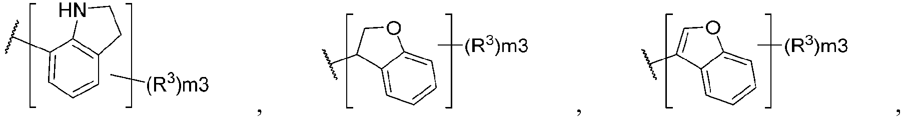 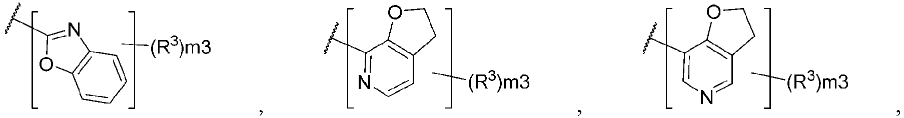 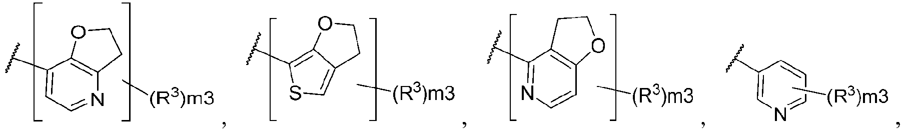 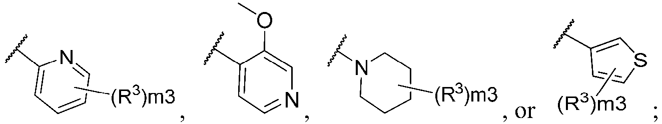
R³ is independently oxo (=O), C₁-C₆ alkyl, C₁-C₆ alkoxy, -NR⁴R⁵, -C(O)R⁶, -SR⁷, -S(O)₂R⁸, halogen, C₁-C₆ alkoxy substituted with one or more R⁹, C₁-C₆ alkyl substituted with one or more R¹⁰, CN, "3-12 membered heterocycloalkyl containing 1, 2, or 3 heteroatoms selected from 1, 2, or 3 of N, O, and S", or -OR¹¹;
R⁴ is independently H or C₁-C₆ alkyl;
R⁵ is independently H, C₁-C₆ alkyl, or -C(O)R⁵⁻¹.
R⁶, R⁵⁻¹, R⁷, and R⁸ are independently C₁-C₆ alkyl;
R⁹ and R¹⁰ are independently OH, -NR⁴R⁵, or halogen;
R¹¹ is H or "5-10 membered heteroaryl containing 1, 2, or 3 heteroatoms selected from 1, 2, or 3 of N, O, and S";
m3 is 0, 1, 2, 3, or 4;
m4 is 0, 1, or 2.

12. The tetracyclic compound represented by formula I or the pharmaceutically acceptable salt thereof as defined in claim 11, meeting one or more of the following conditions:
(1) X¹ is -NR^{X1}-;
(2) X³ is CR¹⁻¹;
(3) X⁴ is CR¹⁻²;
(4) R¹⁻¹, R¹⁻², and R¹⁻³ are independently H, CN, halogen, or C₁-C₆ alkoxy; for example, R¹⁻¹ and R¹⁻³ are H, and R¹⁻² is H, F, CN, or C₁-C₆ alkoxy; in another example, R¹⁻¹, R¹⁻², and R¹⁻³ are independently H;
(5) m4 is 1;
(6) n2 is 1 or 2;
(7) n3 is 0 or 1;
(8) R^{L3} is H;
(9) R² is independently deuterium, C₁-C₆ alkyl, oxo, thio, or hydroxyl, e.g., oxo;
(10) m2 is 0, 1, 2, or 3; for example, m2 is 1;
(11) when L is -(CR^{L1}R^{L2})n1-, -(CR^{L1}R^{L2})n2-O-, or -(CR^{L1}R^{L2})n2-C(O)-, 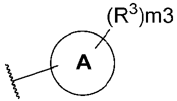 is 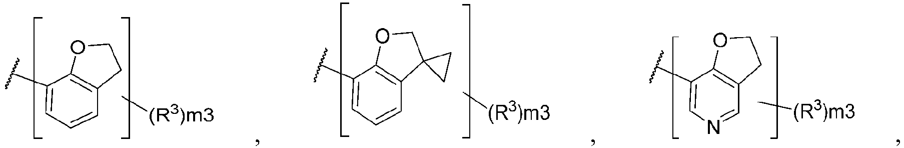 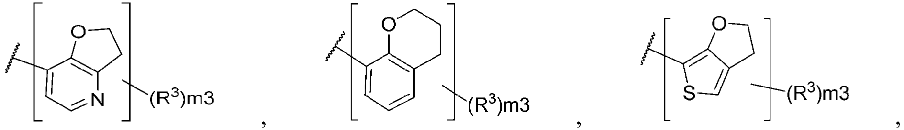 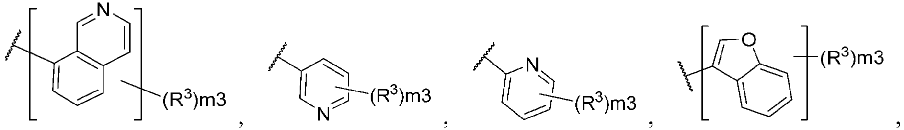 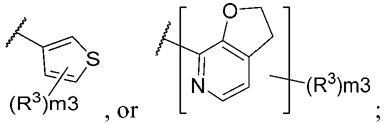 and
(12) R³ is independently -OR¹¹, C₁-C₆ alkyl, C₁-C₆ alkoxy, C₁-C₆ alkoxy substituted with one or more R⁹, or halogen.

13. The tetracyclic compound represented by formula I or the pharmaceutically acceptable salt thereof as defined in claim 11, meeting one or more of the following conditions:
(1) each C₃-C₆ cycloalkyl is independently cyclopropyl, cyclobutyl, cyclopentyl, or cyclohexyl, e.g., cyclobutyl;
(2) in ring A, the "8-12 membered bicyclic heterocyclyl containing 1, 2, or 3 heteroatoms selected from 1, 2, or 3 of N, O, and S" is 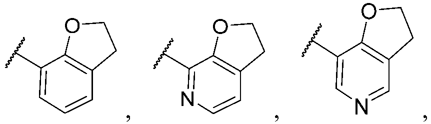 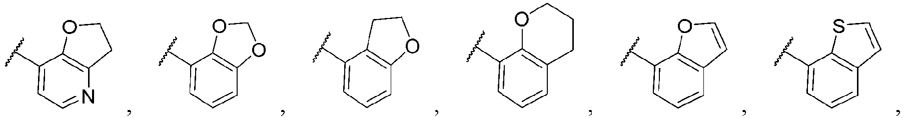 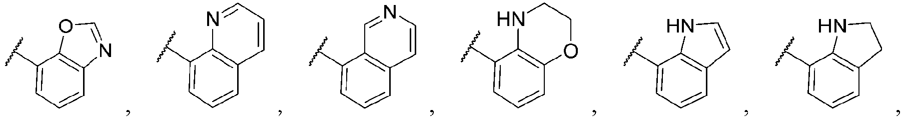 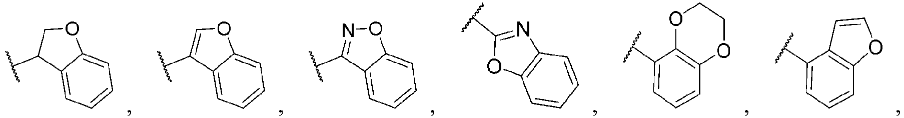 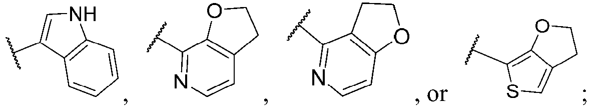
(3) in ring A, the "9-16 membered polycyclic heterocyclyl containing 1, 2, or 3 heteroatoms selected from 1, 2, or 3 of N, O, and S" is "10-12 membered tricyclic heterocyclyl containing 1 or 2 heteroatoms selected from 1 or 2 of N and O", e.g., 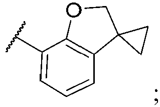 and
(4) 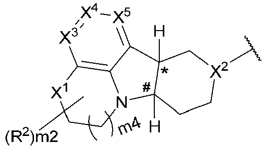 is one or more of 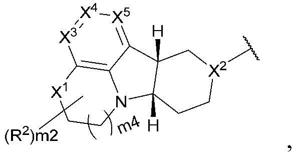 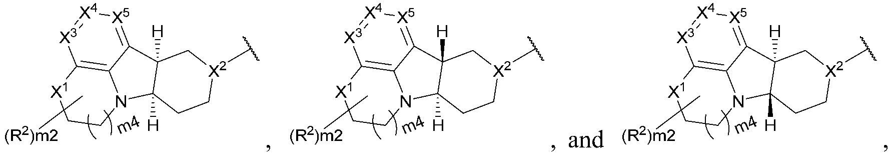 e.g., 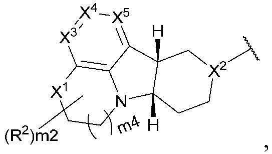 e.g.,

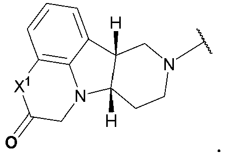

14. The tetracyclic compound represented by formula I or the pharmaceutically acceptable salt thereof as defined in claim 11, meeting one or more of the following conditions:
(1) 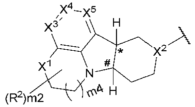 is 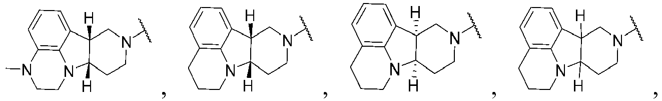 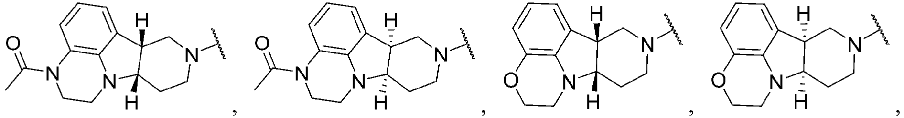 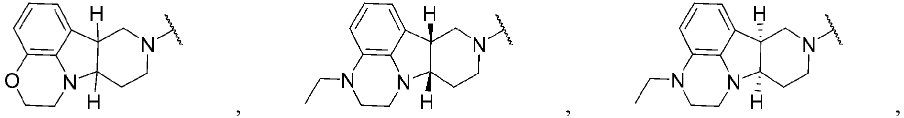 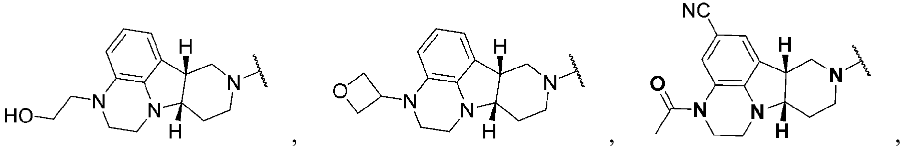 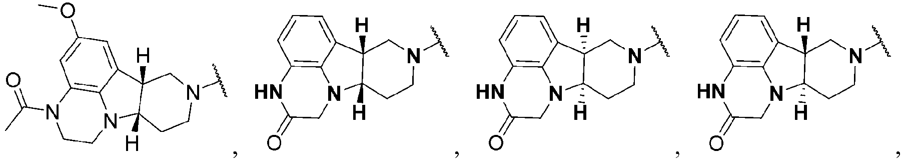 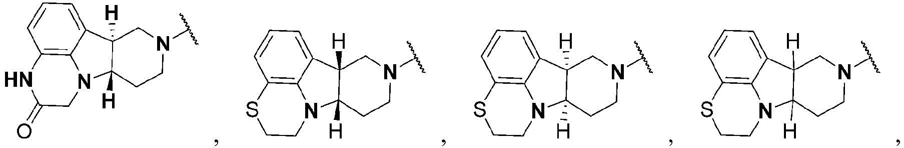 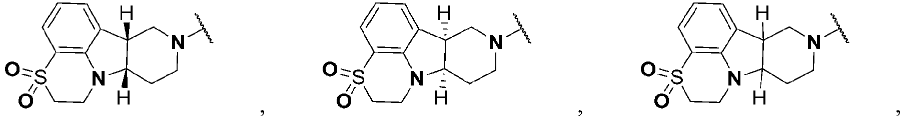 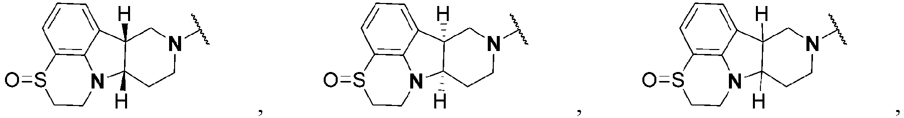 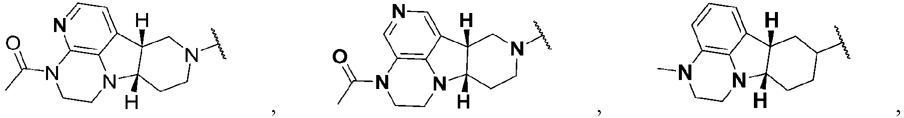 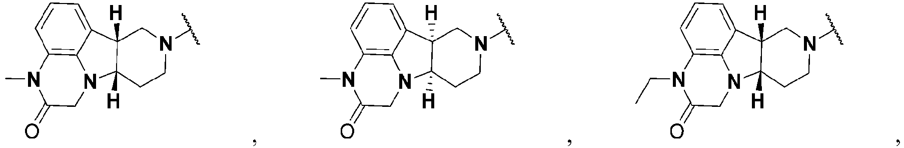 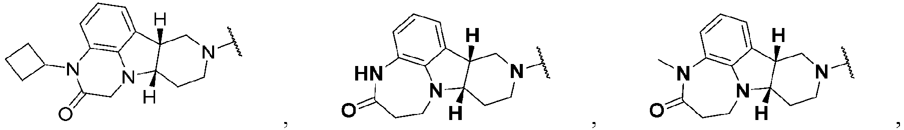 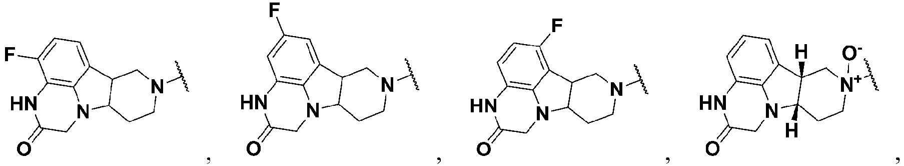 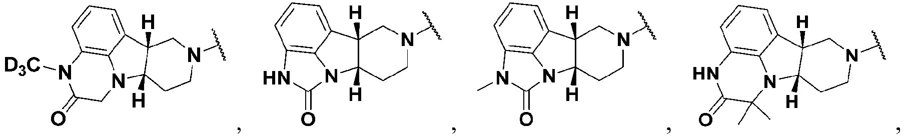 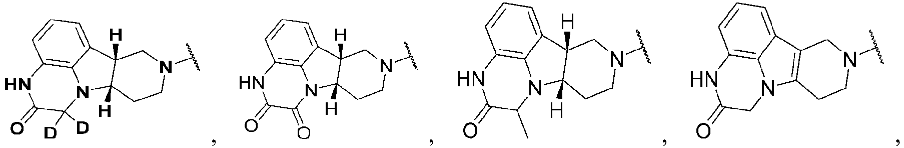 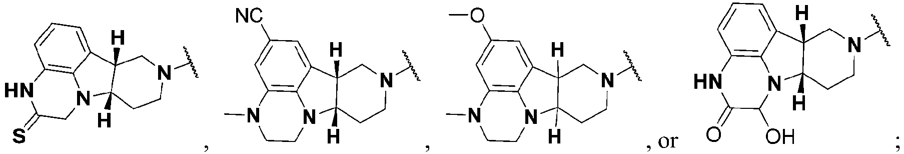
(2) L is 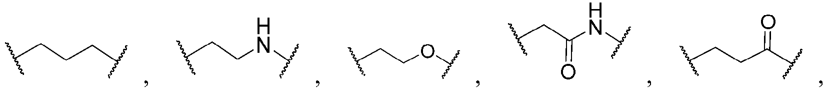 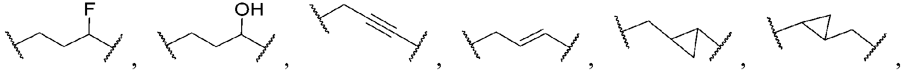 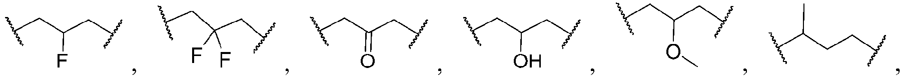 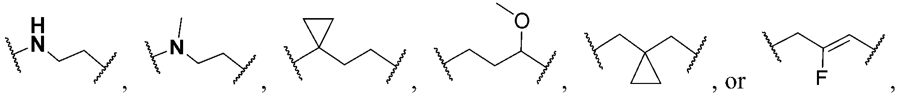 e.g., 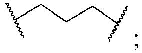 and
(3) 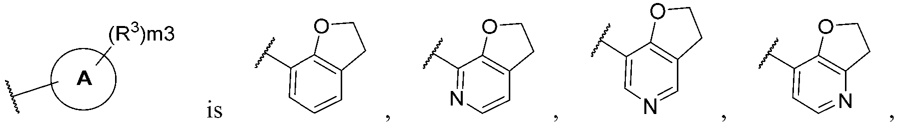 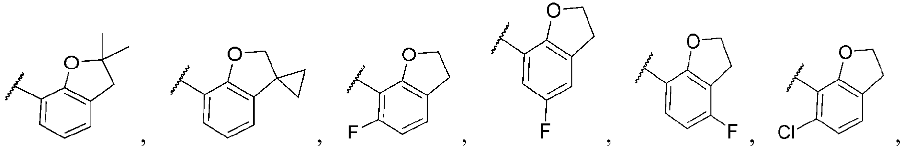 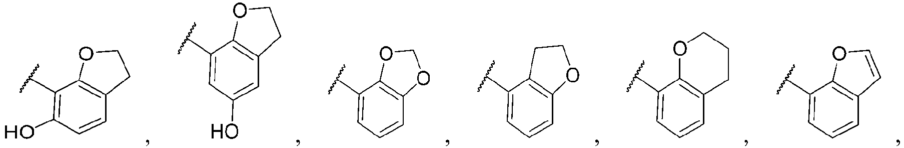 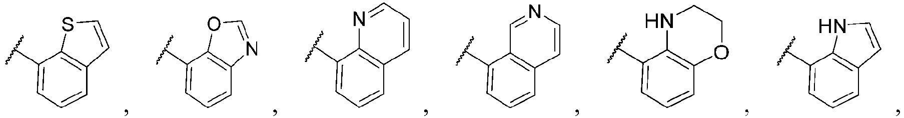 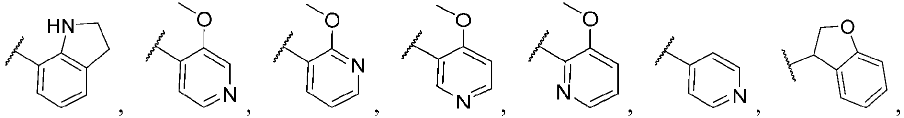 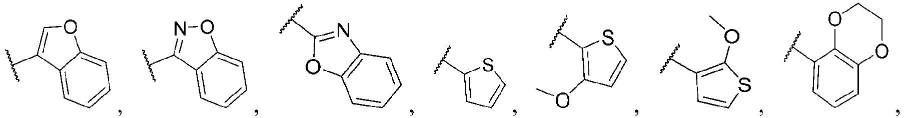 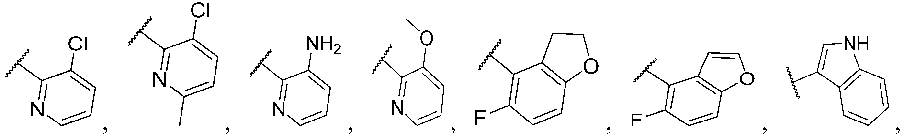 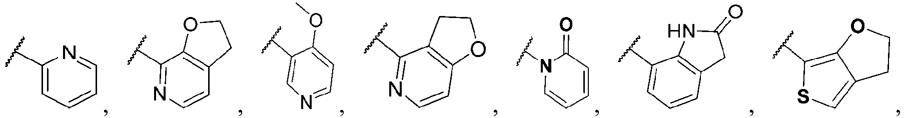 or

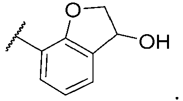

15. The tetracyclic compound represented by formula I or the pharmaceutically acceptable salt thereof as defined in claim 11, wherein 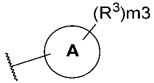 is

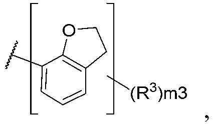

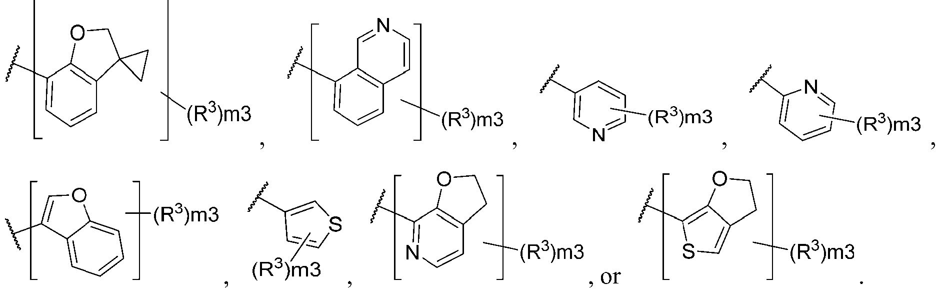

16. The tetracyclic compound represented by formula I or the pharmaceutically acceptable salt thereof as defined in claim 11, wherein 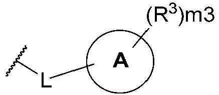 is

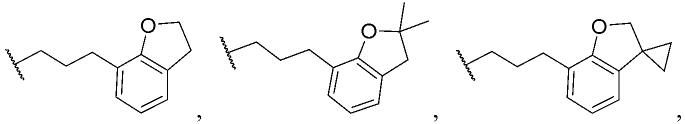

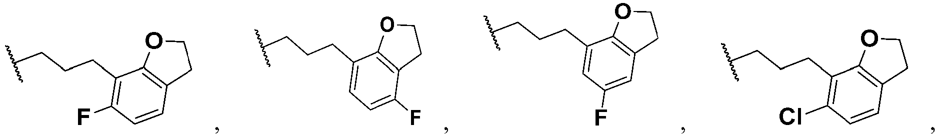

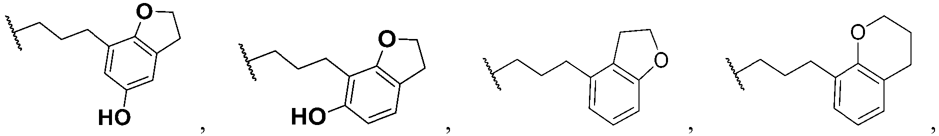

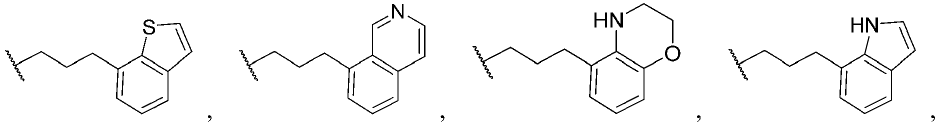

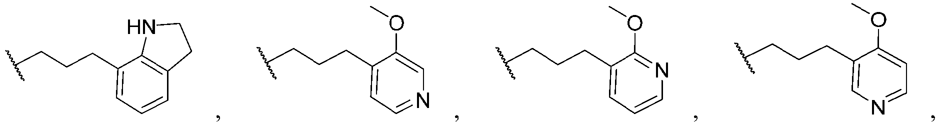

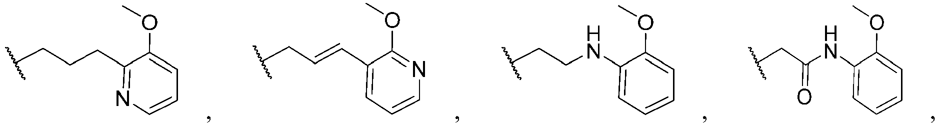

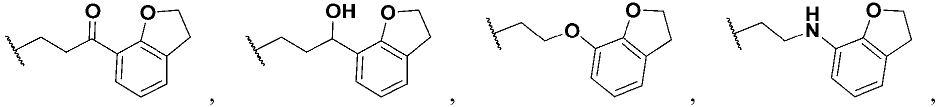

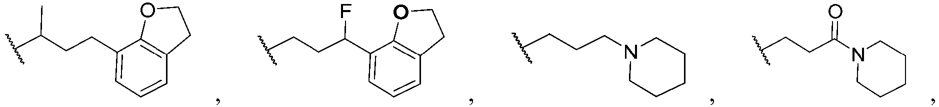

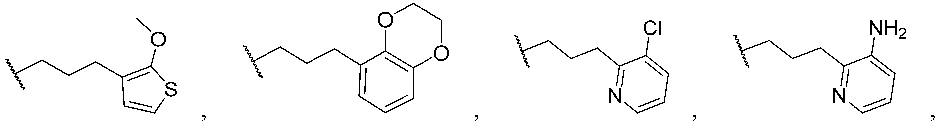

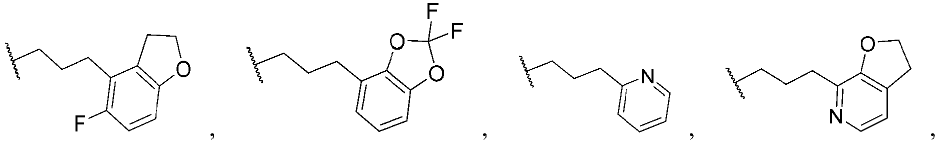

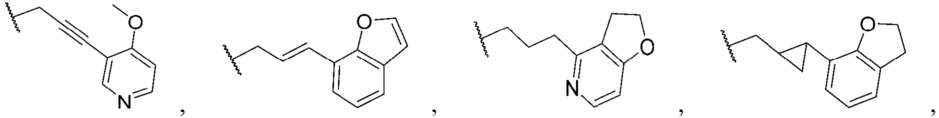

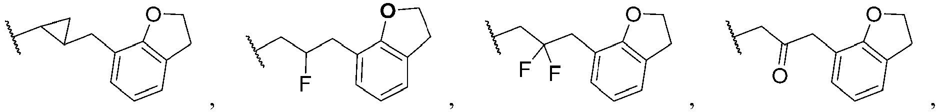

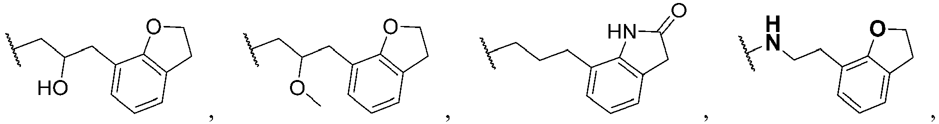

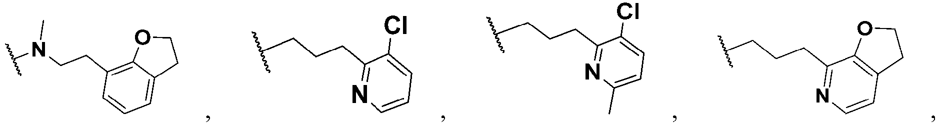

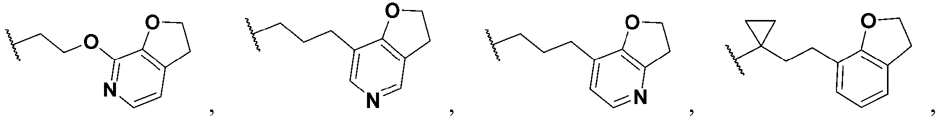

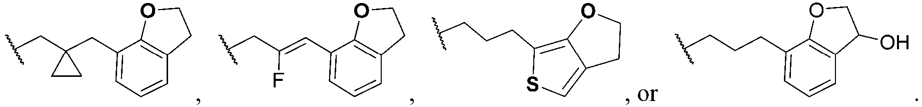

17. The tetracyclic compound represented by formula I or the pharmaceutically acceptable salt thereof as defined in claim 11, wherein the tetracyclic compound is a compound represented by formula I-4: 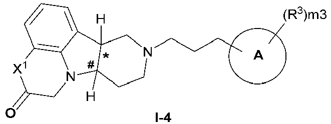 wherein *, #, X¹, ring A, R³, and m3 are as defined in any one of claims 11-16.

18. A tetracyclic compound represented by any one of the following or a pharmaceutically acceptable salt thereof:

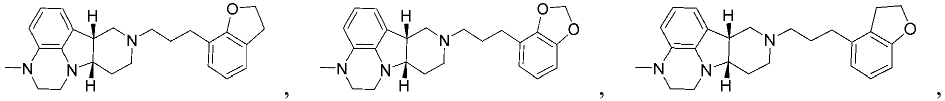

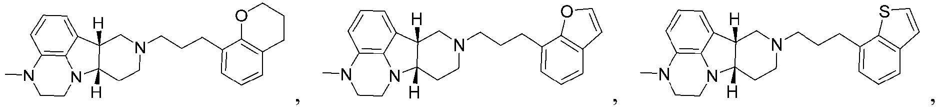

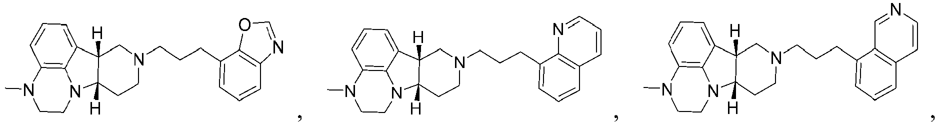

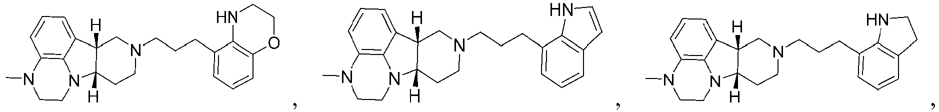

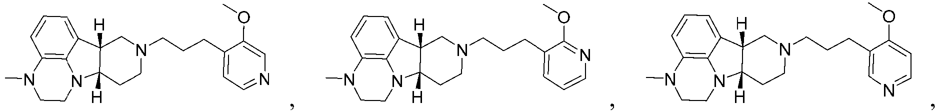

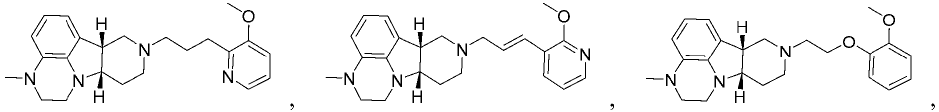

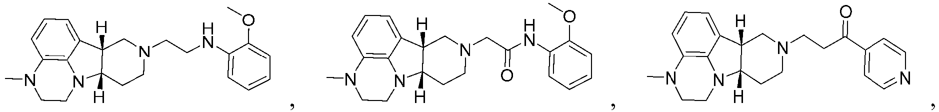

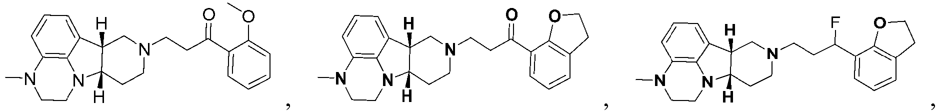

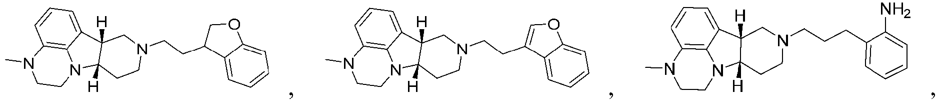

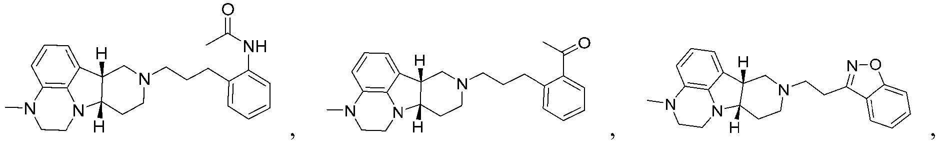

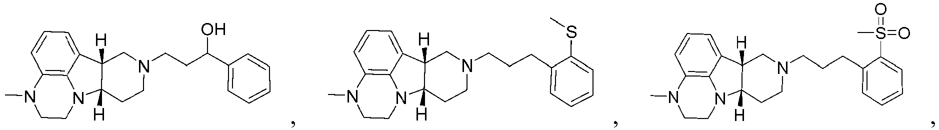

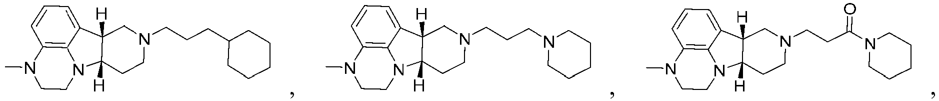

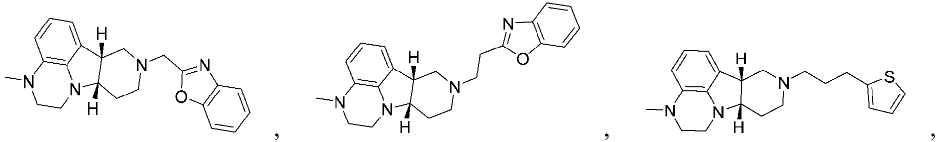

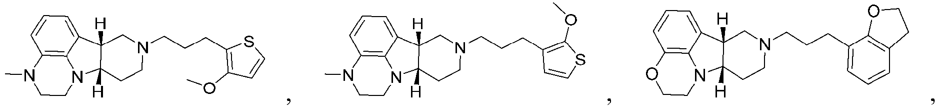

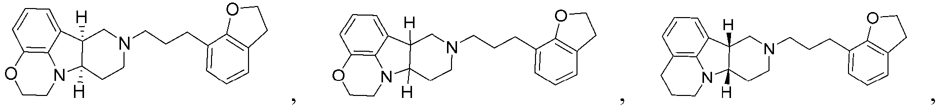

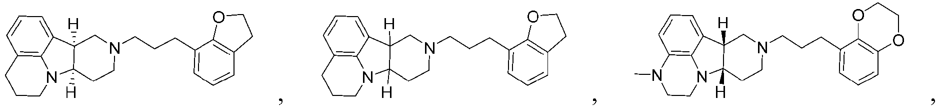

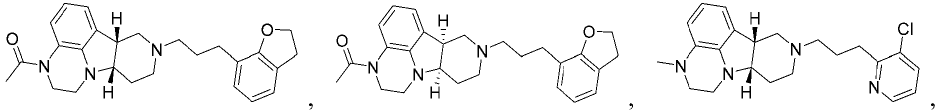

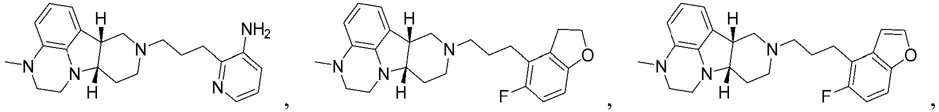

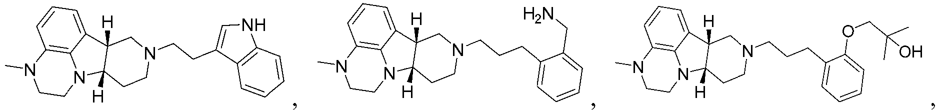

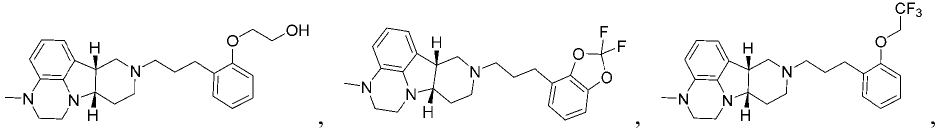

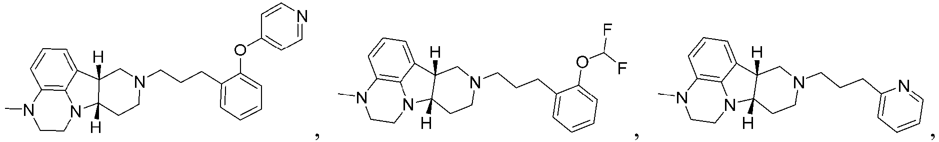

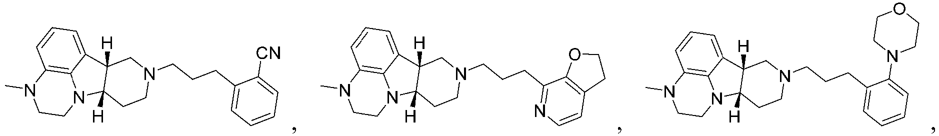

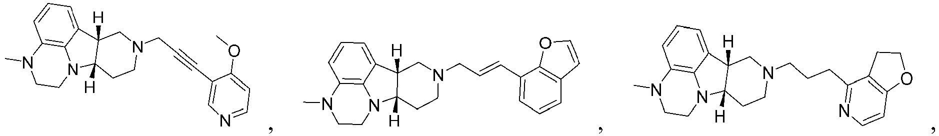

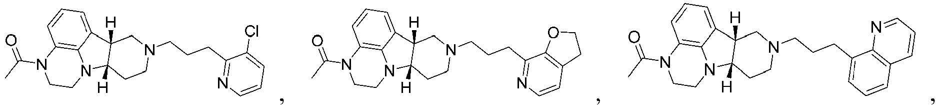

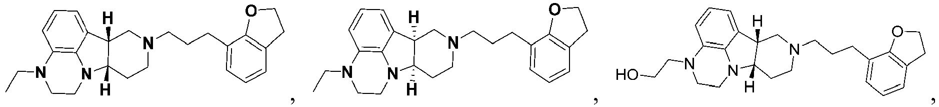

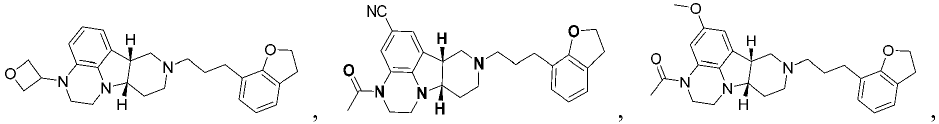

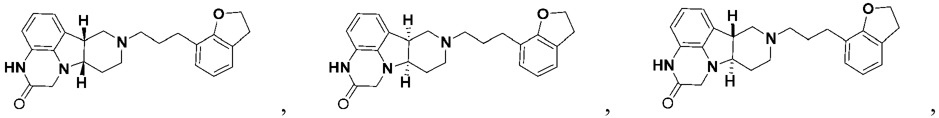

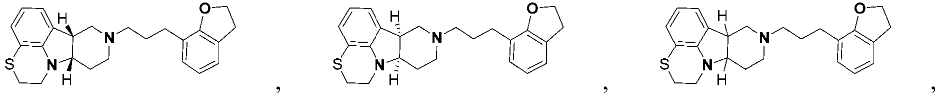

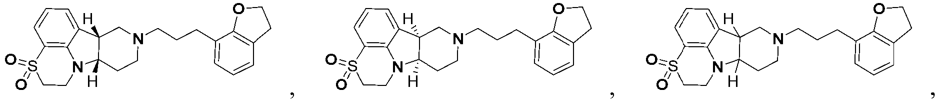

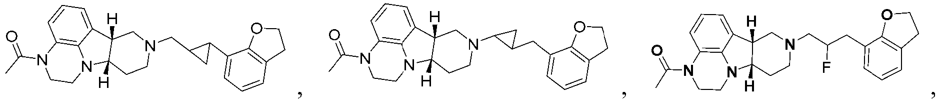

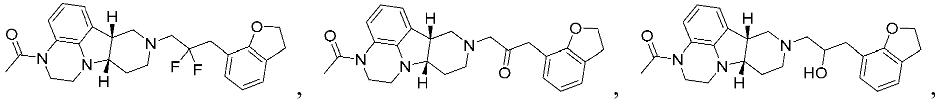

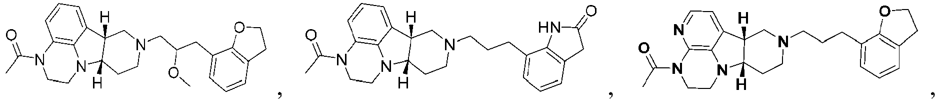

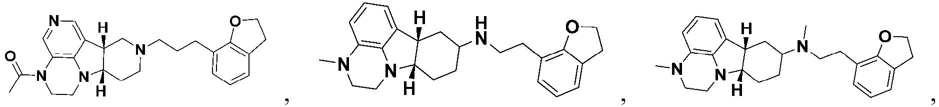

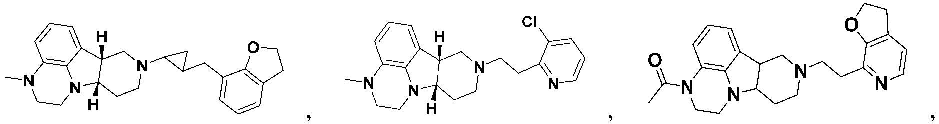

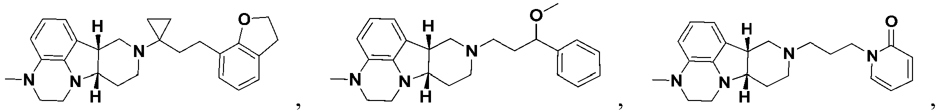

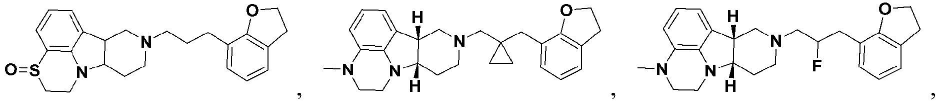

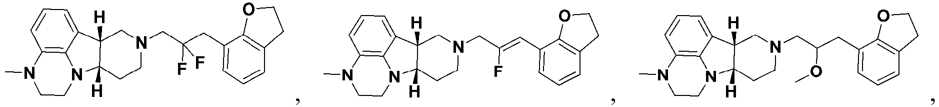

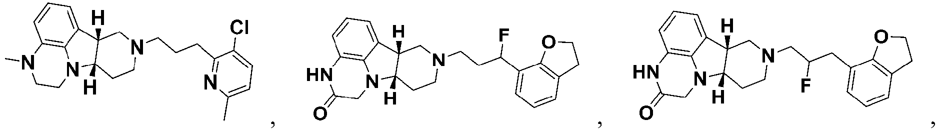

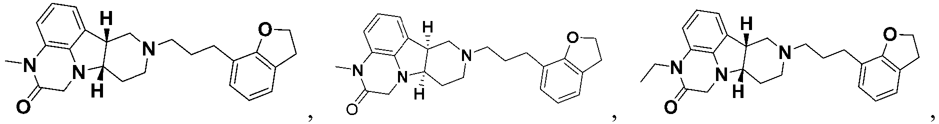

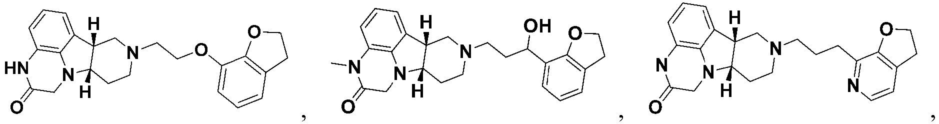

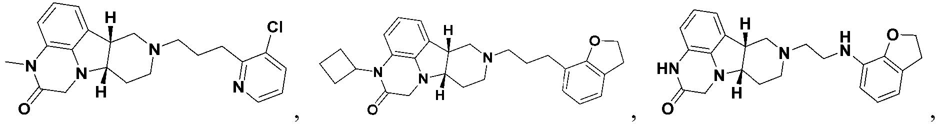

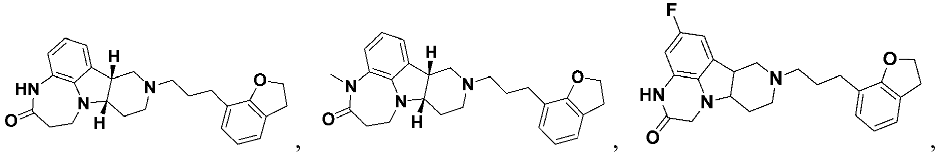

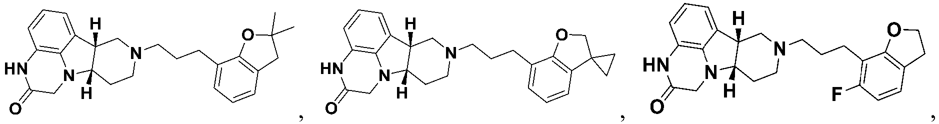

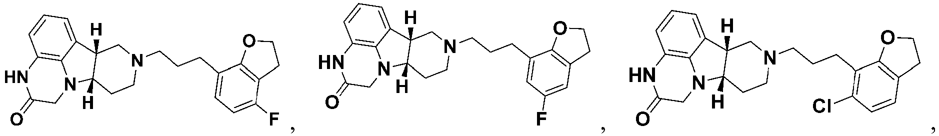

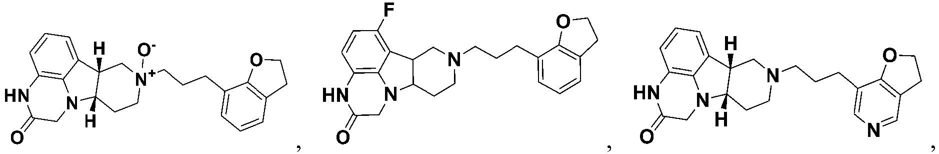

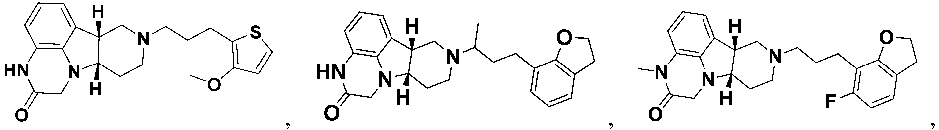

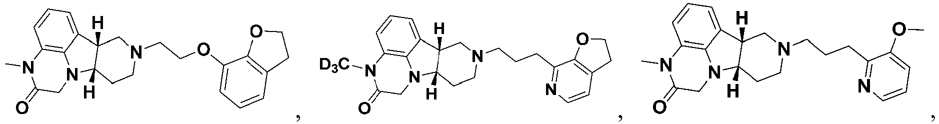

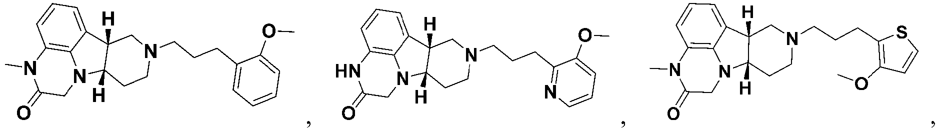

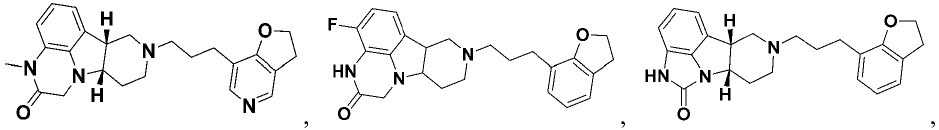

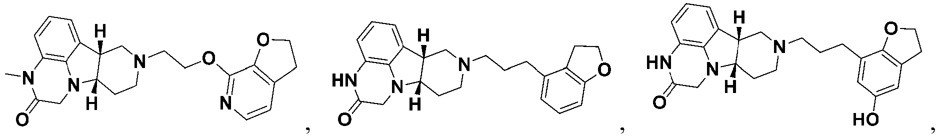

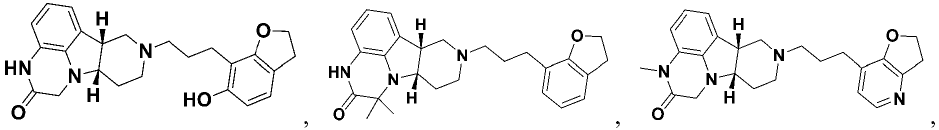

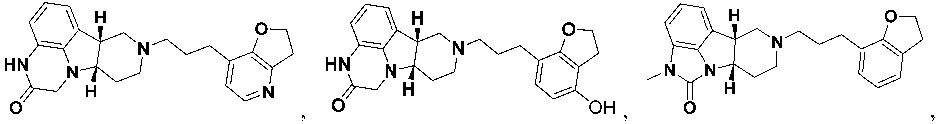

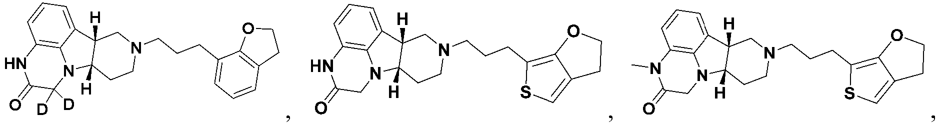

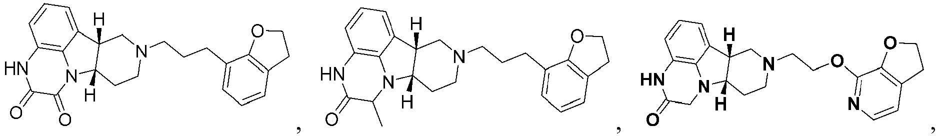

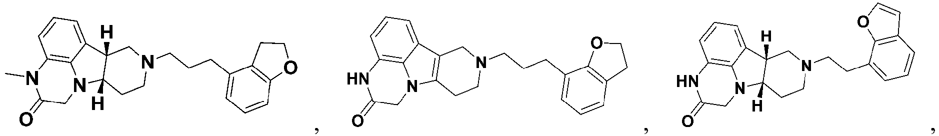

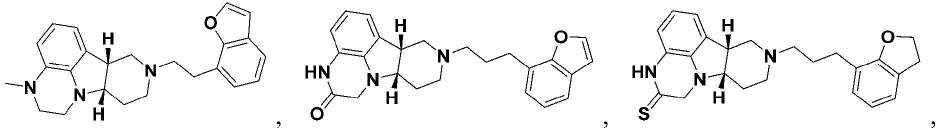

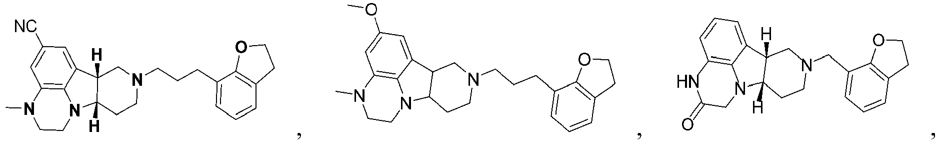

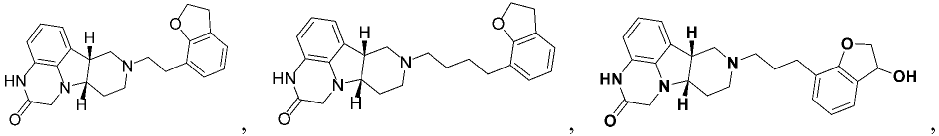

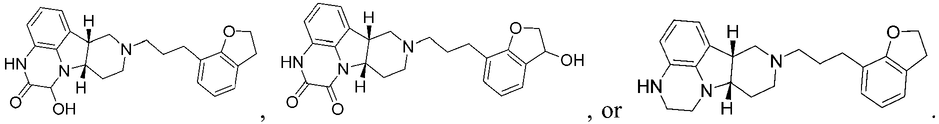

19. A pharmaceutical composition, comprising:
(1) the tetracyclic compound or the pharmaceutically acceptable salt thereof as defined in any one of claims 1-18, and
(2) a pharmaceutically acceptable auxiliary material.

20. Use of the tetracyclic compound or the pharmaceutically acceptable salt thereof as defined in any one of claims 1-18 or the pharmaceutical composition as defined in claim 19, wherein the use is selected from:
(1) use for preparing a 5-HT_{2A} receptor agonist;
(2) use for preparing a medicament for treating and/or preventing a 5-HT_{2A} receptor-related disease; for example, the 5-HT_{2A} receptor-related disease is depression; and
(3) use for preparing a medicament for treating and/or preventing depression.
